(19) Europäisches Patentamt
European Patent Office
Office européen des brevets

(11) EP 2 118 111 B1

(12) **EUROPEAN PATENT SPECIFICATION**

(45) Date of publication and mention of the grant of the patent:
**23.03.2011 Bulletin 2011/12**

(21) Application number: **08702335.4**

(22) Date of filing: **25.01.2008**

(51) Int Cl.:
***C07D 487/04*** *(2006.01)* ***A61K 31/519*** *(2006.01)*
***A61P 35/00*** *(2006.01)*

(86) International application number:
**PCT/IB2008/000200**

(87) International publication number:
**WO 2008/096218 (14.08.2008 Gazette 2008/33)**

(54) **2-AMIN0-5, 7-DIHYDR0-6H- PYRROLO [3, 4-D] PYRIMIDINE DERIVATIVES AS HSP-90 INHIBITORS FOR TREATING CANCER**

2-AMINO-5,7-DIHYDRO-6H-PYRROLO[3,4-D]PYRIMIDINDERIVATE ALS HSP-90-INHIBITOREN ZUR BEHANDLUNG VON KREBS

DÉRIVÉS DE 2-AMINO-5,7-DIHYDRO-6H-PYRROLO [3,4-D] PYRIMIDINE EN TANT QU'INHIBITEURS DE LA HSP 90 POUR LE TRAITEMENT DU CANCER

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **06.02.2007 US 888433 P**
**11.01.2008 US 20661**

(43) Date of publication of application:
**18.11.2009 Bulletin 2009/47**

(73) Proprietor: **Pfizer Inc.**
**New York NY 10017 (US)**

(72) Inventors:
- **BENNETT, Michael, John**
**San Diego, CA 92121 (US)**
- **ZEHNDER, Luke, Raymond**
**San Diego, CA 92121 (US)**
- **NINKOVIC, Sacha**
**San Diego, CA 92121 (US)**
- **KUNG, Pei-Pei**
**San Diego, CA 92121 (US)**
- **MENG, Jerry, Jialun**
**San Diego, CA 92121 (US)**
- **HUANG, Buwen**
**San Diego, CA 92121 (US)**

(74) Representative: **Ruddock, Keith Stephen**
**Pfizer Limited**
**European Patent Department**
**Ramsgate Road**
**Sandwich**
**Kent CT13 9NJ (GB)**

(56) References cited:
**WO-A-03/062225** **WO-A-2006/105372**
**WO-A-2006/118598**

- **BENDERITTER, PASCAL ET AL: "2-Amino-6-iodo-4-tosyloxypyrimidine: A versatile key intermediate for regioselective functionalization of 2-aminopyrimidines in 4- and 6-positions" TETRAHEDRON, vol. 63, no. 50, 2 August 2007 (2007-08-02), pages 12465-12470, XP002480722**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

Field of the Invention

The present invention is directed to compounds, and pharmaceutically acceptable salts and solvates thereof, their synthesis, and their use as modulators or inhibitors of HSP-90. The compounds of the present invention are useful for modulating (e.g. inhibiting) HSP-90 activity and for treating diseases or conditions mediated by HSP-90, such as for example, disease states associated with abnormal cell growth such as cancer.

Background

Molecular chaperones play important roles in cellular function by ensuring proper folding of proteins upon synthesis as well as their refolding under conditions of denaturing stress By regulating the balance between protein synthesis and degradation, molecular chaperones are a significant part of the cellular response to stress. In addition, by regulating the proper folding of various cellular proteins, chaperones play an important role in regulating cellular functions such as cell proliferation and apoptosis. (See, e.g. Jolly, et al., J. Natl. Cancer Inst. 92: 1564-1572 (2000)). Heat shock proteins (HSPs) are a class of chaperones that accumulate in the cell in response to various environmental stresses, such as heat shock, oxidative stress, or the presence of alcohols or heavy metals. In addition to their role in protecting the cell from such environmental stresses, HSPs may also play a significant role as chaperones for a variety of cellular proteins under stress-free conditions. Members of the HSP family are classified according to their molecular weight (e.g. HSP-27, HSP-70, and HSP-90). Evidence of differential expression of HSPs in various stages of tumor progression suggests HSPs play a role in cancer. (See, e.g. Martin, et al., Cancer Res. 60:2232-2238 (2000)).

HSP-90 is a homodimer with ATPase activity and functions in a series of complex interactions with a variety of substrate proteins (Young, et al., J. Cell Biol. 154: 267-273 (2001)). HSP-90 is unique with regard to other chaperones, however, since most of its know substrate proteins are signal transduction proteins. Thus, HSP-90 plays an essential role in regulating cellular signal transduction networks. (See, e.g. Xu, et al., Proc. Natl. Acad. Sci 90:7074-7078 (1993)). In particular, substrate proteins of HSP-90 include many mutated or over-expressed proteins implicated in cancer such as p53, Bcr-Ab1 kinase, Raf-1 kinase, Akt kinase, Npm-Alk kinase $p185^{ErbB2}$ transmembrane kinase, Cdk4, Cdk6, Wee1 (a cell cycle-dependent kinase), HER2/Neu (ErbB2), and hypoxia inducible factor-1$\alpha$ (HIF-1$\alpha$). Thus inhibition of HSP-90 results in selective degradation of these important signaling proteins involved in apoptosis, cell proliferation, and cell cycle regulation (Holstein, et al., Cancer Res. 61:4003-4009 (2001)). Accordingly, HSP-90 is an attractive therapeutic target because of the important roles played by these signaling proteins in disease states involving abnormal cell growth, such as cancer. It is thus desirable to discover and develop new inhibitors of HSP-90 activity that can provide a therapeutic benefit to patients suffering from disease states related to abnormal cell growth such as cancer.

Summary

In one embodiment, the present invention provides a compound of formula (I)

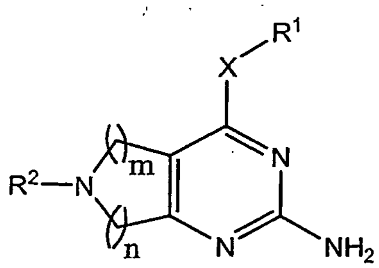

(I)

wherein:

m is 1 or 2, n is 1 or 2, when m is 2, n is 1;

X is a bond or a diradical selected from the group consisting of -O-, -S-, -($C_1$-$C_3$ alkylene)-, -O-($C_1$-$C_3$ alkylene)-, -NH-($C_1$-$C_3$ alkylene)-, -S-($C_1$-$C_3$ alkylene)-, -C(O)-, C(O)-O-, -C(O)-NH-, -OC(O)-NH-, -NH-C(O)-NH-, -S(O)-, -S(O)$_2$-, -S(O)$_2$-O- and -S(O)$_2$-NH-, wherein each end of the diradical may be connected to $R^1$ or to the aminopyrimidine ring of formula I;

where permissible, each nitrogen or carbon atom of X is optionally further substituted by one group selected from $-(C_1-C_3\ alkylene)_t-CN$, $-(C_1-C_3\ alkylene)_t-F$, $-(C_1-C_3\ alkylene)_t-(C_1-C_3\ perfluoroalkyl)$, $-(C_1-C_3\ alkylene)_t-O-(C_1-C_6\ alkyl)$, $-(C_1-C_3\ alkylene)_t-OH$, $-(C_1-C_3\ alkylene)_t-NH_2$, $-(C_1-C_3\ alkylene)_t-NH(C_1-C_3\ alkyl)$ and $-(C_1-C_3\ alkylene)_t-N(C_1-C_3\ alkyl)(C_1-C_3\ alkyl)$, and t is 0 or 1;

$R^1$ is selected from the group consisting of $C_6-C_{12}$ aryl, 5 to 12 member heteroaryl, $C_3-C_{12}$ cycloalkyl, 3-12 member heterocyclyl and $C_5-C_{12}$ unsaturated nonaromatic carbocyclyl, and each $R^1$ is optionally further substituted with 1-5 R, provided that when $R^1$ is phenyl, then $R^1$ is further substituted with at least two R and at least one of the R is not a halogen;

R is selected from the group consisting of $R^x$, $-(C_1-C_6\ alkylene)_p-O-(C_1-C_6$ alkylene)p-$(C_6-C_{10}$ aryl), $-(C_1-C_6\ alkylene)_p-O-(C_1-C_6\ alkylene)_p-(C_7-C_{10}$ cycloalkyl), $-(C_1-C_6\ alkylene)_p-O-(C_1-C_6$ alkylene)p-(7-10 member heteroaryl), $-(C_1-C_6\ alkylene)_p-O-(C_1-C_6\ alkylene)_p$-(7-10 member heterocyclyl), $-(C_1-C_6\ alkylene)_p-O-(C_2-C_6$ alkenyl), $-(C_1-C_6\ alkylene)_p-O-(C_2-C_6\ alkenylene)_p-(C_6-C_{10}$ aryl), $-(C_1-C_6\ alkylene)_p-O-(C_2-C_6\ alkenylene)_p-(C_3-C_{10}$ cycloalkyl), $-(C_1-C_6\ alkylene)_p-O-(C_2-C_6\ alkenylene)_p$-(5-10 member heteroaryl), $-(C_1-C_6\ alkylene)_p-O-(C_2-C_6\ alkenylene)_p$-(3-10 member heterocyclyl), $-(C_1-C_6\ alkylene)_p-O-(C_2-C_6$ alkynyl), $-(C_1-C_6\ alkylene)_p-O-(C_2-C_6\ alkynylene)_p-(C_6-C_{10}$ aryl), - $(C_1-C_6\ alkylene)_p-O-(C_2-C_6\ alkynylene)_p-(C_3-C_{10}$ cycloalkyl), $-(C_1-C_6\ alkylene)_p-O-(C_2-C_6\ alkynylene)_p$-(5-10 member heteroaryl) and $-(C_1-C_6\ alkylene)_p-O-(C_2-C_6\ alkynylene)_p$-(3-10 member heterocyclyl);

$R^2$ is selected from the group consisting of $-(C_1-C_6\ alkylene)_p-C(O)-R^b$, $-(C_1-C_6\ alkylene)_p-C(O)-O-R^a$, $-(C_1-C_6\ alkylene)_p-C(O)-N(R^a)_2$,$-(C_1-C_6\ alkylene)_p-S(O)-R^a$, $-(C_1\ C_6\ alkylene)_p-S(O)_2-R^a$, $-(C_1-C_6\ alkylene)_p-S(O)_2-N(R^a)_2$, $-(C_1-C_6\ alkylene)_p-S(O)_2-O-R^a$, and $R^3$, wherein $R^3$ is selected from $-(C_1-C_6\ alkylene)-(C_1-C_3$ perfluoroalkyl), $C_2-C_8$ alkenyl, $C_2-C_8$ alkynyl, $-(C_1-C_6\ alkylene)_p-(C_3-C_{12}$ cycloalkyl), $-(C_1-C_6\ alkylene)_p$-(3-12 member heterocyclyl), $-(C_1-C_6\ alkylene)_p$-(5-12 member heteroaryl) and $-(C_1-C_6\ alkylene)_p-(C_5-C_{12}$ unsaturated nonaromatic carbocyclyl);

each $R^a$ is independently selected from the group consisting of H, $C_1-C_8$ alkyl, $C_2-C_8$ alkenyl, $C_2-C_8$ alkynyl, $C_1-C_8$ perfluoroalkyl, $-(C_1-C_6\ alkylene)_p-(C_6-C_{12}$ aryl), $-(C_1-C_6\ alkylene)_p$-(5 to 12 member heteroaryl), $-(C_1-C_6\ alkylene)_p-(C_3-C_{12}$ cycloalkyl), $-(C_1-C_6\ alkylene)_p$-(3-12 member heterocyclyl), $-(C_1-C_6\ alkylene)_p-(C_5-C_{12}$ unsaturated nonaromatic carbocyclyl);

two $R^a$ attached to the same nitrogen atom, together with the nitrogen atom, may optionally form a 3-12 member heterocyclyl or a 5-12 member heteroaryl; the said 3-12 member heterocyclyl and the said 5-12 member heteroaryl is optionally further substituted by 1-5 $R^x$;

$R^b$ is selected from the group consisting of $-NR^aN(R^a)_2$, $-NR^aOR^a$; $C_2-C_8$ alkenyl, $C_2-C_8$ alkynyl, $C_1-C_8$ perfluoroalkyl, $-(C_3-C_6\ alkylene)-(C_1-C_3$ perfluoroalkyl), $-(C_1-C_6\ alkylene)_p-(C_6-C_{12}$ aryl), $-(C_1-C_6\ alkylene)_p-(C_3-C_{12}$ cycloalkyl), $-(C_1-C_6\ alkylene)_p$-(3-12 member heterocyclyl), $-(C_1-C_6\ alkylene)_p$-(5-12 member heteroaryl), $-(C_1-C_6\ alkylene)_p-(C_5-C_{12}$ unsaturated nonaromatic carbocyclyl);

p is 0 or 1;

each R, $R^a$, $R^b$ and $R^3$ is optionally further substituted with 1-5 $R^x$;

each $R^x$ is independently selected from the group consisting of -oxo-, $-(C_1-C_4$ alkylene)-, halogen, -CN, -OH, $C_1-C_6$ alkyl, $C_2-C_6$ alkenyl, $C_2-C_6$ alkynyl, $C_1-C_6$ perfluoroalkyl, $-(C_1-C_6$ alkylene)-halogen, $-(C_1-C_6$ alkylene)-OH, $-(C_1-C_6$ alkylene)-CN, - $(C_1-C_6\ alkylene)_q-(C_3-C_6$ cycloalkyl), $-(C_1-C_6\ alkylene)_q$-(3-6 member heterocyclyl), $-(C_1-C_6\ alkylene)_q$-(5-6 member heteroaryl), $-(C_1-C_6\ alkylene)_q-C(O)-(C_1-C_6$ alkyl), $-(C_1-C_6\ alkylene)_q-C(O)-(C_3-C_6$ cycloalkyl), $-(C_1-C_6$ alkylene)q-C(O)-$(C_1-C_6$ alkylene)-$(C_3-C_6$ cycloalkyl), $-(C_1-C_6\ alkylene)_q-C(O)-O-(C_1-C_6$ alkyl), $-(C_1-C_6\ alkylene)_q-C(O)-NH-(C_1-C_6$ alkyl), $-(C_1-C_6\ alkylene)_q-C(O)-N(C_1-C_6\ alkyl)(C_1-C_6$ alkyl), $-(C_1-C_6\ alkylene)_q-O-(C_1-C_6$ alkyl), $-(C_1-C_6\ alkylene)_q-O-(C_1-C_6$ alkylene)-halogen, $-(C_1-C_6\ alkylene)_q-O-(C_1-C_6$ alkylene)-$(C_1-C_3$ perfluoroalkyl), $-(C_1-C_6\ alkylene)_q-O-(C_1-C_6$ alkylene)q-$(C_3-C_6$ cycloalkyl), $-(C_1-C_6\ alkylene)_q-O-(C_1-C_6\ alkylene)_q$-(3-6 member heterocyclyl), $-(C_1-C_6\ alkylene)_q-O-(C_1-C_6\ alkylene)_q$-(5-6 member heteroaryl), $-(C_1-C_6\ alkylene)_q-O-(C_1-C_6\ alkylene)-NH_2$, $-(C_1-C_6\ alkylene)_q-O-(C_1-C_6\ alkylene)-NH-(C_1-C_6$ alkyl), $-(C_1-C_6\ alkylene)_q-O-(C_1-C_6\ alkylene)-NH-(C_3-C_6$ cycloalkyl), $-(C_1-C_6\ alkylene)_q-O-(C_1-C_6\ alkylene)-N(C_1-C_6\ alkyl)_2$, $-(C_1-C_6\ alkylene)_q-NH_2$, $-(C_1-C_6\ alkylene)_q-NH-(C_1-C_6$ alkyl), - $(C_1-C_6\ alkylene)_q-NH-(C_3-C_6$ cycloalkyl), $-(C_1-C_6\ alkylene)_q-N(C_1-C_6\ alkyl)(C_1-C_6$ alkyl), - $(C_1-C_6$ alkylene)q- NHC(O)-$(C_1-C_6$ alkyl), $-(C_1-C_6\ alkylene)_q-NH-SO_2-(C_1-C_6$ alkyl), $-(C_1-C_6\ alkylene)_q-SO_2-(C_1-C_6$ alkyl), $-(C_1-C_6\ alkylene)_q-SO_2-(C_1-C_3\ alkylene)_q-(C_3-C_6$ cycloalkyl), $-(C_1-C_6\ alkylene)_q-SO_2-NH_2$, $-(C_1-C_6\ alkylene)_q-SO_2-NH(C_1-C_3$ alkyl), $-(C_1-C_6\ alkylene)_q-SO_2-NH-(C_1-C_3\ alkylene)_q-(C_3-C_6$ cycloalkyl) and $-(C_1-C_6\ alkylene)_q-SO_2-N(C_1-C_3\ alkyl)_2$; each q is independently 0 or 1; where permissible, each carbon atom of $R^x$ is optionally further substituted by 1-3 fluorine;

or a pharmaceutically acceptable salt thereof.

In a preferred aspect of this embodiment, and in combination of any other aspects not inconsistent, m is 1, n is 1, and the compound is of formula II,

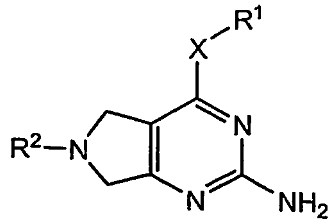

II

In another preferred aspect of this embodiment, and in combination of any other aspects not inconsistent, X is a bond or -O-, $R^1$ is $C_6$-$C_{12}$ aryl, 5 to 12 member heteroaryl, or 3-12 member heterocyclyl, and $R^1$ is further substituted with 2-5 R. More preferably, X is a bond and $R^1$ is a $C_6$-$C_{12}$ aryl further substituted with 2-5 R. Even more preferably, $R^1$ is phenyl further substituted with 2-5 R and at least one of the R is not a halogen.

In another preferred aspect of this embodiment, and in combination of any other aspects not inconsistent, R is selected from the group consisting of F, Cl, Br, -OH, -CN, $C_1$-$C_3$ alkyl, $C_1$-$C_3$ perfluoroalkyl, -($C_1$-$C_6$ alkylene)-OH, -O-($C_1$-$C_6$ alkyl), -($C_1$-$C_6$ alkylene)-O-($C_1$-$C_6$ alkyl), -($C_1$-$C_6$ alkylene)$_p$-O-($C_1$-$C_6$ alkylene)$_p$-($C_6$-$C_{10}$ aryl), -($C_1$-$C_6$ alkylene)p-O-($C_1$-$C_6$ alkylene)$_p$-($C_3$-$C_{10}$ cycloalkyl), -($C_1$-$C_6$ alkylene)$_p$-O-($C_1$-$C_6$ alkylene)$_P$-(5-10 member heteroaryl), -($C_1$-$C_6$ alkylene)$_p$-O-($C_1$-$C_6$ alkylene)$_p$-(3-10 member heterocyclyl), -($C_1$-$C_6$ alkylene)$_p$-O-($C_2$-$C_6$ alkenyl), -($C_1$-$C_6$ alkylene)$_p$-O-($C_2$-$C_6$ alkenylene)$_p$-($C_6$-$C_{10}$ aryl), -($C_1$-$C_6$ alkylene)$_p$-O-($C_2$-$C_6$ alkenylene)$_p$-($C_3$-$C_{10}$ cycloalkyl), -($C_1$-$C_6$ alkylene)$_p$-O-($C_2$-$C_6$ alkenylene)$_p$-(5-10 member heteroaryl), -($C_1$-$C_6$ alkylene)$_p$-O-($C_2$-$C_6$ alkenylene)$_p$-(3-10 member heterocyclyl), -($C_1$-$C_6$ alkylene)$_p$-O-($C_2$-$C_6$ alkynyl), -($C_1$-$C_6$ alkylene)$_p$-O-($C_2$-$C_6$ alkynylene)$_p$-($C_6$-$C_{10}$ aryl), -($C_1$-$C_6$ alkylene)$_p$-O-($C_2$-$C_6$ alkynylene)$_p$-($C_3$-$C_{10}$ cycloalkyl), -($C_1$-$C_6$ alkylene)$_p$-O-($C_2$-$C_6$ alkynylene)$_p$-(5-10 member heteroaryl) and -($C_1$-$C_6$ alkylene)$_p$-O-($C_2$-$C_6$ alkynylene)$_p$-(3-10 member heterocyclyl); wherein each R is optionally further substituted by 1-5 $R^x$.

In another preferred aspect of this embodiment, and in combination with any other aspects not inconsistent, m is 1, n is 1 and the compound is of formula V,

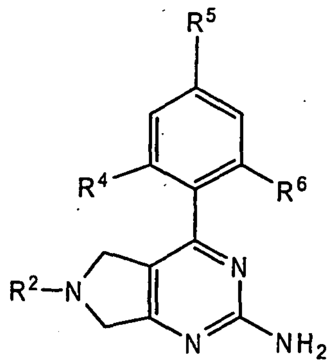

V

wherein

$R^4$ and $R^5$ are independently F, Cl, Br, -OH, -CN, unsubstituted $C_1$-$C_3$ alkyl, $C_1$-$C_3$ perfluoroalkyl, unsubstituted -($C_1$-$C_6$ alkylene)-OH or unsubstituted -O-($C_1$-$C_6$ alkyl);

$R^6$ is selected from the group consisting of -($C_1$-$C_6$ alkylene)-OH, -O-($C_1$-$C_6$ alkyl), -($C_1$-$C_6$ alkylene)-O-($C_1$-$C_6$ alkyl), -($C_1$-$C_6$ alkylene)$_p$-O-($C_1$-$C_6$ alkylene)$_p$-($C_6$-$C_{10}$ aryl), -($C_1$-$C_6$ alkylene)$_p$-O-($C_1$-$C_6$ alkylene)$_p$-($C_3$-$C_{10}$ cycloalkyl), -($C_1$-$C_6$ alkylene)$_p$-O-($C_1$-$C_6$ alkylene)$_p$-(5-10 member heteroaryl), -($C_1$-$C_6$ alkylenl)$_p$-O-($C_1$-$C_6$ alkylene)$_p$-(3-10 member heterocyclyl), -($C_1$-$C_6$ alkyiene)$_p$-O-($C_2$-$C_6$ alkenyl), -($C_1$-$C_6$ alkylene)$_p$-O-($C_2$-$C_6$ alkenylenO)$_p$-($C_6$-$C_{10}$ aryl), - -($C_1$-$C_6$ alkylene)$_p$-O-($C_2$-$C_6$ alkenylene)$_p$-($C_6$-$C_{10}$ cycloalkyl), -($C_1$-$C_6$ alkyiene)$_p$-O-($C_2$-$C_6$ alkenylene)$_p$-(5-10 member heferoaryl); -($C_1$-$C_6$ alkylene)$_p$-O-($C_2$-$C_6$ alkenyiene)$_p$-(3-10 member heterocyclyl), -($C_1$-$C_6$ alkylene)$_p$-O-($C_2$-$C_6$ alkynyl), -($C_1$-$C_6$ alkylene)$_p$-O-($C_2$-$C_6$ alkynylene)$_p$-($C_6$-$C_{10}$ aryl), -($C_1$-$C_6$ alkylene)$_p$-O-($C_2$-$C_6$ alkynylene)$_p$-($C_3$-$C_{10}$ cycloalkyl), -($C_1$-$C_6$ alkylene)$_p$-O-($C_2$-$C_6$ alkynylene)$_p$-(5-10 member heteroaryl) and -($C_1$-$C_6$ alkylene)$_P$-O-($C_2$-$C_6$ alkynylene)$_p$-(3-10 member heterocyclyl); and $R^6$ is optionally further substituted with 1-5 $R^x$.

In another preferred aspect of this embodiment, and in combination with any other aspects not inconsistent, m is 1, n is 1 and the compound is of formula VI,

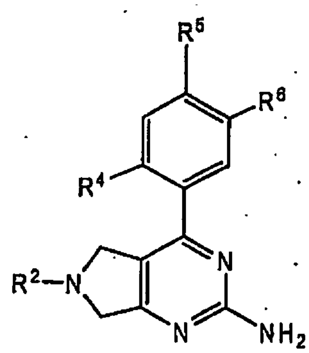

VI

wherein

$R^4$ and $R^5$ are Independently F, Cl, Br, -OH, -CN, unsubstituted $C_1$-$C_3$ alkyl, $C_1$-$C_3$ perfluoroalkyl, unsubstituted -($C_1$-$C_6$ alkylene)-OH or unsubstituted -O-($C_1$-$C_6$ alkyl);
$R^6$ is selected from the group consisting of -($C_1$-$C_6$ alkylene)-OH, -O-($C_1$-$C_6$, alkyl), -($C_1$-$C_6$ alkylene)-O-($C_1$-$C_6$ alkyl), -($C_1$-$C_6$ alkylene)$_p$-O-($C_1$-$C_6$ alkylene)$_p$($C_6$-$C_{10}$ aryl), -($C_1$-$C_6$ alkylene)$_p$-O-($C_1$-$C_6$ alkylene)$_p$-($C_3$-$C_{10}$ cycloalkyl), -($C_1$-$C_6$ alkylene)$_p$-O-($C_1$-$C_6$ alkylene)$_p$-(5-10 member heteroaryl), -($C_1$-$C_6$ alkylene)$_p$-O-($C_1$-$C_6$ alkylene)$_p$-(3-10 member heterocyclyl), -($C_1$-$C_6$ alkylene)p-O-($C_2$-$C_6$ alkenyl), -($C_1$-$C_6$ alkylene)$_p$-O-($C_2$-$C_6$ alkenylene)$_p$-($C_6$-$C_{10}$ aryl), -($C_1$-$C_6$ alkylene)$_p$-O-($C_2$-$C_6$ alkenylene)$_p$-($C_3$-$C_{10}$ cycloalkyl), -($C_1$-$C_6$ alkylene)$_p$-O-($C_2$-$C_6$ alkenylene)$_p$-(5-10 member heteroaryl), -($C_1$-$C_6$ alkylene)$_p$-O-($C_2$-$C_6$ alkenylene)$_p$-(3-10 member heterocyclyl), -($C_1$-$C_6$ alkylene)$_p$-O-($C_2$-$C_6$ alkynyl), -($C_1$-$C_6$ alkylene)$_p$-O-($C_2$-$C_6$ alkynylene)$_p$-($C_6$-$C_{10}$ aryl), -($C_1$-$C_6$ alkylene)p-O-($C_2$-$C_6$ alkynylene)$_p$-($C_3$-$C_{10}$ cycloalkyl), -($C_1$-$C_6$ alkylene)$_p$-O-($C_2$-$C_6$ alkynylene)$_p$-(5-10 member heteroaryl) and -($C_1$-$C_6$ alkylene)$_p$-O-($C_2$-$C_6$ alkynylene)$_p$-(3-10 member heterocyclyl); and $R^6$ is optionally further substituted with 1-5 $R^x$.

In formula V and formula VI, preferably, $R^6$ is -O-($C_1$-$C_6$ alkylene)-(5-10 member heteroaryl), and $R^6$ is optionally further substituted with 1-5 $R^x$; more preferably, $R^6$ is - O-($C_1$-$C_6$ alkylene)-(5 member heteroaryl), and $R^6$ is optionally further substituted with 1-5 $R^x$; also preferably, $R^6$ is -O-($C_1$-$C_6$ alkylene)-(3-10 member heterocyclyl), and $R^6$ is optionally further substituted with 1-5 $R^x$; also preferably, $R^6$ is -O-($C_1$-$C_6$ alkylene)-phenyl, and $R^6$ is optionally further substituted with 1-5 $R^x$; also prefereably, $R^6$ is -O-($C_1$-$C_6$ alkyl) or -O-($C_2$-$C_6$ alkenyl), and $R^6$ is optionally further substutituted with 1-5 $R^X$. Preferrred substituent of $R^6$ is F, Cl, $C_1$-$C_3$ alkyl, -O-($C_1$-$C_3$ alkyl), -OH, -CN and -($C_1$-$C_3$ alkylene)-CN. Most preferred $R^6$ includes the following group, wherein each group is unsubstituted or substituted with 1-5 $R^x$; preferably, the following group is unsubstituted or substituted with 1-5 groups of F, Cl, $C_1$-$C_3$ alkyl, -O-($C_1$-$C_3$ alkyl), -OH, -CN or -($C_1$-$C_3$ alkylene)-CN:

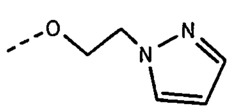
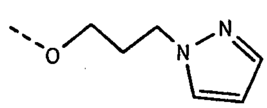
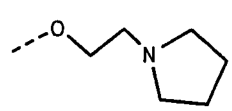
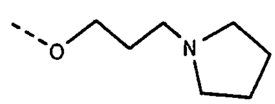

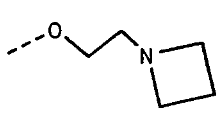
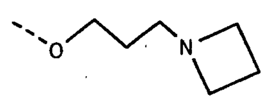

-O-($C_1$-$C_4$ alkyl), -O-($C_2$-$C_4$ alkenyl)

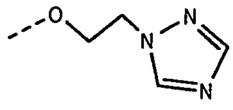 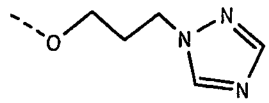 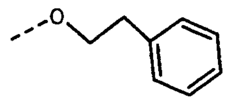 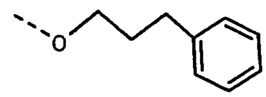

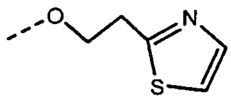 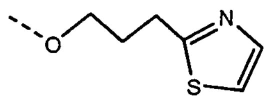 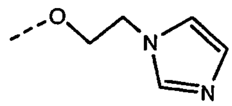 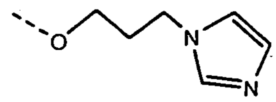

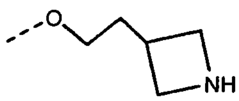 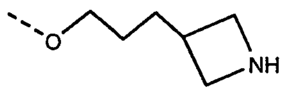 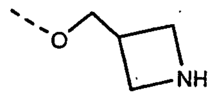 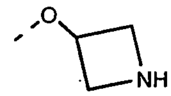

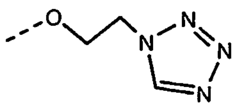 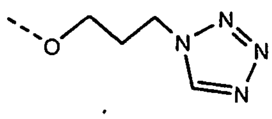 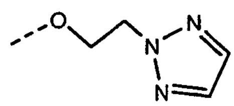 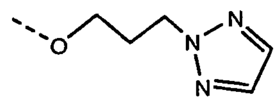

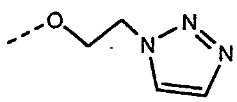 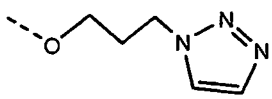 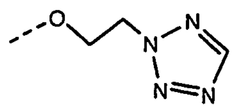 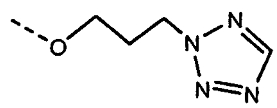

In another preferred aspect of this embodiment, and in combination with any other aspects not inconsistent, particularly in combination with the aspect wherein the compound is of formula V, also particularly in combination with the aspect wherein the compound is of formula VI, $R^2$ is -C(O)-N($R^a$)$_2$. Preferably, a first $R^a$ is H or unsubstituted $C_1$-$C_3$ alkyl; and a second $R^a$ is selected from $C_1$-$C_6$ alkyl, cyclopropyl, cyclobutyl, bicyclo[1.1.1]pent-1-yl, (1S,5R)-3-aza-bicyclo[3.1.0]hex-6-yl, 5-6 membered heteroaryl, 3-7 member heterocyclyl and $C_2$-$C_6$ alkenyl, and the second $R^a$ is optionally further substituted by 1-5 groups selected from F, Cl, $C_1$-$C_3$ alkyl, -O-($C_1$-$C_3$ alkyl), -OH, -CN and -($C_1$-$C_3$ alkylene)-CN. More preferably, the first $R^a$ is H.

In another preferred aspect of this embodiment, and in combination with any other aspects not inconsistent, particularly in combination with the aspect wherein the compound is of formula V, also particularly in combination with the aspect wherein the compound is of formula VI, $R^2$ is -C(O)-O$R^a$. Preferably, $R^a$ is selected from $C_1$-$C_6$ alkyl, cyclopropyl, cyclobutyl, bicyclo[1.1.1] pent-1-yl, 5-6 membered heteroaryl, 3-7 member heterocyclyl and $C_2$-$C_6$ alkenyl, and $R^a$ is optionally further substituted by 1-5 groups selected from F, Cl, $C_1$-$C_3$ alkyl, -O-($C_1$-$C_3$ alkyl), -OH, -CN and -($C_1$-$C_3$ alkylene)-CN.

In another embodiment, the present invention provides a compound of formula (I)

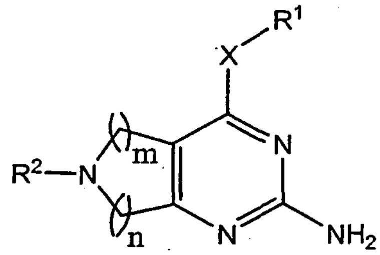

(I)

wherein:

m is 1 or 2, n is 1 or 2, when m is 2, n is 1;
X is a bond or a diradical selected from the group consisting of -O-, -S-, -($C_1$-$C_3$ alkylene)-, -O-($C_1$-$C_3$ alkylene)-, -NH-($C_1$-$C_3$ alkylene)-, -S-($C_1$-$C_3$ alkylene)-, -C(O)-, - C(O)-O-, -C(O)-NH-, -OC(O)-NH-, -NH-C(O)-NH-, -S(O)-, -S (O)$_2$-, -S(O)$_2$-O- and -S(O)$_2$-NH-, wherein each end of the diradical may be connected to $R^1$ or to the aminopyrimidine ring of formula I;
where permissible, each nitrogen or carbon atom of X is optionally further substituted by one group selected from -($C_1$-$C_3$ alkylene)$_t$-CN, -($C_1$-$C_3$ alkylene)$_t$-F, -($C_1$-$C_3$ alkylene)$_t$-($C_1$-$C_3$ perfluoroalkyl), -($C_1$-$C_3$ alkylene)$_t$-O-($C_1$-$C_6$ alkyl), -($C_1$-$C_3$ alkylene)$_t$-OH, -($C_1$-$C_3$ alkylene)$_t$-NH$_2$, -($C_1$-$C_3$ alkylene)$_t$-NH($C_1$-$C_3$ alkyl) and -($C_1$-$C_3$ alkylene)$_t$-N ($C_1$-$C_3$ alkyl)($C_1$-$C_3$ alkyl), and t is 0 or 1;
$R^1$ is selected from the group consisting of $C_6$-$C_{12}$ aryl, 5 to 12 member heteroaryl, $C_3$-$C_{12}$ cycloalkyl, 3-12 member heterocyclyl and $C_5$-$C_{12}$ unsaturated nonaromatic carbocyclyl, and each $R^1$ is optionally further substituted with 1-5 $R^x$, provided that when $R^1$ is phenyl, $R^1$ is further substituted with at least two $R^x$ and one of the $R^x$ is not a halogen; preferred substituents of $R^1$ are selected from F, Cl, B.r, -OH, -CN, $C_1$-$C_3$ alkyl, $C_1$-$C_3$ perfluoroalkyl, -($C_1$-$C_6$ alkylene)-OH, -O-($C_1$-$C_6$ alkyl), -O-($C_1$-$C_6$ alkylene)-halogen, -O-($C_1$-$C_6$ alkylene)-($C_1$-$C_3$ perfluoroalkyl), -O-($C_1$-$C_6$ alkylene)-N($C_1$-$C_6$ alkyl)$_2$, -O-($C_1$-$C_6$ alkylene)-NH$_2$ and -O-($C_1$-$C_6$ alkylene)-NH($C_1$-$C_6$ alkyl);
$R^2$ is selected from the group consisting of -($C_1$-$C_6$ alkylene)$_p$-C(O)-$R^b$ , -($C_1$-$C_6$ alkylene)$_p$-C(O)-O-$R^a$, -($C_1$-$C_6$ alkylene)$_p$-C(O)-N($R^a$)$_2$, -($C_1$-$C_6$ alkylene)$_p$-S(O)-$R^a$, -($C_1$-$C_6$ alkylene)p-S(O)$_2$-$R^a$, -($C_1$-$C_6$ alkylene)$_p$-S(O)$_2$-N ($R^a$)$_2$, -($C_1$-$C_6$ alkylene)$_p$-S(O)$_2$-O-$R^a$, and $R^3$, wherein $R^3$ is selected from -($C_1$-$C_6$ alkylene)-($C_1$-$C_3$ perfluoroalkyl), $C_2$-$C_8$ alkynyl, -($C_1$-$C_6$ alkylene)$_p$-($C_3$-$C_{12}$ cycloalkyl), -($C_1$-$C_6$ alkylene)$_p$-(3-12 member heterocyclyl), -($C_1$-$C_6$ alkylene)$_p$-(5-12 member heteroaryl) and -($C_1$-$C_6$ alkylene)$_p$-($C_5$-$C_{12}$ unsaturated nonaromatic carbocyclyl);
each $R^a$ is independently selected from the group consisting of H, $C_1$-$C_6$ alkyl, $C_2$-$C_8$ alkenyl, $C_2$-$C_8$ alkynyl, $C_1$-$C_6$ perfluoroalkyl, -($C_1$-$C_6$ alkylene)$_p$-($C_6$-$C_{12}$ aryl), -($C_1$-$C_6$ alkylene)$_p$-(5 to 12 member heteroaryl), -($C_1$-$C_6$ alkylene)$_p$-($C_3$-$C_{12}$ cycloalkyl), -($C_1$-$C_6$ alkylene)$_p$-(3-12 member heterocyclyl), -($C_1$-$C_6$ alkylene)$_p$-($C_5$-$C_{12}$ unsaturated nonaromatic carbocyclyl);
two $R^a$ attached to the same nitrogen atom, together with the nitrogen atom, may optionally form a 3-12 member heterocyclyl or a 5-12 member heteroaryl; the said 3-12 member heterocyclyl and the said 5-12 member heteroaryl is optionally further substituted by 1-5 $R^x$;
$R^b$ is selected from the group consisting of $C_2$-$C_8$ alkenyl, $C_2$-$C_8$ alkynyl, $C_1$-$C_8$ perfluoroalkyl, -($C_3$-$C_6$ alkylene)-($C_1$-$C_3$ perfluoroalkyl), -($C_1$-$C_6$ alkylene)$_p$-($C_6$-$C_{12}$aryl), - ($C_1$-$C_6$ alkylene)$_p$-($C_3$-$C_{12}$ cycloalkyl), -($C_1$-$C_6$ alkylene)$_p$-(3-12 member heterocyclyl), - ($C_1$-$C_6$ alkylene)$_p$-(5-12 member heteroaryl), -($C_1$-$C_6$ alkylene)$_p$-($C_5$-$C_{12}$ unsaturated nonaromatic carbocyclyl);
p is 0 or 1; each $R^a$, $R^b$ and $R^3$ is optionally further substituted with 1-5 $R^x$;
each $R^x$ is independently selected from the group consisting of -oxo-, -($C_1$-$C_4$ alkylene)-, halogen, -CN, -OH, $C_1$-$C_6$ alkyl, $C_2$-$C_6$ alkenyl, $C_2$-$C_6$ alkynyl, $C_1$-$C_6$ perfluoroalkyl, -($C_1$-$C_6$ alkylene)-halogen, -($C_1$-$C_6$ alkylene)-OH, -($C_1$-$C_6$ alkylene)-CN, - ($C_1$-$C_6$ alkylene)$_q$-($C_3$-$C_6$ cycloalkyl), -($C_1$-$C_6$ alkylene)$_q$-(3-6 member heterocyclyl), -($C_1$-$C_6$ alkylene)$_q$-(5-6 member heteroaryl), -($C_1$-$C_6$ alkylene)$_q$-C(O)-($C_1$-$C_6$ alkyl), -($C_1$-$C_6$ alkylene)$_q$-C(O)-($C_3$-$C_6$ cycloalkyl), -($C_1$-$C_6$ alkylene)$_q$-C(O)-($C_1$-$C_6$ alkylene)-($C_3$-$C_6$ cycloalkyl), -($C_1$-$C_6$ alkylene)$_q$-C(O)-O-($C_1$-$C_6$ alkyl), -($C_1$-$C_6$ alkylene)$_q$-C(OFNH-($C_1$-$C_6$ alkyl), -($C_1$-$C_6$ alkylene)$_q$-C(O)-N($C_1$-$C_6$ alkyl)($C_1$-$C_6$ alkyl), -($C_1$-$C_6$ alkylene)$_q$-O-($C_1$-$C_6$ alkyl), -($C_1$-$C_6$ alkylene)$_q$-O-($C_1$-$C_6$ alkylene)-halogen, -($C_1$-$C_6$ alkylene)$_q$-O-($C_1$-$C_6$ alkylene)-($C_1$-$C_3$ perfluoroalkyl), -($C_1$-$C_6$ alkylene)$_q$-O-($C_1$-$C_6$ alkylene)$_q$-($C_3$-$C_6$ cycloalkyl), -($C_1$-$C_6$ alkylene)$_q$-O-($C_1$-$C_6$ alkylene)$_q$-(3-6 member heterocyclyl), -($C_1$-$C_6$ alkylene)$_q$-O-($C_1$-$C_6$ alkylene)$_q$-(5-6 member heteroaryl), -($C_1$-$C_6$ alkylene)$_q$-O-($C_1$-$C_6$ alkylene)-NH$_2$, -($C_1$-$C_6$ alkylene)$_q$-O-($C_1$-$C_6$ alkylene)-NH-($C_1$-$C_6$ alkyl), -($C_1$-$C_6$ alkylene)$_q$-O-($C_1$-$C_6$ alkylene)-NH-($C_3$-$C_6$ cycloalkyl), -($C_1$-$C_6$ alkylene)$_q$-O-($C_1$-$C_6$ alkylene)-N($C_1$-$C_6$

alkyl)$_2$, -($C_1$-$C_6$ alkylene)$_q$-$NH_2$, -($C_1$-$C_6$ alkylene)$_q$-NH-($C_1$-$C_6$ alkyl), - ($C_1$-$C_6$ alkylene)q-NH-($C_3$-$C_6$ cycloalkyl). -($C_1$-$C_6$ alkylene)$_q$-N($C_1$-$C_6$ alkyl)($C_1$-$C_6$ alkyl),-($C_1$-$C_6$ alkylene)$_q$- NHC(O)-($C_1$-$C_6$ alkyl), -($C_1$-$C_6$ alkylene)$_q$-NH-$SO_2$-($C_1$-$C_6$ alkyl), -($C_1$-$C_6$ alkylene)$_q$-$SO_2$-($C_1$-$C_6$ alkyl), -($C_1$-$C_6$ alkylene)$_q$-$SO_2$-($C_1$-$C_3$ alkylene)$_q$-($C_3$-$C_6$ cycloalkyl), -($C_1$-$C_6$ alkylene)$_q$-$SO_2$-$NH_2$, -($C_1$-$C_6$ alkylene)$_q$-$SO_2$-NH($C_1$-$C_3$ alkyl), -($C_1$-$C_6$ alkylene)$_q$-$SO_2$-NH-($C_1$-$C_3$ alkylene)$_q$-($C_3$-$C_6$ cycloalkyl) and -($C_1$-$C_6$ alkylene)$_q$-$SO_2$-N($C_1$-$C_3$ alkyl)$_2$; each q is independently 0 or 1; where permissible, each carbon atom of $R^x$ is optionally further substituted by 1-3 fluorine;

or a pharmaceutically acceptable salt thereof.

In the first particular aspect of this embodiment, and in combination with any other particular aspects not inconsistent, m is 1 and n is 1, and the compound is of formula II,

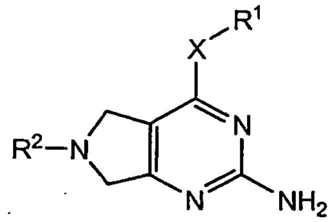

II

In the second particular aspect of this embodiment, and in combination with any other particular aspects not inconsistent, m is 1 and n is 2, and the compound is of formula III,

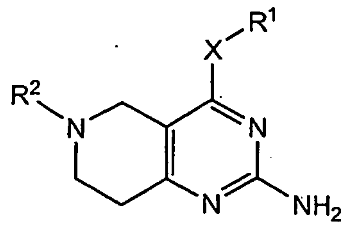

III

In the third particular aspect of this embodiment, and in combination with any other particular aspects not inconsistent, m is 2 and n is 1, and the compounds is of formula IV,

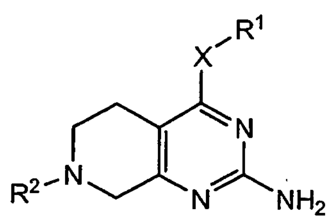

IV

In the fourth particular aspect of this embodiment, and in combination with any other particular aspects not inconsistent, especially in combination with particular aspect one, two or three, X is a bond and $R^1$ is a $C_6$-$C_{12}$ aryl optionally substituted with 1-5 $R^x$. Preferably, $R^1$ is phenyl substituted with 2-5 $R^x$ and at least one of the $R^x$ is not a halogen. More preferably, $R^1$ is phenyl substituted with 2-3 groups selected from F, Cl, Br, -OH, -CN, $C_1$-$C_3$ alkyl, $C_1$-$C_3$ perfluoroalkyl, -($C_1$-$C_6$ alkylene)-OH, -O-($C_1$-$C_6$ alkyl), -O-($C_1$-$C_6$ alkylene)-halogen, -O-($C_1$-$C_6$ alkylene)-($C_1$-$C_3$ perfluoroalkyl), -O-($C_1$-$C_6$ alkylene)-N($C_1$-$C_6$ alkyl)$_2$, -O-($C_1$-$C_6$ alkylene)-$NH_2$ and -O-($C_1$-$C_6$ alkylene)-NH($C_1$-$C_6$ alkyl), provided that at least one of the said 2-3 groups is not a halogen.

In the fifth particular aspect of this embodiment, and in combination with any other particular aspects not

inconsistent, especially in combination with particular aspect one, two or three, X is a bond, $R^1$ is 5-12 member heteroaryl, and $R^1$ is optionally further substituted with 1-5 $R^x$

In the sixth particular aspect of this embodiment, and in combination with any other particular aspects not inconsistent, especially in combination with particular aspect one, two or three, X is a bond, $R^1$ is 3-12 member heterocyclyl, and $R^1$ is optionally further substituted with 1-5 $R^x$.

In the seventh particular aspect of this embodiment, and in combination with any other particular aspects not inconsistent, especially in combination with particular aspect one, two or three, X is a bond, $R^1$ is $C_3$-$C_{12}$ cycloalkyl, and $R^1$ is optionally further substituted with 1-5 $R^x$.

In the eighth particular aspect of this embodiment, and in combination with any other particular aspects not inconsistent, especially in combination with particular aspect one, two or three, X is -O- or unsubstituted -O-($C_1$-$C_3$ alkylene)-, $R^1$ is $C_6$-$C_{12}$ aryl, and $R^1$ is optionally further substituted with 1-5 $R^x$.

In the ninth particular aspect of this embodiment, and in combination with any other particular aspects not inconsistent, especially in combination with particular aspect one, two or three, X is -O- or unsubstituted -O-($C_1$-$C_3$ alkylene)-, $R^1$ is 5-12 member heteroaryl, and $R^1$ is optionally further substituted with 1-5 $R^x$.

In the tenth particular aspect of this embodiment, and in combination with any other particular aspects not inconsistent, especially in combination with particular aspect one, two or three, X is -O- or unsubstituted -O-($C_1$-$C_3$ alkylene)-, $R^1$ is 3-12 member heterocyclyl; and $R^1$ is optionally further substituted with 1-5 $R^x$.

In the eleventh particular aspect of this embodiment, and in combination with any other particular aspects not inconsistent, especially in combination with particular aspect one, two or three, X is -O- or unsubstituted -O-($C_1$-$C_3$ alkylene)-, $R^1$ is $C_3$-$C_{12}$ cycloalkyl, and $R^1$ is optionally further substituted with 1-5 $R^x$.

In the twelfth particular aspect of this embodiment, and in combination with any other particular aspects not inconsistent, especially in combination with particular aspect one, two or three, X is unsubstituted -NH-($C_1$-$C_3$ alkylene)-, $R^1$ is $C_6$-$C_{12}$ aryl, and $R^1$ is optionally further substituted with 1-5 $R^x$.

In the thirteenth particular aspect of this embodiment, and in combination with any other particular aspects not inconsistent, especially in combination with particular aspect one, two or three, X is unsubstituted -NH-($C_1$-$C_3$ alkylene)-, $R^1$ is 5-12 member heteroaryl, and $R^1$ is optionally further substituted with 1-5 $R^x$.

In the fourteenth particular aspect of this embodiment, and in combination with any other particular aspects not inconsistent, especially in combination with particular aspect one, two or three, X is unsubstituted -NH-($C_1$-$C_3$ alkylene)-, $R^1$ is 3-12 ember heterocyclyl, and $R^1$ is optionally further substituted with 1-5 $R^x$.

In the fifteenth particular aspect of this embodiment, and in combination with any other particular aspects not inconsistent, especially in combination with particular aspect one, two or three, X is unsubstituted -NH-($C_1$-$C_3$ alkylene)-, $R^1$ is $C_3$-$C_{12}$ cycloalkyl, and $R^1$ is optionally further substituted with 1-5 $R^x$.

In the sixteenth particular aspect of this embodiment, and in combination with any other particular aspects not inconsistent, especially in combination with particular aspect one, two or three, wherein X is unsubstituted -($C_1$-$C_3$ alkylene)-, $R^1$ is $C_6$-$C_{12}$ aryl, and $R^1$ is optionally further substituted with 1-5 $R^x$.

In the nineteenth particular aspect of this embodiment, and in combination with any other particular aspects not inconsistent, especially in combination with particular aspect one, two or three, X is unsubstituted -($C_1$-$C_3$ alkylene)-, $R^1$ is 5-12 member heteroaryl, and $R^1$ is optionally further substituted with 1-5 $R^x$.

In the twentieth particular aspect of this embodiment, and in combination with any other particular aspects not inconsistent, especially in combination with particular aspect one, two or three, X is unsubstituted -($C_1$-$C_3$ alkylene)-, $R^1$ is 3-12 member heterocyclyl, and $R^1$ is optionally further substituted with 1-5 $R^x$.

In the twenty-first particular aspect of this embodiment, and in combination with any other particular aspects not inconsistent, especially in combination with particular aspect one, two or three, X is unsubstituted -($C_1$-$C_3$ alkylene)-, $R^1$ is $C_3$-$C_{12}$ cycloalkyl, and $R^1$ is optionally further substituted with 1-5 $R^x$.

In the twenty-second particular aspect of this embodiment, and in combination with any other particular aspects not inconsistent, including any of the particular aspect one to twenty-one, $R^2$ is -C(O)-OR$^a$ or -($C_1$-$C_6$ alkyleneyC(O)-O-R$^a$.

In the twenty-third particular aspect of this embodiment, and in combination with any other particular aspects not inconsistent, including any of the particular aspect one to twenty-one, $R^2$ is -C(O)-N($R^a$)$_2$ or -($C_1$-$C_6$ alkylene)-C(O)-N($R^a$)2.

In the twenty-fourth particular aspect of this embodiment, and in combination with any other particular aspects not inconsistent, including any of the particular aspect one to twenty-one,$R^2$ is selected from -S(O)$_2$-R$^a$, -S(O)$_2$-N($R^a$)$_2$, -S(O)$_2$-O-R$^a$, -($C_1$-$C_6$ alkylene)-S(O)$_2$-R$^a$, -($C_1$-$C_6$ alkylene)-S(O)$_2$-O-R$^a$ and -($C_1$-$C_6$ alkylene)-S(O)$_2$-N(R)$_2$.

In the twenty-fifth particular aspect of this embodiment, and in combination with any other particular aspects not inconsistent, including any of the particular aspect one to twenty-one,$R^2$ is -C(O)-R$^b$ or -($C_1$-$C_6$ alkylene)-C(O)-R$^b$.

In another embodiment, the present invention provides a compound of formula 1,

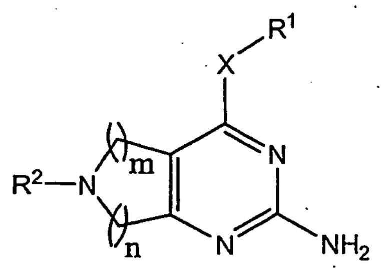

I

wherein:

m is 1 or 2, n is 1 or 2 , when m is 2, n is 1;
X is a bond or a diradical selected from the group consisting of -O- and unsubstituted -O-($C_1$-$C_3$ alkylene)-, wherein each end of the diradical may be connected to $R^1$ orto the aminopyrimidine ring of formula 1;
$R^1$ is phenyl, 5-12 member heteroaryl or 3-12 member heterocyclyl;
when $R^1$ is phenyl, $R^1$ is further substituted with 2-5 groups selected from F, Cl, Br, -OH, -CN, $C_1$-$C_3$ alkyl, $C_1$-$C_3$ perfluoroalkyl, -($C_1$-$C_6$ alkylene)-OH, -O-($C_1$-$C_6$ alkyl), -O-($C_1$-$C_6$ alkylene)-halogen, -O-($C_1$-$C_6$ alkylene)-($C_1$-$C_3$ perfluoroalkyl), -O-($C_1$-$C_6$ alkylene)-N($C_1$-$C_6$ alkyl)$_2$, -O=($C_1$-$C_6$ alkylene)-$NH_2$ and -O-(C1-C6 alkylene)-NH($C_1$-$C_6$ alkyl); and at least one of the substituent of $R^1$ is not a halogen;
when $R^1$ is 5-12 member heteroaryl or 3-12 member heterocyclyl, $R^1$ is optionally further substituted with 1-5 groups selected from F, Cl, Br, -OH, -CN, $C_1$-$C_3$ alkyl, $C_1$-$C_3$ perfluoroalkyl, -($C_1$-$C_6$ alkylene)-OH, -O-($C_1$-$C_6$ alkyl), -O-($C_1$-$C_6$ alkylene)-halogen, -O-($C_1$-$C_6$ alkylene)-($C_1$-$C_3$ perfluoroalkyl), -O-($C_1$-$C_6$ alkylene)-N($C_1$-$C_6$ alkyl)$_2$, -O-($C_1$-$C_6$ alkylene)-$NH_2$ and -O-($C_1$-$C_6$ alkylene)-NH($C_1$-$C_6$ alkyl);
$R^2$ is selected from the group consisting of -($C_1$-$C_6$ alkylene)$_P$-C(O)-$OR^a$, -($C_1$-$C_6$ alkylene)p-C(O)-N($R^a$)$_2$, -($C_1$-$C_6$ alkylene)p-C(O)-$R^b$ and -($C_1$-$C_6$ alkylene)$_p$-$SO_2$-$R^a$;
$R^a$ is selected from the-group consisting of H, $C_1$-$C_8$ alkyl, $C_2$-$C_8$ alkenyl, $C_2$-$C_8$ alkynyl, $C_1$-$C_8$ perfluoroalkyl, -($C_1$-$C_6$ alkylene)$_p$-($C_6$-$C_{12}$ aryl), -($C_1$-$C_6$ alkylene)$_p$-(5 to 12 member heteroaryl), -($C_1$-$C_6$ alkylene)p-($C_3$-$C_{12}$ cycloalkyl), -($C_1$-$C_6$ alkylene)$_p$-(3-12 member heterocyclyl) and -($C_1$-$C_6$ alkylene)$_p$-($C_5$-$C_{12}$ unsaturated nonaromatic carbocyclyl);
two $R^a$ attached to the same nitrogen atom, together with the nitrogen atom, may optionally form a 3-12 member heterocyclyl or a 5-12 member heteroaryl; the said 3-12 member heterocyclyl and the said 5-12 member heteroaryl is optionally further substituted by 1-5 groups selected from $C_1$-$C_3$ alkyl, -CN, -F, -Cl, -Br, -O-($C_1$-$C_3$ alkyl) and $C_1$-$C_3$ perfluoroalkyl;
$R^b$ is selected from the group consisting of $C_2$-$C_8$ alkenyl, $C_2$-$C_8$ alkynyl, $C_1$-$C_8$ perfluoroalkyl, -($C_3$-$C_6$ alkylene)-($C_1$-$C_3$ perfluoroalkyl), -($C_1$-$C_6$ alkylene)$_p$-($C_6$-$C_{12}$ aryl), - ($C_1$-$C_6$ alkylene)$_p$-($C_3$-$C_{12}$ cycloalkyl), -($C_1$-$C_6$ alkylene)$_p$-(3-12 member heterocyclyl), - ($C_1$-$C_6$ alkylene)$_p$-(5-12 member heteroaryl), -($C_1$-$C_6$ alkylene)p-($C_5$-$C_{12}$ unsaturated nonaromatic carbocyclyl);
each $R^a$ and $R^b$ is independently optionally further substituted by 1-5 groups selected from $C_1$-$C_3$ alkyl, -CN, -F, -Cl, -Br, -O-($C_1$-$C_3$ alkyl), $C_1$-$C_3$ perfluoroalkyl, -$NH_2$, - NH-($C_1$-$C_3$ alkyl), -N($C_1$-$C_3$ alkyl)$_2$ and OH;
p is 0 or 1;

or a pharmaceutically acceptable salt thereof.

In the first particular aspect of this embodiment, and in combination with any other particular aspects not inconsistent, m is 1 and n is 1.

In the second particular aspect of this embodiment, and in combination with any other particular aspects not inconsistent, m is 1 and n is 2.

In the third particular aspect of this embodiment, and in combination with any other particular aspects not inconsistent, m is 2 and n is 1.

In the fourth particular aspect of this embodiment, and in combination with any other particular aspects not inconsistent, X is a bond.

In the fifth particular aspect of this embodiment, and in combination with any other particular aspects not inconsistent, X is diradical of -O- or unsubstituted -O-($C_1$-$C_3$ alkylene)-.

In another embodiment, the present invention provides a a compound of formula II,

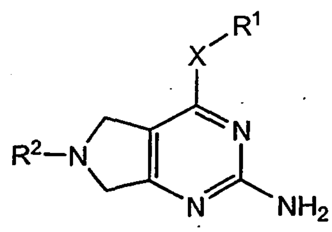

II

wherein:

X is a bond or a diradical selected from the group consisting of -O-, -NH-, -S-, - ($C_1$-$C_3$ alkylene)-, -O-($C_1$-$C_3$ alkylene)-, -NH-($C_1$-$C_3$ alkylene)-, -S-($C_1$-$C_3$ alkylene)-, - C(O)-, -C(O)-O-, -C(O)-NH-, -OC(O)-NH-, -NH-C(O)-NH-, -S(O)-, -$S(O)_2$-, -$S(O)_2$-O- and -$S(O)_2$-NH-, wherein each end of the diradical may be connected to $R^1$ or the aminopyrimidine ring of formula II;

where permissible, each nitrogen or carbon atom of X is optionally further substituted by a group selected from -($C_1$-$C_3$ alkylene)$_t$-CN, -($C_1$-$C_3$ alkylene)$_t$-F, -($C_1$-$C_3$ alkylene)$_t$-($C_1$-$C_3$ perfluoroalkyl), -($C_1$-$C_3$ alkylene)t-O-($C_1$-$C_6$ alkyl), -($C_1$-$C_3$ alkylene)$_t$-OH, -($C_1$-$C_3$ alkylene)$_t$-$NH_2$, -($C_1$-$C_3$ alkylene)$_t$-NH($C_1$-$C_3$ alkyl), -($C_1$-$C_3$ alkylene)$_t$-N($C_1$-$C_3$ alkyl)($C_1$-$C_3$ alkyl), and t is 0 or 1;

$R^1$ is selected from the group consisting of $C_6$-$C_{12}$ aryl, 5 to 12 member heteroaryl, $C_3$-$C_{12}$ cycloalkyl, 3-12 member heterocyclyl and $C_5$-$C_{l2}$ unsaturated nonaromatic carbocyclyl, and each $R^1$ is optionally further substituted with 1-5 $R^x$;

$R^2$ is selected from the group consisting of -($C_1$-$C_6$ alkylene)$_p$-$R^a$, -($C_1$-$C_6$ alkylene)$_p$-C(O)-$R^a$, -($C_1$-$C_6$ alkylene)$_p$-C (O)-O-$R^a$, -($C_1$-$C_6$ alkylene)$_p$-C(O)-N($R^a$)$_2$, -($C_1$-$C_6$ alkylene)$_p$-S(O)-$R^a$, -($C_1$-$C_6$ alkylene)$_p$-$S(O)_2R^a$, -($C_1$-$C_6$ alkylene)$_p$-$S(O)_2$-N($R^a$)$_2$ and - ($C_1$-$C_6$ alkylene)$_p$-S(O)2-O-$R^a$;

each $R^a$ is independently selected from the group consisting of H, $C_1$-$C_8$ alkyl, $C_2$-$C_8$ alkenyl, $C_2$-$C_8$ alkynyl, $C_1$-$C_8$ perfluoroalkyl, -($C_1$-$C_6$ alkylene)$_p$-($C_6$-$C_{12}$ aryl), -($C_1$-$C_6$ alkylene)$_p$-(5 to 12 member heteroaryl), -($C_1$-$C_6$ alkylene)$_p$-($C_3$-$C_{12}$ cycloalkyl), -($C_1$-$C_6$ alkylene)$_p$-(3-12 member heterocyclyl), and -($C_1$-$C_6$ alkylene)p-($C_5$-$C_{12}$ unsaturated nonaromatic carbocyclyl), $R^a$ is optionally further substituted with 1-5 $R^x$;

two $R^a$ attached to the same nitrogen atom, together with the nitrogen atom, may optionally form a 3-12 member heterocyclyl or a 5-12 member heteroaryl; the said 3-12 member heterocyclyl and the said 5-12 member heteroaryl is optionally further substituted by 1-5 $R^x$;

each p is independently 0 or 1;

each $R^x$ is independently selected from the group consisting of -oxo-, -($C_1$-$C_4$ alkylene)-, halogen, -CN, -OH, $C_1$-$C_6$ alkyl, $C_2$-$C_6$ alkenyl, $C_2$-$C_6$ alkynyl, $C_1$-$C_6$ perfluoroalkyl, -($C_1$-$C_6$ alkylene)-halogen, -($C_1$-$C_6$ alkylene)-OH, -($C_1$-$C_6$ alkylene)-CN, - ($C_1$-$C_6$ alkylene)$_q$-($C_3$-$C_6$ cycloalkyl), -($C_1$-$C_6$ alkylene)$_q$-(3-6 member heterocyclyl), -($C_1$-$C_6$ alkylene)$_q$-(5-6 member heteroaryl), -($C_1$-$C_6$ alkylene)$_q$-C(O)-($C_1$-$C_6$ alkyl), -($C_1$-$C_6$ alkylene)$_q$-C(O)-($C_3$-$C_6$ cycloalkyl), -($C_1$-$C_6$ alkylene)$_q$-C(O)-($C_1$-$C_6$ alkylene)-($C_3$-$C_6$ cycloalkyl), -($C_1$-$C_6$ akylene)$_q$-C(O)-O-($C_1$-$C_6$ alkyl), -($C_1$-$C_6$ alkylene)$_q$-C(O)-NH-($C_1$-$C_6$ alkyl), -($C_1$-$C_6$ alkylene)$_q$-C(O)-N($C_1$-$C_6$ alkyl)($C_1$-$C_6$ alkyl), -($C_1$-$C_6$ alkylene)$_q$-O-($C_1$-$C_6$ alkyl), -($C_1$-$C_6$ akylene)$_q$-C(O)-O-($C_1$-$C_6$ alkylene)-halogen, -($C_1$-$C_6$ alkylene)$_q$-O-($C_1$-$C_6$ alkylene)-($C_1$-$C_3$ perfluoroalkyl), -($C_1$-$C_6$ alkylene)$_q$-O-($C_1$-$C_6$ alkylene)$_q$-($C_3$-$C_6$ cycloalkyl), -($C_1$-$C_6$ alkylene)$_q$-O-($C_1$-$C_6$ alkylene)$_q$-(3-6 member heterocyclyl). -($C_1$-$C_6$ alkylene)q-O-($C_1$-$C_6$ alkylene)$_q$-(5-6- member heteroaryl), -($C_1$-$C_6$ alkylene)$_q$-O-($C_1$-$C_6$ alkylene)-$NH_2$, -($C_1$-$C_6$ alkylene)$_q$-O-($C_1$-$C_6$ alkylene)-NH-($C_1$-$C_6$ alkyl), -($C_1$-$C_6$ alkylene)$_q$-O-($C_1$-$C_6$ alkylene)-NH-($C_3$-$C_6$ cycloalkyl), -($C_1$-$C_6$ alkylene)$_q$-O-($C_1$-$C_6$ alkylene)-N($C_1$-$C_6$ alkyl)$_2$, -($C_1$-$C_6$ alkylene)$_q$-$NH_2$, -($C_1$-$C_6$ alkylene)$_q$-NH-($C_1$-$C_6$ alkyl), - ($C_1$-$C_6$ alkylene)$_q$-NH-($C_3$-$C_6$ cycloalkyl), -($C_1$-$C_6$ alkylene)$_q$-N($C_1$-$C_6$ alkyl)($C_1$-$C_6$ alkyl), - ($C_1$-$C_6$ alkylene)$_q$- NHC(O)-($C_1$-$C_6$ alkyl), -($C_1$-$C_6$ alkylene)$_q$-NH-$SO_2$-($C_1$-$C_6$ alkyl), -($C_1$-$C_6$ alkylene)$_q$-$SO_2$-($C_1$-$C_6$ alkyl), -($C_1$-$C_6$ alkylene)$_q$-$SO_2$-($C_1$-$C_3$ alkylene)$_q$-($C_3$-$C_6$ cycloalkyl), -($C_1$-$C_6$ alkylene)$_q$-$SO_2$-$NH_2$, -($C_1$-$C_6$ alkylene)$_q$-$SO_2$-NH($C_1$-$C_3$ alkyl), -($C_1$-$C_6$ alkylene)$_q$-$SO_2$-NH-($C_1$-$C_3$ alkylene)$_q$-($C_3$-$C_6$ cycloalkyl) and -($C_1$-$C_6$ alkylene)$_q$-$SO_2$-N($C_1$-$C_3$ alkyl)$_2$; q is independently 0 or 1; where permissible, each carbon atom of $R^X$ is optionally further substituted by 1-3 fluorine;

or a pharmaceutically acceptable salt thereof.

In the first particular aspect of this embodiment, and in combination with any other particular aspects not inconsistent, X is a bond or a diradical selected from the group consisting of -O- and unsubstituted -O-($C_1$-$C_3$ alkylene)-; $R^x$ is selected from the group consisting of F, Cl, Br, -OH, -CN, $C_1$-$C_3$ alkyl, $C_1$-$C_3$ perfluoroalkyl, -($C_1$-$C_6$ alkylene)-OH, -O-($C_1$-$C_6$ alkyl), -O-($C_1$-$C_6$ alkylene)-halogen, -O-($C_1$-$C_6$ alkylene)-($C_1$-$C_3$ perfluoroalkyl), -O-($C_1$-$C_6$ alkylene)-N($C_1$-$C_6$

alkyl)$_2$, -O-($C_1$-$C_6$ alkylene)-$NH_2$ and -O-($C_1$-$C_6$ alkylene)-NH($C_1$-$C_6$ alkyl. Preferably, X is a bond, $R^1$ is selected from the group consisting of phenyl, 5-12 member heteroaryl and 3-12 member heterocyclyl, $R^1$ is optionally further substituted by 1-5 $R^x$. More preferably, $R^1$ is phenyl optionally further substituted by 1-5 $R^x$.

In the second particular aspect of this embodiment, and in combination with any other particular aspects not inconsistent, X is a diradical -O-($C_1$-$C_3$ alkylene)-, $R^1$ is phenyl or 5-12 member heteroaryl, $R^1$ is optionally further substituted by 1-5 $R^x$.

In the third particular aspect of this embodiment, and in combination with any other particular aspects not inconsistent, X is a diradical -O-, $R^1$ is phenyl optionally further substituted by 1-5 $R^x$.

In another embodiment, the present invention provides a pharmaceutical composition comprising a compound of formule - I, II, III, IV, V or VI, or a pharmaceutically acceptable salt thereof, and a pharmaceutically acceptable carrier.

In another embodiment, the present invention provides a use of a compound of formula I, II, III, IV, V or VI, or a pharmaceutically acceptable salt thereof, in the preparation of a medicament for the treatment of cancer.

In further embodiments of the invention are provided:

A compound of the formula (I), or a pharmaceutically acceptable salt thereof, or a pharmaceutical composition thereof, for use in the treatment of cancer.

• A compound a compound of the formula (I), or a pharmaceutically acceptable salt thereof, or a pharmaceutical composition thereof, for use as a modulator or inhibitor of the activity of HSP-90.

• A compound of the formula (I), or a pharmaceutically acceptable salt thereof, or a pharmaceutical composition thereof, for use in the treatment of a disease associated with abnormal cell growth:

• The use of a compound of the formula (I), or a pharmaceutically acceptable salt thereof, or a pharmaceutical composition thereof, for the manufacture of a medicament for the treatment of cancer.

• The use of a compound of the formula (I), or a pharmaceutically acceptable salt thereof, or a pharmaceutical composition thereof, for the manufacture of a medicament for use as a modulator or inhibitor of the activity of HSP-90.

• The use of a compound of the formula (I), or a pharmaceutically acceptable salt thereof, or a pharmaceutical composition thereof, for the manufacture of a medicament for the treatment of a disease associated with abnormal cell growth.

In one embodiment of this use, the abnormal cell growth is cancer, including, but not limited to, mesothelioma, hepatobilliary (hepatic and billiary duct), a primary or secondary CNS tumor, a primary or secondary brain tumor, lung cancer (NSCLC and SCLC), bone cancer, pancreatic cancer, skin cancer, cancer of the head or neck, cutaneous or intraocular melanoma, ovarian cancer, colon cancer, rectal cancer, cancer of the anal region, stomach cancer, gastrointestinal (gastric, colorectal, and duodenal), breast cancer, uterine cancer, carcinoma of the fallopian tubes, carcinoma of the endometrium, carcinoma of the cervix, carcinoma of the vagina, carcinoma of the vulva, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, testicular cancer, chronic or acute leukemia, chronic myeloid leukemia, lymphocytic lymphomas, cancer of the bladder, cancer of the kidney or ureter, renal cell carcinoma, carcinoma of the renal pelvis, neoplasms of the central nervous system. (CNS), primary CNS lymphoma, non hodgkins's lymphoma, spinal axis tumors, brain stem glioma, pituitary adenoma, adrenocortical cancer, gall bladder cancer, multiple myeloma, cholanglocarcinoma, fibrosarcoma, neuroblastoma; retinoblastoma, or a combination of one or more of the foregoing cancers.

In another embodiment of said use, said abnormal cell growth is a benign proliferative disease, including, but not limited to, psoriasis, benign prostatic hypertrophy or restinosis.

In a preferred embodiment of the present invention the cancer is selected from lung cancer (NSCLC and SCLC), cancer of the head or neck, ovarian cancer, colon cancer, rectal cancer, cancer of the anal region, stomach cancer, breast cancer, cancer of the kidney or ureter, renal cell carcinoma, carcinoma of the renal pelvis, neoplasms of the central nervous system (CNS), primary CNS lymphoma, non hodgkins's lymphoma, spinal axis tumors, or a combination of one or more of the foregoing cancers.

In another preferred embodiment of the present invention the cancer is selected from lung cancer (NSCLC and SCLC), ovarian cancer, colon cancer, rectal cancer, cancer of the anal region, or a combination of one or more of the foregoing cancers. In a more preferred embodiment of the present invention the cancer is selected from lung cancer (NSCLC and SCLC), ovarian cancer, colon cancer, rectal cancer, or a combination of one or more of the foregoing cancers.

In another embodiment of said use, said abnormal cell growth is a benign proliferative disease, including, but

not limited to, psoriasis, benign prostatic hypertrophy or restinosis.

This invention also relates to a pharmaceutical composition for the treatment of abnormal cell growth in a mammal, including a human, comprising an amount of a compound of the Formula I, as defined above, or a pharmaceutically acceptable salt or solvate thereof, that is effective in treating abnormal cell growth, and a pharmaceutical acceptable carrier. In one embodiment of said composition, said abnormal cell growth is cancer, including, but not limited to, mesothelioma, hepatobilliary (hepatic and billiary duct), a primary or secondary CNS tumor, a primary or secondary brain tumor, lung cancer (NSCLC and SCLC), bone cancer, pancreatic cancer, skin cancer, cancer of the head or neck, cutaneous or intraocular melanoma, ovarian cancer, colon cancer, rectal cancer, cancer of the anal region, stomach cancer, gastrointestinal (gastric, colorectal, and duodenal), breast cancer, uterine cancer, carcinoma of the fallopian tubes, carcinoma of the endometrium, carcinoma of the cervix, carcinoma of the vagina, carcinoma of the vulva, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, testicular cancer, chronic or acute leukemia, chronic myeloid leukemia, lymphocytic lymphomas, cancer of the bladder, cancer of the kidney or ureter, renal cell carcinoma, carcinoma of the renal pelvis, neoplasms of the central nervous system (CNS), primary CNS lymphoma, non hodgkins's lymphoma, spinal axis tumors, brain stem glioma, pituitary adenoma, adrenocortical cancer, gall bladder cancer, multiple myeloma, cholangiocarcinoma, fibrosarcoma, neuroblastoma, retinoblastoma, or a combination of one or more of the foregoing cancers. In another embodiment of said pharmaceutical composition, said abnormal cell growth is a benign proliferative disease, including, but not limited to, psoriasis, benign prostatic hypertrophy or restinosis.

As used herein, the symbol [------] when incorporated into the chemical structure of a substituent means that the atom to which [------] is attached is the point of attachment of that substitutent to some position on another molecule. For example, X in the hypothetical molecule $CH_3CH_2$-x might be defined as X is

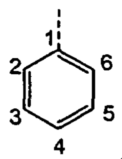

In which case, the placement of [------j attached to the arbitrarily numbered position C-1, means that C-1 of the phenyl ring is attached to the methylene carbon.

The symbols "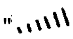" and "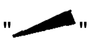" when used together in a single molecule without further indication otherwise, for example, chemical name or accompanying description, merely indicate relative stereochemistry of *trans* or cis where applicable. The symbol "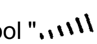" and the symbol" 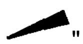", used together or separately, in combination with an indication of them representing the absolute stereochemistry, for example, an indication of "*S*" or "*R*" in the corresponding chemical structure or the accompanying chemical name, indicate the absolute stereochemistry of the corresponding chiral center.

"Aliphatic" refers to straight-chain, branched or cyclic $C_1$-$C_{12}$ hydrocarbons which are completely saturated or which contains one or more units of unsaturation but which are not aromatic. Examples of aliphatic groups include linear, branched or cyclic alkyl, alkenyl, alkynyl groups-and hybrids thereof such as (cycloalkyl)alkyl, (cycloalkenyl)alkyl, etc. An aliphatic group may be optionally substituted by 1-6 substituents. Suitable substituents on an aliphatic group include: 3-12 member heterocyclyl, $C_6$-$C_{10}$ aryl, 5-12 member heteroaryl, halogen, $-NO_2$; $NH_2$, $NR_2$, -CN, -COR, -COOR, $-CONR_2$, -OH, -OR, - OCOR, -SR, -SOR, $-SO_2R$, $-SONR_2$, $-SO_2NR_2$, wherein R is H, $C_1$-$C_{10}$ alkyl, 3-10 member heterocyclyl, $C_6$-$C_{10}$ aryl, 5-12 member heteroaryl.

"$C_1$-$C_{12}$ alkyl" refers to a straight chain or branched saturated hydrocarbon radical having from 1 to 12 carbon atoms. A $C_1$-$C_{12}$ alkyl group may be optionally substituted by at least one substituent. Suitable substituents on a $C_1$-$C_{12}$ alkyl group include, but are not limited to, 3-12 member heterocyclyl, $C_6$-$C_{10}$aryl, 5-12 member heteroaryl, halogen, $-NO_2$, $-NR_2$, -CN, -COR, -COOR, $-CONR_2$, -OH, -OR, -OCOR, -SR, -SOR, $-SO_2R$, - $SONR_2$, $-SO_2NR_2$, wherein each R is independently -H, $C_1$-$C_{10}$ alkyl, 3-12 member heterocyclyl, $C_6$-$C_{10}$ aryl, 5-12 member heteroaryl. Examples of $C_1$-$C_{12}$ alkyl groups include, but are not limited to methyl, ethyl, propyl, isopropyl, butyl, sec-butyl, iso-butyl, tert-butyl, pentyl, neo-pentyl, sec-pentyl, hexyl, heptyl, octyl, and the like, including substitute forms thereof. Further, the term "alkyl," refers to a straight chain or branched saturated hydrocarbon radical of 1 to 20 carbon atoms, or 1 to 12 carbon atoms, or 1 to 8 carbon atoms, or 1 to 6 carbon atoms, or 1 to 4 carbon atoms. "Lower alkyl" refers specifically to an alkyl group having 1 to 4 carbon atoms. Alkyl may be substituted or unsubstituted. Suitable substituents on an alkyl group are the same as those described for a $C_1$-$C_{12}$ alkyl group.

"cycloalkyl" refers to a cyclic saturated hydrocarbon radical having from 3 to 20 carbon atoms. A cycloalkyl

group may be monocyclic and where permissible may be bicyclic or polycyclic. A cycloalkyl group may be optionally substituted by at least one substituent. Suitable substituents on a cycloalkyl group are the same as those described for an alkyl group. Examples of cycloalkyl groups include, but are not limited to cyclopropyl, cyclobutyl, cyclopentyl, cyclohexyl, cycloheptyl, cyclooctyl, nobornyl, adamantyl, and the like, including substituted forms thereof.

"Nonaromatic carbocyclyl" refers to a 3 to 12 member all-carbon monocyclic ring group, all-carbon bicyclic on multicyclic ring system group wherein one or more of the rings may contain one or more double bonds or an aromatic ring as part of the bicyclic or multicyclic ring system, but the monocyclic ring, the bicyclic or multicyclic ring system does not have a completely conjugated pi-electron system. Examples, without limitation, of nonaromatic carbocyclyl are cyclopropyl, cyclobutyl, cydopentyl, cyclopentenyl, cyclohexyl, cyclohexadienyl, adamantanyl, cycloheptyl, cycloheptatrienyl and the like. A nonaromatic carbocyclyl may be substituted or unsubstituted. Typical substituent groups are the same with those of alkyl group, as defined herein. Illustrative examples of nonaromatic carbocyclyl are derived from, but not limited to, the following:

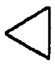 , 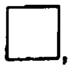 , 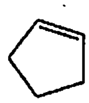 , 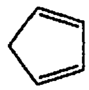 , 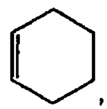 , 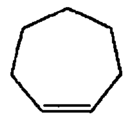 , 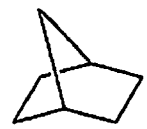

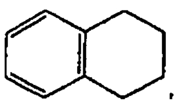 , 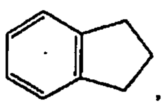 , 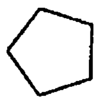 , 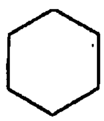 , 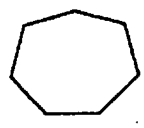 , 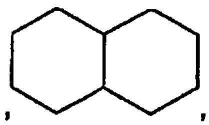 ,

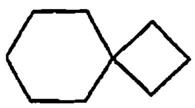 , 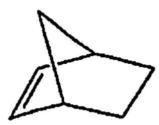 , 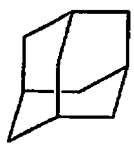 and 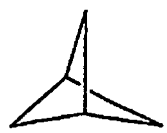 .

"Unsaturated nonaromatic carbocyclyl" or "nonaromatic unsaturated carbocyclyl" both refer to a nonaromatic carbocyclyl, as defined herein, that contains at least one carbon carbon double bond or one carbon carbon triple bond, or an aromatic ring as part of the bicyclic or multicyclic ring system.

"$C_2$-$C_{12}$ alkenyl" refers to a straight chain or branched unsaturated hydrocarbon radical having from 2 to 12 carbon atoms. A $C_2$-$C_{12}$ alkenyl group may have one or more points of unsaturation (i.e.- one or more carbon-carbon double bonds). In the case where $C_2$-$C_{12}$ alkenyl has more than one carbon-carbon double bond, the carbon-carbon double bonds can be conjugated or unconjugated. A $C_2$-$C_{12}$ alkenyl group may be optionally substituted by at least one substituent. Suitable substituents on a $C_2$-$C_{l2}$ alkenyl group are the same as those described for a $C_1$-$C_{12}$ alkyl group. Examples of $C_2$-$C_{12}$ alkenyl include, but are not limited to, ethenyl, 1-propenyl, 2-propenyl, 1-butenyl, 2-butenyl, isobutenyl, and the like, including substituted forms thereof. Further, the term "alkenyl" refers to a straight chain or branched unsaturated hydrocarbon radical having from 2 to 20 carbon atoms, or 2 to 12 carbon atoms, or 2 to 8 carbon atoms, or 2 to 6 carbon atoms, or 2 to 4 carbon atoms. An alkenyl group may have one or more points of unsaturation (i.e.- one or more carbon-carbon double bonds). In the case where an alkenyl group has more than one carbon-carbon double bond, the carbon-carbon double bonds can be conjugated or unconjugated. An alkenyl group may be substituted or unsubstituted. Suitable substituents on an alkenyl group are the same as those described for a $C_1$-$C_{12}$ alkyl group.

"Alkoxy" or "alkoxyl" refers to -$OR^c$ wherein $R^c$ is $C_1$-$C_{12}$ alkyl, $C_2$-$C_{12}$ alkenyl, $C_2$-$C_{12}$ alkynyl, $C_3$-$C_{12}$ cycloalkyl or ($C_1$-$C_6$ alkylene)-($C_3$-$C_{12}$ cycloalkyl). A "$C_1$-$C_{12}$ alkoxy" or "$C_1$-$C_{12}$ alkoxyl" refers to an alkoxy group, as defined herein, wherein $R^c$ has 1 to 12 total carbon atoms. "Alkoxyalkyl" refers to an alkyl, as defined herein, that is substituted by at least one alkoxy group as defined herein. A "$C_2$-$C_6$ alkylalkoxy" refers an alkylalkoxy wherein the total carbon number of the alkyl and its alkoxy substituents are from 2 to 6.

"Alkylamino" refers to -$NR^pR^q$ wherein each $R^p$ and $R^q$ is independently H, $C_1$-$C_{12}$ alkyl, $C_2$-$C_{12}$ alkenyl, $C_2$-$C_{12}$ alkynyl, $C_3$-$C_{12}$ cycloalkyl, ($C_1$-$C_6$ alkylene)-($C_3$-$C_{12}$ cycloalkyl) provided $R^p$ and $R^q$ are not both H. A "monoalkylamino" refers to an alkylamino group, as defined herein, wherein one of $R^p$ and $R^q$ is H. A "dialkylamino" refers to an alkylamino group, as defined herein, wherein none of $R^p$ and $R^q$ is H. A "$C_{1-12}$ alkylamino" refers to an alkylamino group that contains 1 to 10 carbon atoms.

"$C_2$-$C_{12}$ alkynyl" refers to a straight chain or branched hydrocarbon radical having from 2-12 carbon atoms and

at least one carbon-carbon triple bond. In the case where $C_2$-$C_{12}$ alkynyl has more than one carbon-carbon double bond, the carbon-carbon double bonds can be conjugated or unconjugated. A $C_2$-$C_{12}$ alkynyl group may be optionally substituted by at least one substituent. Suitable substituents on a $C_2$-$C_{12}$ alkynyl group are the same as those described for a $C_1$-$C_{12}$ alkyl group. Examples of $C_2$-$C_{12}$ alkynyl include, but are not limited to, ethynyl, 1-propynyl, 2-propynyl, 1-butynyl, 2-butynyl, and the like, including substituted forms thereof. Further, the term "alkynyl" refers to a straight chain or branched hydrocarbon radical of 2 to 20 carbon atoms, or 2 to 12 carbon atoms, or 2 to 8 carbon atoms, or 2 to 6 carbon atoms, or 2 to 4 carbon atoms, and having at least one carbon-carbon triple bond. Alkynyl may be substituted or unsubstituted. Suitable substituents on an alkynyl group are the same as those described for a $C_1$-$C_{12}$ alkyl group.

"Amino" refers to $-NH_2$.

"$C_6$-$C_{10}$ aryl" refers to an all-carbon monocyclic ring or polycyclic ring of 6 to 10 carbon atoms having a completely conjugated pi-electron system. A $C_6$-$C_{10}$ aryl group may be optionally substituted by at least one substituent. Suitable substituents on a $C_6$-$C_{10}$ aryl group are the same as those described or a $C_1$-$C_{12}$ alkyl group. Examples of $C_6$-$C_{10}$ aryl include, but are not limited to, phenyl and naphthyl. Further, the term "aryl" refers to an all-carbon monocyclic ring or polycyclic ring of 6 to 20 carbon atoms having a completely conjugated pi-electron system. The, aryl group may be substituted or unsubstituted. Examples of aryl include, but are not limited to, anthracenyl, phenanthreneyl and perylenyl.

"Aralkyl" refers to alkyl, as defined herein, that is substituted with an $C_{6-10}$ aryl group as defined above; e.g., $-CH_2$Phenyl, $-(CH_2)_2$phenyl, $-(CH_2)_3$phenyl, $CH_3CH(CH_3)CH_2$phenyl, and the like and derivatives thereof. A $C_1$-$C_6$ aralkyl refers to a $C_1$-$C_6$ alkyl that is substituted with a $C_6$-$C_{10}$ aryl group.

"Heteroaralkyl" group means alkyl, as defined herein, that is substituted with a 5-12 member heteroaryl group; e.g., $-CH_2$pyridinyl, $-(CH_2)_2$pyrimidinyl, $-(CH_2)_3$imidazolyl, and the like, and derivatives thereof. A $C_1$-$C_6$ heteroaralkyl refers to a $C_1$-$C_6$ alkyl that is substituted with an 5-12 member heteroaryl group.

"Heteroaryl" refers to a monocyclic or fused ring group of 5 to 12 ring atoms containing one, two, three or four ring heteroatoms selected from N, O, and S, the remaining ring atoms being C, and, in addition, having a completely conjugated pi-electron system. Examples, without limitation, of unsubstituted heteroaryl groups are pyrrole, furan, thiophene, imidazole, oxazole, thiazole, pyrazole, pyridine, pyrimidine, quinoline, isoquinoline, purine, tetrazole, triazine, and carbazole. The heteroaryl group may be substituted or unsubstituted. Typical substituents include $C_{1-12}$ aliphatic, 3-10 member heterocyclyl, 6-10 member aryl, halogen, $-NO_2$, NH2, $NR_2$, -CN, -COR, -COOR, $-CONR_2$, -OH, -OR, -OCOR, -SR, -SOR, $-SO_2R$, $-SONR_2$, $-SO_2NR_2$, wherein R is a $C_{1-10}$ aliphatic, 3-10 member heterocyclyl, $C_{6-10}$ aryl, 5-10 member heteroaryl.

A "pharmaceutically acceptable heteroaryl" is one that is sufficiently stable to be attached to a compound of the invention, formulated into a pharmaceutical composition and subsequently administered to a patient in need thereof.

Examples of typical monocyclic heteroaryl groups include, but are not-limited to:

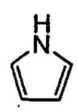
pyrrole (pyrrolyl)

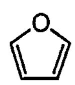
furan (furanyl)

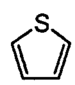
thiophene (thiophenyl)

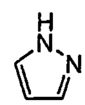
pyrazole (pyrazolyl)

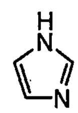
imidazole (imidazolyl)

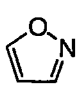
isoxazole (isoxazolyl)

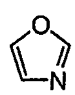
oxazole (oxazolyl)

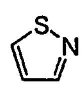
isothiazole (isothiazolyl)

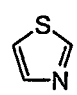
thiazolyl (thiazolyl)

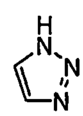
1,2,3-triazole (1,2,3-triazolyl)

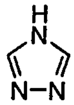
1,3,4-triazole (1,3,4-triazolyl)

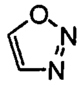
1-oxa-2,3-diazole (1-oxa-2,3-diazolyl)

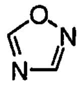
1-oxa-2,4-diazole (1-oxa-2,4-diazolyl)

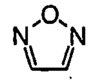
1-oxa-2,5-diazole (1-oxa-2,5-diazolyl)

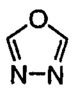
1-oxa-3,4-diazole (1-oxa-3,4-diazolyl)

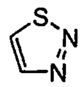
1-thia-2,3-diazole (1-thia-2,3-diazolyl)

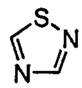
1-thia-2,4-diazole (1-thia-2,4-diazolyl)

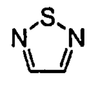
1-thia-2,5-diazole (1-thia-2,5-diazolyl)

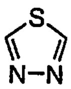
1-thia-3,4-diazole (1-thia-3,4-diazolyl)

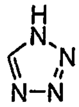
tetrazole (tetrazolyl)

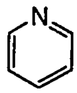
pyridine (pyridinyl)

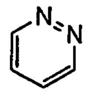
pyridazine (pyridazinyl)

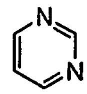
pyrimidine (pyrimidinyl)

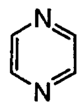
pyrazine (pyrazinyl)

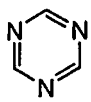
1,3,5-triazine triazinyl

Examples of bicyclic heteroaryl groups include, but are not limited to:

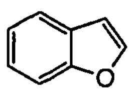
benzofuran (benzofuranyl)

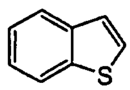
benzothiophene (benzothiophenyl)

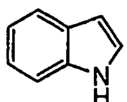
indole (indolyl)

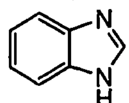
benzimidazole (benzimidazolyl)

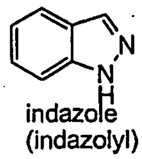
indazole (indazolyl)

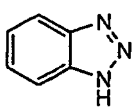
benzotriazole (benzotriazolyl)

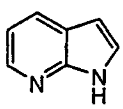
pyrrolo[2,3-b]pyridine (pyrrolo[2,3-b]pyridinyl)

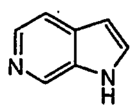
pyrrolo[2,3-c]pyridine (pyrrolo[2,3-c]pyridinyl)

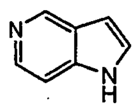
pyrrolo[3,2-c]pyridine (pyrrolo[3,2-c]pyridinyl)

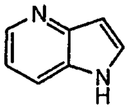
pyrrolo[3,2-b]pyridine (pyrrolo[3,2-b]pyridinyl)

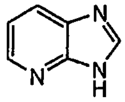
imidazo[4,5-b]pyridine (imidazo[4,5-b]pyridinyl)

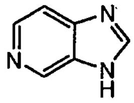
imidazo[4,5-c]pyridine (imidazo[4,5-c]pyridinyl)

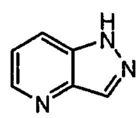
pyrazolo[4,3-d]pyridine (pyrazolo[4,3-d]pyidinyl)

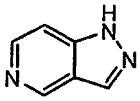
pyrazolo[4,3-c]pyridine (pyrazolo[4,3-c]pyidinyl)

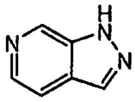
pyrazolo[3,4-c]pyridine (pyrazolo[3,4-c]pyidinyl)

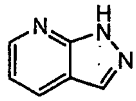
pyrazolo[3,4-b]pyridine (pyrazolo[3,4-b]pyidinyl)

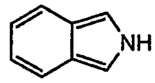
isoindole (isoindolyl)

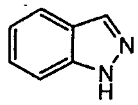
indazole
(indazolyl)

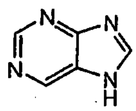
purine
(purinyl)

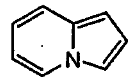
indolizine
(indolininyl)

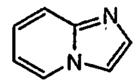
imidazo[1,2-a]pyridine
(imidazo[1,2-a]pyridinyl)

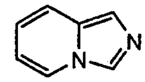
imidazo[1,5-a]pyridine
(imidazo[1,5-a]pyridinyl)

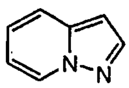
pyrazolo[1,5-a]pyridine
(pyrazolo[1,5-a]pyridinyl)

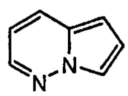
pyrrolo[1,2-b]pyridazine
(pyrrolo[1-2,b]pyridazinyl)

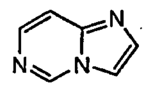
imidazo[1,2-c]pyrimidine
(imidazo[1,2-c]pyrimidinyl)

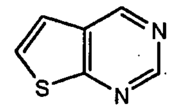
thienopyrimidine
(thienopyrimidinyl)

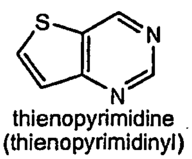
thienopyrimidine
(thienopyrimidinyl)

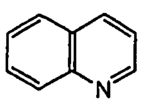
quinoline
(quinolinyl)

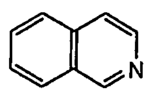
isoquinoline
(isoquinolinyl)

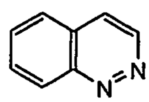
cinnoline
(cinnolinyl)

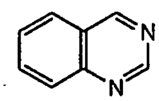
quinazoline
(azaquinazoline)

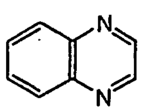
quinoxaline
(quinoxalinyl)

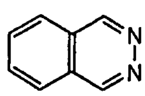
phthalazine
(phthalazinyl)

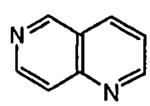
1,6-naphthyridine
(1,6-naphthyridinyl)

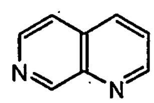
1,7-naphthyridine
(1,7-naphthyridinyl)

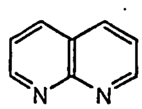
1,8-naphthyridine
(1,8-naphthyridinyl)

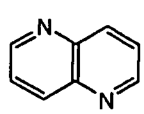
1,5-naphthyridine
(1,5-naphthyridinyl)

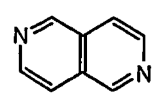
2,6-naphthyridine
(2,6-naphthyridinyl)

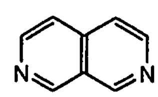
2,7-naphthyridine
(2,7-naphthyridinyl)

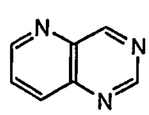
pyrido[3,2-d]pyrimidine
(pyrido[3,2-d]pyrimidinyl)

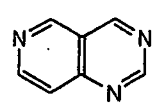
pyrido[4,3-d]pyrimidine
(pyrido[4,3-d]pyrimidinyl)

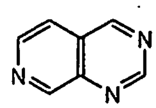
pyrido[3,4-d]pyrimidine
(pyrido[3,4-d]pyrimidinyl)

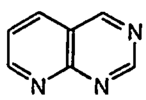

pyrido[2,3-d]pyrimidine (pyrido[2,3-d]pyrimidinyl)

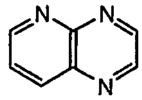

pyrido[2,3-b]pyrazine (pyrido[2,3-b]pyrazinyl)

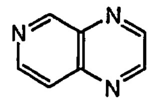

pyrido[3,4-b]pyrazine (pyrido[3,4-b]pyrazinyl)

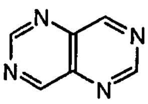

pyrimido[5,4-d]pyrimidine (pyrimido[5,4-d]pyrimidinyl)

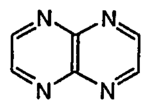

pyrazino[2,3-b]pyrazine (pyrazino[2,3-b]pyrazinyl)

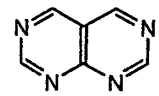

pyrimido[4,5-d]pyrimidine (pyrimido[4,5-d]pyrimidinyl)

"Heteroalicyclic" or "heterocyclyl" refers to a monocyclic or polycyclic group having from 3 to 12 ring atoms, wherein from 1 to 4 ring atoms are heteroatoms selected from N, O, and S. "Heteroalicyclic" or "heterocyclyl" may also have one or more double bonds. However, "Heteroalicyclic" or "heterocyclyl" do not have a completely conjugated pi-electron system. "Heteroalicyclic" or "heterocyclyl" can be substituted or unsubstituted. Typical substituents include, but are not limited to, $C_1$-$C_{12}$ aliphatic, 6-10 member aryl, 6-10 member aryl, halogen, $-NO_2$, $NH_2$, $NR_2$, -CN, -COR, - COOR, $-CONR_2$, -OH, -OR, -OCOR, -SR, -SOR, $-SO_2R$, wherein R is a $C_1$-$C_{10}$ alkyl, 3-10 member heterocyclyl, $C_6$-$C_{10}$ aryl, 5-10 member heteroaryl.

Examples of saturated heterocyclyl groups include, but are not limited to:

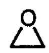

oxirane (oxiranyl)

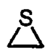

thiarane (thiaranyl)

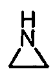

aziridine (aziridinyl)

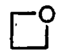

oxetane (oxetanyl)

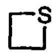

thiatane (thiatanyl)

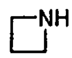

azetidine (azetidinyl)

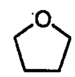

tetrahydrofuran (tetrahydrofuranyl)

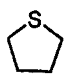

tetrahydrothiophene (tetrahydrothiophenyl)

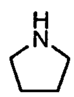

pyrrolidine (pyrrolidinyl)

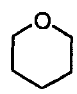

tetrahydropyran (tetrahydropyranyl)

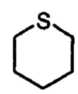

tetrahydrothiopyran (tetrahydrothiopyranyl)

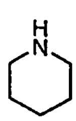

piperidine (piperidinyl)

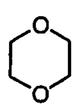

1,4-dioxane (1,4-dioxanyl)

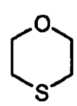

1,4-oxathiane (1,4-oxathianyl)

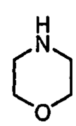

morpholine (morpholinyl)

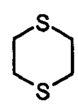

1,4-dithiane (1,4-dithianyl)

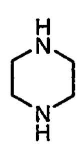

piperazine (piperazinyl)

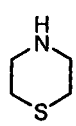

1,4-azathiane (1,4-azathianyl)

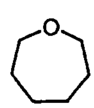

oxepane (oxepanyl)

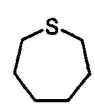

thiepane (thiepanyl)

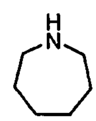

azepane (azepanyl)

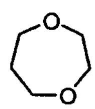

1,4-dioxepane (1,4-dioxepanyl)

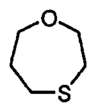

1,4-oxathiepane (1,4-oxathiepanyl)

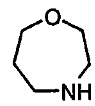

1,4-oxaazepane (1,4-oxaazepanyl)

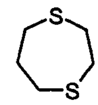

1,4-dithiepane (1,4-dithiepanyl)

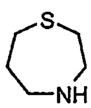

1,4-thieazepane (1,4-thieazepanyl)

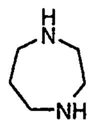

1,4-diazepane (1,4-diazepanyl)

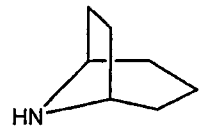

tropane (tropanyl)

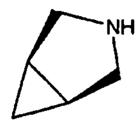

(1*S*,5*R*)-3-aza-bicyclo[3.1.0]hexane (1*S*,5*R*)-3-aza-bicyclo[3.1.0]hexyl

Examples of partially unsaturated heterocyclyl groups include, but are not limited to:

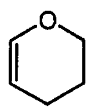

3,4-dihydro-2H-pyran (3,4-dihydro-2H-pyranyl)

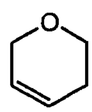

5,6-dihydro-2H-pyran (5,6-dihydro-2H-pyranyl)

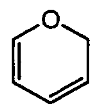

2H-pyran (2H-pyranyl)

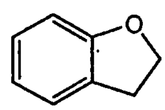

2,3-dihydrobenzofuran 2,3-dihydrobenzofuranyl

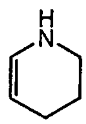

1,2,3,4-tetrahydropyridine (1,2,3,4-tetrahydropyridinyl)

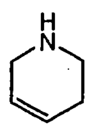

1,2,5,6-tetrahydropyridine (1,2,5,6-tetrahydropyridinyl)

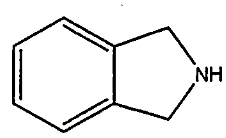

isoindoline isoindolinyl

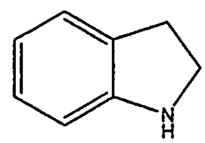

indoline indolinyl

A "diradical" refers to a group that has two open valences and is further connected to two other groups, or forms a double bond with the same atom of one group, or forms two single bonds with the same atom of one group. Examples of diradicals are, but are not limited to $-CH_2-$, -O-, $-O-CH_2-$, -($C_1$-$C_3$ alkylene)-NH- and - $CH_2$-$CH_2$-. When a diradical is referred to as, for example, $-O-CH_2-$ or -($C_1$-$C_3$ alkylene)-NH-, it is understood that each end of the diradical can equally connect to another moiety. For example, if K is defined as A-L-B, and L is a diradical selected from $-0-CH_2-$ and -($C_1$-$C_3$ alkylene)-, it is understood that K is therefore selected from A-O-$CH_2$-B, A-$CH_2$-O-B, .and A-($C_1$-$C_3$ alkylene)-B. A and B herein are referred to as different organic moieties.

When "ene" is added after the "yl" at the end of any of the previously defined terms to form a new term, the new term refers to a diradical formed by removing one hydrogen atom from the original term of which the new term derived from. For example; an alkylene refers to a diradical group formed by removing one hydrogen atom from an alkyl group and that a "methylene" refers to a divalent radical $-CH_2-$ derived from removing one hydrogen atom from methyl. More examples of such diradicals include, but are not limited to: alkenylene, alkynylene, cycloalkylene, phenylene, heterocyclylene, heteroarylene and (nonaromatic unsaturated carbocyclylene), which are derived from alkenyl, alkynyl, cycloalkyl, phenyl, heterocyclyl, heteroaryl and (nonaromatic unsaturated carbocyclyl), respectively. For example, "cyclopropylene" refers to both

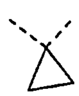, and 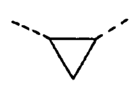.

For example, "$C_1$-$C_2$ alkylene" refers to all of the following: $-CH_2-$, $-CH(CH_3)-$ and $-CH_2-CH_2-$.

"oxo" or "-oxo-" refers to an oxygen double bond "=O" substitution.

"Hydroxy" or "hydroxyl" both refer to -OH.

"Perfluoroalkyl" refers to an alkyl group in which all of its hydrogen atoms are replaced by fluorine atoms.

"Optional" or "optionally" means that the subsequently described event or circumstance may but need not occur, and that the description includes instances where the event or circumstance occurs and instances in which it does not. For example, "heterocyclyl group optionally substituted with an alkyl group" means that the alkyl may but need not be present, and the description includes situations where the heterocyclyl group is substituted with an alkyl group and situations where the heterocyclyl group is not substituted with the alkyl group.

When a group is "optionally substituted" or "optionally further substituted" by some substituents, it means a carbon or a nitrogen atom of this group wherein one or more hydrogen atoms are attached to the carbon or nitrogen atom, such carbon or nitrogen atom is optionally substituted by some other substituents. For example, "R is H, $C_1$-$C_3$ alkyl or phenyl, and R is optionally further substituted by 1-3 groups selected from -F, oxo and $C_1$-$C_3$ perfluoroalkyl", means that R is 1) H (when R is H, R cannot be further substituted); 2) $C_1$-$C_3$ alkyl optionally further substituted by 1-3 groups selected from -F, oxo and $C_1$-$C_3$ perfluoroalkyl; and 3) phenyl optionally further substituted by 1-3 groups selected from -F and $C_1$-$C_3$ perfluoroalkyl. Optional substitution of oxo does not apply when R is phenyl because no single atom of the phenyl group possess two hydrogen atoms to be substituted by oxo, i.e. =O bond When a group is further substituted by a "-($C_1$-$C_4$ alkylene)-"; it means the "-($C_1$-$C_4$ alkylene)-", together with the nitrogen atom or the carbon atom of the group to which "$C_1$-$C_4$ alkylene" is attached to, form a carbo or hetero spiro code.

A "pharmaceutical composition" refers to a mixture of one or more of the compounds described herein, or physiologically/pharmaceutically acceptable salts, solvates or hydrates thereof, with other chemical components, such as physiologically/pharmaceutically acceptable carriers and excipients. The purpose of a pharmaceutical composition is to facilitate administration of a compound to an organism.

As used herein, a "physfoiogically/pharmaceutically acceptable carrier" refers to a carrier or diluent that does not cause significant irritation to an organism and does not abrogate the biological activity and properties of the administered compound.

A "pharmaceutically acceptable excipient" refers to an inert substance added to a pharmaceutical composition to further facilitate administration of' a compound. Examples, without limitation, of excipients include calcium carbonate, calcium phosphate, various sugars and types of starch, cellulose derivatives, gelatin, vegetable oils and polyethylene glycols.

As used herein, the term "pharmaceutically acceptable salt" refers to those salts that retain the biological effectiveness and properties of the parent compound. Such salts include:

(1) acid addition salts, which can be obtained by reaction of the free base of the parent compound with inorganic acids such as hydrochloric acid, hydrobromic acid, nitric acid, phosphoric acid, sulfuric acid, and perchloric acid and the like, or with organic acids such as acetic acid, oxalic acid, (D) or (L) malic acid, maleic acid, methanesulfonic acid, ethanesulfonic acid, p-toluenesulfonic acid, salicylic acid, tartaric acid, citric acid, succinic acid or malonic acid and the like; or

(2) salts formed when an acidic proton present in the parent compound either is replaced by a metal ion, e.g., an alkali metal ion, an alkaline earth ion, or an aluminum ion; or coordinates with an organic base such as ethanolamine, diethanolamine, triethanolamine, tromethamine, N-methylglucamine, and the like.

"PK" refers to receptor protein tyrosine kinase (RTKs), non-receptor or "cellular" tyrosine kinase (CTKs) and serine-threonine kinases (STKs). "Modulation" or "modulating" refers to the alteration of the catalytic activity of RTKs, CTKs and STKs. In particular, modulating refers to the activation of the catalytic activity of RTKs, CTKs and STKs, preferably the activation or inhibition of the catalytic activity of RTKs, CTKs and STKs, depending on the concentration of the compound or salt to which the RTK, CTK or STK is exposed or, more preferably, the inhibition of the catalytic activity of RTKs, CTKs and STKs.

"Catalytic activity" refers to the rate of phosphorylation of tyrosine under the influence, direct or indirect, of RTKs and/or CTKs or the phosphorylation of serine and threonine under the influence, direct or indirect, of STKs.

"Contacting" refers to bringing a compound of the present teachings and a target PK together in such a manner that the compound can affect the catalytic activity of the PK, either directly, i.e., by interacting with the kinase' itself, or indirectly, i.e., by interacting with another molecule on which the catalytic activity of the kinase is dependent. Such "contacting" can be accomplished "*in vitro,*" i.e., in a test tube, a petri dish or the like. In a test tube, contacting may involve only a compound and a PK of interest or it may involve whole cells. Cells may also be maintained or grown in cell culture dishes and contacted with a compound in that environment. In this context, the ability of a particular compound to affect a PK related disorder, i.e., the $IC_{50}$ of the compound, can be determined before use of compounds *in vivo* with more complex living organisms is attempted. For cells outside the organism, multiple methods exist, and are well-known to those skilled in the art, to get the PKs in contact with the compounds including, but not limited to, direct cell microinjection

and numerous transmembrane carrier techniques.

"*In vitro*" refers to procedures performed in an artificial environment such as, e.g., without limitation, in a test tube or culture medium.

"*In vivo*" refers to procedures performed within a living organism such as, without limitation, a mouse, rat or rabbit.

"PK related disorder," "PK driven disorder," and "abnormal PK activity" all refer to a condition characterized by inappropriate, i.e., under or, more commonly, over, PK catalytic activity, where the particular PK can be an RTK, a CTK or an STK. Inappropriate catalytic activity can arise as the result of either: (1) PK expression in cells which normally do not express PKs, (2) increased PK expression leading to unwanted cell proliferation, differentiation and/or growth, or, (3) decreased PK expression leading to unwanted reductions in cell proliferation, differentiation and/or growth. Over-activity of a PK refers to either amplification of the gene encoding a particular PK or production of a level of PK activity which can correlate with a cell proliferation, differentiation and/or growth disorder (that is, as the level of the PK increases, the severity of one or more of the symptoms of the cellular disorder increases). Under-activity is, of course, the converse, wherein the severity of one or more symptoms of a cellular disorder increase as the level of the PK activity decreases.

"Treat", "treating" and "treatment" refer to a method of alleviating or abrogating a PK mediated cellular disorder and/or its attendant symptoms. With regard particularly to cancer, these terms simply mean that the life expectancy of an individual affected with a cancer will be increased or that one or more of the symptoms of the disease will be reduced.

"Organism" refers to any living entity comprised of at least one cell. A living organism can be as simple as, for example, a single eukariotic cell or as complex as a mammal, including a human being.

"Therapeutically effective amount" refers to that amount of the compound being administered which will relieve to some extent one or more of the symptoms of the disorder being treated. In reference to the treatment of cancer, a therapeutically effective amount refers to that amount which has at least one of the following effects:

(1) reducing the size of the tumor;
(2) inhibiting (that is, slowing to some extent, preferably stopping) tumor metastasis;
(3) inhibiting to some extent (that is, slowing to some extent, preferably stopping) tumor growth, and
(4) relieving to some extent (or, preferably, eliminating) one or more symptoms associated with the cancer.

"Monitoring" means observing or detecting the effect of contacting a compound with a cell expressing a particular PK. The observed or detected effect can be a change in cell phenotype, in the catalytic activity of a PK or a change in the interaction of a PK with a natural binding partner. Techniques for observing or detecting such effects are well-known in the art. The effect is selected from a change or an absence of change in a cell phenotype, a change or absence of change in the catalytic activity of said protein kinase or a change or absence of change in the interaction of said protein kinase with a natural binding partner in a final aspect of this invention.

"Cell phenotype" refers to the outward appearance of a cell or tissue or the biological function of the cell or tissue. Examples, without limitation, of a cell phenotype are cell size, cell growth, cell proliferation, cell differentiation, cell survival, apoptosis, and nutrient uptake and use. Such phenotypic characteristics are measurable by techniques well-known in the art.

"Natural binding partner" refers to a polypeptide that binds to a particular PK in a cell. Natural binding partners can play a role in propagating a signal in a PK-mediated signal transduction process. A change in the interaction of the natural binding partner with the PK can manifest itself as an increased or decreased concentration of the PK/natural binding partner complex and, as a result, in an observable change in the ability of the PK to mediate signal transduction.

The term "stereoisomers" refers to compounds that have identical chemical constitution, but differ with regard to the arrangement of their atoms or groups in space. In particular, the term "enantiomers" refers to two stereoisomers of a compound that are non-superimposable mirror images of one another. The terms "racemic" or "racemic mixture," as used herein, refer to a 1:1 mixture of enantiomers of a particular compound. The term "diastereomers", on the other hand, refers to the relationship between a pair of stereoisomers that comprise two or more asymmetric centers and are not mirror images of one another.

Detailed Description

The compounds of the current invention, i.e., the compounds of formula I, as well as compounds of formula II, III and IV, can be made following reaction Scheme 1, 2 and 3.

## Scheme 1

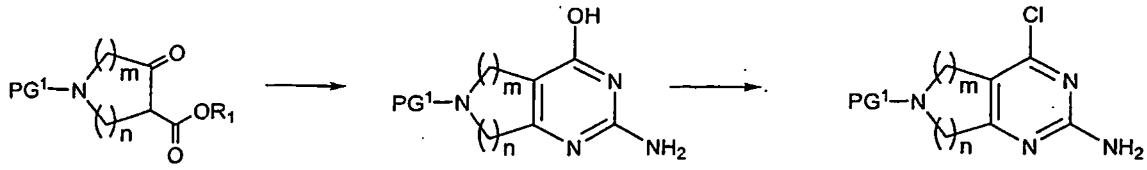

I(A) I(B) I(C)

## Scheme 2

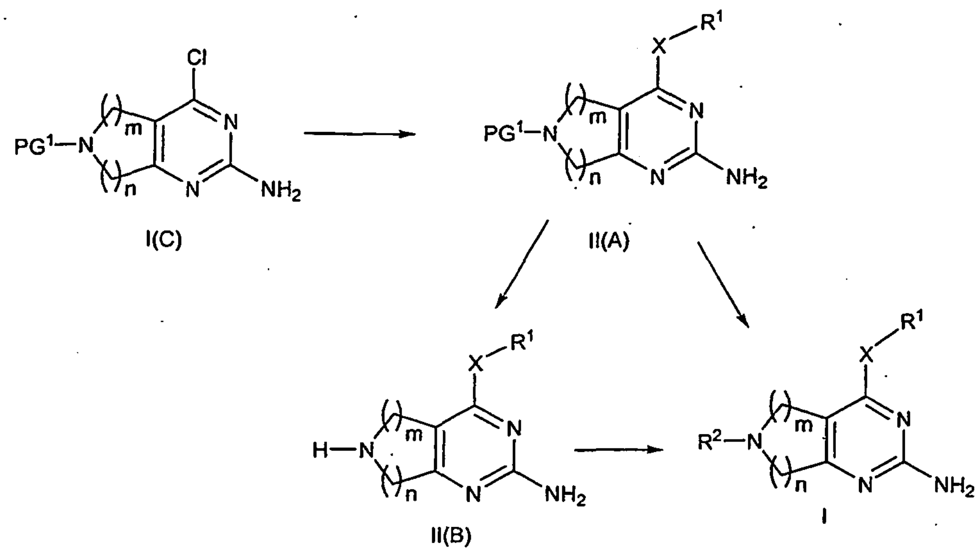

I(C) II(A) II(B) I

## Scheme 3

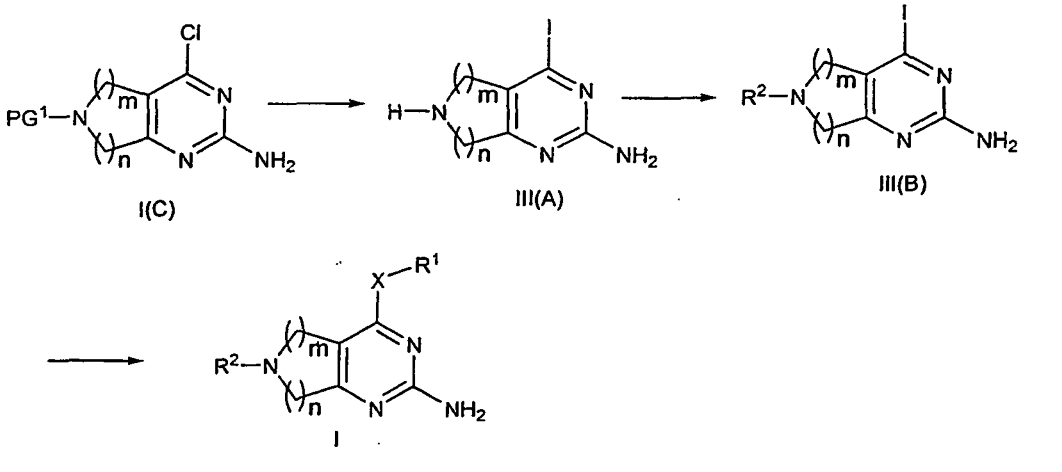

I(C) III(A) III(B) I

Scheme 1 illustrates the synthesis of intermediate **I(C)** used to make compounds of formula **I**. The beta keto ester **I(A)** can be prepared based on a known procedures (see, e.g. Viscontini and Buhler Helvetica Chimica Acta, 50 (5): 1289-93; (1967), Rosowsky et. al. J. Heterocyclic Chem., 26: 509-16 (1989)). $PG^1$, the nitrogen protecting group, can be selected for compatability with subsequent chemistry. Protecting groups and general considerations for their use are described in T. Greene and P. Wuts, "Protective Groups in Organic Synthesis", 3rd Edition 1999, John Wiley & Sons and are well known to those skilled in the art. Compound **I(A)** is condensed with guanidine to give compound **I(B).** This

can typically be done by heating compound **I(A)** with guanidine or guanidine equivalent a protic solvent. A typical reaction condition would be to reflux compound **I(A)** with guandidine carbonate in *tert*-butanol as a solvent. Conversion of the hydroxyl group of compound **I(B)** to chloro gives **I(C).** This can typically be done by heating compound **I(B)** with $POCl_3$ in an aprotic solvent. A typical reaction condition would be to reflux compound **I(B)** together with excess $POCl_3$ either neat or in dry acetonitrile as solvent.

Scheme 2 illustrates the route through which compounds of formula I can be made from intermediate **I(C).** In Scheme 2, the chloro leaving group of compound **I(C)** is replaced by a $R^1$-X- group to give compound **II(A).** This reaction can typically be carried out by displacement of the chloro leaving group using a variety of nucleophiles such as carbon, oxygen, nitrogen, sulfur, or other nucleophiles. A typical nucleophilic displacement reaction for the transformation of compound **I(C)** to compound **II(A)** would be to treat compound **I(C)** with a nucleophile in the presence of base such as cesium carbonate using DMF or DMSO as solvent to give compound **II(A).** Alternatively, the displacement of the chloro leaving group of compound **I(C)** by $R^1$-X- to give compound **II(A)** can also be carried out using cross coupling methodology utilizing Suzuki, Stille, Negishi or similar conditions. A typical cross coupling reaction for the transformation of compound **I(C)** to compound **II(A)** would be to treat compound **I(C)** with a boronic acid or ester in the presence of a base such as sodium carbonate and Pd(0) catalyst in a solvent mixture such as water and 1,4-dioxane to give compound **II(A)**. The nitrogen protecting group, $PG^1$, of compound **II(A)** is then removed to give compound **II(B).** This can typically be done, when $PG^1$ forms an ethyl carbamate protecting group, by refluxing compound **II(A)** with trimethylsilyliodide in a solvent such as $CH_3CN$. Alternatively HBr in acetic acid or KOH in isopropanol can also be used. A typical condition for the transformation of compound **II(A)** to compound **II(B)** would be to treat compound II(A) with TMSI (5 equivalents) in refluxing $CH_3CN$ to give compound **II(B).** The dihydropyrrolo amino moiety of compound **II(B)** then acts as a nucleophile in reactions with an electrophilic $R^2$ moiety to give compound **I.** This nucleophilic reaction can be alkylation, acylation, sulfonylation, and other reactions applicable to secondary alkyl amines. An alkylation reaction of compound **II(B)** can be carried out by reacting compound **II(B)** with an alkylating $R^2$ moiety. A typical alkylation reaction condition is to react compound **II(B)** with an $R^2$ alkyl bromide moiety in the presence of TEA at room temperature to give compound **I** as an N-alkyl. An acylation reaction of compound **II(B)** can be carried out by reacting compound **II(B)** with an acylating $R^2$ moiety. A typical acylation reaction condition is to react compound **II(B)** with an $R^2$ activated ester moiety or $R^2$ acyl halide in the presence of TEA to give compound **I** as an amide. Another typical acylation reaction condition is to react compound **II(B)** with an $R^2$ isocyanate or isocyanate equivalent moiety in the presence of TEA to give compound I as a urea. A sulfonylation reaction of compound **II(B)** can be carried out by reacting compound **II(B)** with an sulfonylating $R^2$ moiety. A typical sulfonylation reaction condition is to react compound **II(B)** with an $R^2$ sulfonyl chloride moiety in the presence of TEA to give compound **I** as a sulfonamide.

Another method for the preparation of compound **I** as a urea takes compound **II(A),** when $PG^1$ forms an ethyl carbamate protecting group, and directly converts compound **II(A)** into compound **I**. This reaction can typically be carried out using a nucleophilic amine in the presence of trimethylaluminum in a solvent such as toluene. A typical condition is to treat a nucleophilic amine in toluene at 0˚C with a solution of trimethylaluminum in hexanes. After warming to room temperature, compound **II(A)** is added and the mixture is heated under microwave radiation to prepare compound I.

Scheme 3 illustrates another route through which compounds of formula **I** can be made from intermediate **I (C).** In Scheme 3 when $PG^1$ forms an ethyl carbamate protecting group, the protecting group, $PG^1$, of compound **I(C)** is removed with concurrent conversion of chloro to iodo in one step. This can typically be carried out by heating compound **I(C)** with TMSI in an aprotic solvent. A typical reaction condition is to reflux compound **I(C)** in $CH_3CN$ with five equivalents of TMSI. Following a methanol quench, compound **III(A)** is obtained as an HI salt. The dihydropyrrolo amino moiety of compound **III(A)** then acts as a nucleophile in reactions with an electrophilic $R^2$ moiety to give compound **III(B).** This nucleophilic reaction can be alkylation, acylation, sulfonylation, and other reactions applicable to secondary alkyl amines. An alkylation reaction of compound **III(A)** can be carried out by reacting compound **III(A)** with an alkylating $R^2$ moiety. A typical alkylation reaction condition is to react compound **III(A)** with an $R^2$ alkyl bromide moiety in the presence of TEA at room temperature to give compound **III(B)** as an N-alkyl. An acylation reaction of compound **III(A)** can be carried out by reacting compound **III(A)** with an acylating $R^2$ moiety. A typical acylation reaction condition is to react compound **III(A)** with an $R^2$ activated ester moiety or $R^2$ acyl halide in the presence of TEA to give compound **III(B)** as an amide. Another typical acylation reaction condition is to react compound **III(A)** with an $R^2$ isocyanate or isocyanate equivalent moiety in the presence of TEA to give compound **III(B)** as a urea. A sulfonylation reaction of compound **III(A)** can be carried out by reacting compound **III(A)** with an sulfonylating $R^2$ moiety. A typical sulfonylation reaction condition is to react **III(A)** with an $R^2$ sulfonyl chloride moiety in the presence of TEA at 0˚C to give compound **III(B)** as a sulfonamide. The iodo group of compound **III(B)** is then displaced by $R^1$-X-using cross coupling methodology to give compound **I**. This reaction can typically be carried out using Suzuki, Stille, Negishi or similar conditions. A typical cross coupling reaction for the transformation of compound **III(B)** to compound **I** would be to treat compound **III(B)** with a boronic acid or ester in the presence of a base such as sodium carbonate and Pd(0) catalyst in a solvent mixture such as water and 1,4-dioxane to give compound **I**.

The compounds of the present invention may have asymmetric carbon atoms. The carbon-carbon bonds of

the compounds of the present invention may be depicted herein using a solid line (-------), a solid wedge ( ), or a dotted wedge ( ). The use of a solid line to depict bonds to asymmetric carbon atoms is meant to indicate that all possible stereoisomers (e.g. specific enantiomers, racemic mixtures, etc.) at that carbon atom are included. The use of either a solid or dotted wedge to depict bonds to asymmetric carbon atoms is meant to indicate that only the stereoisomer shown is meant to be included. It is possible that compounds of the invention may contain more than one asymmetric carbon atom. In those compounds, the use of a solid line to depict bonds to asymmetric carbon atoms is meant to indicate that all possible stereoisomers are meant to be included. For example, unless stated otherwise, it is intended that the compounds of the present invention can exist as enantiomers and diastereomers or as racemates and mixtures thereof. The use of a solid line to depict bonds to one or more asymmetric carbon atoms in a compound of the invention and the use of a solid or dotted wedge to depict bonds to other asymmetric carbon atoms in the same compound is meant to indicate that a mixture of diastereomers is present.

Conventional techniques for the preparation/isolation of individual enantiomers include chiral synthesis from a suitable optically pure precursor or resolution of the racemate using, for example, chiral high pressure liquid chromatography (HPLC). Alternatively, the racemate (or a racemic precursor) may be reacted with a suitable optically active compound, for example, an alcohol, or, in the case where the compound contains an acidic or basic moiety, an acid or base such as tartaric acid or 1-phenylethylamine. The resulting diastereomeric mixture may be separated by chromatography and/or fractional crystallization and one or both of the diastereoisomers converted to the corresponding pure enantiomer(s) by means well known to one skilled in the art. Chiral compounds of the invention (and chiral precursors thereof) may be obtained in enantiomerically-enriched form using chromatography, typically HPLC, on an asymmetric resin with a mobile phase consisting of a hydrocarbon, typically heptane or hexane, containing from 0 to 50% isopropanol, typically from 2 to 20%, and from 0 to 5% of an alkylamine, typically 0.1 % diethylamine. Concentration of the eluate affords the enriched mixture. Stereoisomeric conglomerates may be separated by conventional techniques known to those skilled in the art. See, e.g. "Stereochemistry of Organic Compounds" by E. L. Eliel (Wiley, New York, 1994), the disclosure of which is incorporated herein by reference in its entirety.

Where a compound of the invention contains an alkenyl or alkenylene group, geometric *cis*/*trans* (or Z/E) isomers are possible. Cis/trans isomers may be separated by conventional techniques well known to those skilled in the art, for example, chromatography and fractional crystallization. Where structural isomers are interconvertible *via* a low energy barrier, tautomeric isomerism ('tautomerism') can occur. This can take the form of proton tautomerism in compounds of the present invention containing, for example, an imino, keto, or oxime group, or so-called valence tautomerism in compounds which contain an aromatic moiety. It follows that a single compound may exhibit more than one type of isomerism. Included within the scope of the invention are all stereoisomers, geometric isomers and tautomeric forms of the inventive compounds, including compounds exhibiting more than one type of isomerism, and mixtures of one or more thereof.

Salts of the present invention can be prepared according to methods known to those of skill in the art. Examples of salts include, but are not limited to, acetate, acrylate, benzenesulfonate, benzoate (such as chlorobenzoate, methylbenzoate, dinitrobenzoate, hydroxybenzoate, and methoxybenzoate), bicarbonate, bisulfate, bisulfite, bitartrate, borate, bromide, butyne-1,4-dioate, calcium edetate, camsylate, carbonate, chloride, caproate, caprylate, clavulanate, citrate, decanoate, dihydrochloride, dihydrogenphosphate, edetate, edislyate, estolate, esylate, ethylsuccinate, formate, fumarate, gluceptate, gluconate, glutamate, glycollate, glycollylarsanilate, heptanoate, hexyne-1,6-dioate, hexylresorcinate, hydrabamine, hydrobromide, hydrochloride, $\gamma$-hydroxybutyrate, iodide, isobutyrate, isothionate, lactate, lactobionate, laurate, malate, maleate, malonate, mandelate, mesylate, metaphosphate, methane-sulfonate, methylsulfate, monohydrogenphosphate, mucate, napsylate, naphthalene-1-sulfonate, naphthalene-2-sulfonate, nitrate, oleate, oxalate, pamoate (embonate), palmitate, pantothenate, phenylacetates, phenylbutyrate, phenylpropionate, phthalate, phospate/diphosphate, polygalacturonate, propanesulfonate, propionate, propiolate, pyrophosphate, pyrosulfate, salicylate, stearate, subacetate, suberate, succinate, sulfate, sulfonate, sulfite, tannate, tartrate, teoclate, tosylate, triethiodode, and valerate salts.

The compounds of the present invention that are basic in nature are capable of forming a wide variety of salts with various inorganic and organic acids. Although such salts must be pharmaceutically acceptable for administration to animals, it is often desirable in practice to initially isolate the compound of the present invention from the reaction mixture as a pharmaceutically unacceptable salt and then simply convert the latter back to the free base compound by treatment with an alkaline reagent and subsequently convert the latter free base to a pharmaceutically acceptable acid addition salt. The acid addition salts of the base compounds of this invention can be prepared by treating the base compound with a substantially equivalent amount of the selected mineral or organic acid in an aqueous solvent medium or in a suitable organic solvent, such as methanol or ethanol. Upon evaporation of the solvent, the desired solid salt is obtained. The desired acid salt can also be precipitated from a solution of the free base in an organic solvent by adding an appropriate mineral or organic acid to the solution.

Those compounds of the present invention that are acidic in nature are capable of forming base salts with

various pharmacologically acceptable cations. Examples of such salts include the alkali metal or alkaline-earth metal salts and particularly, the sodium and potassium salts. These salts are all prepared by conventional techniques. The chemical bases which are used as reagents to prepare the pharmaceutically acceptable base salts of this invention are those which form non-toxic base salts with the acidic compounds of the present invention. Such non-toxic base salts include those derived from such pharmacologically acceptable cations as sodium, potassium calcium and magnesium, etc. These salts may be prepared by any suitable method, for example, treatment of the free acid with an inorganic or organic base, such as an amine (primary, secondary or tertiary), an alkali metal hydroxide or alkaline earth metal hydroxide, or the like. Illustrative examples of suitable salts include organic salts derived from amino acids, such as glycine and arginine, ammonia, primary, secondary, and tertiary amines, and cyclic amines, such as piperidine, morpholine and piperazine, and inorganic salts derived from sodium, calcium, potassium, magnesium, manganese, iron, copper, zinc, aluminum and lithium. These salts can also be prepared by treating the corresponding acidic compounds with an aqueous solution containing the desired pharmacologically acceptable cations, and then evaporating the resulting solution to dryness; preferably under reduced pressure. Alternatively, they may also be prepared by mixing lower alkanolic solutions of the acidic compounds and the desired alkali metal alkoxide together, and then evaporating the resulting solution to dryness in the same manner as before. In either case, stoichiometric quantities of reagents are preferably employed in order to ensure completeness of reaction and maximum yields of the desired final product.

If the inventive compound is a base, the desired pharmaceutically acceptable salt may be prepared by any suitable method available in the art, for example, treatment of the free base with an inorganic acid, such as hydrochloric acid, hydrobromic acid, sulfuric acid, nitric acid, phosphoric acid and the like, or with an organic acid, such as acetic acid; maleic acid, succinic acid, mandelic acid, fumaric acid, malonic acid, pyruvic acid, oxalic acid, glycolic acid, salicylic acid, a pyranosidyl acid, such as glucuronic acid or galacturonic acid, an alpha-hydroxy acid, such as citric acid or tartaric acid, an amino acid, such as aspartic acid or glutamic acid, an aromatic acid, such as benzoic acid or cinnamic acid, a sulfonic acid, such as p-toluenesulfonic acid or ethanesulfonic acid, or the like.

In the case of compounds that are solids, it is understood by those skilled in the art that the inventive compounds and salts may exist in different crystalline or polymorphic forms, or in an amorphous form, all of which are intended to be within the scope of the present invention.

The invention also includes isotopically-labeled compounds of the invention, wherein one or more atoms is replaced by an atom having the same atomic number, but an atomic mass or mass number different from the atomic mass or mass number usually found in nature. Examples of isotopes suitable for inclusion in the compounds of the invention include isotopes of hydrogen, such as $^{2}H$ and $^{3}H$, carbon, such as $^{11}C$, $^{13}C$ and $^{14}C$, chlorine, such as $^{36}Cl$, fluorine, such as $^{18}F$, iodine, such as $^{123}I$ and $^{125}I$, nitrogen, such as $^{13}N$ and $^{15}N$, oxygen, such as $^{15}O$, $^{17}O$ and $^{18}O$, phosphorus, such as $^{32}P$, and sulfur, such as $^{35}S$. Certain isotopically-labeled compounds of the invention, for example, those incorporating a radioactive isotope, are useful in drug and/or substrate tissue distribution studies. The radioactive isotopes tritium, $^{3}H$, and carbon-14, $^{14}C$, are particularly useful for this purpose in view of their ease of incorporation and ready means of detection. Substitution with heavier isotopes such as deuterium, $^{2}H$, may afford certain therapeutic advantages resulting from greater metabolic stability, for example, increased *in vivo* half-life or reduced dosage requirements, and hence may be preferred in some circumstances. Substitution with positron emitting isotopes, such as $^{11}C$, $^{18}F$, $^{15}O$ and $^{13}N$, can be useful in Positron Emission Topography (PET) studies for examining substrate receptor occupancy. Isotopically-labeled compounds of the invention can generally be prepared by conventional techniques known to those skilled in the art or by processes analogous to those described herein, using an appropriate isotopically-labeled reagent in place of the non-labeled reagent otherwise employed.

The compounds of the invention may exist in both unsolvated and solvated forms. The term 'solvate' is used herein to describe a molecular complex comprising a compound of the invention and an amount of one or more pharmaceutically acceptable solvent molecules, The term 'hydrate' is employed when said solvent is water. Examples of solvate forms include, but are not limited to, compounds of the invention in association with water, isopropanol, ethanol, methanol, dimethylsulfoxide (DMSO), ethyl acetate, acetic acid, ethanolamine, or mixtures thereof. It is specifically contemplated that in the present invention one solvent molecule can be associated with one molecule of the compounds of the present invention, such as a hydrate.

Furthermore, it is specifically contemplated that in the present invention, more than one solvent molecule may be associated with one molecule of the compounds of the present invention, such as a dihydrate. Additionally, it is specifically contemplated that in the present invention less than one solvent molecule may be associated with one molecule of the compounds of the present invention, such as a hemihydrate. Furthermore, solvates of the present invention are contemplated as solvates of compounds of the present invention that retain the biological effectiveness of the non-hydrate form of the compounds.

Prodrugs of the compounds described herein may also be used. Such produgs may have little or no pharmacological activity themselves, but when administered into or onto the body may be converted Into compounds of the present invention having the desired activity, for example, by hydrolytic cleavage. Such derivatives are referred to as 'prodrugs'. Further information on the use of prodrugs may be found in Pro-drugs as Novel Delivery Systems, Vol. 14,

ACS Symposium Series (T: Higuchi and W. Stella) and Bioreversible Carriers in Drug Design, Pergamon Press, 1987 (ed. E. B. Roche, American Pharmaceutical Association).

Prodrugs can, for example, be produced by replacing appropriate functionalities present in the compounds of the present invention with certain moieties known to those skilled in the art as 'pro-moieties' as described, for example, in Design of Prodrugs by H. Bundgaard (Elsevier, 1985).

Some examples of prodrugs include:

(i) where the compounds of the present invention contain a carboxylic acid functionality (-COOH), a prodrug compound wherein the hydrogen of the carboxylic acid functionality of the compound is replaced by ($C_1$-$C_8$)alkyl to form the corresponding ester;

(ii) where the compounds of the present invention contain an alcohol functionality (-OH), a prodrug compound wherein the hydrogen of the alcohol functionality of the compound is replaced by ($C_1$-$C_6$) alkanoyloxymethyl to form the corresponding ether; and

(iii) where the compounds of the present invention contain a primary or secondary amino functionality (-$NH_2$ or -NHR where $R \neq H$), a prodrug compound wherein, as the case may be, one or both hydrogens of the amino functionality of the compound I is/are replaced by ($C_1$-$C_{10}$) alkanoyl to form the corresponding amide.

Further examples of replacement groups in accordance with the foregoing examples and examples of other prodrug types may be found in the aforementioned references. Moreover, certain compounds of the present invention may themselves act as prodrugs of other compounds of the present invention.

Metabolites of the compounds of the present invention exist, that is, compounds formed *in vivo* upon administration of the drug. Some examples of metabolites are:

(i) where the compounds of the present invention contain a methyl group, a hydroxymethyl derivative thereof (e.g. -$CH_3$ -> -$CH_2OH$);

(ii) where the compounds of the present invention contain an alkoxy group, a hydroxy derivative thereof (e.g. -OR -> -OH);

(iii) where the compounds of the present invention contain a tertiary amino group, a secondary amino derivative thereof (e.g. -$NR^1R^2$-> -$NHR^1$ or -$NHR^2$);

(iv) where the compounds of the present invention contain a secondary amino group, a primary derivative thereof (e.g. -$NHR^1$-> -$NH_2$);

(v) where the compounds of the present invention contain a phenyl moiety, a phenol derivative thereof (e.g. -Ph -> -PhOH); and

(vi) where the compounds of the present invention contain an amide group, a carboxylic acid derivative thereof (e.g. -$CONH_2$ -> COOH).

Compounds of the invention intended for pharmaceutical use may be administered as crystalline or amorphous products, or mixtures thereof. They may be obtained, for example, as solid plugs, powders, or films by methods such as precipitation, crystallization, freeze drying, spray drying, or evaporative drying. Microwave or radio frequency drying may be used for this purpose.

The compounds can be administered alone or in combination with one or more other compounds of the invention, or in combination with one or more other drugs (or as any combination thereof). Generally, they will be administered as' a formulation in association with one or more pharmaceutically acceptable excipients. The term "excipient" is used herein to describe any ingredient other than the compound(s) of the invention. The choice of excipient will to a large extent depend on factors such as the particular mode of administration, the effect of the excipient on solubility and stability, and the nature of the dosage form.

Pharmaceutical compositions suitable for the delivery of compounds of the invention and methods for their preparation will be readily apparent to those skilled in the art. Such compositions and methods for their preparation can be found, for example, in Remington's Pharmaceutical Sciences', 19th Edition (Mack Publishing Company, 1995), the disclosure of which is incorporated herein by reference in its entirety.

Oral Administration

The compounds of the invention may be administered orally. Oral administration may involve swallowing, so that the compound enters the gastrointestinal tract, or buccal or sublingual administration may be employed by which the compound enters the blood stream directly from the mouth.

Formulations suitable for oral administration include solid formulations such as tablets, capsules containing particulates, liquids, or powders, lozenges (including liquid-filled), chews, multi- and nano-particulates, gels, solid solution,

liposome, films (including muco-adhesive), ovules, sprays and liquid formulations.

Liquid formulations include suspensions, solutions, syrups and elixirs. Such formulations may be used as fillers in soft or hard capsules and typically include a carrier, for example, water, ethanol, polyethylene glycol, propylene glycol, methylcellulose, or a suitable oil, and one or more emulsifying agents and/or suspending agents. Liquid formulations may also be prepared by the reconstitution of a solid, for example, from a sachet.

The compounds of the invention may also be used in fast-dissolving, fast-disintegrating dosage forms such as those described in Expert Opinion in Therapeutic Patents, 11 (6), 981-986 by Liang and Chen (2001), the disclosure of which is incorporated herein by reference in its entirety.

For tablet dosage forms, depending on dose, the drug may make up from 1 wt% to 80 wt% of the dosage form, more typically from 5 wt% to 60 wt% of the dosage form. In addition to the drug, tablets generally contain a disintegrant. Examples of disintegrants include sodium starch glycolate, sodium carboxymethyl cellulose, calcium carboxymethyl cellulose, croscarmellose sodium, crospovidone, polyvinylpyrrolidone, methyl cellulose, microcrystalline cellulose, lower alkyl-substituted hydroxypropyl cellulose, starch, pregelatinized starch and sodium alginate. Generally, the disintegrant will comprise from 1 wt% to 25 wt%, preferably from 5 wt% to 20 wt% of the dosage form.

Binders are generally used to impart cohesive qualities to a tablet formulation. Suitable binders include microcrystalline cellulose, gelatin, sugars, polyethylene glycol, natural and synthetic gums, polyvinylpyrrolidone, pregelatinized starch, hydroxypropyl cellulose and hydroxypropyl methylcellulose. Tablets may also contain diluents, such as lactose (monohydrate, spray-dried monohydrate, anhydrous and the like), mannitol, xylitol, dextrose, sucrose, sorbitol, microcrystalline cellulose, starch and dibasic calcium phosphate dihydrate.

Tablets may also optionally include surface active agents, such as sodium lauryl sulfate and polysorbate 80, and glidants such as silicon dioxide and talc. When present, surface active agents are typically in amounts of from 0.2 wt% to 5 wt% of the tablet, and glidants typically from 0.2 wt% to 1 wt% of the tablet.

Tablets also generally contain lubricants such as magnesium stearate, calcium stearate, zinc stearate, sodium stearyl fumarate, and mixtures of magnesium stearate with sodium lauryl sulphate. Lubricants generally are present in amounts from 0.25 wt% to 10 wt%, preferably from 0.5 wt% to 3 wt% of the tablet.

Other conventional ingredients include anti-oxidants, colorants, flavoring agents, preservatives and taste-masking agents.

Exemplary tablets contain up to about 80 wt% drug, from about 10 wt% to about 90 wt% binder, from about 0 wt% to about 85 wt% diluent, from about 2 wt% to about 10 wt% disintegrant, and from about 0.25 wt% to about 10 wt% lubricant.

Tablet blends may be compressed directly or by roller to form tablets. Tablet blends or portions of blends may alternatively be wet-, dry-, or melt-granulated, melt congealed, or extruded before tabletting. The final formulation may include one or more layers and may be coated or uncoated; or encapsulated.

The formulation of tablets is discussed in detail in "Pharmaceutical Dosage Forms: Tablets, Vol. 1", by H. Lieberman and L. Lachman, Marcel Dekker, N.Y., N.Y., 1980 (ISBN 0-8247-6918-X), the disclosure of which is incorporated herein by reference in its entirety.

Solid formulations for oral administration may be formulated to be immediate and/or modified release. Modified release formulations include delayed-, sustained-, pulsed-, controlled-, targeted and programmed release.

Suitable modified release formulations are described in U.S. Patent No. 6,106,864. Details of other suitable release technologies such as high energy dispersions and osmotic and coated particles can be found in Verma et al, Pharmaceutical Technology On-line, 25(2), 1-14 (2001). The use of chewing gum to achieve controlled release is described in WO 00/35298. The disclosures of these references are incorporated herein by reference in their entireties.

Parenteral Administration

The compounds of the invention may also be administered directly into the blood stream, into muscle, or into an internal organ. Suitable means for parenteral administration include intravenous, intraarterial, intraperitoneal, intrathecal, intraventricular, intraurethral, intrasternal, intracranial, intramuscular and subcutaneous. Suitable devices for parenteral administration include needle (including microneedle) injectors, needle-free injectors and infusion techniques.

Parenteral formulations are typically aqueous solutions which may contain excipients such as salts, carbohydrates and buffering agents (preferably to a pH of from 3 to 9), but, for some applications, they may be more suitably formulated as a sterile non-aqueous solution or as a dried form to be used in conjunction with a suitable vehicle such as sterile, pyrogen-free water.

The preparation of parenteral formulations under sterile conditions, for example, by lyophilization, may readily be accomplished using standard pharmaceutical techniques well known to those skilled in the art.

The solubility of compounds of the invention used in the preparation of parenteral solutions may be increased by the use of appropriate formulation techniques, such as the incorporation of solubility-enhancing agents.

Formulations for parenteral administration may be formulated to be immediate and/or modified release. Modified

release formulations include delayed-, sustained-, pulsed-, controlled-, targeted and programmed release. Thus compounds of the invention may be formulated as a solid, semi-solid, or thixotropic liquid for administration as an implanted depot providing modified release of the active compound. Examples of such formulations include drug-coated stents and PGLA microspheres.

Topical Administration

The compounds of the invention may also be administered topically to the skin or mucosa, that is, dermally or transdermally. Typical formulations for this purpose include gels, hydrogels, lotions, solutions, creams, ointments, dusting powders, dressings, foams, films, skin patches, wafers, implants, sponges, fibers, bandages and microemulsions. Liposomes may also be used. Typical carriers include alcohol, water, mineral oil, liquid petrolatum, white petrolatum, glycerin, polyethylene glycol and propylene glycol. Penetration enhancers may be incorporated; see, for example, J Pharm Sci, 88 (10), 955-958 by Finnin and Morgan (October 1999). Other means of topical administration include delivery by electroporation, iontophoresis, phonophoresis, sonophoresis and microneedle or needle-free (*e.g*. Powderject™, Bioject™, *etc.*) injection. The disclosures of these references are incorporated herein by reference in their entireties.

Formulations for topical administration may be formulated to be immediate and/or modified release. Modified release formulations include delayed-, sustained-, pulsed-, controlled-, targeted and programmed release.

Inhaled/Intranasal Administration

The compounds of the invention can also be administered intranasally or by inhalation, typically in the form of a dry powder (either alone, as a mixture, for example, in a dry blend with lactose, or as a mixed component particle, for example, mixed with phospholipids, such as phosphatidylcholine) from a dry powder inhaler or as an aerosol spray from a pressurized container, pump, spray, atomizer (preferably an atomizer using electrohydrodynamics to produce a fine mist), or nebulizer, with or without the use of a suitable propellant, such as 1,1,1,2-tetrafluoroethane or 1,1,1,2,3,3,3-heptafluoropropane. For intranasal use, the powder may include a bioadhesive agent, for example, chitosan or cyclodextrin.

The pressurized container, pump, spray, atomizer, or nebulizer contains a solution or suspension of the compound(s) of the invention comprising, for example, ethanol, aqueous ethanol, or a suitable alternative agent for dispersing, solubilizing, or extending release of the active, a propellant(s) as solvent and an optional surfactant, such as sorbitan trioleate, oleic acid, or an oligolactic acid.

Prior to use in a dry powder or suspension formulation, the drug product is micronized to a size suitable for delivery by inhalation (typically less than 5 microns). This may be achieved by any appropriate comminuting method, such as spiral jet milling, fluid bed jet milling, supercritical fluid processing to form nanoparticles, high pressure homogenisation, or spray drying.

Capsules (made, for example, from gelatin or HPMC), blisters and cartridges for use in an inhaler or insufflator may be formulated to contain a powder mix of the compound of the invention, a suitable powder base such as lactose or starch and a performance modifier such as /-leucine, mannitol, or magnesium stearate. The lactose may be anhydrous or in the form of the monohydrate, preferably the latter. Other suitable excipients include dextran, glucose, maltose, sorbitol, xylitol, fructose, sucrose and trehalose.

A suitable solution formulation for use in an atomizer using electrohydrodynamics to produce a fine mist may contain from 1μg to 20mg of the compound of the invention per actuation and the actuation volume may vary from 1μL to 100μL. A typical formulation includes a compound of the invention, propylene glycol, sterile water, ethanol and sodium chloride. Alternative solvents which may be used instead of propylene glycol include glycerol and polyethylene glycol.

Suitable flavors, such as menthol and levomenthol, or sweeteners, such as saccharin or saccharin sodium, may be added to those formulations of the invention intended for inhaled/intranasal administration.

Formulations for inhaled/intranasal administration may be formulated to be immediate and/or modified release using, for example, poly(DL-lactic-coglycolic acid (PGLA). Modified release formulations include delayed-, sustained-, pulsed-, controlled-, targeted and programmed release.

In the case of dry powder inhalers and aerosols, the dosage unit is determined by means of a valve which delivers a metered amount. Units in accordance with the invention are typically arranged to administer a metered dose or "puff" containing a desired mount of the compound of the invention. The overall daily dose may be administered in a single dose or, more usually, as divided doses throughout the day.

Rectal/Intravaginal Administration

Compounds of the invention may be administered rectally or vaginally, for example, in the form of a suppository, pessary, or enema. Cocoa butter is a traditional suppository base, but various alternatives may be used as appropriate.

Formulations for rectal/vaginal administration may be formulated to be immediate and/or modified release. Modified release formulations include delayed-, sustained-, pulsed-, controlled-, targeted and programmed release.

Ocular Administration

Compounds of the invention may also be administered directly to the eye or ear, typically in the form of drops of a micronized suspension or solution in isotonic, pH-adjusted, sterile saline. Other formulations suitable for ocular and aural administration include ointments, biodegradable (*e.g.* absorbable gel sponges, collagen) and non-biodegradable (*e.g.* silicone) implants, wafers, lenses and particulate or vesicular systems, such as niosomes or liposomes. A polymer such as crossed-linked polyacrylic acid, polyvinylalcohol, hyaluronic acid, a cellulosic polymer, for example, hydroxypropylmethylcellulose, hydroxyethylcellulose, or methyl cellulose, or a heteropolysaccharide polymer, for example, gelan gum, may be incorporated together with a preservative, such as benzalkonium chloride. Such formulations may also be delivered by iontophoresis.

Formulations for ocular/aural administration may be formulated to be immediate and/or modified release. Modified release formulations include delayed-, sustained-, pulsed-, controlled-, targeted, or programmed release.

Other Technologies

Compounds of the invention may be combined with soluble macromolecular entities, such as cyclodextrin and suitable derivatives thereof or polyethylene glycol-containing polymers, in order to improve their solubility, dissolution rate, taste-masking, bioavailability and/or stability for use in any of the aforementioned modes of administration.

Drug-cyclodextrin complexes; for example, are found to be generally useful for most dosage forms and administration routes. Both inclusion and non-inclusion complexes may be used. As an alternative to direct complexation with the drug, the cyclodextrin may be used as an auxiliary additive, *i.e*. as a carrier, diluent, or solubilizer. Most commonly used for these purposes are alpha-, beta- and gamma-cyclodextrins, examples of which may be found in PCT Publication Nos. WO 91/11172, WO 94/02518 and WO 98/55148, the disclosures of which are Incorporated herein by reference in their entireties.

The amount of the active compound administered will be dependent on the subject being treated, the severity of the disorder or condition, the rate of administration, the disposition of the compound and the discretion of the prescribing physician. However, an effective dosage is.typically in the range of about 0.001 to about 100 mg per kg body weight per day, preferably about 0.01 to about 35 mg/kg/day, in single or divided doses. For a 70 kg human, this would amount to about 0.07 to about 7000 mg/day, preferably about 0.7 to about 2500 mg/day. In some instances, dosage levels below the lower limit of the aforesaid range may be more than adequate, while in other cases still larger doses may be used without causing any harmful side effect, with such larger doses. typically divided into several smaller doses for administration throughout the day.

This invention also relates to a combination for the treatment of abnormal cell growth in a mammal which comprises administering to said mammal an amount of a compound of the present invention, or a salt or solvate thereof, that is effective in treating abnormal cell growth in combination with an anti-tumor agent selected from the group consisting of mitotic inhibitors, alkylating agents, anti-metabolites, intercalating antibiotics, growth factor inhibitors, cell cycle inhibitors, enzymes, topoisomerase inhibitors, biological response modifiers, antibodies, cytotoxics, anti-hormones, and anti-androgens.

In one embodiment of the present invention the anti-tumor agent used in conjunction with a compound of the present invention and pharmaceutical compositions described herein is an anti-angiogenesis agent, kinase inhibitor, pan kinase inhibitor or growth factor inhibitor. Preferred pan kinase inhibitors include Sutent™ (sunitinib), described in U.S. Patent. No. 6,573,293 (Pfizer, Inc, NY, USA). Anti-angiogenesis agents, include but are not limited to the following agents, such as EGF inhibitors, EGFR inhibitors, VEGF inhibitors, VEGFR inhibitors, TIE2 inhibitors, IGF1R inhibitors, COX-II (cyclooxygenase II) inhibitors, MMP-2 (matrix-metalloprotienase 2) inhibitors, and MMP-9 (matrix-metalloprotienase 9) inhibitors.

Preferred VEGF inhibitors, include for example, Avastin (bevacizumab), an anti-VEGF monoclonal antibody of Genentech, Inc. of South San Francisco, California. Additional VEGF inhibitors include CP-547,632 (Pfizer Inc., NY, USA), AG13736 (Pfizer Inc.), ZD-6474 (AstraZeneca), AEE788 (Novartis), AZD-2171, VEGF Trap (Regeneron/Aventis), Vatalanib (also known as PTK-787, ZK-222584: Novartis & Schering AG), Macugen' (pegaptanib octasodium, NX-1838, EYE-001, Pfizer Inc./Gilead/Eyetech), IM862 (Cytran Inc. of Kirkland, Washington, USA); and angiozyme, a synthetic ribozyme from Ribozyme (Boulder, Colorado) and Chiron (Emeryville, California) and combinations thereof.

VEGF inhibitors useful in the practice of the present invention are described in US Patent No. 6,534,524 and 6,235,764, both of which are incorporated in their entirety for all purposes. Additional VEGF inhibitors are described in, for example in WO 99/24440, in WO 95/21613, WO 99/61422, U.S. Patent 5,834,504, WO 98/50356, U.S. Patent 5,883,113 U.S. Patent 5,886,020, U.S. Patent 5,792,783, U.S. Patent 6,653,308, WO 99/10349, WO 97/32856, WO

97/22596, WO 98/54093, WO 98/02438, WO 99/16755, and WO 98/02437.

Other anti-angiogenic compounds include acitretin, fenretinide, thalidomide, zoledronic acid, angiostatin, aplidine, cilengtide, combretastatin A-4, endostatin, halofuginone, rebimastat, removab, Revlimid, squalamine, ukrain, Vitaxin and combinations thereof.

Other antiproliferative agents that may be used in combination with the compounds of the present invention include inhibitors of the enzyme farnesyl protein transferase and inhibitors of the receptor tyrosine kinase PDGFr, including the compounds disclosed and claimed in the following: U.S. Patent 6,080,769; U.S. Patent 6,194,438; U.S. Patent 6,258,824; U.S. Patent 6,586447; U.S. Patent 6,071,935; U.S. Patent 6,495,564; and U.S. Patent 6,150,377; U.S. Patent 6,596,735; U.S. Patent 6,479,513; WO 01/40217; U.S. 2003-0166675.

PDGRr inhibitors include but are not limited to those disclosed in international patent application publication numbers WO01/40217 and WO2004/020431, Preferred PDGFr inhibitors include Pfizer's CP-673,451 and CP-868,596 and its salts.

Preferred GARF inhibitors include Pfizer's AG-2037 (pelitrexol and its salts). GARF inhibitors useful in the practice of the present invention are disclosed in US' Patent No. 5,608,082.

Examples of useful COX-II inhibitors which can be used in conjunction with a compound of Formula (I) and pharmaceutical compositions disclosed herein include CELEBREX™ (celecoxib), parecoxib, deracoxib, ABT-963, MK-663 (etoricoxib), COX-189 (Lumiracoxib), BMS 347070, RS 57067, NS-398, Bextra (valdecoxib), paracoxib, Vioxx (rofecoxib), SD-8381, 4-Methyl-2-(3,4-dimethylphenyl)-1-(4-sulfamoyl-phenyl)-1H-pyrrole, 2-(4-Ethoxyphenyl)-4-methyl-1-(4-sulfamoylphenyl)-1H-pyrrole, T-614, JTE-522, S-2474, SVT-2016, CT-3, SC-58125 and Arcoxia (etoricoxib). Additonally, COX-II inhibitors are disclosed In U.S. Patent Applications US 2005-0148627 and US 2005-0148777.

In a particular embodiment the anti-tumor agent is celecoxib (U.S. Patent No. 5,466,823), valdecoxib (U.S. Patent No. 5,633,272), parecoxib (U.S. Patent No. 5,932,598), deracoxib (U.S. Patent No. 5,521,207), SD-8381 (U.S. Patent No. 6,034,256, Example 175), ABT-963 (WO 2002/24719), rofecoxib (CAS No. 162011-90-7), MK-663 (or etoricoxib) as disclosed in WO 1998/03484, COX-189 (Lumiracoxib) as disclosed in WO 1999/11605, BMS-347070 (U.S. Patent 6,180,651), NS-398 (CAS 123653-11-2), RS 57067 (CAS 17932-91-3), 4-Methyl-2-(3,4-dimethylphenyl)-1-(4-sulfamoyl-phenyl)-1H-pyrrole, 2-(4-Ethoxyphenyl)-4-methyl-1-(4-sulfamoylphenyl)-1H-pyrrole, or meloxicam.

Other useful inhibitors as anti-tumor agents used in combination with a compound of the present invention and pharmaceutical compositions disclosed herein include aspirin, and non-steroidal anti-inflammatory drugs (NSAIDs) which inhibit the enzyme that makes prostaglandins (cyclooxygenase I and II), resulting in lower levels of prostaglandins, include but are not limited to the following, Salsalate (Amigesic), Diflunisal (Dolobid), Ibuprofen (Motrin), Ketoprofen (Orudis), Nabumetone (Relafen), Piroxicam (Feldene), Naproxen (Aleve, Naprosyn), Diclofenac (Voltaren), Indomethacin (Indocin), Sulindac (Clinoril), Tolmetin (Tolectin), Etodolac (Lodine), Ketorolac (Toradol), Oxaprozin (Daypro) and combinations thereof.

Preferred COX-I inhibitors include ibuprofen (Motrin), nuprin, naproxen (Aleve), indomethacin (Indocin), nabumetone (Relafen) and combinations thereof.

Targeted agents used in combination with a compound of the present invention and pharmaceutical compositions disclosed herein include EGFr inhibitors such as Iressa (gefitinib, AstraZeneca), Tarceva (erlotinib or OSI-774, OSI Pharmaceuticals Inc.), Erbitux (cetuximab, Imclone Pharmaceuticals, Inc.), EMD-7200 (Merck AG), ABX-EGF (Amgen Inc. and Abgenix Inc.), HR3 (Cuban Government), IgA antibodies (University of Erlangen-Nuremberg), TP-38 (IVAX), EGFR fusion protein, EGF-vaccine, anti-EGFr immunoliposomes (Hermes Biosciences Inc.) and combinations thereof. Preferred EGFr inhibitors include Iressa, Erbitux, Tarceva and combinations thereof.

Other anti-tumor agents include those selected from pan erb receptor inhibitors or ErbB2 receptor inhibitors, such as CP-724,714 (Pfizer, Inc.), CI-1033 (canertinib, Pfizer, inc.), Herceptin (trastuzumab, Genentech Inc.), Omitarg (2C4, pertuzumab, Genentech Inc.), TAK-165 (Takeda), GW-572016 (Ionafarnib, GlaxoSmithKline), GW-282974 (GlaxoSmithKline), EKB-569 (Wyeth), PKI-166 (Novartis), dHER2 (HER2 Vaccine, Corixa and GlaxoSmithKline), APC8024 (HER2 Vaccine, Dendreon), anti-HER2/neu bispecific antibody (Decof Cancer Center), B7.her2.IgG3 (Agensys), AS HER2 (Research Institute for Rad Biology & Medicine), trifunctional bispecific antibodies (University of Munich) and mAB AR-209 (Aronex Pharmaceuticals inc) and mAB 2B-1 (Chiron) and combinations thereof.

Preferred erb selective anti-tumor agents include Herceptin, TAK-165, CP-724,714, ABX-EGF, HER3 and combinations thereof. Preferred pan erbb receptor inhibitors include GW572016, CI-1033, EKB-569, and Omitarg and combinations thereof.

Additional erbB2 inhibitors include those disclosed in WO 98/02434, WO 99/35146, WO 99/35132, WO 98/02437, WO 97/13760, WO 95/19970, U.S. Patent 5,587,458, and U.S. Patent 5,877,305. ErbB2 receptor inhibitors useful in the present invention are also disclosed in U.S. Patents 6,465,449, and 6,284,764, and in WO 2001/98277.

Additionally, other anti-tumor agents may be selected from the following agents, BAY-43-9006 (Onyx Pharmaceuticals Inc.), Genasense (augmerosen, Genta), Panitumumab (Abgenix/Amgen), Zevalin (Schering), Bexxar (Corixa/GlaxoSmithKline), Abarelix, Alimta, EPO 906 (Novartis), discodermolide (XAA-296), ABT-510 (Abbott), Neovastat (Aetema), enzastaurin (Eli Lilly), Combrestatin A4P (Oxigene), ZD-6126 (AstraZeneca), flavopiridol (Aventis), CYC-202

(Cyclacel), AVE-8062 (Aventis), DMXAA (Roche/Antisoma), Thymitaq (Eximias), Temodar (temozolomide, Schering Plough) and Revilimd (Celegene) and combinations thereof.

Other anti-tumor agents may be selected from the following agents, CyPat (cyproterone acetate), Histerelin (histrelin acetate), Plenaixis (abarelix depot), Atrasentan (ABT-627), Satraplatin (JM-216), thalomid (Thalidomide), Theratope, Temilifene (DPPE), ABI-007 (paclitaxel), Evista (raloxifene), Atamestane (Biomed-777), Xyotax (polyglutamate paclitaxel), Targetin (bexarotine) and combinations thereof.

Additionally, other anti-tumor agents may be selected from the following agents, Trizaone (tirapazamine), Aposyn (exisulind), Nevastat (AE-941), Ceplene (histamine dihydrochloride), Orathecin (rubitecan), Virulizin, Gastrimmune (G17DT), DX-8951f (exatecan mesylate), Onconase (ranpirnase), BEC2 (mitumoab), Xcytrin (motexafin gadolinium) and combinations thereof.

Further anti-tumor agents may be selected from the following agents, CeaVac (CEA), NeuTrexin (trimetresate glucuronate) and combinations thereof. Additional anti-tumor agents may be selected from the following agents, OvaRex (oregovomab), Osidem (IDM-1), and combinations thereof. Additional anti-tumor agents may be selected from the following agents, Advexin (ING 201), Tirazone (tirapazamine), and combinations thereof. Additional anti-tumor agents may be selected from the following agents, RSR13 (efaproxiral), Cotara (1311 chTNT 1/b), NBI-3001 (IL-4) and combinations thereof. Additional anti-tumor agents may be selected from the following agents, Canvaxin, GMK vaccine, PEG Interon A, Taxoprexin (DHA/paciltaxel), and combinations thereof.

Other anti-tumor agents include Pfizer's MEK1/2 inhibitor PD325901, Array Biopharm's MEK inhibitor ARRY-142886, Bristol Myers' CDK2 inhibitor BMS-387,032, Pfizer's CDK inhibitor PD0332991 and AstraZeneca's AXD-5438, and combinations thereof.

Additionally, mTOR inhibitors may also be utilized such as CCI-779 (Wyeth) and rapamycin derivatives RAD001 (Novartis) and AP-23573 (Ariad), HDAC inhibitors, SAHA (Merck Inc./Aton Pharmaceuticals) and combinations thereof. Additional anti-tumor agents include aurora 2 inhibitor VX-680 (Vertex), and Chk1/2 inhibitor XL844 (Exilixis).

The following cytotoxic agents, e.g., one or more selected from the group consisting of epirubicin (Ellence), docetaxel (Taxotere), paclitaxel, Zinecard (dexrazoxane), rituximab (Rituxan) imatinib mesylate (Gleevec), and combinations thereof, may be used in combination with a compound of the present invention and pharmaceutical compositions disclosed herein. The invention also contemplates the use of the compounds of the present invention together with hormonal therapy, including but not limited to, exemestane (Aromasin, Pfizer Inc.), leuprorelin (Lupron or Leuplin, TAP/ Abbott/Takeda), anastrozole (Arimidex, Astrazeneca), gosrelin (Zoladex, AstraZeneca), doxercalciferol, fadrozole, formestane, tamoxifen citrate (tamoxifen, Nolvadex, AstraZeneca), Casodex (AstraZeneca), Abarelix (Praecis), Trelstar, and combinations thereof.

The invention also relates to the use of the compounds of the present invention together with hormonal therapy agents such as anti-estrogens including, but not limited to fulvestrant, toremifene, raloxifene, lasofoxifene, letrozole (Femara, Novartis), anti-androgens such as bicalutamide, flutamide, mifepristone, nilutamide, Casodex™(4'-cyano-3-(4-fluorophenylsulphonyl)-2-hydroxy-2-methyl-3'-(trifluoromethyl) propionanilide, bicalutamide) and combinations thereof.

Further, the invention provides a compound of the present invention alone or in combination with one or more supportive care products, e.g., a product selected from the group consisting of Filgrastim (Neupogen), ondansetron (Zofran), Fragmin, Procrit, Aloxi, Emend, or combinations thereof.

Particularly preferred cytotoxic agents include Camptosar, Erbitux, Iressa, Gleevec, Taxotere and combinations thereof.

The following topoisomerase I inhibitors may be utilized as anti-tumor agents: camptothecin; irinotecan HCl (Camptosar); edotecarin; orathecin (Supergen); exatecan (Daiichi); BN-80915 (Roche); and combinations thereof. Particularly preferred toposimerase II inhibitors include epirubicin (Ellence).

Alkylating agents include, but are not limited to, nitrogen mustard N-oxide cyclophosphamide, ifosfamide, melphalan, busulfan, mitobronitol, carboquone, thiotepa, ranimustine, nimustine, temozolomide, AMD-473, altretamine, AP-5280, apaziquone, brostallicin, bendamustine, carmustine, estramustine, fotemustine, glufosfamide, ifosfamide, KW-2170, mafosfamide, and mitolactol; platinum-coordinated alkylating compounds include but are not limited to, cisplatin, Paraplatin (carboplatin), eptaplatin, lobaplatin, nedaplatin, Eloxatin (oxaliplatin, Sanofi) or satrplatin and combinations thereof. Particularly preferred alkylating agents include Eloxatin (oxaliplatin).

Antimetabolites include but are not limited to, methotrexate, 6-mercaptopurine riboside, mercaptopurine, 5-fluorouracil (5-FU) alone or in combination with leucovorin, tegafur, UFT, doxifluridine, carmofur, cytarabine, cytarabine ocfosfate, enocitabine, S-1, Alimta (premetrexed disodium, LY231514, MTA), Gemzar (gemcitabine, Eli Lilly), fludarabin, 5-azacitidine, capecitabine, cladribine, clofarabine, decitabine, eflornithine, ethynylcytidine, cytosine arabinoside, hydroxyurea, TS-1, melphalan, nelarabine, nolatrexed, ocfosfate, disodium premetrexed, pentostatin, pelitrexol, raltitrexed, triapine, trimetrexate, vidarabine, vincristine, vinorelbine; or for example, one of the preferred anti-metabolites disclosed in European Patent Application No. 239362 such as N-(5-[N-(3,4-dihydro-2-methyl-4-oxoquinazolin-6-ylmethyl)-N-methylamino]-2-thenoyl)-L-glutamic acid and combinations thereof.

Antibiotics include intercalating antibiotics and include, but are not limited to: aclarubicin, actinomycin D, amrubicin, annamycin, adriamycin, bleomycin, daunorubicin, doxorubicin, elsamitrucin, epirubicin, galarubicin, idarubicin, mitomycin C, nemorubicin, neocarzinostatin, peplomycin, pirarubicin, rebeccamycin, stimalamer, streptozocin, valrubicin, zinostatin and combinations thereof.

Plant derived anti-tumor substances include for example those selected from mitotic inhibitors, for example vinblastine, docetaxel (Taxotere), paditaxel and combinations thereof.

Cytotoxic topoisomerase inhibiting agents include one or more agents selected from the group consisting of aclarubicn, amonafide, belotecan, camptothecin, 10-hydroxycamptothecin, 9-aminocamptothecin, diflomotecan, irinotecan HCl (Camptosar), edotecarin, epirubicin (Ellence), etoposide, exatecan, gimatecan, lurtotecan, mitoxantrone, pirarubicin, pixantrone, rubitecan, sobuzoxane, SN-38, tafluposide, topotecan, and combinations thereof.

Preferred cytotoxic topoisomerase inhibiting agents include one or more agents selected from the group consisting of camptothecin, 10-hydroxycamptothecin, 9-aminocamptothecin, irinotecan HCl (Camptosar), edotecarin, epirubicin (Ellence), etoposide, SN-38, topotecan, and combinations thereof.

Immunologicals include interferons and numerous other immune enhancing agents. Interferons include interferon alpha, interferon alpha-2a, interferon, alpha-2b, interferon beta, interferon gamma-1a, interferon gamma-1b (Actimmune), or interferon gamma-n1 and combinations thereof. Other agents include filgrastim, lentinan, sizofilan, TheraCys, ubenimex, WF-10, aldesleukin, alemtuzumab, BAM-002, dacarbazine, daclizumab, denileukin, gemtuzumab ozogamicin, ibritumomab, imiquimod, lenograstim, lentinan, melanoma vaccine (Corixa), molgramostim, OncoVAX-CL, sargramostim, tasonermin, tecleukin, thymalasin, tositumomab, Virulizin, Z-100, epratuzumab, mitumomab, oregovomab, pemtumomab. (Y-muHMFG1), Provenge (Dendreon) and combinations thereof.

Biological response modifiers are agents that modify defense mechanisms of living organisms or biological responses, such as survival, growth, or differentiation of tissue cells to direct them to have anti-tumor activity. Such agents include krestin, lentinan, sizofiran, picibanil, ubenimex and combinations thereof.

Other anticancer agents that can be used in combination with a compound of the present invention include alitretinoin, ampligen, atrasentan bexarotene, bortezomib. Bosentan, calcitriol, exisulind, finasteride,fotemustine, ibandronic acid, miltefosine, mitoxantrone, I-asparaginase, procarbazine, dacarbazine, hydroxycarbamide, pegaspargase, pentostatin, tazarotne, Telcyta (TLK-286, Telik Inc.), Velcade (bortemazib, Millenium), tretinoin, and combinations thereof.

Platinum-coordinated compounds include but are not limited to, cisplatin, carboplatin, nedaplatin, oxaliplatin, and combinations thereof.

Camptothecin derivatives include but are not limited to camptothecin, 10hydroxycamptothecin, 9-aminocamptothecin, irinotecan, SN-38, edotecarin, topotecan and combinations thereof.

Other antitumor agents include mitoxantrone, I-asparaglnase, procarbazine, dacarbazine, hydroxycarbamide, pentostatin, tretinoin and combinations thereof.

Anti-tumor agents capable of enhancing antitumor immune responses, such as CTLA4 (cytotoxic lymphocyte antigen 4) antibodies, and other agents capable of blocking CTLA4 may also be utilized, such as MDX-010 (Medarex) and CTLA4 compounds disclosed in U.S. Patent 6,682,736; and anti-proliferative agents such as other farnesyl protein transferase inhibitors, for example the famesyl protein transferase inhibitors. Additionally, specific CTLA4 antibodies that can be used in combination with compounds of the present invention include those disclosed in U.S. Patents 6,682,736 and 6,682,736.

Specific IGF1R antibodies that can be used in the combination methods of the present invention include those disclosed in WO 2002/053596.

Specific CD40 antibodies that can be used in the present invention include those disclosed in WO 2003/040170. Gene therapy agents may also be employed as anti-tumor agents such as TNF6tade (GeneVec), which express TNFalpha in response to radiotherapy.

In one embodiment of the present invention statins may be used in combination with a compound of the present invention and pharmaceutical compositions thereof. Statins (HMG-CoA reducatase inhibitors) may be selected from the group consisting of Atorvastatin (Lipitor™, Pfizer Inc.), Provastatin (Pravachol™, Bristol-Myers Squibb), Lovastatin (Mevacor™, Merck Inc.), Simvastatin (Zocor™, Merck Inc.), Fluvastatin, (Lescol™ , Novartis), Cerivastatin (Baycol™, Bayer), Rosuvastatin (Crestor™, AstraZeneca), Lovostatin and Niacin (Advicor™, Kos Pharmaceuticals), derivatives and combinations thereof.

In a preferred embodiment the statin is selected from the group consisting of Atovorstatin and Lovastatin, derivatives and combinations thereof. Other agents useful as anti-tumor agents include Caduet.

Inasmuch as it may desirable to administer a combination of active compounds, for example, for the purpose of treating a particular, disease or condition, two or more pharmaceutical compositions, at least one of which contains a compound in accordance with the invention, may conveniently be combined in the form of a kit suitable for coadministration of the compositions. Thus the kit includes two or more separate pharmaceutical compositions, art least one of which contains a compound of the invention, and means for separately retaining said compositions, such as a container, divided bottle, or divided foil packet. An example of such a kit is the familiar blister pack used for the packaging of tablets,

capsules and the like.

The kit is particularly suitable for administering different dosage forms, for example, oral and parenteral, for administering the separate compositions at different dosage intervals, or for titrating the separate compositions against one another. To assist compliance, the kit typically includes directions for administration and may be provided with a memory aid.

Examples

In the following examples molecules with a single chiral center, unless otherwise, noted or indicated by the structural formula or chemical name, exist as a racemic mixture. Those molecules with two or more chiral centers, unless otherwise noted or indicated by the structural formula or chemical name, exist as a racemic mixture of diastereomers. Single enantiomers/diastereomers may be obtained by methods known to those skilled in the art,

$^{1}$H-NMR spectra were recorded on a Bruker instrument operating either at 300 MHz, or 400 MHz and $^{13}$C-NMR spectra were recorded operating at 75 MHz.

The following abbreviations may be used herein: $Et_2O$ (diethyl ether); DMF *(N,N*-dimethylformamide); THF (tetrahydrofuran); DCM (dichloro-methane); DMA (dimethyl acetal); DBU (1,8-diazabicyclo[5.4.0]undec-7-ene); LiHMDS or LHMDS (lithium hexamethyldisilazide); TBME (*tert*-butyl methyl ether); LDA (lithium diisopropylamide); DMSO (dimethylsulfoxide); MeOH (methanol); EtOH (ethanol); BuOH (butanol); EtOAc (ethyl acetate); THF (tetrahydrofuran); Ac (acetyl); Me (methyl); Et (ethyl); Ph (phenyl); TMSI (trimethylsilyliodide); DSC (N,N'-disuccinimidyl carbonate); CDI (1,1'-carbonyldiimidazole); Boc (*tert*-butoxycarbonyl); nBuLi (n-butyl lithium); EDC (1-(3-dimethylaminopropyl)-3-ethylcarbodiimide hydrochloride); HOBt (N-hydroxybenzitriazole hydrate); DME (1,2-dimethoxyethane); $Pd(dba)_2$ (bis(dibenzylideneacetone)palladium(0)); and RT or rt (room temperature).

**Preparation of Compound (i): ethyl 2-amino-4-chloro-5,7-dihydro-6*H*-pyrrolo[3,4-*d*]pyrimidine-6-carboxylate.**

Intermediate (**f**) was prepared in three steps using known procedures (see, e.g. Viscontini and Buhler Helvetica Chimica Acta, 50(5): 1289-93; (1967)) and then converted to compound (i) in two additional steps.

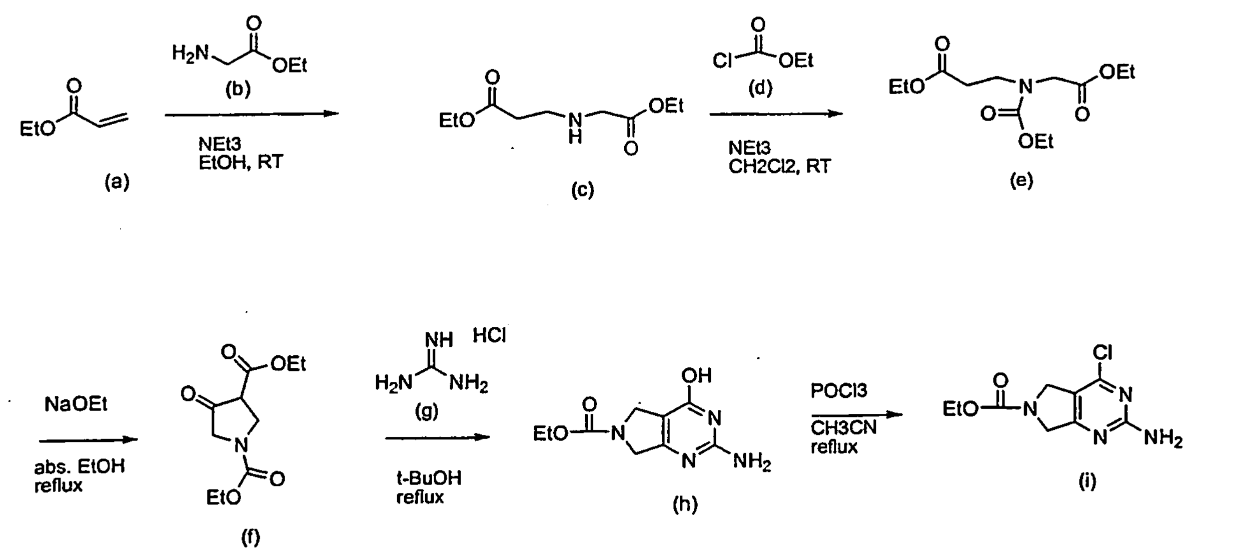


**Step 1. ethyl *N*-(ethoxycarbonyl)-β-alaninate (c)**

Ethyl acrylate (**a**) (50 mL, 460 mmol, 1.1eq), glycine ethyl ester hydrochloride (**b**) (58.4 g, 418 mmol, 1eq), and triethylamine (58.3 mL, 418 mmol, 1eq) in absolute EtOH (960 mL) was stirred at ambient temperature for approximately 72h. After reaction was complete, volatile components were removed under vacuum and the crude intermediate (c) was carried on directly.

**Step2. ethyl *N*-ethoxycarbonyl-*N*-(2-ethoxy-2-oxoethyl-β-alanmate (e)**

Crude intermediate (**c**) (418 mmol) was dissolved in $CH_2Cl_2$ (275 mL) and triethylamine (58.3 mL, 418 mmol) was added followed by ethyl chloroformate (**d**) (39.8 mL, 418 mmol). The reaction was stirred at ambient temperature

for about 24 h. After the reaction was complete, the volatile components were removed under vacuum. The crude product was then distilled under vacuum (about 5mmHg) and dissolved in EtOAc which was washed with aqueous saturated $KHSO_4$ x3, with brine x1 and dried over $Na_2SO_4$. Following filtration, the volatile components were removed under vacuum to afford intermediate (**e**) as a clear oil (74.8g, 272 mmol) in 65% yield over two steps.

**Step 3. diethyl 4-oxopyrrolidine-1,3-dicarboxylate (f)**

Intermediate (e) (18.0g, 65.2 mmol) was added to an ice bath cooled solution of NaOEt (32.6 mL) (21% by weight in EtOH) in absolute EtOH (41.7 mL) under a nitrogen atmosphere.The ice bath was removed and the mixture was heated at 80˚C for about 12h until the condensation was complete as observed by TLC. The mixture was poured onto ice/water and extracted into EtOAc. The solvent was dried with $Na_2SO_4$, filtered, and evaporated to afford crude intermediate (**f**) as an off white solid (14.05g) which was carried on without purification.

**Step 4. ethyl 2-amino-4-hydroxy-5,7-dihydro-6*H*-pyrrolo[3,4-d]pyrimidine-6- , carboxylate** (**h**)

A suspension of intermediate (**f**) (14.05g) and guanidine carbonate (**g**) (16.6g, 91.9 mmol) was refluxed in t-butanol (147 mL) for about 6h. The mixture was allowed to cool to ambient temperature for about 2h. The volatile components were removed under vacuum and water was added. The pH was adjusted to about 6-7 using $KHS0_4$. The resulting slurry was filtered to collect the solids which were washed with water followed by EtOAc. The solids were dried under vacuum to afford intermediate (**h**) as cream solids (11.9g, 53.1 mmol) in 87% yield. 1H NMR (400 MHz, DMSO-D6) δppm 11.01 (s, 1H), 6.97 (s, 0.5H, possible tautomer), 6.70 (s, 2H), 4.25 (s, 4H), 4.13-4.03 (m, 2H), 1.22 (t, 3H). LCMS $(M+H)^+$: 225.2.

**Step 5. ethyl 2-amino-4-chloro-5,7-dihydro-6*H*-pyrrolo[3,4-*d*]pyrimidine-6-carboxylate (i)**

Intermediate (**h**) (11g, 49 mmol) was azeotroped x2 with toluene on the rotovap to assure dryness. Anhydrous acetonitrile (250 mL) and $POCl_3$ (25 mL, 270 mmol) were added and the mixture was refluxed for about 2.5h. Additional $POCl_3$ (50 mL) was added and the mixture was refluxed for an additional 2h. The volatile components were concentrated under vacuum at 40 ˚C to give a red solution. A minimum amount of dry acetonitrile was added until the solution was readily transferable whereupon it was poured onto ice in a large beaker. The flask was further rinsed with a small amount of acetonitrile which was added to the ice. Water (about 50 mL) was added to the ice mixture to help it with stirring. Concentrated $NH_4OH$ (25 mL) was added slowly with stirring until the ice slurry mixture was strongly basic, then 50% aqueous NaOH (25 mL) was also added to the still stirring slurry of ice. Additional ice was added. After about 5 minutes stirring as ice slurry, EtOAc was added. After stirring in the beaker for several more minutes, water was added to help melt the ice. The mixture was poured into a separatory funnel and the layers were allowed to partition. The aqueous layer was extracted with EtOAc x3. The combined EtOAc extracts were washed x2 with saturated aqueous $KHSO_4$, x2 with saturated aqueous $NaHCO_3$, **x1** with brine, dried over $Na_2SO_4$, filtered and evaporated to afford a pale pink powder which was triturated with ethyl acetate to give compound (i) as pale pink solids (6.8g, 28 mmol) in 57% yield. HPLC/ LCMS purity was greater than 90%. $^1$H NMR (400 MHz, DMSO-D6) δ ppm 7.20 (s, 2H), 4.48 (s, 2H), 4.45 (s, 2H), 4.17-4.08 (m, 2H), 1.24 (t, 3H). LCMS $(M+H)^+$: 243.2, 245.2.

**Example 1: ethyl 2-amino-4-(4-bromo-2-chloro-5-methoxyphenyl)-5,7-dihydro-6H-pyrrolo[3,4-d]pyrimidine-6-carboxylate**

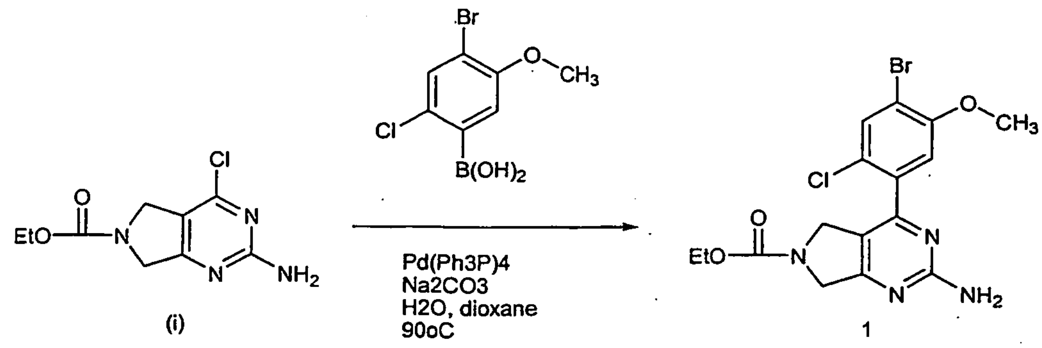


To compound (i) (437 mg, 1.80 mmol), (4-bromo-2-chloro-5-methoxyphenyl)boronic acid (340 mg, 1.28 mmol), and sodium carbonate (382 mg, 3.60 mmol) in an Ar purged round bottom flask was added 1,4- dioxane (8.6 mL) and water (5.2 mL). Preceding the addition, Ar had been bubbled through both solvents to deoxygenate. Palladium tetrakis (triphenylphosphine) (220 mg, 0.19 mmol) was then added and Ar was bubbled through the mixture using a needle and septum for about 3 min. at which point the Ar needle was removed and the mixture was placed in a 90˚C oil bath and heated 2.5h. After cooling to ambient temperature, water was added and the resulting solid was collected by filtration. The solid was washed with water x2, EtOAc x2 and dried at 35˚C for 72h to afford the title compound 1 (300 mg, 0.70 mmol) as a tan powder. $^1$H NMR (400 MHz, DMSO-D6) δ ppm 7.88 (s, 1H), 7.16 (s, 1H), 6.98 (br s, 2H), 4.55-4.47 (m, 2H), 4.39-4.32 (m, 2H), 4.17-4.03 (m, 2H), 3.87 (s, 3H), 1.27-1.15 (m, 3H). LCMS $(M+H)^+$: 429.0, 427.2.

**Example 2: 4-(4-bromo-2-chloro-5-methoxyphenyl)-6,7-dihydro-5H-pyrrolo[3,4-d]pyrimidin-2-amine**

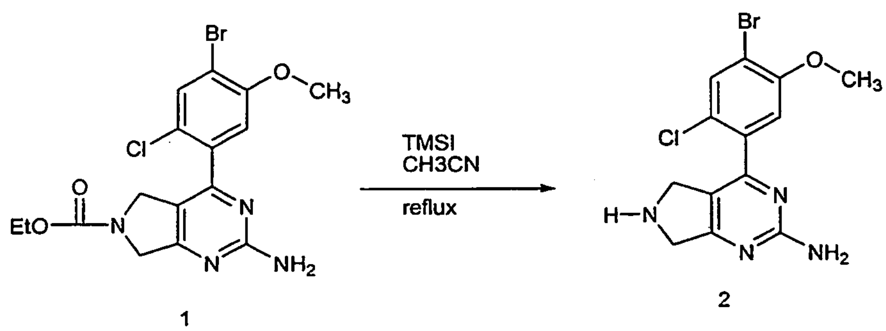


To compound **1** (300 mg, 0.70 mmol) was added anhydrous $CH_3CN$ (4 mL) followed by trimethylsilyliodide (0.5 mL, 4.0 mmol). The mixture was refluxed for about 1h and then cooled to ambient temperature whereupon a small amount of methanol was added to quench. The volatile components were then reduced under vacuum and a small amount of $CH_3CN$ was added followed by $Et_2O$ to precipitate yellow solids which gradually turned orange. The solids were washed with $Et_2O$ and then dried under vacuum at 30 ˚C overnight. Assuming a salt containing 2 HI, the title compound **2** (404 mg, 0.66 mmol) was obtained as orange solids in about 95% yield and was carried on without further purification. $^1$H NMR (400 MHz, DMSO-D6) δ ppm 3.81 - 3.90 (m, 3 H) 4.30 (s, 2 H) 4.39 (s, 2 H) 7.12 (s, 1H) 7.16 (s, 2 H) 7.89 (s, 1H) 9.40 (s, 1H). LCMS $(M+H)^+$: 357.0, 355.0.

**Example 3: 2-amino-4-(4-bromo-2-chloro-5-methoxyphenyl)-N-cyclobutyl-5,7-dihydro-6H-pyrrolo[3,4-d]pyrimidine-6-carboxarnide**

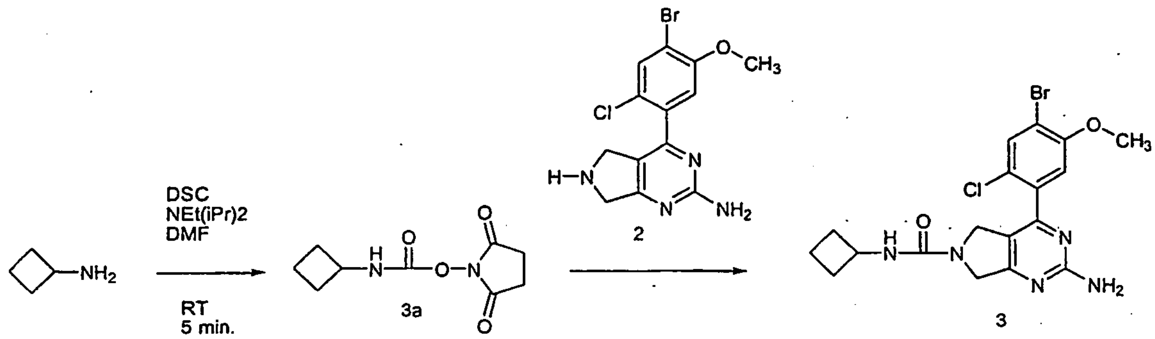


At ambient temperature to stirring N,N'-disuccinimidyl carbonate (67 mg, 0.26 mmol) in DMF (1 mL) was added cyclobutylamine (0.022 mL, 0.26 mmol). Diisopropyl ethylamine (0.152 mL, 0.873 mmol) was then added and the mixture was allowed to stir for about 5 min. With continued stirring, compound **2** (177 mg, 0.289 mmol) in DMF (2 mL) was added. The mixture was allowed to stir overnight and then purified by preparative HPLC. Following lyophilization of the combined purified fractions, the title compound 3 was obtained (57 mg, 0.13 mmol) as a fluffy white solid in 48% yield. $^1$H NMR (400 MHz, DMSO-D6) δ ppm 7:80 (s, 1H), 7.09 (s, 1H), 6.83 (s, 2H), 6.46 (d, 1H), 4.35 (s, 2H), 4.20 . (s, 2H), 4.06 (m, 1H), 3.79m (s, 3H), 2.07-1.97 (m, 2H), 1.92-1.79 (m, 2H), 1.53-1.41 (m, 2H). LCMS $(M+H)^+$: 454.2, 452.2.

**Example 4: 4-(4-chloro-2-methylphenyl)-6-(propylsulfonyl)-6,7-dihydro-5H-pyrrolo[3,4-d]pyrimidin-2-amine**

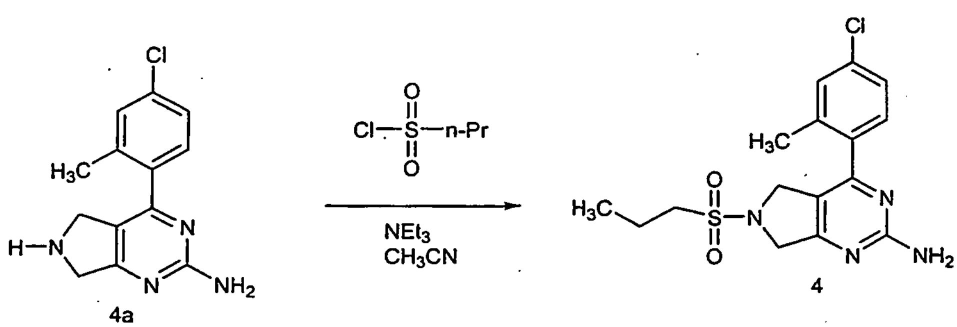


4a

4

The preparation of 4-(4-chloro-2-methylphenyl)-6,7-dihydro-5H-pyrrolo[3,4-d]pyrimidin-2-amine **4a** from compound (i) and 4-chloro-2methylphenylboronic acid was carried out in two steps in a manner analogous to that described for Examples 1 and 2. To a suspension of crude **4a** (100.7 mg, ~0.200 mmol) in acetonitrile (4.0 mL) at 0° C was added triethylamine (0.15 mL, 1.1 mmol) and 1-propanesulfonyl chloride (0.025 mL, 0.22 mmol). After 30 minutes at 0° C, the reaction mixture was concentrated under vacuum, redisolved in dichloromethane (10 mL), washed with water (10 mL)), and concentrated. The residue was purified by preparative HPLC and following lyophilization of the combined purified fractions, the title compound was obtained (16 mg, 0.04 mmol) as a white solid in 22% yield. $^1$H NMR (300 MHz, DMSO-D6) δ ppm 7.43 (d, J = 1.9 Hz, 1 H), 7.38 (d, J = 8.1 Hz, 1H). 7.33 (dd, J = 8.1 and 1.7 Hz, 1 H), 6.86 (brs, 2H), 4.49 (s, 2H), 4.36 (s, 2H), 3.22-3.17 (m, 2H), 2.26 (s, 3H), 1.75-1.63 (m, 2H), 0.97 (t, J = 7.4 Hz, 3H). LCMS $(M+H)^+$: 367.2.

**Example 5: 6-benzyl-4-(4-bromo-2-chloro-5-methoxyphenyl)-6,7-dihydro-5H-pyrrolo[3,4-d]pyrimidin-2-amine trifluoroacetate**

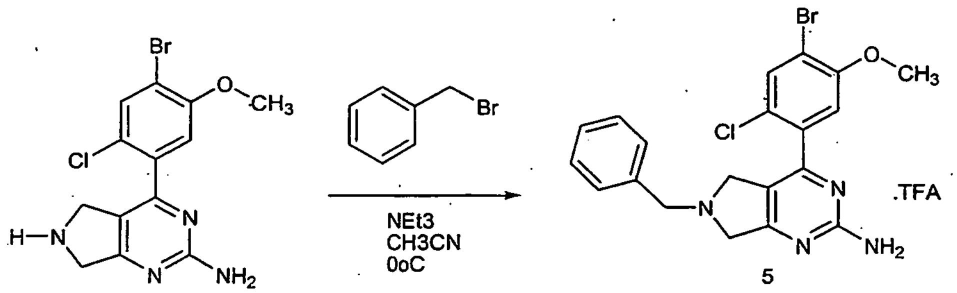


2

5

To compound **2** (91 mg, 0.15 mmol) in DMSO (1 mL), cooled in ice bath, was added N,N-diisopropylethylamine (0.078 mL, 0.45 mmol) followed by benzyl bromide (0.016 mL, 0.13 mmol). The frozen mixture was allowed to thaw and warmed to ambient temperature with stirring. After after about 20 min., the clear red solution was analyzed by LCMS. An approximately 3:2 mixture of the desired mono alkylated product and the quaternary salt was observed. The mixture was purified by preparative HPLC and following lyophilization of the combined purified fractions, the title compound **5** was obtained (29 mg, 0.05 mmol) as a white solid TFA salt in 32% yield. The bis-alkylated quaternary salt was also isolated as a by-product (15 mg). $^1H$ NMR (400 MHz, DMSO-D6) δ ppm 7.90 (s, 1H), 7.58-7.39 (m, 5H), 7.29-7.14 (m, 2H), 7.08 (s, 1H), 4.68-4.25 (m, 6H), 3.89 (s, 3H). LCMS $(M+H)^+$: 447.2, 445.2.

**Example 6: 4-(2,4-dichlorophenyl)-6-[(35-difluoropyridin-2-yl)carbonyl]-6,7-dihydro-5H-pyrrolo[3,4-d]pyrimidin-2-amine**

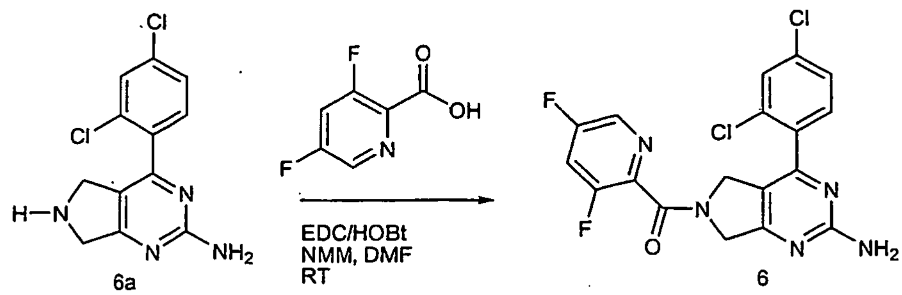


6a

6

The preparation of 4-(2,4-dichlorophenyl)-6,7-dihydro-5H-pyrrolo[3,4-d]pyrimidin-2-amine **6a** from compound (i) and 2,4-dichlorophenylboronic acid was carried out in two steps in a manner analogous to that described for Examples 1 and 2. To a mixture of compound **6a** (54 mg, 0.1 mmol) and 3,5-difluoropyridine-2-carboxylic acid (24 mg, 0.15 mmol) in DMF (2.0 mL) was added 4-methylmorpholine (0.1 mL, 1.0 mmol), EDC (58 mg, 0.3 mmol) and HOBt (46 mg, 0.3 mmol). The resulting mixture was stirred at room temperature for 16 hours. The mixture was partitioned between EtOAc (200 mL) and washed with saturated aqueous $NaHCO_3$ (50 mL) and brine (50 mL). The organic layer was dried over $Na_2SO_4$, filtered and concentrated under vacuum. The residue was purified by preparative HPLC and following lyophilization of the combined purified fractions, the title compound **6** was obtained (25 mg, 0.06 mmol) as a white solid (solvated with 0.4 equivalents of HOAc) in 56% yield. $^1H$ NMR (400 MHz, DMSO-d6) δ ppm 4.54 (d, J=32.08 Hz, 2 H) 4.70 (d, J=29.56 Hz, 2 H) 6.98 (d, J=16.93 Hz, 2 H) 7.38 - 7.62 (m, 2 H) 7.77 (dd, J=36.38, 2.02 Hz, 1H) 8.02 - 8.21 (m, 1H) 8.57 (dd, J=30.69, 2.15 Hz, 1H). LCMS $(M+H)^+$: 424, 422.

**Example 7: 2-amino-4-(4-chloro-2-methylphenyl)-N-cyclopropyl-5,7-dihydro-6H-pyrrolo[3,4-d]pyrimidine-6-carboxamide**

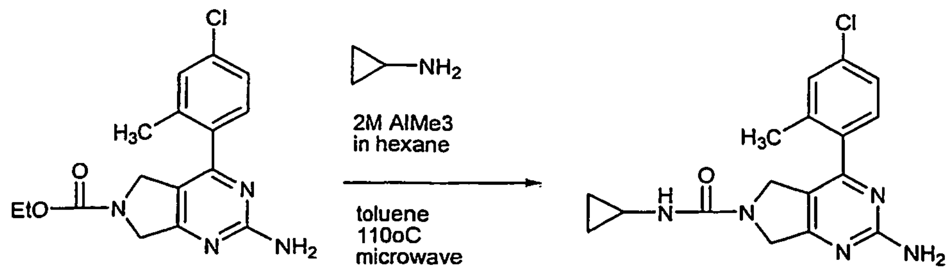


7a 7

The preparation of ethyl 2-amino-4-(4-chloro-2-methylphenyl)-5,7-dihydro-6H-pyrrolo[3,4-d]pyrimidine-6-carboxylate **7a** from compound (i) and 4-chloro-2-methylphenylboronic acid was carried out in a manner analogous to that described for Example **1.** To a solution of cyclopropanamine (0.15 mL, 2.2 mmol) in toluene (2.5 mL) at 0° C was added trimethyl aluminum (2.0 M in hexanes, 0.60 mL, 1.2 mmol) dropwise and the reaction mixture was allowed to warm to room temperature. After 30 minutes, compound **7a** (75.0 mg, 0.225 mmol) was added in one portion, and the reaction was warmed to 110° C in the microwave for 30 minutes. The mixture was then cooled to room temperature, quenched with THF/water (2.5 mL/0.5 mL), and the precipitate was removed by filtration. The filtrate was concentrated under vacuum and purified by preparative HPLC. Following lyophilization of the combined purified fractions, the title compound **7** was obtained (55 mg, 0.16 mmol) as a white solid in 71% yield. $^1$H NMR (300 MHz, DMSO-D6) δ ppm 7.43 (d, J = 1.9 Hz, 1H), 7.34 (dd, J = 8.3 and 1.9 Hz, 1H), 7.31 (d, J = 8.1 Hz, 1 H), 6.77 (br s, 2H), 6.46 (d, J = 2.8 Hz, 1 H), 4.38 (s, 2H), 4.21 (s, 2H), 2.55-2.48 (m, 1H), 2.22 (s, 3H), 0.56-0.50 (m, 2H), 0.41-0.36 (m, 2H). LCMS $(M+H)^+$: 344.2.

**Example 8: 2-amino-4-(4-bromo-2-chlorophenyl)-N-isopropyl-5,7-dihydro-6H-pyrrolo[3,4-d]pyrimidine-6-carboxamide hydrochloride**

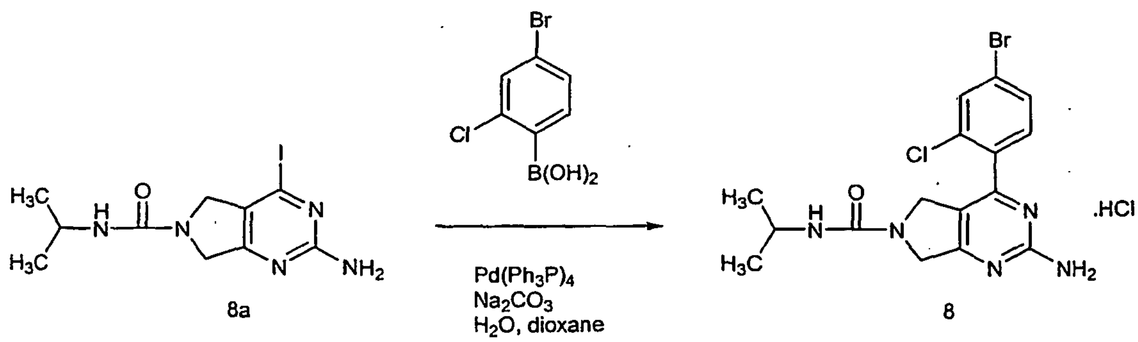


8a 8

4-Bromo-2-chlorophenylboronic acid was prepared by treating 1-bromo-3-chloro-4-iodobenzene (1.339 g, 4.219 mmol) in THF (20.0 mL) at -78°C with nBuLi (2.30 mL, 4.60 mmol) dropwise. After 1h, trimethylborate (1.00 mL, 8.97 mmol) was added dropwise and the reaction was allowed to warm to rt. The reaction mixture was diluted with water (20 mL) and 3N NaOH (10 mL) and stirred for 30 minutes. The mixture was poured into $Et_2O$ (50 mL) and the layers were separated. The aqueous layer was then acidified with 1 N aq. $KH_2PO_4$ (15 mL) and boronic acid extracted into EtOAc. Following isolation, the 4-bromo-2-chlorophenylboronic acid (26.3 mg, 0.112 mmol) was combined with compound **8a** (43.1 mg, 0.124 mmol), and aqueous sodium carbonate (2.0M, 0.125 mL, 3.4 mmol) in DME (2.0 mL). Nitrogen gas was bubbled into the mixture for ten minutes to deoxygenate. Tetrakis(triphenylphosphino)palladium(0) (12.1 mg, 0.0105 mmol) was added and the reaction was warmed to 85°C. After 4h the reaction mixture was cooled to ambient temperature, poured into EtOAc (50 mL), washed with water (50 mL), concentrated under vacuum and purified by preparative HPLC. The purified fractions were combined and 1N HCl (about 3 eq.) was added before lyophilization to prepare the hydrochloride. The title compound **8** was obtained (15 mg, 0.11 mmol) in 30% yield. $^1$H NMR (300 MHz, DMSO-D6) δ ppm 7.92 (d, J = 1.6 Hz, 1H), 7.69 (dd,J = 8.0 and 1.9 Hz, 1H), 7.41 (d, J = 8.0 Hz, 1H), 4.41 (s, 2H), 4.24 (s, 2H), 3.82-3.74 (m, 1H), 1.05 (d, J = 6.6 Hz, 6H). LCMS $(M+H)^+$: 412.2, 410.2.

**Preparation of Compound 8a, 2-amino-4-iodo-*N*-isopropyl-5,7-dihydro-6*H*-pyrrolo[3,4-*d*]pyrimidine-6-carboxamide**

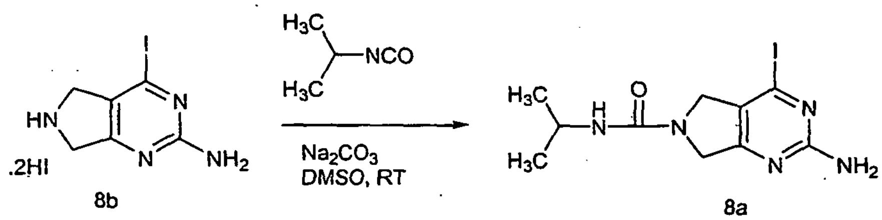


8b

8a

To a solution of 4-iodo-6*H*-pyrrolo[3,4-*d*]pyrimidin-2-amine **8b** (520 mg, 1.0 mmol) in DMSO at ambient temperature was added sodium carbonate (256 mg, 2.4 mmol) and isopropyl isocyanate (0.13 mL, 1.1 mmol). After 3h, the reaction was complete. The DMSO solution was filtered and the filtrate purified by preparative HPLC. Following lyophilization of the combined purified fractions, the title compound **8a** was obtained (204 mg, 0.59 mmol) in 59% yield. $^{1}H$ NMR (300 MHz, DMSO-D6) δ ppm 6.96 (br s, 2H), 6.08 (d, J = 7.7 Hz, 1H), 4.34 (s, 2H), 4.20 (s, 2H), 3.78-3.67 (m, 1H), 1.02 (d, J = 6.6 Hz, 6H). LCMS $(M+H)^+$: 348.2.

**Preparation of Compound 8b, 4-iodo-6*H*-pyrrolo[3,4-d]pyrimidin-2-amine**

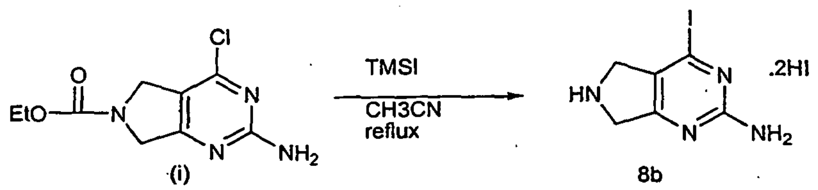


(i)

8b

To a suspension compound (i) (1.00 g, 4.12 mmol) in $CH_3CN$ (25.0 mL) was added iodotrimethylsilane TMSI (3.0 mL, 21.1 mmol). The mixture was refluxed 5 h and then cooled to ambient temperature. Methanol (5 mL) was added to quench the excess TMSI and the mixture was concentrated under vacuum to about 10 mL. Diethyl ether (40 mL) was added and the resulting precipitate was collected by filtration. The solids were then stirred in refluxing EtOAc (50 mL) for a few minutes at which point the mixture was cooled. The light tan solids (1.8g, 3.4 mmol) were collected by filtration to afford the title compound **8b** in 83% yield (assuring 2 HI salt). $^{1}H$ NMR (300 MHz, DMSO-D6) δ ppm 9.47 (br s, 2H), 4.37 (s, 2H), 4.25 (s, 2H). LCMS $(M+H)^+$: 263.2.

**Example 9: *tert*-butyl 2-amino-4-(4-bromo-2-chloro-5-methoxyphenyl)-5,7-dihydro-6H-pyrrolo[3,4-d]pyrimidine-6-carboxylate**

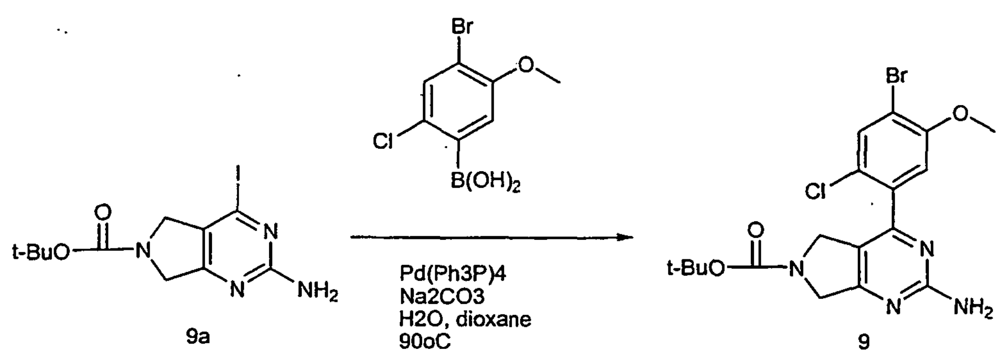


9a

9

Impure compound **9a** (113 mg) in 1,4- dioxane (2 mL) and water (1.2 mL) was treated with (4-bromo-2-chloro-5-methoxyphenyl)boronic acid (385 mg, 0.32 mmol), sodium carbonate (89 mg, 0.84 mmol) and tetrakis(triphenylphosphino)palladium(0) (50 mg, 0.04 mmol) and heated under conditions similar to those described in Example 1. After cooling to ambient temperature, water and EtOAc was added. The water layer was extracted twice with EtOAc and the combined extracts were washed with brine, dried with $Na_2SO_4$, filtered, and volatile components removed under vacuum. The residue was purified by preparative HPLC. Following lyophylization of the combined purified fractions, the *tert*-butoxycarbonyl (Boc) protected title compound **9** was obtained (33 mg, 0.07 mmol) as a white solid in greater than 23% yield. $^1$H NMR (400 MHz, DMSO-D6) δ ppm 7.88 (s, 1H), 7.17 (s, 1H), 6.95 (s, 2H), 4.48-4.42 (m, 2H), 4.33-4.27 (m, 2H), 3.87 (s, 3H), 1.43 (d, 9H). LCMS $(M+H)^+$: 457.2, 455.2.

**Preparation of Compound 9a, *tert*-butyl 2-amino-4-iodo-5,7-dihydro-6*H*-pyrrolo[3,4-*d*]pyrimidine-6-carboxylate**

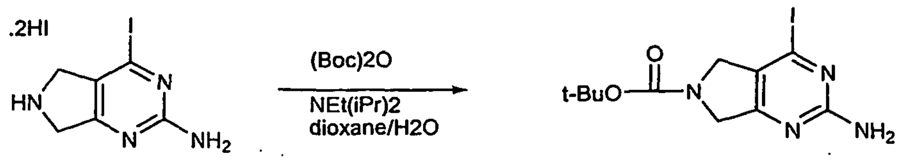


8b 9a

To a stirring mixture of impure compound **8b** (113 mg) in 1,4 dioxane (1 mL) and water (1 mL) was added di-*tert*-butyldicarbonate (0.16 mL, 0.70 mmol) and diisopropylethylamine (0.25 mL, 1.4 mmol). After stirring over night the volatile components were reduced under vacuum and EtOAC and aqueous saturated $KHSO_4$ were added. The resulting two phase mixture was extracted twice with EtOAC and the combined extracts were washed with aqueous saturated $KHSO_4$ x1, with aqueous saturated $NaHCO_3$ x2, with brine x1, and dried over $Na_2SO_4$. Following filtration, the volatile components were removed under vacuum to afford the title compound **9a** as a tan solid (77 mg) which was combined with additional material **9a** (36 mg) from a previous reaction and carried on directy to the next step. $^1$H NMR (400 MHz, DMSO-D6) δ ppm 1.44 (d, J=4.80 Hz, 9 H) 4.25 (d, J=13.14 Hz, 2 H) 4.42 (d, J=8.34 Hz, 2 H) 7.06 (s, 2 H). LCMS $(M+H)^+$: 348.2.

**Example 10: ethyl 2-amino-4-(4-chlorophenoxy)-5,7-dihydro-6H-pyrrolo[3,4-d]pyrimidine-6-carboxylate**

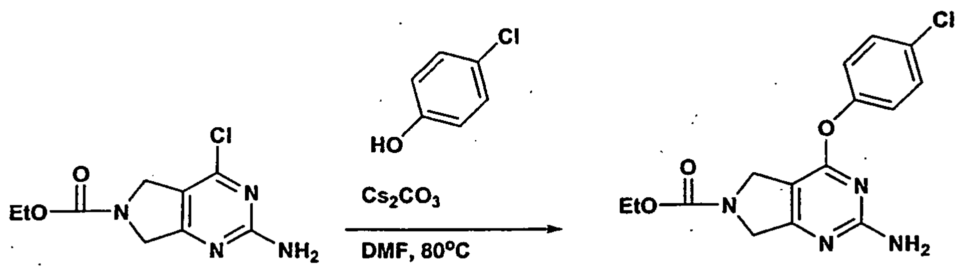


(i) 10

To compound (i) (99 mg, 0.41 mmol) and p-chlorophenol (52 mg, 0.41 mmol) in DMF (2 mL) was added cesium carbonate (266 mg, 0.82 mmol). The mixture was heated at 80 °C for 1.5 h and allowed to cool to ambient temperature. The mixture was filtered through a 0.45μM teflon syringe filter and the filtrate submitted for preparative RPHPLC. Following lyophilization of the combined purified fractions, the title compound was obtained (48 mg, 0.14 mmol) as a white solid in 34% yield. $^1$H NMR (400 MHz, DMSO-D6) δ ppm 7.49 (d, 2H), 7.26 (d, 2H), 6.75 (s, 2H), 4.50 (s, 1 H), 4.46-4.39 (m, 3H), 4.17-4.08 (m, 2H), 1.24 (t, 3H). LCMS $(M+H)^+$: 335.2.

**Example 11: ethyl 2-amino-4-[(4-methoxy-3,5-dimethylpyridin-2-yl)methoxy]-5,7-dihydro-6H-pyrrolo[3,4-d]pyrimidine-6-carboxylate**

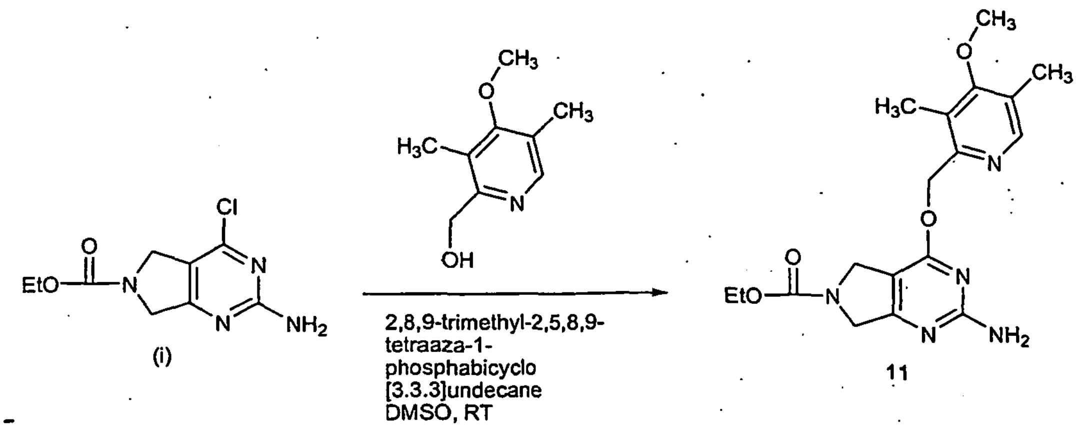


(i) 11

Compound (i) (54 mg, 0.22 mmol), 3,5-dimethyl-4-methoxy-2-pyridinemethanol (45 mg, 0.268 mmol) and 2,8,9-trimethyl-2,5,8,9-tetraaza-1-phosphabicyclo[3.3.3]undecane (58 mg, 0.27 mmol) were stirred in DMSO (1.5 mL) at ambient temperature for 2 h. The mixture was then purified by preparative HPLC, and following lyophilization of the combined purified fractions, the title compound **11** was obtained (37 mg, 0.10 mmol) as a white solid in 44% yield. $^1H$ NMR (300 MHz, DMSO-D6) (conformers) δ ppm 8.20 (s, 1H), 6.72 (br s, 2H), 5.39 (s, 1H). 5.38 (s, 1H), 4.36-4.29 (m, 4H), 4.11-4.03 (m, 2H), 3.75 (s, 3H), 2.22 (s, 6H), 1.23-1.17 (m, 3H). LCMS $(M+H)^+$: 374.2.

**Example 12: ethyl 2-amino-4-(1,3-dihydro-2H-isoindol-2-yl)-5,7-dihydro-6H-pyrrolo[3,4-d]pyrimidine-6-carboxylate**

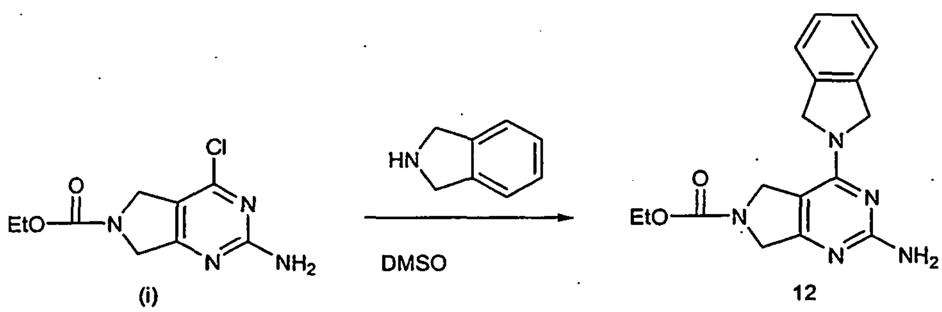


(i) 12

Compound (i) (74.8 mg, 0.308 mmol) and isoindoline (105 mg, 0.881 mmol) in DMSO (1 mL) were stirred and heated to 80°C for 20 minutes. After cooling, the mixture was poured into water and extracted into $CH_2Cl_2$. Following evaporation of the volatile components, the residue was dissolved in DMSO and submitted for preparative RPHPLC. Following lyophilization of the combined purified fractions, the title compound **12** was obtained (68 mg, 0.19 mmol) in 62% yield.

$^1H$ NMR (300 MHz, DMSO-D6) (conformers) δ ppm 7.42-7.39 (m, 2H), 7.32-7.29 (m, 2H), 6.13 (br s, 2H), 4.96-4.87 (br m, 4H), 4.28-4.23 (m, 2H), 4.16-4.10 (m, 2H), 3.31 (br s, partially obscured by H2O peak, 2H), 1.28-1.21 (m, 3H). LCMS $(M+H)^+$: 326.2.

**Example 13: ethyl 2-amino-4-(4-bromo-2-chloro-5-hydroxyphenyl)-5,7-dihydro-6H-pyrrolo[3,4-d]pyrimidine-6-carboxylate**

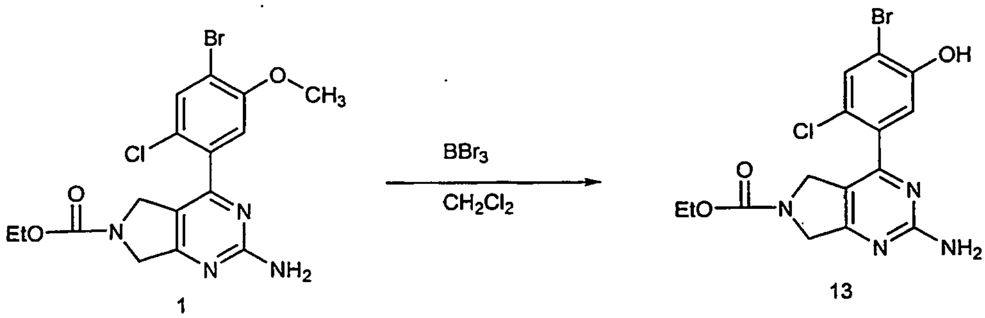


1

13

To a stirring suspension of compound **1** (428 mg, 1.0 mmol) in $CH_2Cl_2$ (10 mL) at 0 °C was added $BBr_3$ (0.5 mL, 5.0 mmol). The mixture was stirred overnight and allowed to warm to ambient temperature whereupon ice and water (10 mL) were added and the resulting precipitate was collected by filtration. After washing with water and $CH_2Cl_2$, the solid was dried under vacuum to give the title compound **13** (375 mg, 0.91 mmol) as a yellow solid in 91% yield. $^1H$ NMR (300 MHz, DMSO-D6) (conformers) δ ppm 1.09 - 1.28 (m, 3 H) 3.99 - 4.16 (m, 2 H) 4.33 (d, J=8.34 Hz, 2 H) 4.47 (d, J=11.12 Hz, 2 H) 6.93 (d, J=1.77 Hz, 1H) 7.74 (s, 1H) 10.89 (s, 1H). LCMS $(M+H)^+$: 417.0, 415.0.

**Example 14: ethyl 2-amino-4-[4-bromo-2-chloro-5-(2-chloroethoxy)phenyl]-5,7-dihydro-6H-pyrrolo[3,4-d]pyrimidine-6-carboxylate**

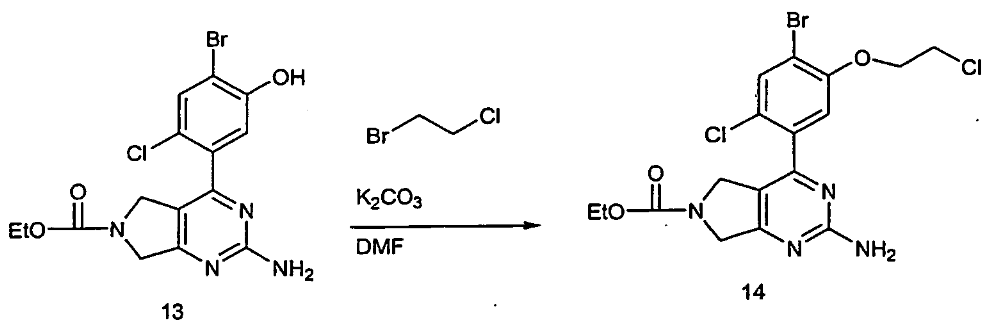


13

14

To a stirring mixture of compound **13** (360 mg, 0.87 mmol) and potassium carbonate (361 mg, 2.61 mmol) in anhydrous DMF (4 mL) was added 1-bromo-3-chloropropane (0.15 mL, 1.74 mmol). The mixture was heated at 50°C for 12 hours whereupon the mixture was cooled and filtered to remove suspended solids, and the solids were washed well with EtOAc. The washes and filtrate were combined and additional EtOAc (200 mL) was added. The combined organic layer was washed with saturated aqueous $NaHCO_3$ (50 mL), and brine (50 mL), and then dried over $Na_2SO_4$, filtered, and concentrated under vacuum. The yellow residue was treated with $CH_2Cl_2$ (30 mL), and the resulting solids were collected by filtration. After washing with $CH_2Cl_2$ and hexane, the solids were dried under vacuum to give the title compound **14** (240 mg, 0.5 mmol) as a white solid in 58% yield. $^1H$ NMR (300 MHz, DMSO-D6) δ ppm 1.11 - 1.27 (m, 3 H) 3.90 - 4.00 (m, 2 H) 4.08 (dd, J=14.53, 7.20 Hz, 2 H) 4.29 - 4.40 (m, 4 H) 4.48 (d, J=10.61 Hz, 2 H) 6.95 (s, 2 H) 7.20 (s, 1H) 7.88 (s; 1H). LCMS $(M+H)^+$: 477.0, 475.0.

**Example 15: 2-amino-4-{4-bromo-2-chloro-5-[2-(dimethylamino)ethoxy]phenyl}-N-isopropyl-5,7-dihydro-6H-pyrrolo[3,4-d]pyrimidine-6 carboxamide**

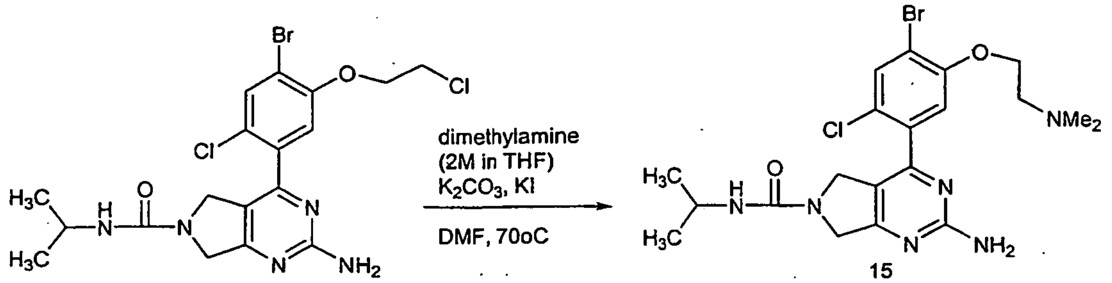


15a

15

To a mixture of compound 15a (90 mg, 0.18 mmol), $K_2CO_3$ (76 mg, 0.55 mmol), and KI (61 mg, 0.37 mmol) in DMF (2.0 mL) was added a 2M solution of dimethylamine in THF (0.3 mL, 0.55 mmol). After stirring at 70°C for 12 h, the mixture was cooled and filtered to remove suspended solids. The solids were washed with EtOAc. The washes and filtrate were combined and additional EtOAc (200 mL) was added. The combined organic layer was washed with saturated aqueous $NaHCO_3$ (50 mL), and brine (50 mL), and then dried over $Na_2SO_4$, filtered, and concentrated under vacuum. The residue was purified by preparative HPLC and following lyophilization of the combined purified fractions, the title compound 15 was obtained (16 mg, 0.03 mmol) as a yellow solid in 17% yield. $^1$H NMR (400 MHz, DMSO-d6) δ ppm 1.06 (t, J=7.07 Hz, 6 H) 2.22 (s, 6 H) 2.65 (t, J=5.68 Hz, 2 H) 3.78 (dd, J= 13.89, 6.82 Hz, 1H) 4.14 (t, J=5.56 Hz, 2 H) 4.25 (s, 2 H) 4.42 (s, 2 H) 6.08 (d, J=7.83 Hz, 1H) 6.88 (s, 2 H) 7.19 (s, 1H) 7.85 (s, 1H). LCMS $(M+H)^+$: 501.2, 499.2.

**Preparation of compound 15a, 2-amino-4-[4-bromo-2-chloro-5-(2-chloroethoxy)phenyl]-N-isopropyl-5,7-dihydro-6H-pyrrolo[3,4-d]pyrimidine-6-carboxamide**

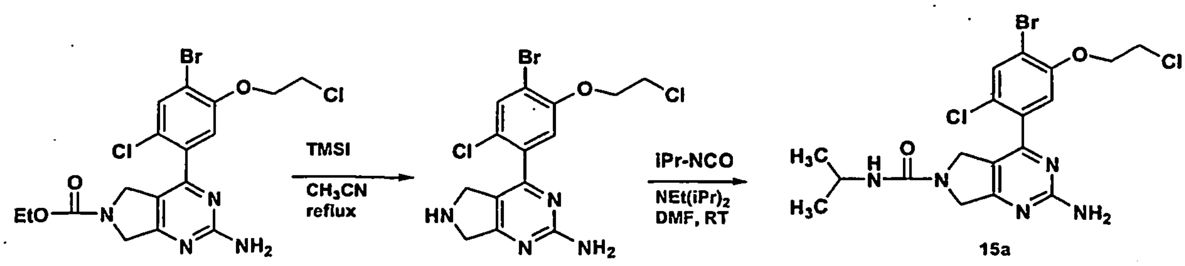


14

15b

15a

Compound **14** was deprotected in a manner similar to that described for Example 2. Extractive work up from EtOAc and saturated aqueous $NaHCO_3$ gave compound **15b,** 4-[4-bromo-2-chloro-5-(2-chloroethoxy)phenyl]-6,7-dihydro-5H-pyrrolo[3,4-d]pyrimidin-2-amine, (191 mg, 0.473 mmol) which was dissolved in DMF (2.0 mL) and treated sequentially with diisopropylethylamine (0.4 mL, 2.36 mmol) and isopropylisocyanate (40 mg, 0.473 mmol) at ambient temperature. After 3 h, methanol was added and the mixture was concentrated under vacuum. The residue was dissolved in EtOAc and washed with saturated aqueous $NaHCO_3$ and brine. The organic layers were dried over $Na_2SO_4$, filtered, and concentrated under vacuum. The residue was chromatographed on silica gel eluting with 10% methanol in $CH_2Cl_2$. The purest fractions were combined and the volatile components removed under vacuum to give the title compound **15a** (180 mg, 0.37 mmol) as a brown foam in 78% yield. $^1$H NMR (400 MHz, DMF-d7) δ ppm 1.04 - 1.11 (m, 6 H) 3.81 (s, 1H) 3.92 - 4.04 (m, 2 H) 4.28 (s, 2 H) 4.39 (d, J=10.11 Hz, 2 H) 4.44 (s, 2 H) 6.09 (d, J=8.08 Hz, 1H) 6.91 (s, 2 H) 7.24 (s, 1H) 7.91 (s, 1H). LCMS $(M+H)^+$: 488.0, 490.0.

**Example 17: 6-[(4-chlorophenyl)acetyl]-4-(4-fluoro-2-methoxyphenyl)-5,6,7,8-tetrahydropyrido[4,3-d]pyrimidin-2-amine**

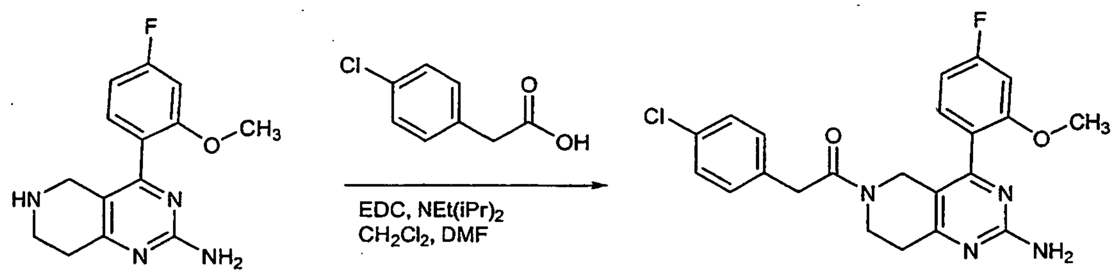


17a

17

Compound **17a** (32 mg, 0.19 mmol) was coupled to (4-chlorophenyl) acetic acid (32 mg, 0.19 mmol), using EDC (40 mg, 0.21 mmol) and $NEt(iPr)_2$ (0.051 mL, 0.29 mmol) in DMF (0.5 mL) and $CH_2Cl_2$ (0:5 mL) in a manner similar to that described for Example 6. Purification on silica gel (eluting with 10% MeOH in $CH_2Cl_2$) gave, after isolation, the title compound **17** (48 mg, 0.11 mmol) in 59% yield. 1H NMR (400 MHz, DMSO-D6) (conformers) δ ppm 7.41-6.82 (m, 7H), 6.58-6.47 (m, 2H), 4.17 (s, 2H), 3.85-3.55 (m, 7H), 2.71 (br s, 2H). LCMS $(M+H)^+$: 429.2, 427.2.

**Preparation of compound 17a, 4-(4-fluoro-2-methoxyphenyl)-5,6,7,8-tetrahydropyrido[4,3-d]pyrimidin-2-amine**

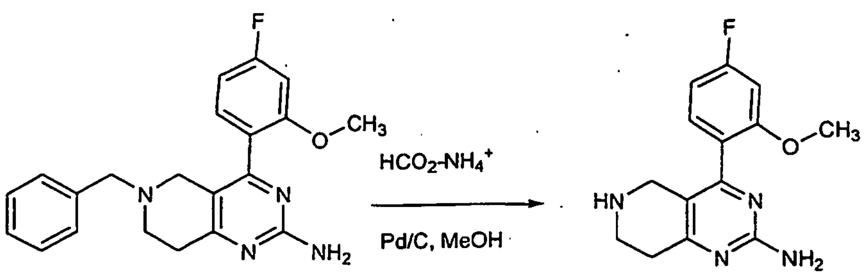


17b

17a

Compound **17b** (971 mg, 2.66 mmol) in methanol was treated with ammonium formate (1.9 g, 29.4 mmol) and 10% palladium on carbon (490 mg) After stirring for several days, the palladium on carbon was removed through two successive filtrations- first through filter paper and then through a 0.45 micron teflon syringe filter. The filtrate was reduced under vacuum and a small amount of water was added. The resulting precipitate was collected by filtration to afford the title compound **17a** (438 mg, 1.6 mmol) in 60% yield after drying under vacuum. $^1H$ NMR (400 MHz, DMSO-D6) (conformers) δ ppm 8.26 (s, 1H), 7.19 (m, 1H), 7.03 (m, 1H), 6.87 (m, 1H), 6.41 (s, 2H), 3.78 (s, 3H), 3.53-3.39 (m, 2H, partially obscured), 3,12-3.02 (m, 2H), 2.71-2.62 (m, 2H). LCMS $(M+H)^+$: 275.0.

**Preparation of compound 17b, 6-benzyl-4-(4-fluoro-2-methoxyphenyl)-5,6,7,8-tetrahydropyrido[4,3-d]pyrimidin-2-amine**

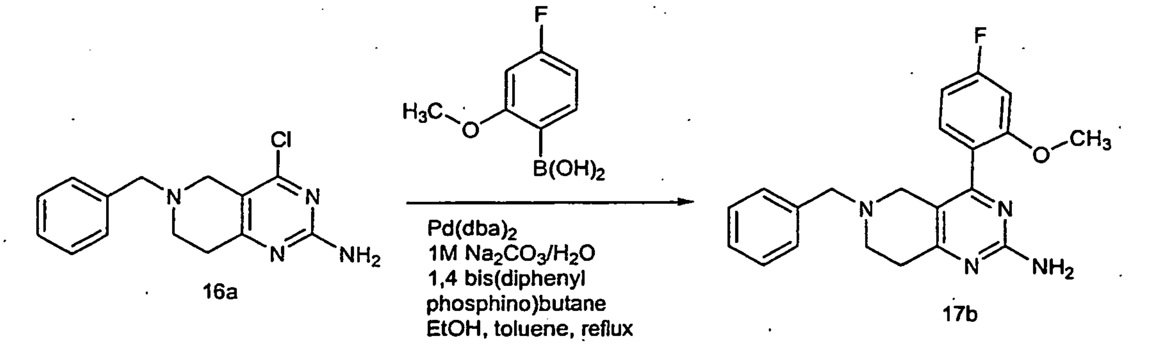


Argon was bubbled through a mixture of compound **16a** (286 mg, 1.04 mmol), 4-fluoro-2-methoxyphenyl) boronic acid (194 mg, 1.14 mmol), 1M aqueous $Na_2CO_3$ (1 mL) in toluene (2 mL) and ethanol (0.5 mL). $Pd(dba)_2$ (30 mg, 0.052 mmol) and 1,4 bis (diphenylphosphino)butane (44 mg, 0.104 mmol) were added and additional argon was bubbled through the mixture which was then refluxed for approximately 19h. After cooling to ambient temperature, water was added and the crude product was extracted into EtOAc, washed with brine, dried over $Na_2SO_4$ and filtered. The volatile components were removed under vacuum and the residue was purified on silica eluting with EtOAc followed by acetone. The purest fractions were combined and reduced to dryness whereupon the solids were dissolved in THF and filtered through a 0.45 micron teflon filter. Again the volatile components were removed under vacuum to afford the title compound **17b** (228 mg, 0.62 mmol) in 60% yield. $^1H$ NMR (400 MHz, DMSO-D6) (conformers) δ ppm 7.32-7.20 (m, 5H), 7.15 (m, 1H), 6.94 (d, 1H), 6.80 9m, 1H), 6.37 (s, 2H), 3.63-3.42 (m, 5H; (contains 3.63 (s, 3H)), 3.31-2.92 (m, 2H), 2.74-2.59 (m, 4H (contains 2.66 (s, 2H)). LCMS $(M+H)^+$: 365.0.

**Example 18: 2-amino-N-cyclobutyl-4-[2,4-dichloro-6-(4,4,4-trifluorobutoxy)phenyl]-5,7-dihydro-6H-pyrrolo [3,4-d]pyrimidine-6-carboxamide**

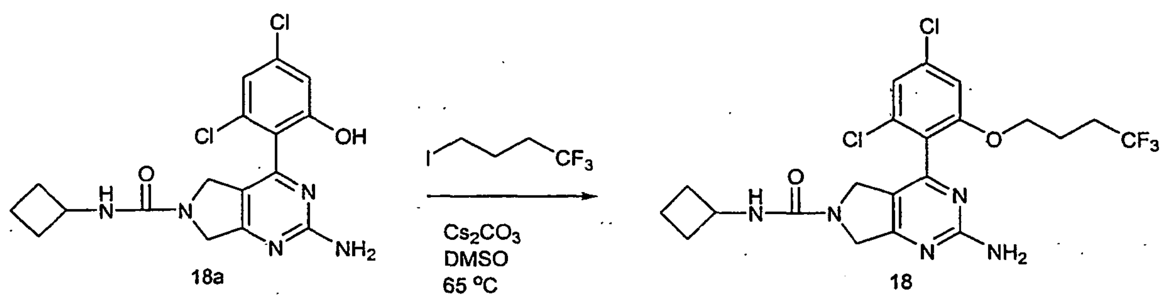


A solution of compound 18a 1 (91 mg, 0.231 mmol) in DMSO (1.5 mL) was treated with $Cs_2CO_3$ (174 mg, 0.533 mmol) and 1-iodo-4,4,4-trifluorobutane (89.2 mg, 0.375 mmol) and warmed to 65˚C. After 30 minutes, the reaction mixture was filtered and the DMSO solution was subjected to preparative HPLC. The purified fractions were combined any lyophilized. The resulting white powder was then dissolved in a solution of $CH_3CN$, (2 mL), 1 N HCl (0.45 mL) and water (5 mL). Following lyophilization, compound 18 (46 mg, 0.085 mmol) was obtained as a white powder in 37% yield. $^1H$ NMR (300 MHz, DMSO-D6) δ ppm 7.32 (d, J = 1.8 Hz, 1H), 7.26 (d, J = 1.8 Hz, 1H), 6.46 (d, J = 7.3 Hz, 1H), 4.40-4.30 (m, 2H), 4.11-4.02 (m, 5H), 2.12-1.99 (m, 4H), 1.91-1.81 (m, 2H), 1.74-1.67 (m, 2H), 1.52-1.43 (m,2H) . LCMS $(M+H)^+$: 504.0, 506.0. Anal. Calcd for $C_{21}H_{22}N_5Cl_2O_2F_3$- 0.7 HCl. 0.6 H2O: C, 46.65; H, 4.46; N12.95; Cl, 17.70. Found: C, 46.75; H, 4.26; N, 12.69; Cl, 17.43.

**Preparation of Compound 18a, 2-amino-*N*-cyclobutyl-4-(2,4-dichloro-6-hydroxyphenyl)-5,7-dihydro-6*H*-pyrrolo[3,4-d]pyrimidine-6-carboxamide**

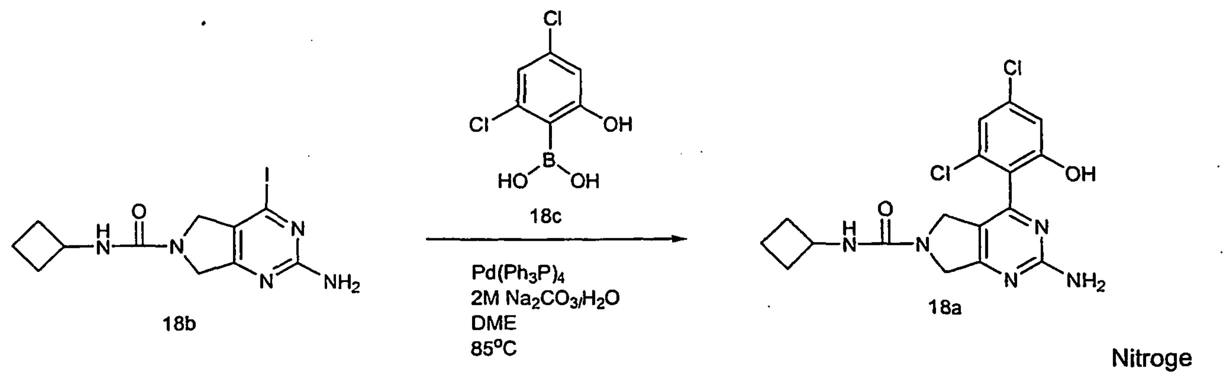


Nitroge

n was bubbled through a solution of compound 18b (1350 mg, 3.75 mmol), compound 18c (775 mg, 3.75 mmol) and 2.0M aqueous sodium carbonate (0.60 mL, 1.20 mmol) in 1,4-dioxane (7.0 mL) for 15 minutes. To this was added tetrakis(triphenylphosphino)palladium(0) (431 mg, 0.373 mmol) and the mixture was warmed to 85˚C. After 5h, the mixture was cooled to ambient temperature and EtOAc (20 mL) and water (20 mL) were added. The aqueous layer was extracted with EtOAc and the EtOAc layer was discarded. The pH of the aqueous solution was adjusted to about 5 with 1 N $KH_2PO_3$ and then extracted with a mixture of EtOAc and iPrOH. The volatile components were remove under vacuum to give compound 18a (869 mg, 2.2 mmol) in 59% yield which was carried on without further purification. $^1$H NMR (300 MHz, DMSO-D6) δ ppm 10.64 (s, 1H), 7.16 (d, J = 1.7 Hz, 1H), 6.97 (d, J = 1.7 Hz, 1H), 6.79 (br, 2H), 6.52(d, J = 7.5 Hz, 1H), 4.40 (br s, 2H), 4.15 (br s, 2H), 4.08-3.99 (m, 1H), 2.15-2.03 (m, 2H), 1.97-1.86 (m, 2H), 1.60-1.47 (m, 2H). LCMS $(M+H)^+$: 394.2 and 396.2

**Preparation of Compound 18b: 2-amino-*N*-cyctobutyl-4-iodo-5,7-dihydro-6*H*-pyrrolo[3,4-*d*]pyrimidine-6-carboxamide**

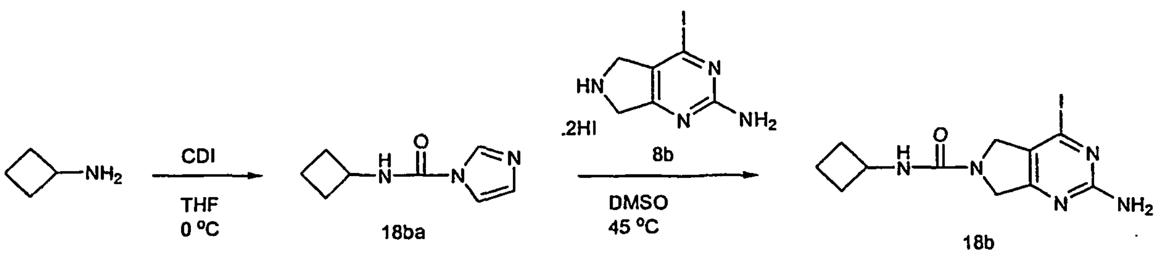


To a solution of 1,1-carbonyldiimidazole (3.54 g, 21.8 mmol) in THF (100 mL) at 0 ˚C was added a solution of cyclobutylamine (1.00 g, 14.1 mmol) in THF (9.0 mL) dropwise over 10 minutes. The mixture was allowed to warm to ambient temperature and stirred for 2h. The volatile components were removed under vacuum and the resulting oil was purified on silica gel, eluting with a gradient of 0-10% MeOH in $CH_2Cl_2$. The purest' fractions were combined and the volatile components removed under vacuum to give compound 18ba, *N*-cyclobutyl-1H-imidazole-1-carboxamide, (2.2 g, 13.4 mmol) as clear oil. Compound 18ba (1.39 g, 8.41 mmol) and compound 8b (3.79 g, 7.32 mmol) were then dissolved in DMSO (20 mL) and $Cs_2CO_3$ (4.92 g, 15.1 mmol) was added. The mixture was stirred for 1h at ambient temperature and then heated at 45˚C for 4h whereupon the mixture was cooled to ambient temperature and water (150 mL) was added. The aqueous suspension was extracted with a mixture of EtOAc and EtOH (200 mL) and the volatile components were removed under vacuum to give the title compound 18b (2 g, 5.6 mmol) in 66% yield which was carried on without further purification. $^1$H NMR (300 MHz, DMSO-D6) δ ppm 7.02 (br s, 2H), 6.60 (d, J = 7.7 Hz, 1H), 4.41 (br s, 2H), 4.26 (br s, 2H), 4.19-4.07 (m, 1H), 2.18-2.07 (m, 2H), 2.04-1.91 (m, 2H), 1.64-1.50 (m, 2H). LCMS $(M+H)^+$ 360.2.

**Preparation of Compound 18c, 2,4-dichloro-6-hydroxyphenylboronic acid**

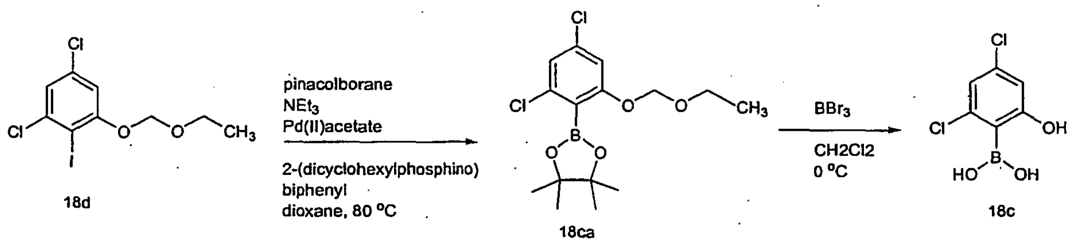


18d 18ca 18c

A solution of compound 18d (4.33 g, 12.5 mmol), pinacolborane (3.80 mL, 26.2 mmol), and triethylamine (5.2 mL, 37 mmol) in 1,4-dioxane (80 mL) was purged with $N_2$ for 15 minutes. Palladium (II) acetate (142 mg, 0.632 mmol) and 2-(dicyclohexylphosphino)biphenyl (439 mg, 1.25 mmol) were added and the mixture was warmed to 80˚C for 1.5h. After cooling to room temperature, the mixture was poured into EtOAc (200 mL), and washed with saturated aqueous NH4Cl (100 mL) and water (200 mL). Following concentation under vacuum, the residue was purified on silica gel, eluting with a gradient of 10-70% $CH_2Cl_2$ in hexane. The purest fractions were combined and the volatile components removed under vacuum to give the compound 18ca, 2,4-dichloro-6-(ethoxymethoxy)phenylboronic acid pinacol ester, (1.55 g, 4.47 mmol) which was then dissolved in dichloromethane (50.0 mL) and cooled to 0 ˚C. A 1.0 M solution of boron tribromide in $CH_2Cl_2$ (10.0 mL, 10.0 mmol) was added slowly over 5 minutes. After 15 minutes, the mixture was poured into ice water. The biphasic mixture was stirred vigorously and the pH of the aqueous phase was adjusted to about 10 with 3N NaOH. The layers were separated and the dichloromethane phase discarded. The pH of the aqueous phase was then adjusted to about 3 with 1 N HCl and extracted with a mixture of EtOAc and iPrOH. The organic layer was then concentrated to give the title compound 18c (778 mg, 3.8 mmol) as a tan powder that was carried on without further purification.

**Preparation of Compound 18d, 1,5-dichloro-3-(ethoxymethoxy)-2-iodobenzene**

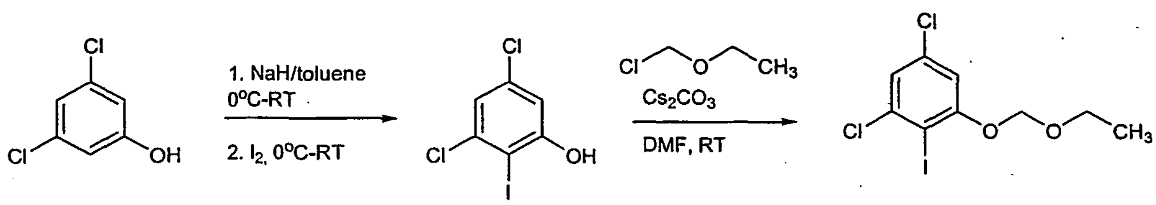


18da 18d

To a solution of 3,5-dichlorophenol (5.98 g, 36.7 mmol) in toluene (200 mL) at 0˚C was added NaH (60% in oil, 4.50 g, 117 mmol) in portions. The reaction mixture was then allowed to warm to ambient temperature and stir for 20 minutes. The suspension was then cooled back to 0˚C, and iodine (7.91 g, 31.2 mmol) was added slowly. The mixture was allowed to warm to ambient temperature and stirred overnight. Aqueous 1 N HCl (200 mL) was added, and the mixture was exrtacted twice with $Et_2O$ (300 mL total). The combined organic extracts were concentrated under vacuum, and the residue was purified on silica gel, eluting with a gradient of 0-30% $CH_2Cl_2$ in hexane. The purest fractions were combined, and the volatile components removed under vacuum to give 3,5-dichloro-2-iodophenol, compound 18da, (6.53g, 22.7 mmol) in 62% yield. Compound 18da (3.726 g, 12.90 mmol) in DMF (50 mL) was then treated with $Cs_2CO_3$ (3.45 g, 10.6 mmol) and chloromethylethyl ether (1.50 mL, 16.2 mmol). After 3h at ambient temperature, $Et_2O$ (150 mL) was added, and the organic layer was washed with water (3 x 100 mL). The organic layer was concentrated under vacuum and the residue was purified on silica gel eluting with a gradient of 0-40% $CH_2Cl_2$ in hexane. The purest fractions were combined and the volatile components removed under vacuum to give the title compound 18d (4.3 g, 12.5 mmol) as a clear oil. Estimated purity (by NMR) was 86%. 1H NMR (300 MHz, $CDCl_3$) δ ppm 7.00 (d, J = 1.6 Hz, 1H), 6.98 (d, J = 1.5 Hz, 1H), 5.18 (s, 2H), 3.71 (q, J = 7.2 Hz, 2H), 1.39 (s, 12H), 1.22 (t, J = 7.2 Hz, 3H). Compounds of Examples 20-98 were prepared followng the methods of Examples 1-18, as shown in the following Table 1.

**Table 1**

| Ex. No. | Structure | Name | Synthetic Method | $^1$H NMR | MS |
|---|---|---|---|---|---|
| 20 | 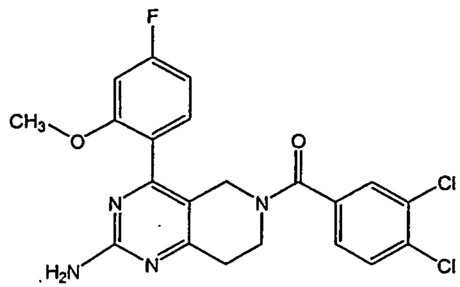 | 6-(3,4-dichlorobenzo yl)-4-(4-fluoro-2-methoxypheny 1)-5,6,7,8-tetrahydropyrido[4,3-d]pyrimidin-2-amine | Example 17 | (400 MHz, DMSO-D6) (conformers) δ ppm 7.80-7.57 (m, 2H), 7.46 (m, 1H), 7.27 (m, 1H), 7.11 (m, 1H), 6.89 (m, 1H), 6.59 (s, 2H), 4.29 br s, 2H), 3.82, br s, 3H), 3.64-3.37 (m, 2H), 2.85-2.73 (m, 2H). | 449.2, 447.2 |
| 22 | 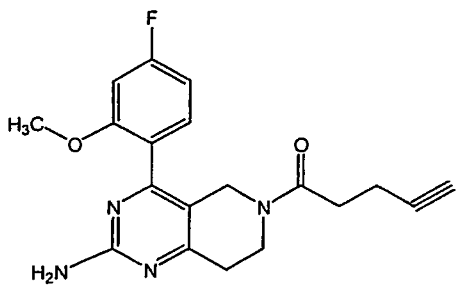 | 4-(4-fluoro-2-methoxyphenyl)-6-pent-4-ynoyl-5,6,7,8-tetrahydropyri do[4,3-d]pyrimidin-2-amine | Example 17 | (400 MHz, DMSO-D6) (conformers) δ ppm 7.22 (m, 1H), 7.08 (m, 1H), 6.90 (m, 1H), 6.52 (m, 2H), 4.15 (br s, 2H), 3.86-3.67 (m, 5H, contains 3.78 (s)), 2.84-2.49 (m, 5H, partially obscured), 2.45-2.27 (m, 2H). | 355.2 |
| 23 | 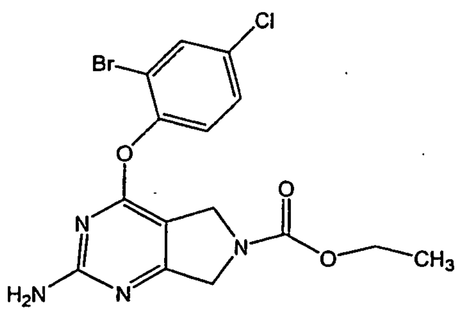 | ethyl 2-amino-4-(2-bromo-4-chlorophenoxy)-5,7-dihydro-6H-pyrrolo[34-d]pyrimidine-6-carboxylate | Example 10 | (400 MHz, DMSO-D6) δ ppm 7.91 (s, 1H), 7.56 (m, 1H), 7.43 (m, 1H). 6.82 (br s, 2H), 4.55 (s, 1H), 4.52-4.42 (m, 3H), 4.19-4.09 (m, 2H), 1.24 (t, 3H). | 412.8, 414.8 |

(continued)

| Ex. No. | Structure | Name | Synthetic Method | $^1$H NMR | MS |
|---|---|---|---|---|---|
| 24 | 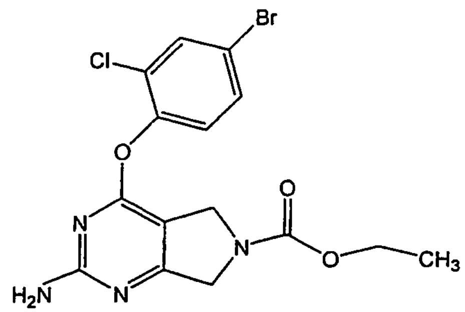 | ethyl 2-amino-4-(4-bromo-2-chlorophenoxy)-5,7-dihydro-6H-pyrrolo[34-d]pyrimidine-6-carboxylate | Example 10 | (400 MHz, DMSO-D6) δ ppm 7.92 (s, 1H), 7.66 (d, 1H), 7.39 (d, 1H), 6.82 (br s, 2H), 4.54 (s, 1H), 4.50 (s, 1H), 4.44 (d, 2H), 4.18-4.09 (m, 2H), 1.25 (t, 3H). | 412.8, 414.8 |
| 25 | 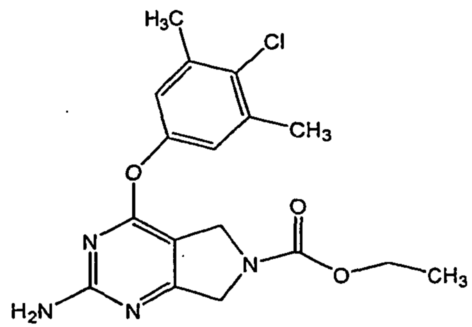 | ethyl 2-amino-4-(4-chloro-35-dimethylphenoxy)-5,7-dihydro-6H-pyrrolo[34-d]pyrimidine-6-carboxylate hydrochloride | Example 10 | (400 MHz, DMSO-D6) 6 ppm 7.09 (s, 2H); 4.50 (s, 1H), 4.46-4.42 .(m, 3H), 4.17-4.10 (m, CH2, partially obscured), 2.35 (s, 6H), 1.25 (t, 3H). | 363.2 |
| 26 | 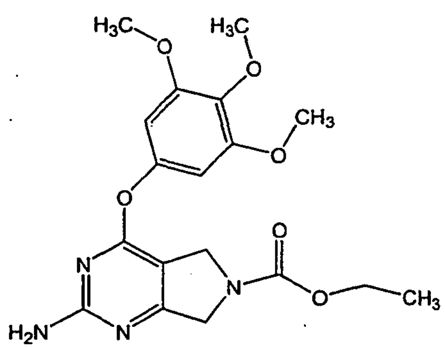 | ethyl 2-amino-4-(345-trimethoxyphe noxy)-5,7-dihydro-6H-pyrrolo[34-d] pyrimidine-6-carboxylate | Example 10 | (400 MHz, DMSO-D6) 6 ppm 6.59 (s, 2H), 4.49-4.40 (m, 4H), 4.17-4.09 (m, 2Hz, partially obscured), 3.78 (s, 6H), 3.67 (s, 3H), 1.28-1.21 (m, 3H). | 391.2 |

(continued)

| Ex. No. | Structure | Name | Synthetic Method | $^1$H NMR | MS |
|---|---|---|---|---|---|
| 27 | 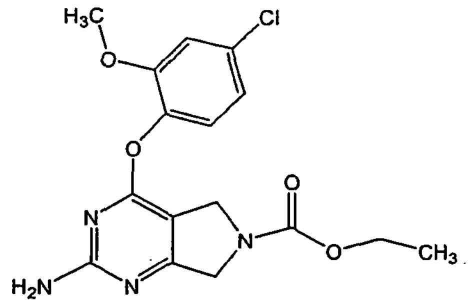 | ethyl 2-amino-4-(4-chloro-2-methoxyphenoxy)-5,7-dihydro-6H-pyrrolo[3,4-d]pyrimidine-6-carboxylate | Example 10 | (400 MHz, DMSO-D6) δ ppm 7.30-7.18 (m, 2H), 7.05 (m, 1H). 6.71 (s, 2H), 4.49 9s, 1H), 4.43-4.38 (m, 3H), 4.17-4.08 (m, 2H), 3.77 (s, 3H), 1.23 (t, 3H).. | 365.0 |
| 29 | 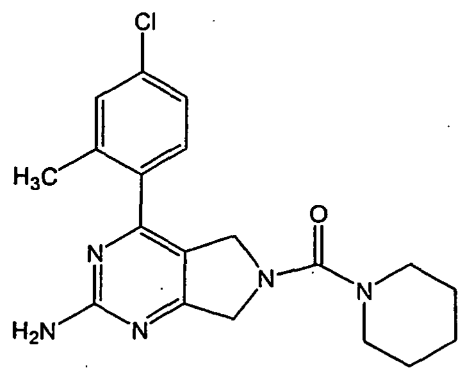 | 4-(4-chloro-2-methylphenyl)-6-(piperidin-1-ylcarbonyl)-6,7-dihydro-5H-pyrrolo[3,4-d]pyrimidin-2-amine | Example 7 | (300 MHz, DMSO-D6) δ ppm 7.42 (br s, 1H), 7.35-7.33 (m, 2H), 6.75 (br s, 2H), 4.52 (s, 2H), 4.39 (s, 2H), 3.18-3.15 (m, 4H), 2.24 (s, 3H), 1.56-1.39 (m, 6H). | 372.2 |
| 30 | 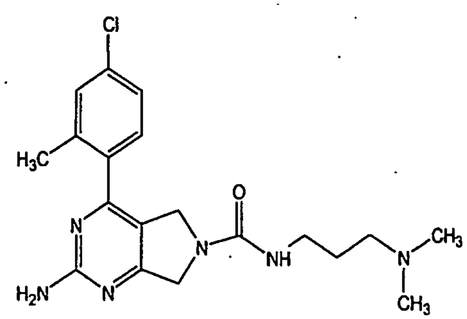 | 2-amino-4-(4-chloro-2-methylphenyl)-N-[3-(dimethylamin o) propyl]-5,7-dihydro-6H-pyrrolo[3,4-dlpyrimidine-6-carboxamide | Example 7 | (300 MHz, DMSO-D6) δ ppm 7.43 (br s, 1H), 7.35-7.32 (m, 2H), 6.77 (br s, 2H), 6.44 (t, J=5.5 Hz, 1H). 4.40 (s, 2H), 4.24 (s, 2H), 3.07-3.00 (m, 2H), 2.23 (s, 3H), 2.18 (t, J = 7.1 Hz, 2H), 2.09 (s, 6H),1.57-1.48 (m, 2H). | 389.2 |

(continued)

| Ex. No. | Structure | Name | Synthetic Method | $^1$H NMR | MS |
|---|---|---|---|---|---|
| 31 | 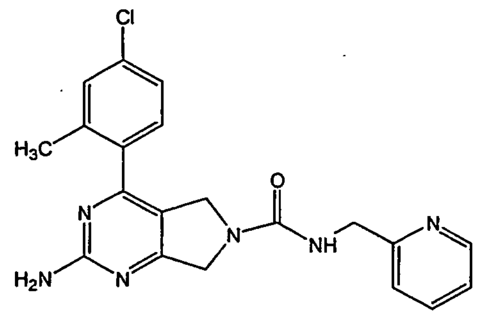 | 2-amino-4-(4-chloro-2-methylphenyl)-N-(pyridin-2-ylmethyl)-5,7-dihydro-6H-pyrrolo[3,4-.d] pyrimidine-6-carboxamide | Example 7 | (300 MHz, DMSO-D6) δ ppm 8.48-8.44 (m, 1H), 7.72 (dt, J = 7.7 and 1.9 Hz, 1H), 7.43 (brs, 1H), 7.36-7.33 (m, 2H), 7.31 (d, J = 7.9 Hz, 1H), 7.25-7.18 .(m, 1H), 7.07 (t, J = 5.9 Hz, 1H), 6.79 (br s, 2H), 4.48 (s, 2H), 4.36-4.31 (m, 4H), 2.24 (s, 3H). | 395.2 |
| 32 | 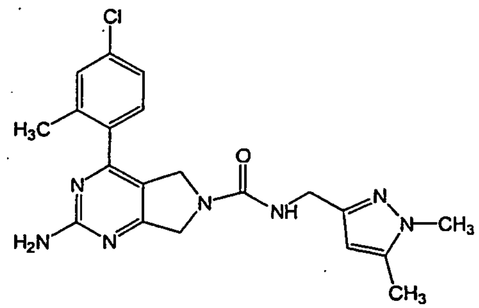 | 2-amino-4-(4-chloro-2-methylphenyl)-N-[(15-dimethyl-1H-pyrazol-3-yl)methyl]-5,7-dihydro-6H-pyrrolo[3,4-d]pyrimidine-6-carboxamide | Example 7 | (300 MHz, DMSO-D6) δ ppm 7.42 (br s, 1H), 7.34-7.32 (m, 2H), 6.77 (br s, 2H), 6.73 (t, J = 5.8 Hz, 1H), 5.88 (s, 1H), 4.42 (s, 2H), 4.26 (s, 2H), 4.08 (d, J = 5.7 Hz, 2H), 3.62 (s, 3H), 2.23 (s, 3H), 2.17 (s, 3H). | 412.2 |
| 33 | 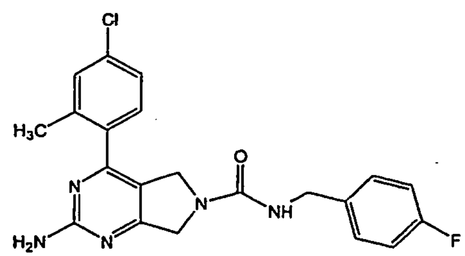 | 2-amino-4-(4-chloro-2-methylphenyl)-N-(4-fluorobenzyl)-5,7-dihydro-6H-pyrrolo[3,4-d]pyrimidine-6-carboxamide | Example 7 | (300 MHz, DMSO-D6) δ ppm 7.42 (br s, 1H), 7.34-7.27 (m, 4H), 7.14-7.06 (m, 2H), 6.99 (t, J = 5.8 Hz, 1H), 6.78 (br s, 2H), 4.45 (s, 2H), 4.30 (s, 2H), 4.22 (d, J = 5.7 Hz, 2H), 2.23 (s, 3H). | 412.2 |

(continued)

| Ex. No. | Structure | Name | Synthetic Method | $^1$H NMR | MS |
|---|---|---|---|---|---|
| 34 | 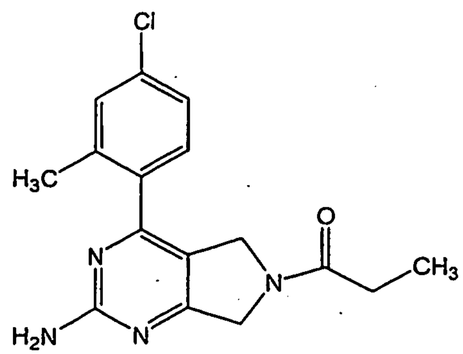 | 4-(4-chloro-2-methylphenyl)-6-propionyl-6,7-dihydro-5H-pyrrolo[3,4--d]pyrimidin-2-amine | Example 6 | (300 MHz, DMSO-D6) δ ppm 7.43 (br s, 1H), 7.35-7.32 (m, 2H), 6.79 (br s, 2H), 4.67 (s, 1H), 4.52 (s, 1H), 4.45 (s, 1H), 4.29 (s, 1H), 2.36-2.23 (m, 5H), 1.03-0.95 (m, 3H). | 317.2 |
| 35 | 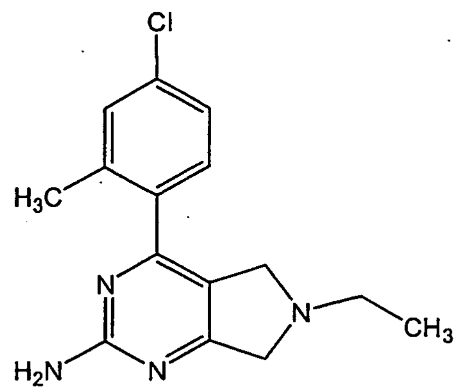 | 4-(4-chloro-2-methylphenyl)-6-ethyl-6,7-dihydro-5H-pyrrolo[3,4-d] pyimidin-2-amine | Example 5 | (300 MHz, DMSO-D6) δ ppm 7.40 (d, J = 2.1 Hz, 1H), 7.31 (dd, J = 8.3 and 2.1 Hz, 1H), 7.25 (d, J = 8.3 Hz, 1H), 6.58 (br s, 2H), 3.70 (s, 2H), 3.58 (s, 2H), 2.65 (q, J = 7.2 Hz, 2H), 2.24 (s, 3H), 1:04 (t, J = 7.2 Hz, 3H). | 289.2 |

(continued)

| Ex. No. | Structure | Name | Synthetic Method | $^1$H NMR | MS |
|---|---|---|---|---|---|
| 36 | 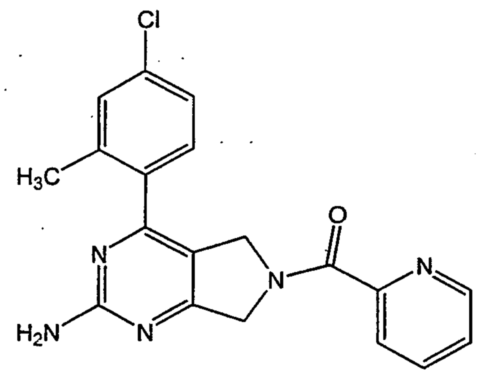 | 4-(4-chloro-2-methylphenyl)-6-(pyridin-2-ylcarbonyl)-6,7-dihydro-5H-pyrrolo[3,4-d]pyrimidin-2-amine | Example 6 | (300 MHz, DMSO-D6) (conformers) δ ppm 8: 68 (d, J = 4.3 Hz, 0.5H), 8.57 (d, J = 4.3 Hz, 0.5H), 8.01-7.91 (m, 1H), 7.88-7.78 (m, 1H), 7.58-7.37 (m, 3H), 7.29 (s, 1H), 6.82 (br s, 1H), 6.79 (br s, 1H), 4.99 (s, 1H), 4.80 (s, 1H), 4.74 (s, 1H), 4.60 (s, 1H), 2.27 (s, 1.5H), 2.22 (s, 1.5H). | 366.2 |
| 37 | 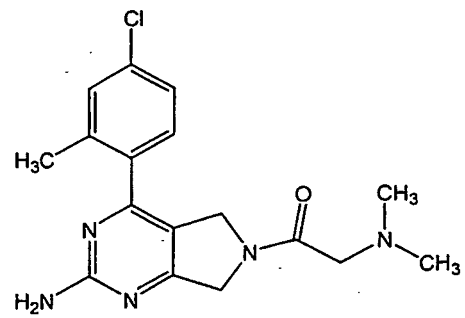 | 4-(4-chloro-2-methylphenyl)-6-[(dimethylamin o)acetyl]-6,7-dihydro-5H-pyrrolo[3,4-d]pyrimidin-2-amine | Example 6 | (300 MHz, DMSO-D6) (conformers) δ ppm 7.43 (m,1H), 7.35-7.32 (m, 2H), 6.80 and 6.78 (2 overlapping br s, 2H), 4.75 (s, 1H), 4.58 (s, 1H), 4.46 (s, 1H), 4.32 (s, 1H), 3.12 (s, 1H), 3.08 (s, 1H), 2.25 (s, 1.5H), 2.24 (s, 1.5H), 2.23 (s, 3H), 2.18 (s, 3H). | 346.2 |

(continued)

| Ex. No. | Structure | Name | Synthetic Method | $^1$H NMR | MS |
|---|---|---|---|---|---|
| 38 | 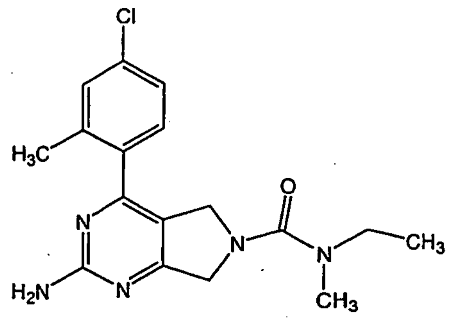 | 2-amino-4-(4-chloro-2-methylphenyl)-N-ethyl-N-methyl-5,7-dihydro-6H-pyrrolo[3,4-d]pyrimidine-6-carboxamide | Example 7 | (300 MHz, DMSO-D6) δ ppm 7.42 (s, 1H), 7.35-7.32 (m, 2H), 6.73 (br s, 2H), 4.51 (s, 2H), 4.38 (s, 2H), 3.15 (q, J = 7.0 Hz, 2H), 2.76 (s, 3H), 2.25 (s, 3H), 1.05 (t, J = 7.0 Hz, 3H). | 346.2 |
| 39 | 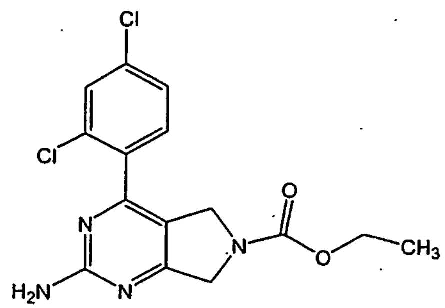 | ethyl 2-amino-4-(2,4-dichlorophenyl )-5,7-dihydro-6H-pyrrolo[3,4-d] pyrimidine-6-carboxylate | Example 1 | (300 MHz, DMSO-D6) (conformers) δ ppm 7.78 (d, J = 1.9 Hz, 1H), 7.56 (dd, J = 8.3 and 1.9 Hz, 1H), 7.48 (d, J = 8.3 Hz, 1H), 6.92 (br s, 2H), 4.49 (s, 1H), 4.47 (s, 1H), 4.34 (s, 1H), 4.31 (s, 1H), 4.13-4.03 (m, 2H), 1.24-1.15 (m, 3H). | 355.2, 353.2 |
| 40 | 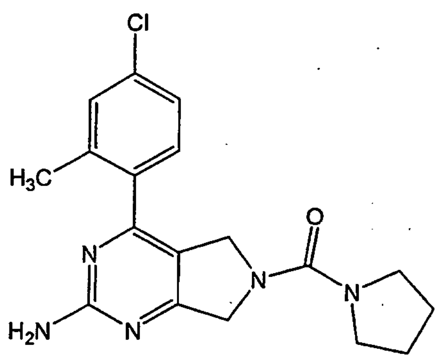 | 4-(4-chloro-2-methylphenyl)-6-(pyrrolidin-1-ylcarbonyl)-6,7-dihydro-5H-pyrrolo[3,4-d]pyrimidin-2-amine | Example 7 | (300 MHz, DMSO-D6) δ ppm 7.42 (s, 1H), 7.35-7.32 (m, 2H), 6.73 (br s, 2H), 4.53 (s, 2H), 4.39 (s, 2H), 3.32-3.27 (m, 4H), 2.24 (s, 3H), 1.79-1.73 (m, 4H). | 358.2 |

(continued)

| Ex. No. | Structure | Name | Synthetic Method | $^1$H NMR | MS |
|---|---|---|---|---|---|
| 41 | 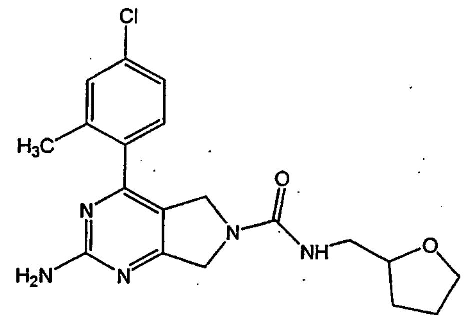 | 2-amino-4-(4-chloro-2-methylphenyl)-N-(tetrahydrofura n-2-ylmethyl)-5,7-dihydro-6H-pyrrolo[3,4-d]pyrimidine-6-carboxamide | Example 7 | (300 MHz, DMSO-D6) δ ppm 7.43 (br s, 1H), 7.35-7.32 (m, 2H), 6.75 (br s, 2H), 6.41 (t, J = 5.8 Hz, 1H), 4.41 (s, 2H), 4.26 (s, 2H), 3.87-3.80 (m, 1H), 3.76-3.69 (m, 1H), 3.62-3.54 (m, 1H), 3.07 (dt, J = 5.8 and 2.3 Hz, 2H), 2.23 (s, 3H), 1.88-1.72 (m, 3H), 1.58-1.50 (m, 1H), | 388.2 |
| 42 | 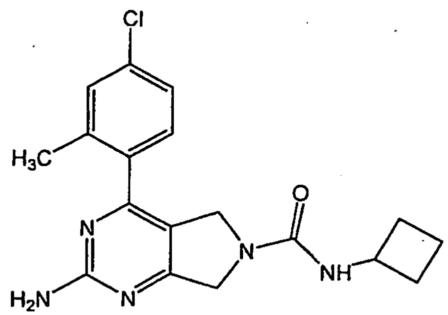 | 2-amino-4-(4-chloro-2-methylphenyl)-N-cyclobutyl-5,7-dihydro-6H-pyrrolo[3,4-d]pyrimidine-6-carboxamide | Example 7 | (300 MHz, DMSO-D6) δ ppm 7.43 (br s, 1H), 7.35-7.32 (m; 2H), 6.75 (br s, 2H), 6.50 (d, J = 8.1 Hz, 1H), 4.40 (s, 2H), 4.24 (s, 2H), 4.18-4.08 (m, 1H), 2.22 (s, 3H), 2.14-2.03 (m, 2H), 1.96-1.89 (m, 2H), 1.59-1.50 (m, 2H). | 358.2 |
| 43 | 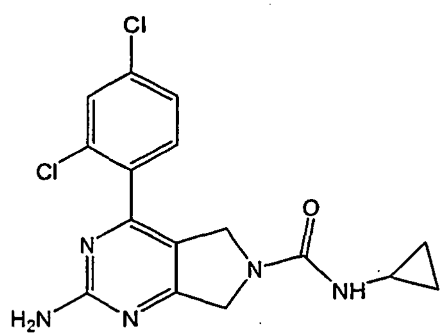 | 2-amino-N-cyclopropyl-4-(24-dichlorophenyl)-5,7-dihydro-6H-pyrrolo[3,4-d]pyrimidine-6-carboxamide | Example 7 | (300 MHz, DMSO-D6) δ ppm 7.79 (d, J = 1.9 Hz, 1H), 7.55 (dd, J = 8.3 and 2.0 Hz, 1H), 7.47 (d, J = 8.3 Hz, 1H), 6.87 (br s, 2H), 6.46 (d, J = 2.6 Hz, 1H), 4.40 (s, 2H), 4.22 (s, 2H), 0.57-0.50 (m, 2H), 0.41-0.36 (m, 2H). | 364.2, 366.2 |

(continued)

| Ex. No. | Structure | Name | Synthetic Method | $^1$H NMR | MS |
|---|---|---|---|---|---|
| 44 | 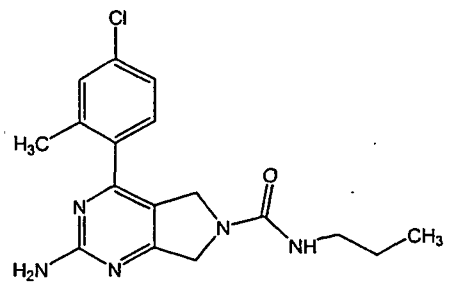 | 2-amino-4-(4-chloro-2-methylphenyl)-N-propyl-5,7-dihydro-6H- . pyrrolo[3,4-d]pyrimidine-6-carboxamide | Example 7 | (300 MHz, DMSO-D6) δ ppm 7.43 (br s, 1H), 7.34-7.32 (m, 2H), 6.77 (br s, 2H), 6.36 (t, J = 6.4 Hz, 1H), 4.40 (s, 2H), 4.24 (s, 2H), 2.98 (m, 2H), 2.23 (s, 3H), 1.47-1.34 (m, 2H), 0.82 (t, J = 7.4 Hz, 3H). | 346.2 |
| 45 | 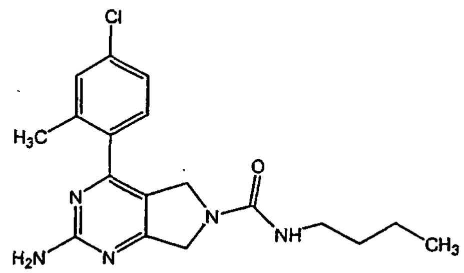 | 2-amino-N-butyl-4-(4-chloro-2-methylphenyl)-5,7-dihydro-6H-pyrrolo [3,4-d]pyrimidine-6-carboxamide | Example 7 | (300 MHz, DMSO-D6) δ ppm 7.42 (br s, 1H), 7.34-7.32 (m, 2H), 6.77 (br s, 2H), 6.32 (t, J = 5.5 Hz, 1H), 4.39 (s, 2H), 4.23 (s, 2H), 3.01 (m, 2H), 2.22 (s, 3H), 1.45-1.36 (m, 2H), 1.33-1.24 (m, 2H), 0.88 (t, J = 7.4 Hz, 3H | 360.2 |
| 46 | 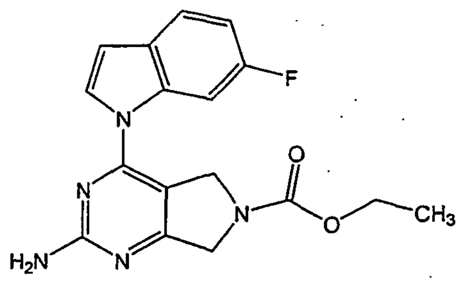 | ethyl 2-amino-4-(6-fluoro-1H-indol-1-yl)-5,7-dihydro-6H-pyrrolo[3,4-d] pyrimidine-6-carboxylate | Example 12 | (300 MHz, DMSO-D6) δ ppm 8.50-8.44 (m, 1H), 7.70-7.63 (m, 2H), 7.14-7.07 (m, 3H), 6.84 (d, J = 3.6 Hz, 1H), 4.82 (s, 1H), 4.79 (s, 1H), 4.47 (s, 1H), 4.44 (s, 1H), 4.13 (q, J = 7.2 Hz, 2H), 1.24 (t, J = 7.2 Hz, 3H) | 342.2 |

(continued)

| Ex. No. | Structure | Name | Synthetic Method | $^1$H NMR | MS |
|---|---|---|---|---|---|
| 47 | 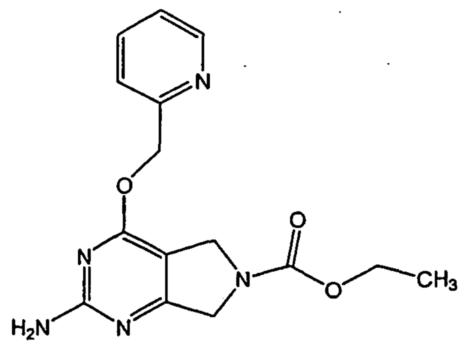 | ethyl 2-amino-4-(pyridin-2-ylmethoxyr 5,7-dihydro-6H-pyrrolo[3,4-d] pyrimidine-6-carboxylate | Example 11 | (300 MHz, DMSO-D6) (conformers) δ ppm 8.56 (m, 1H), 7.82 (dt, J = 7.7 and 1.7 Hz, 1H), 7.47 (d, J = 7.9 Hz, 1H), 7.36-7.32 (m, 1H), 6.72 (br s, 2H), 5.46 (s, 1H), 5.45 (s, 1H), 4.46-4.34 (m, 4H), 4.14-4.05 4m, 2H), 1.25-1.18 (m, 3H). | 316.2 |
| 48 | 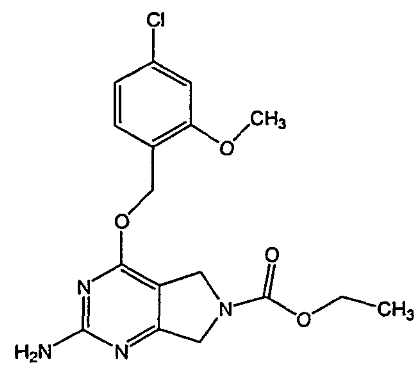 | ethyl 2-amino-4-[(4-chloro-2-methoxybenzyl)oxy]-5,7-dihydro-6H-pyrrolo[3,4-d]pyrimidine-6-carboxylate | Example 11 | (300 MHz, DMSO-D6) (conformers) δ ppm 7.41 (d, J = 8.1 Hz, 1H), 7.13 (d, J =2.0 Hz, 1H), 7.03 (dd, J = 8.1 and 1.8 Hz, 1H), 6.73 (br s, 2H), 5.32 (s, 1H), 5.31 (s, 1H), 4.40-4.32 (m, 4H), 4.12-4.05 (m, 2H),3.84 (s, 3H), 1.23-1.19 (m, 3H). | 379.2 |

(continued)

| Ex. No. | Structure | Name | Synthetic Method | $^1$H NMR | MS |
|---|---|---|---|---|---|
| 49 | 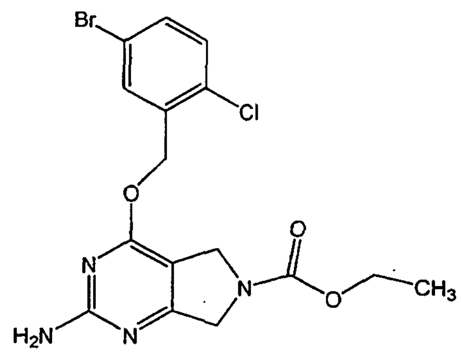 | ethyl 2-amino-4-[(5-bromo-2-chlorobenzyl)oxy]-5,7-dihydro-6H-pyrrolo[3,4-d]pyrimidine-6-carboxylate | Example 11 | (300 MHz, DMSO-D6) (conformers) δ ppm 7.78-7.76 (m, 1H), 7.62-7.59 (m, 1H), 7.49 (d, J = 8.6, 1H), 6.79 (brs, 2H), 5.42 (s, 1H), 5.41 (s, 1H), 4.42-4.33 (m, 4H), 4.12-4.06 (m, 2H), 1.23-1.19 (m, 3H). | 427.0, 429.0 |
| 50 | 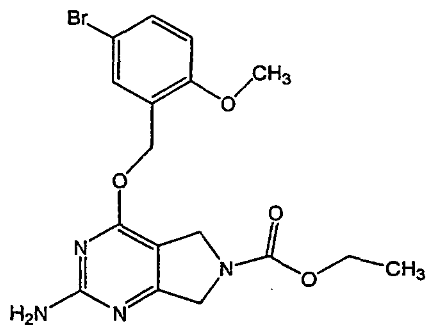 | ethyl 2-amino-4-[(5-bromo-2-methoxybenzyl)oxy]-5,7-dihydro-6H-pyrrolo[3,4-d]pyrimidine-6-carboxylate | Example 11 | (300 MHz, DMSO-D6) (conformers) δ ppm 7.53-7.48 (m, 2H), 7.05-7.00 (m, 1H), 6.75 (br s, 2H), 5.33 (s, 1H), 5.32 (s, 1H), 4.41-4.32 (m, 4H), 4.13-4.05 (m, 2H), 3.81 (s, 3H), 1.24-1.19 (m, 3H). | 423.2, 425.2 |
| 51 | 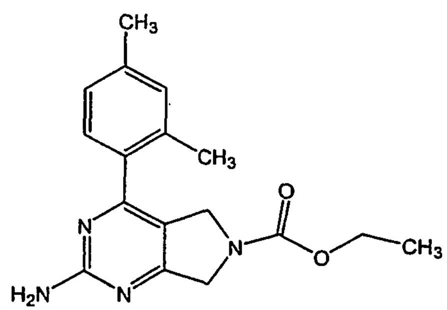 | ethyl 2-amino-4-(2,4-dimethylphenyl)-5,7-dihydro-6H-pyrrolo[3,4-d] pyrimidine-6-carboxylate | Example 1 | (300 MHz, DMSO-D6) (conformers) δ ppm 7.21-7.07 (m, 3H), 4.48 and 4.45 (2 overlapping br s, 2H), 4.32 and 4.29 (2 overlapping br s, 2H), 4.12-4.01 (m, 2H), 2.31 (s, 3H), 2.21 and 2.20 (2 overlapping s, 3H), 1.23-1.14 (m, 3H) | 313.2 |

(continued)

| Ex. No. | Structure | Name | Synthetic Method | $^1$H NMR | MS |
|---|---|---|---|---|---|
| 52 | 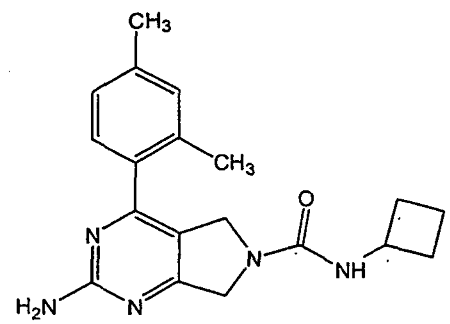 | 2-amino-N-cyclobutyl-4-(2,4-dimethylpheny l)-5,7-dihydro-6H-pyrrolo[3,4-d]pyrimidine-6-carboxamide | Example 7 | (300 MHz, DMSO-D6) δ ppm 7.17-7.06 (m, 3H), 6.67 (br s, 2H), 6.50 (d, J = 7.5 Hz, 1H), 4.38 (br s, 2H), 4.24 (br s, 2H), 4.16-4.08 (m, 1H), 2.31 (s, 3H), 2.19 (s, 3H), 2.13-2.04 (m, 2H), 1.99-1.85 (m, 2H), 1.58-1.48 (m, 2H). | 338.2 |
| 53 | 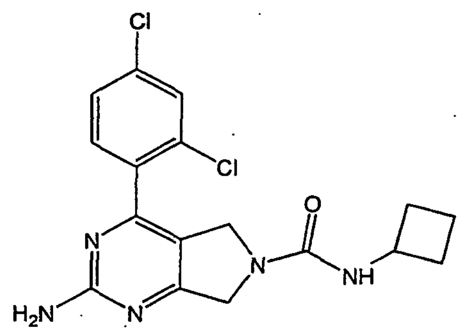 | 2-amino-N-cyclobutyl-4-(2,4-dichlorophenyl )-5,7-dihydro-6H-pyrrolo[3,4-d]pyrimidine-6-carboxamide | Example 3 | (300 MHz, DMSO-D6) δ ppm 7.80 (d, J = 2.0 Hz, 1H), 7.56 (dd, J = 8.1 and 2.0 Hz, 1H), 7.48 (d, J = 8.3 Hz, 1H), 6.89 (br s, 2H), 6.53 (d, J = 7.8 Hz, 1H), 4.40 (br s, 2H), 4.24 (br s, 2H), 4.17-4.07 (m, 1H), 2.12-2.05 (m, 2H), 1.97-1.86 (m, 2H), 1.58-1.48 (m, 2H) | 378 |
| 54 | 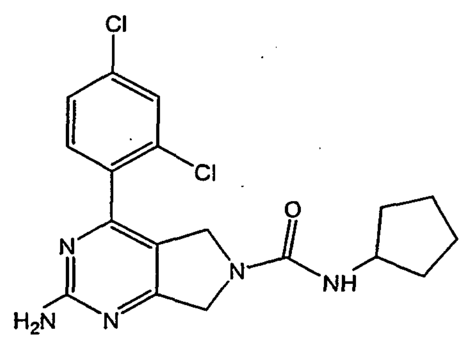 | 2-amino-N-cyclopentyl-4-(2,4-dichlorophenyl )-5,7-dihydro-6H-pyrrolo[3,4-dlpyrimidine-6-carboxamide | Example 15a | (300 MHz, DMSO-D6) δ ppm 7.80 (d, J = 2.0 Hz, 1H), 7.56 (dd, J = 8.3 and 2.0 Hz, 1H), 7.48 (d, J = 8.3 Hz, 1H), 6.88 (br s, 2H), 6.13 (d, J = 7.3 Hz, 1H), 4.42 (br s, 2H), 4.25 (br s, 2H), 3.97-3.88 (m, 1H), 1.81-1.73 (m, 2H), 1.65-1.57 (m, 2H), 1.50-1.34 (m, 4H) | 378.2, 380.2 |

(continued)

| Ex. No. | Structure | Name | Synthetic Method | $^1$H NMR | MS |
|---|---|---|---|---|---|
| 55 | 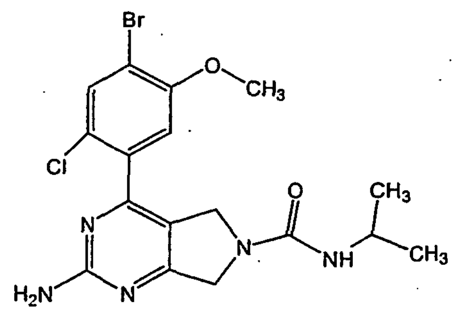 | 2-amino-4-(4-bromo-2-chloro-5-methoxyphenyl)-N-isopropyl-5,7-dihydro-6H-pyrrolo[3,4-d]pyrimidine-6-carboxamide | Example 15a | (300 MHz, DMSO-D6) δ ppm 7.86 (s.1H), 7.16 (s, 1H), 6.89 (br s, 2H), 6.07 (d, J = 7.7 Hz, 1H), 4.42 (br s, 2H), 4.25 (br s, 2H), 3.86 (s, 3H), 3.82-3.75 (m, 1H), 1.05 (d, J = 6.4 Hz, 6H) | 440.2, 442.2 |
| 56 | 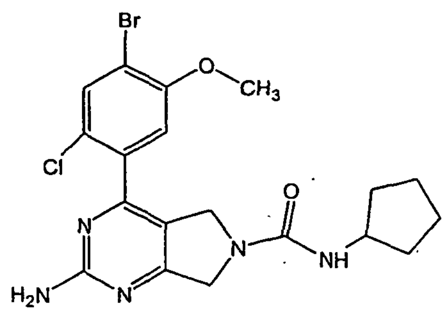 | 2-amino-4-(4-bromo-2-chloro-5-methoxyphenyl)-N-cyclopentyl-5,7-dihydro-6H-pyrrolo[3,4-d]pyrimidine-6-carboxamide | Example 15a | (300 MHz, DMSO-D6) δ ppm 7.86 (s, 1H), 7.16 (s, 1H), 6.90 (br s, 2H), 6.13 (d, J = 7.5 Hz, 1H), 4.42 (s, 2H), 4.26 (s, 2H), 3.97-3.87 (m, 1H), 3.86 (s, 3H), 1.84-1.71 (m, 2H), 1.67-1.56 (m, 2H), 1.52-1.32 (m, 4H) | 466.2, 468.2 |
| 57 | 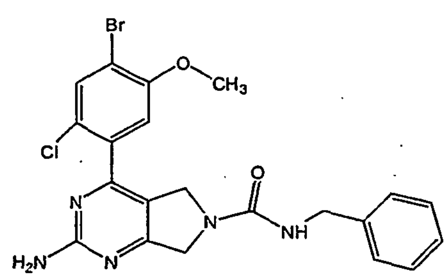 | 2-amino-N-benzyl-4-(4-bromo-2-chloro-5-methoxyphenyl)-5,7-dihydro-6H-pyrrolo[3,4-d]pyrimidine-6-carboxamide | Example 15a | (300 MHz, DMSO-D6) δ ppm 7.86 (s, 1H), 7.31-7.24 (m, 4H), 7.22-7.17 (m, 1H), 7.17 (s, 1H), 7.00 (t, J = 5.7 Hz, 1H), 6.91 (br s, 2H), 4.47 (s, 2H), 4.33 (s, 2H), 4.25 (d, J = 5.7 Hz, 2H), 3.86 (s, 3H) | 488.2, 490.2 |

(continued)

| Ex. No. | Structure | Name | Synthetic Method | $^1$H NMR | MS |
|---|---|---|---|---|---|
| 58 | 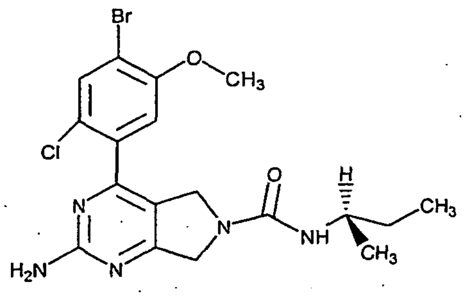 | 2-amino-4-(4-bromo-2-chloro-5-methoxyphenyl)-N-[(1R)-1-methylpropyl]-5,7-dihydro-6H-pyrrolo [3,4-d]pyrimidine-6-carboxamide | Example 3 | (300 MHz, DMSO-D6) δ ppm 7.86 (s, 1H), 7.17 (s, 1H), 6.90 (br s, 2H), 6.00 (d, J = 7.8 Hz, 1H), 4.43 (s, 2H), 4.27 (s, 2H), 3.86 (s, 3H), 3.62-3.49 (m, overlaps with H2O peak, 1H), 1.46-1.31 (m, 2H), 1.02 (d, J = 6.6 Hz, 3H), 0.81 (t, J = 7.3 Hz, 3H) | 454.2, 456.2 |
| 59 | 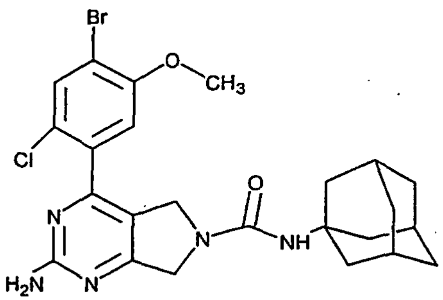 | N-1-adamantyl-2-amino-4-(4-bromo-2-chloro-5-methoxypheny l)-5,7-dihydro-6H-pyrrolo[3,4-d]pyrimidine-6-carboxamide | Example 15a | (300 MHz, DMSO-D6) δ ppm 7.86 (s, 1H), 7.16 (s, 1H), 6.97 (br s, 2H), 5.45 (s, 1H), 4.41 (s, 2H), 4.26 (s, 2H), 3.86 (s, 3H), 1.99 (br s, 3 H), 1.94 (br s, 6H), 1.60 (br s, 6H) | 532.2, 534.2 |
| 60 | 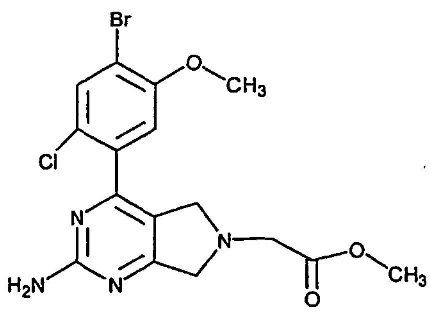 | methyl [2-amino-4-(4-bromo-2-chloro-5-methoxyphenyl)-57-dihydro-6H-pyrrolo[34-d]pyrimidin-6-yl]acetate | Example 5 | (300 MHz, CDCl3) δ ppm 7.66 (s, 1H), 6.98 (s, 1H), 5.76 (br s, 2H), 4.45 (br s, 2H), 4.38 (br s, 2H), 3.97 (s, 3H), 3.88 (s, 2H), 3.82 (s, 3 H) | 427.2, 429.2 |

(continued)

| Ex. No. | Structure | Name | Synthetic Method | $^1$H NMR | MS |
|---|---|---|---|---|---|
| 61 | 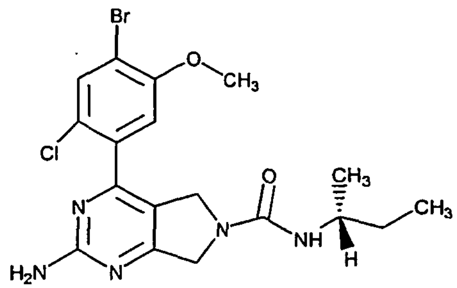 | 2-amino-4-(4-bromo-2-chloro-5-methoxyphenyl)-N-[(1S)-1-methylpropyl]-5,7-dihydro-6H-pyrrolo[3,4-d]pyrimidine-6-carboxamide | Example 3 | (300 MHz, DMSO-D6) δ ppm 7.86 (s, 1H), 7.17 (s, 1H), 6.89 (br s, 2H), 6.00 (d, J = 8.3 Hz, 1H), 4.42 (s, 2H), 4.26 (s, 2H), 3.86 (s, 3H), 3.64-3.51 (m, overlaps with H2O peak, 1H), 1.47-1.30 (m, 2H), 1.02 (d, J = 6.4 Hz, 3H), 0.80 (t, J = 7.3 Hz, 3H). | 454.2, 456.2 |
| 62 | 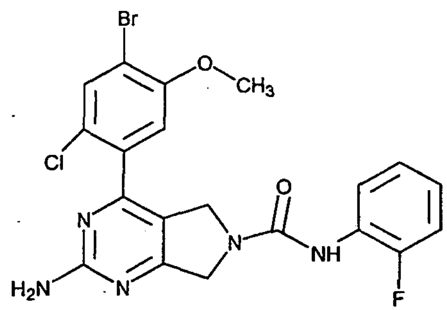 | 2-amino-4-(4-bromo-2-chloro-5-methoxyphenyl)-N-(2-fluorophenyl)-5,7-dihydro-6H-pyrrolo[3,4-d]pyrimidine-6-carboxamide | Example 15a | (300 MHz, DMSO-D6) δ ppm 8.18 (s, 1H), 7.88 (s, 1H), 7.50-7.44 (m, 1H), 7.23-7.10 (m, 4H), 6.95 (br s, 2H), 4.61 (br s, 2H), 4.48 (br s, 2H), 3.88 (s, 3H). | 492.2, 494.2 |
| 63 | 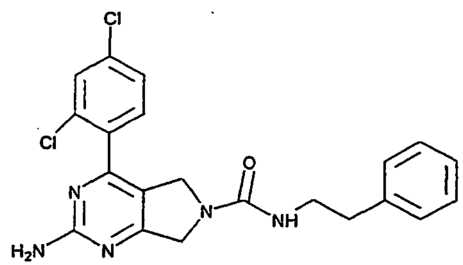 | 2-amino-4-(2,4-dichlorophenyl)-N-(2-phenylethyl)-5,7-dihydro-6H-pyrrolo[3,4-d]pyrimidine-6-carboxamide | Example 15a | (300 MHz, DMSO-D6) δ ppm 7.80 (s, 1H), 7.57 (d, J = 8.3 Hz, 1 H), 7.49 (d, J = 8.1 Hz, 1H), 7.32-7.26 (m, 2H), 7.23-7.15 (m, 3H), 6.90 (br s, 2H), 6.52 (m, 1H), 4.42 (br s, 2H), 4.25 (br s, 2H), 3.32-3.19 (m, overlaps with H2O peak, 2H), 2.72 (t, J = 7.3 Hz, 2H). | 428.2, 430.2 |

(continued)

| Ex. No. | Structure | Name | Synthetic Method | $^1$H NMR | MS |
|---|---|---|---|---|---|
| 64 | 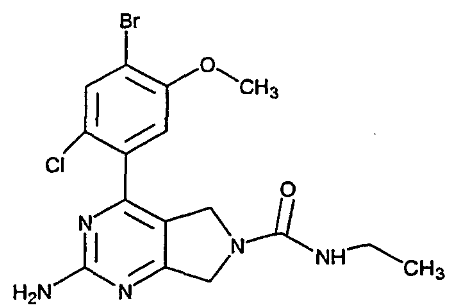 | 2-amino-4-(4-bromo-2-chloro-5-methoxyphenyl)-N-ethyl-5,7-dihydro-6H-pyrrolo[3,4-d]pyrimidine-6-carboxamide | Example 15a | (300 MHz, DMSO-D6) δ ppm 7.86 (s, 1H), 7.17 (s, 1H), 6.90 (br s, 2H), 6.36 (t, J = 5.2 Hz, 1H), 4.42 (br s, 2H), 4.26 (br s, 2H), 3.86 (s, 3H), 3.10-3.01 (m, 2H), 1.01 (t, J = 7.2 Hz, 3H). | 426.2, 428.2 |
| 65 | 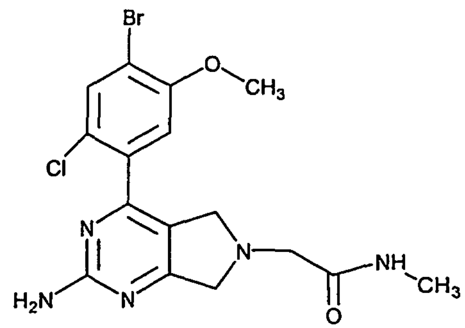 | 2-[2-amino-4-(4-bromo-2-chloro-5-methoxyphenyl)-5,7-dihydro-6H-pyrrolo[34-d]pyrimidin-6-yl]-N-methylacetami de | Example 5 | (300 MHz, DMSO-D6) δ ppm 7.82 (s, 1H), 7.81-7.75 (m, 1H), 7.07 (s, 1H), 6.76 (br s, 2H), 3.85 (s, 5H), 3.73 (s, 2H), 3.28 (s, 2H), 2.57 (d, J = 4.7 Hz, 3H). | 426.2, 428.2 |
| 66 | 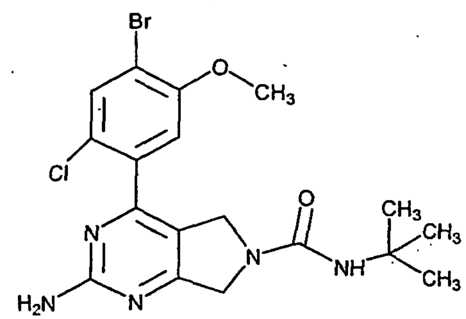 | 2-amino-4-(4-bromo-2-chloro-5-methoxyphenyl)-N-(tert-butyl)-5,7-dihydro-6H-pyrrolo[3,4-d]pyrimidine-6-carboxamide | Example 15a | (300 MHz, DMSO-D6) δ ppm 7.86 (s, 1H), 7.16 (s, 1H), 6.88 (br s, 2H), 5.58 (s, 1H), 4.42 (s, 2H), 4.26 (s, 2H), 3.86 (s, 3H), 1.26 (s, 9H). | 454.2, 456.2 |

(continued)

| Ex. No. | Structure | Name | Synthetic Method | $^1$H NMR | MS |
|---|---|---|---|---|---|
| 67 | 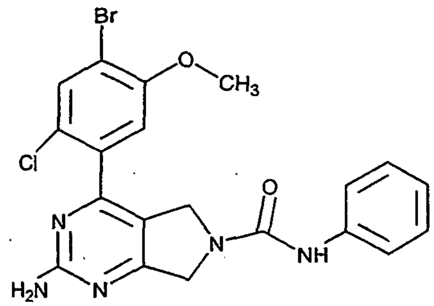 | 2-amino-4-(4-bromo-2-chloro-5-methoxyphenyl)-N-phenyl-5,7-dihydro-6H-pyrrolo[3,4-d]pyrimidine-6-carboxamide | Example 15a | (300 MHz, DMSO-D6) δ ppm 8.34 (s, 1H), 7.89 (s, 1H), 7.51 (d, J = 7.9 Hz, 2H), 7.26-7.19 (m, 3H), 6.98-6.91 (m, 3H), 4.60 (s, 2H), 4.48 (s, 2H), 3.88 (s, 3H). | 474.2, 476.2 |
| 68 | 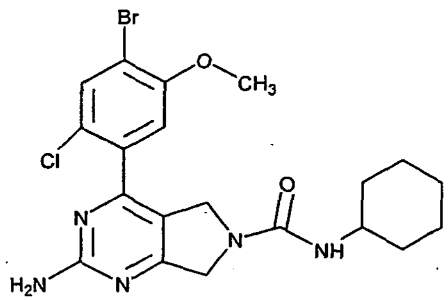 | 2-amino-4-(4-bromo-2-chloro-5-methoxyphenyl)-N-cyclohexyl-5,7-dihydro-6H-pyrrolo[3,4-d]pyrimidine-6-carboxamide | Example 15a | (300 MHz, DMSO-D6) δ ppm 7.86 (s, 1H), 7.16 (s, 1H), 6.89 (br s, 2H), 6.07 (d, J = 7.9 Hz, 1H), 4.42 (s, 2H), 4.25 (s, 2H), 3.49-3.44 (m, under H2O peak, 1H), 3.86 (s, 3H), 1.78-1.00 (M, 10H), | 480.2, 482.2 |
| 69 | 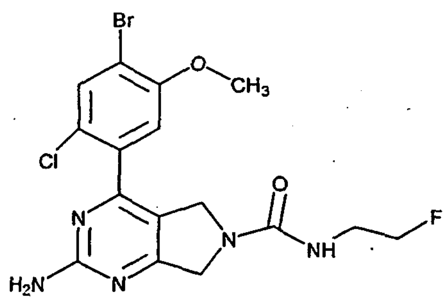 | 2-amino-4-(4-bromo-2-chloro-5-methoxyphenyl)-N-(2-fluoroethyl)-5,7-dihydro-6H-pyrrolo[3,4-d]pyrimidine-6-carboxamide | Example 3 | (300 MHz, DMSO-D6) δ ppm 7.87 (s, 1H), 7.17 (s, 1H), 6.91 (br s, 2H), 6.65 (t, J = 5.4 Hz, 1H), 4.50-4.43 (m, 3H), 4.35-4.28 (m, 3H), 3.86 (s, 3H), 3.36-3.25 (m, under H2O peak, 2H). | 444.2, 446.2 |

(continued)

| Ex. No. | Structure | Name | Synthetic Method | $^1$H NMR | MS |
|---|---|---|---|---|---|
| 70 | 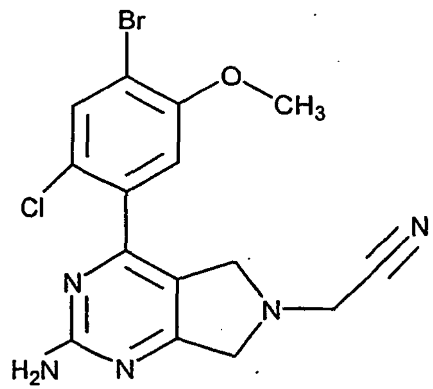 | [2-amino-4-(4-bromo-2-chloro-5-methoxyphenyl)-5,7-dihydro-6H-pyrrolo[3,4-d]pyrimidin-6-y]acetonitrile | Example 5 | (300 MHz, DMSO-D6) δ ppm 7.84 (s, 1H), 7.14 (s, 1H), 6.87 (br s, 2H), 3.96 (s, 2H), 3.86 (s, 3H), 3.85 (s, 2H), 3.74 (s, 2H). | 394.2, 396.2 |
| 71 | 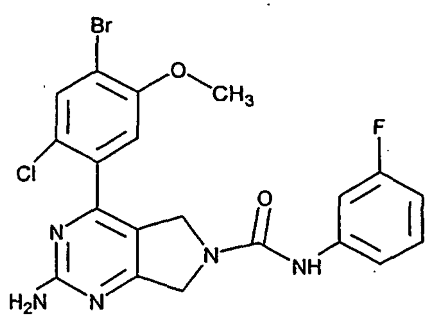 | 2-amino-4-(4-bromo-2-chloro-5-methoxyphenyl)-N-(3-fluorophenyl)-57-dihydro-6H-pyrrolo[34-d] pyrimidine-6-carboxamide | Example 15a | (300 MHz, DMSO-D6) δ ppm 8.54 (s, 1H), 7.88 (s, 1H), 7.50 (dt, J = 12.2 and 1.9 Hz, 1H), 7.33-7.21 (m, 2H), 7.19 (s, 1H), 6.95 (br s, 2H), 6.78-6.72 (m, 1H), 4.60 (s, 2H), 4.48 (s, 2H), 3.87 (s, 3H). | 492.2, 494.2 |
| 72 | 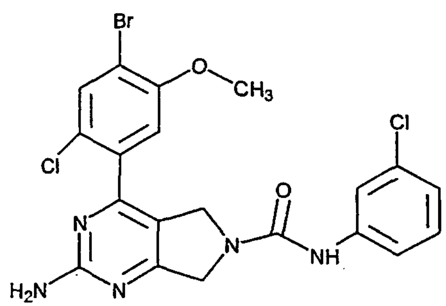 | 2-amino-4-(4-bromo-2-chloro-5-methoxyphenyl)-N-(3-chlorophenyl)-5,7-dihydro-6H-pyrrolo[3,4-d] pyrimidine-6-carboxamide | Example 15a | (300 MHz, DMSO-D6) δ ppm 8.52 (s, 1H), 7.88 (s, 1H), 7.70 (t, J = 1.8 Hz, 1H), 7.48 (d, J = 7.5 Hz, 1H), 7.25 (t, J = 8.10 Hz, 1H), 7.19 (s, 1H), 7.01-6.94 (m, 3H), 4.60 (s, 2H), 4.47 (s, 2H), 3.87 (s, 3H). | 508.0, 510.2, 512.0 |

(continued)

| Ex. No. | Structure | Name | Synthetic Method | $^1$H NMR | MS |
|---|---|---|---|---|---|
| 73 | 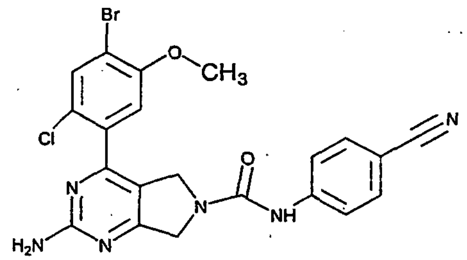 | 2-amino-4-(4-bromo-2-chloro-5-methoxyphenyl)-N-(4-cyanophenyl)-5,7-dihydro-6H-pyrrolo[3,4-d] pyrimidine-6-carboxamide | Example 15a | (300 MHz, DMSO-D6) δ ppm 8.80 (s, 1H), 7.89 (s, 1H), 7.76 (d, J = 8.9 Hz, 2H), 7.69 (d, J = 8.9 Hz, 2H), 7.19 (s, 1H), 6.97 (br s, 2H), 4.63 (s, 2H), 4.51 (s, 2H), 3.87 (s, 3H). | 499.2, 501.2 |
| 74 | 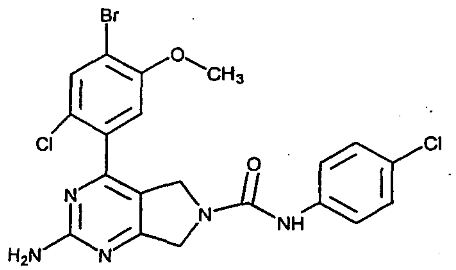 | 2-amino-4-(4-bromo-2-chloro-5-methoxyphenyl)-N-(4-chlorophenyl)-5,7-dihydro-6H-pyrrolo[3,4-d] pyrimidine-6-carboxamide | Example 15a | (300 MHz, DMSO-D6) δ ppm 8.48 (s, 1H), 7.89 (s, 1H), 7.56 (d, J = 8.9 Hz, 2H), 7.29 (d, J = 8.9 Hz, 2H), 7.19 (s, 1H), 6.95 (br s, 2H), 4.60 (s, 2H), 4.47 (s, 2H), 3.87 (s, 3H). | 508.0, 510.0, 512.0 |
| 75 | 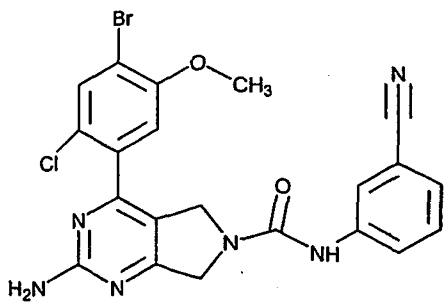 | 2-amino-4-(4-bromo-2-chloro-5-methoxyphenyl)-N-(3-cyanophenyl)-5,7-dihydro-6H-pyrrolo[3,4-d] pyrimidine-6-carboxamide | Example 15a | (300 MHz, DMSO-D6) δ ppm 8.69 (s, 1H), 8.01-7.99 (m, 1H), 7.89 (s, 1H). 7.85-7.80 (m, 1H), 7.49-7.38 (m, 2H), 7.20 (s, 1H), 6.97 (br s, 2H), 4.61 (s, 2H), 4.49 (s, 2H), 3.88 (s, 3H). | 499.2, 501.2 |

(continued)

| Ex. No. | Structure | Name | Synthetic Method | $^1$H NMR | MS |
|---|---|---|---|---|---|
| 76 | 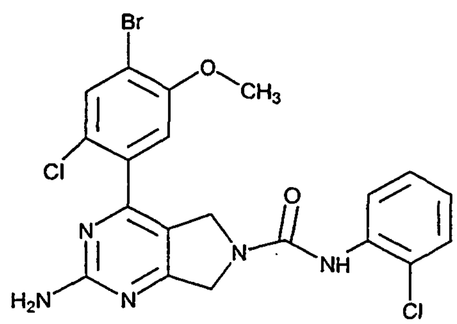 | 2-amino-4-(4-bromo-2-chloro-5-methoxyphenyl)-N-(2-chlorophenyl)-5,7-dihydro-6H-pyrrolo[3,4-d] pyrimidine-6-carboxamide | Example 15a | (300 MHz, DMSO-D6) δ ppm 8.07 (s, 1H), 7.87 (s, 1H), 7.55 (d, J = 7.5 Hz, 1H), 7.46 (d, J = 7.4 Hz, 1H), 7.29 (t, J = 7.3 Hz, 1H), 7.20 (s, 1H), 7.15 (t, J = 7.5 Hz, 1H), 6.95 (br s, 2H), 4.61 (s, 2H), 4.49 (s, 2H), 3.87 (s, 3H). | 508.0, 510.0, 512.0 |
| 77 | 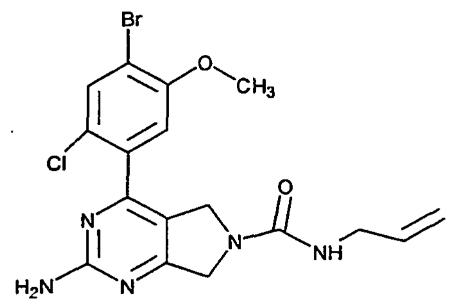 | N-allyl-2-amino-4-(4-bromo-2-chloro-5-methoxyphenyl)-5,7-dihydro-6H-pyrrolo[3,4-d]pyrimidine-6-carboxamide | Example 15a | (300 MHz, DMSO-D6) δ ppm 7.86 (s, 1H), 7.17 (s, 1H), 6.92 (br s, 2H), 6.59 (t, J = 5.4 Hz, 1H), 5.87-5.74 (m, 1H), 5.12-4.97 (m, 2H), 4.45 (s, 2H), 4.30 (s, 2H), 3.86 (s, 3H), 3.67 (m, 2H). | 438.2, 440.2 |
| 78 | 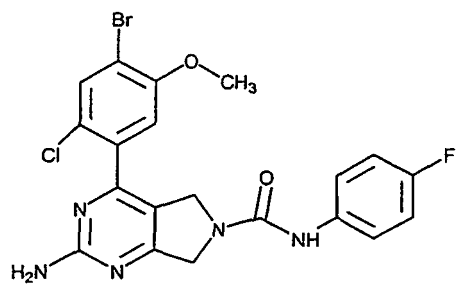 | 2-amino-4-(4-bromo-2-chloro-5-methoxyphenyl)-N-(4-fluorophenyl)-5,7-dihydro-6H-pyrrolo[3,4-d] pyrimidine-6-carboxamide | Example 15a | (300 MHz, DMSO-D6) δ ppm 8.40 (s, 1H), 7.88 (s, 1H). 7.54-7.47 (M, 2H), 7.19 (s, 1H), 7.07 (t, J = 8.7 Hz, 2H), 6.95 (br s, 2H), 4.59 (s, 2H), 4.46 (s, 2H), 3.87 (s, 3H). | 492.2, 494.2 |

(continued)

| Ex. No. | Structure | Name | Synthetic Method | $^1$H NMR | MS |
|---|---|---|---|---|---|
| 79 | 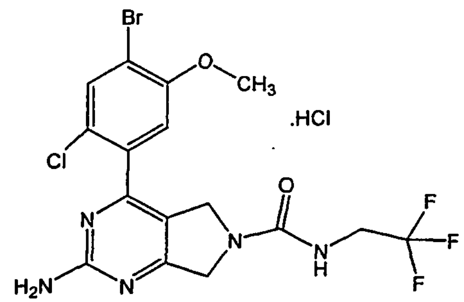 | 2-amino-4-(4-bromo-2-chloro-5-methoxyphenyl)-N-(2,2,2-trifluoroethyl)-5,7-dihydro-6H-pyrrolo[3,4-d]pyrimidine-6-carboxamide hydrochloride | Example 3 | (300 MHz, DMSO-D6) δ ppm 7.86 (s, 1H), 7.17 (s, 1H). 7.09 (t, J = 6.0 Hz, 1H), 6.93 (br s, 2H), 4.47 (s, 2H), 4.32 (s, 2H), 3.86 (s, 3H), 3.86-3.76 (m, 2H). | 480.2, 482.2 |
| 80 | 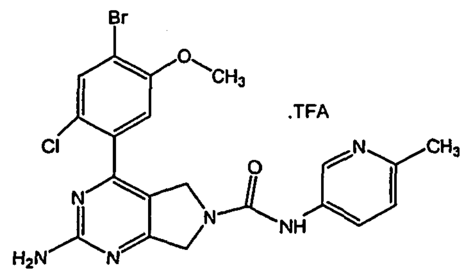 | 2-amino-4-(4-bromo-2-chloro-5-methoxyphenyl)-N-(6-methylpyridin-3-yl)-5,7-dihydro-6H-pyrrolo[3,4-d]pyrimidine-6-carboxamide trifluoroacetate | Example 3 | (300 MHz, DMSO-D6) δ ppm 8.98 (s, 1H), 8.84 (s, 1H), 8.23 (d, J = 8.5 Hz, 1H), 7.90 (s, 1H), 7.61 (d, J = 8.3 Hz, 1H), 7.20 (s, 1H), 6.99 (br s, 2H), 4.63 (s, 2H), 4.51 (s, 2H), 3.88 (s, 3H), 2.55 (s, 3H). | 489.2, 491.2 |
| 81 | 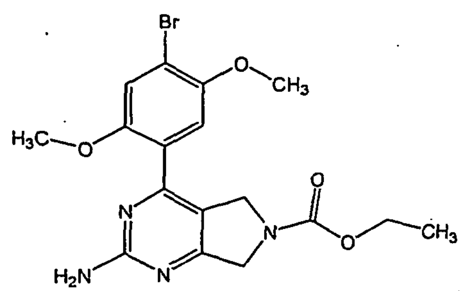 | ethyl 2-amino-4-(4-bromo-2,5-dimethoxyphenyl)-5,7-dihydro-6H-pyrrolo[3,4-d]pyrimidine-6-carboxylate | Example 1 | (300 MHz, DMSO-D6) (conformers) δ ppm 7.39 (s, 1H), 7.05 (s, 1H), 6.82 (br s, 2H), 4.45 (br s, 1H), 4.43 (br s, 1H), 4.31 (br s, 1H), 4.29 (br s, 1H), 4.12-4.02 (m, 2H), 3.79 (s, 3H), 3.77 (s, 3H), 1.23-1.15 (m, 3H). | 423.2, 425.2 |

(continued)

| Ex. No. | Structure | Name | Synthetic Method | $^1$H NMR | MS |
|---|---|---|---|---|---|
| 82 | 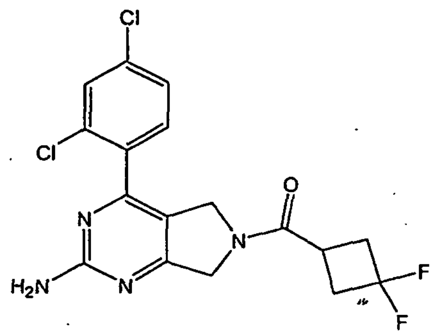 | 4-(2,4-dichlorophenyl )-6-[(3,3-difluorocyclob utyl)carbonyl]-6,7-dihydro-5H-pyrrolo[3,4-d]pyrimidin-2-amine | Example 6 | (400 MHz, DMSO-d6) δ ppm 2.68 - 2.88 (m, 4 H) 3.14 - 3.28 (m, 1H) 4.35 (s, 1H) 4.52 (d, J=14.40 Hz, 2 H) 4.69 (s, 1 H) 6.95 (s, 2 H) 7.44 - 7.52 (m, 1 H) 7.53 - 7.61 (m, 1H) 7.80 (d, J=1.77 Hz, 1H). | 399, 401 |
| 83 | 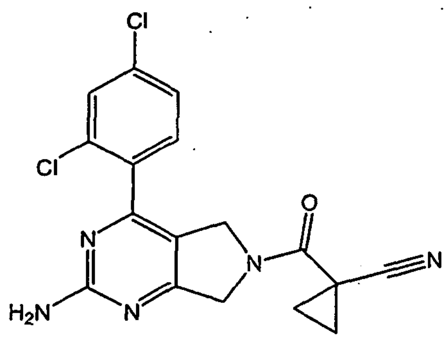 | 1-{[2-amino-4-(2,4-dichlorophenyl )-5,7-dihydro-6H-pyrrolo[3,4-d] pyrimidin-6-yl]carbonyl} cyclopropanecarbonitrile | Example 6 | (400 MHz, DMSO-d6) δ ppm 1.47 - 1.67 (m, 4 H) 4.49 (d, J=58.86 Hz, 2 H) 4.94 (d, J=43.96 Hz, 2 H) 7.00 (s, 2 H) 7.42 - 7.63 (m, 2 H) 7.81 (dd, J=17.94, 2.02 Hz, 1H). | 374, 376 |
| 84 | 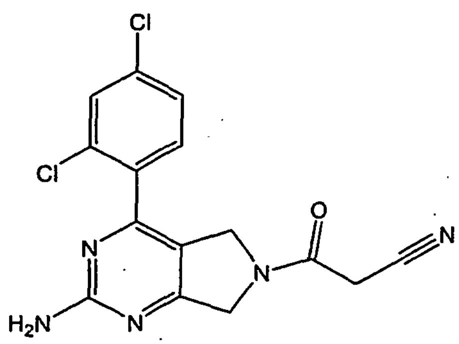 | 3-[2-amino-4-(2,4-dichlorophenyl )-5,7-dihydro-6H-pyrrolo[3,4-d] pyrimidin-6-yl]-3-oxopropanenit rile | Example 6 | (400 MHz, DMSO-d6) δ ppm 4.03 (d, J=27.79 Hz, 2 H) 4.35 (s, 1 H) 4.51 (d, J=3.54 Hz, 2 H) 4.68 (s, 1 H) 6.99 (s, 2 H) 7.48 (dd, J=8.34, 6.32 Hz, 1H) 7.52 - 7.61 (m, 1H) 7.80 (dd, J=7.33, 2.02 Hz, 1H). | 348, 350 |

(continued)

| Ex. No. | Structure | Name | Synthetic Method | $^1$H NMR | MS |
|---|---|---|---|---|---|
| 85 | 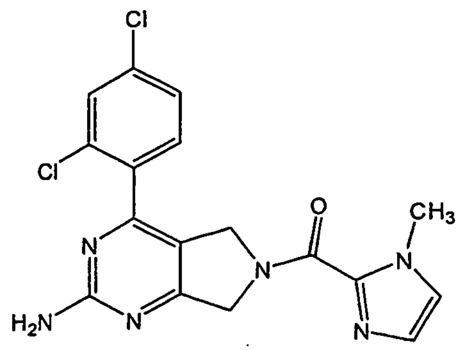 | 4-(2,4-dichlorophenyl )-6-[(1-methyl-1H-imidazol-2-yl)carbonyl]-6,7-dihydro-5H-pyrrolo[3,4-d]pyrimidin-2-amine | Example 6 | (400 MHz, DMSO-d6) δ ppm 3.86 (d, J=1.77 Hz, 3 H) 4.62 (d, J=61.39 Hz, 2 H) 5.04 (d, J=61.64 Hz, 2 H) 6.94 (s, 2 H) 7.03 (dd, J=34.61, 1.01 Hz, 1H) 7.35 (d, J=14.91 Hz, 1H) 7.44 - 7.52 (m, 1 H) 7.54 - 7.62 (m, 1H) 7.80 (d, J=1.77 Hz, 1H). | 389, 391 |
| 86 | 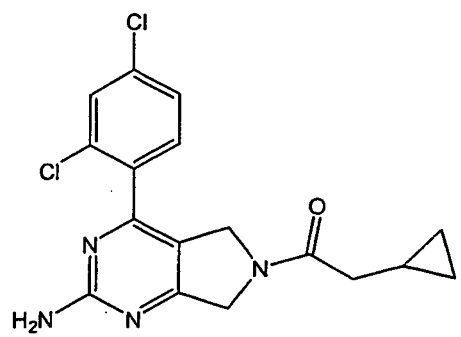 | 6-(cyclopropylacetyl)-4-(2,4-dichlorophenyl)-6,7-dihydro-5H-pyrrolo[3,4-d]pyrimidin-2-amine | Example 6 | (400 MHz, DMSO-d6) δ ppm 0.01 - 0.18 (m, 2 H) 0.36 - 0.52 (m, 2 H) 0.99 (dd, J=7.33, 4.80 Hz, 1 H) 2.25 (dd, J=30.06, 6.57 Hz, 2 H) 4.31 (s, 1H) 4.48 (d, J=9.85 Hz, 2 H) 4.67 (s, 1H) 6.93 (s, 2 H) 7.43 - 7.51 (m, 1H) 7.52 - 7.61 (m, 1H) 7.79 (d, J=1.26 Hz, 1H). | 363, 365 |
| 87 | 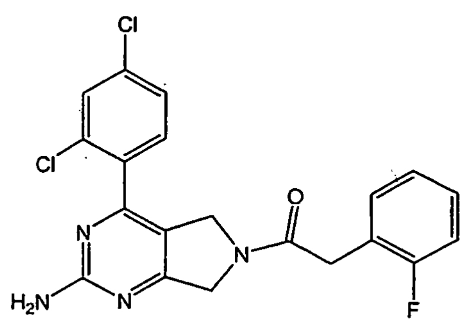 | 4-(2,4-dichlorophenyl )-6-[(2-fluorophenyl)a cetyl]-6,7-dihydro-5H-pyrrolo[3,4-d]pyrimidin-2-amine | Example 6 | (400 MHz, DMSO-d6) δ ppm 3.77 (d, J=32.34 Hz, 2 H) 4.35 (s, 1 H) 4.51 (s, 1H) 4.70 (s, 1H) 4.86 (s, 1H) 6.97 (s, 2 H) 7.08 - 7.21 (m, 2 H) 7.24 - 7.37 (m, 2 H) 7.46 - 7.63 (m, 2 H) 7.80 (dd, J=9.85, 1.77 Hz, 1H). | 417, 419 |

(continued)

| Ex. No. | Structure | Name | Synthetic Method | $^1$H NMR | MS |
|---|---|---|---|---|---|
| 88 | 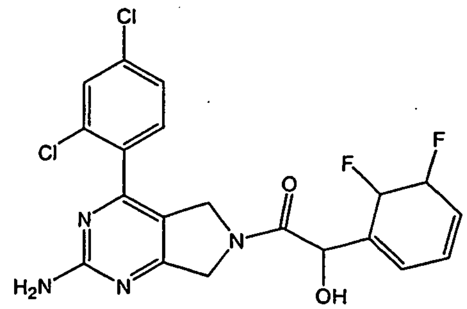 | 2-[2-amino-4-(2,4-dichlorophenyl )-5,7-dihydro-6H-pyrrolo[3,4-d] pyrimidin-6-yl]-1-(2,3-difluorophenyl)-2-oxoethanol | Example 6 | (400 MHz, DMSO-d6) δ ppm 4.44 - 4.60 (m, 2 H) 4.75 - 5.02 (m, 2 H) 5.59 (dd, J=26.02, 7.07 Hz, 1H) 6.18 (dd, J=6.82, 2.27 Hz, 1 H) 6.96 (d, J=8.59 Hz, 2 H) 7.16 - 7.33 (m, 2 H) 7.33 - 7.44 (m, 1H) 7.44 - 7.54 (m, 1 H) 7.53 - 7.61 (m, 1H) 7.79 (dd, | 451, 453 |
| 89 | 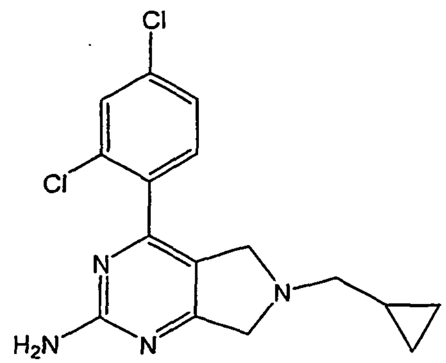 | 6-(cyclopropylmethyl)-4-(2,4-dichlorophenyl)-6,7-dihydro-5H-pyrrolo[3,4-d]pyrimidin-2-amine | Example 5 | (400 MHz, MeOH-d4) δ ppm 0.29 (d, J=6.06 Hz, 2 H) 0.55 - 0.69 (m, 2 H) 0.93 - 1.10 (m, 1 H) 2.84 (d, J=7.07 Hz, 2 H) 4.04 (s, 2 H) 4.14 (s, 2 H) 7.35 - 7.54 (m, 2 H) 7.64 (d, J=1.77 Hz, 1 H). | 335, 337 |
| 90 | 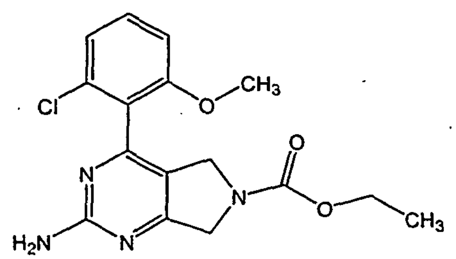 | ethyl 2-amino-4-(2-chloro-6-methoxyphenyl)-5,7-dihydro-6H-pyrrolo[3,4-d]pyrimidine-6-carboxylate | Example 1 | (400 MHz, DMSO-D6) δ ppm 7.45 (dd, 1H, J = 8.4, 8.3 Hz), 7.16-7.12 (m, 2H), 6.84 (br s, 2H), 4.55-4.45 (m, 2H), 4.20-3.98 (m, 4H), 3.74 (s, 3H), 1.27-1.12 (m, 3H) | 349.2 351.2 |

(continued)

| Ex. No. | Structure | Name | Synthetic Method | $^1$H NMR | MS |
|---|---|---|---|---|---|
| 91 | 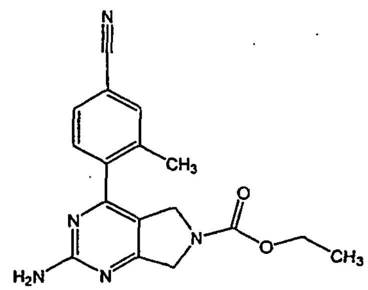 | ethyl 2-amino-4-(4-cyano-2-methylphenyl)-5,7-dihydro-6H-pyrrolo [3,4-d]pyrimidine-6-carboxylate | Example 1 | (400 MHz, DMSO-D6) δ ppm 7.84 (s, 1H), 7.76 (d, 1H, J = 7.9 Hz), 7.68-7.48 (m, 1H), 6.89 (br s, 2H), 4.55-4.40 (m, 2H), 4.35-4.20 (m, 2H), 4.15-3.95 (m, 2H), 2.26 (s, 3H), 1.27-1.12 (m, 3H) | 324 |
| 92 | 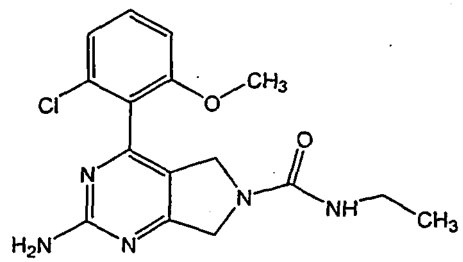 | 2-amino-4-(2-chloro-6-methoxyphenyl)-N-ethyl-5,7-dihydro-6H-pyrrolo[3,4-d]pyrimidine-6-carboxamide | Example 15a | (400 MHz, DMSO-D6) δ ppm 7.44 (dd, 1H, J = 8.4, 8.3 Hz), 7.16-7.12 (m, 2H), 6.79 (br s, 2H), 6.36 (t, 1 H); 4.42 (br s, 2H), 4.10 (br s, 2H), 3.74 (s, 3H), 3.08-2.95 (m, 2H), 1.00 (t, 3H) | 348.2, 350.2 |
| 93 | 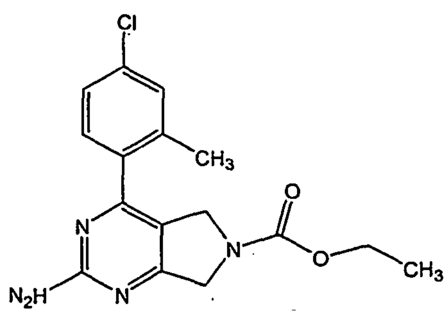 | ethyl 2-amino-4-(4-chloro-2-methylphenyl)-57-dihydro-6H-pyrrolo [34-d]pyrimidine-6-carboxylate | Example 1 | (400 MHz, DMSO-D6) δ ppm 7.43 (s, 1H), 7.34 (s, 2H), 6.82 (bs, 2H), 4.47 (d, 2H, J=8), 4.32 (d, 2H, J= 12 Hz), 4.07 (m, 2H), 2.24 (s, 3H), 1.22 (t, 3H, J= 7.1 Hz). | 333.2, 335.2 |

(continued)

| Ex. No. | Structure | Name | Synthetic Method | $^1$H NMR | MS |
|---|---|---|---|---|---|
| 94 | 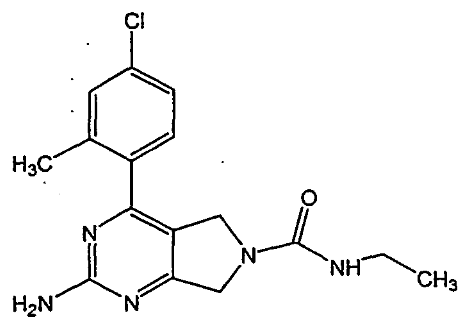 | 2-amino-4-(4-chloro-2-methylphenyl)-N-ethyl-57-dihydro-6H-pyrrolo[34-d]pyrimidine-6-carboxamide | Example 15a | (400 MHz, CD3OD) δ ppm 7.27 (s, 1H), 7.20 (s, 2H), 4.44 (s, 2H), 4.27 (s, 2H), 3.11 (m, 2H), 2.17 (s, 3H), 1.01 (t, 3H, 8 Hz) | 332.4, 334.2 |
| 95 | 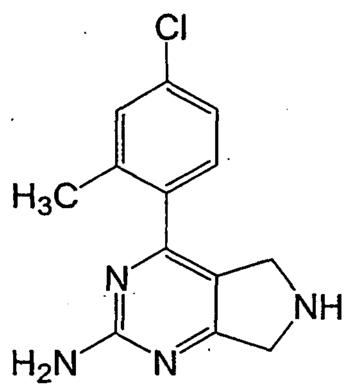 | 4-(4-chloro-2-methylphenyl)-67-dihydro-5H-pyrrolo[34-d]pyrimidin-2-amine | Example 2 | (400 MHz, DMSO-D6) δ ppm 7.41 (s, 1 H), 7.32 (d, 1H, J= 8 Hz), 7.26 (d, 1H, J= 8hz), 6.56 (bs, 2H), 3.88 (s, 2H), 3.80 (s, 2H), 2.24 (s, 3H) | 261 |
| 96 | 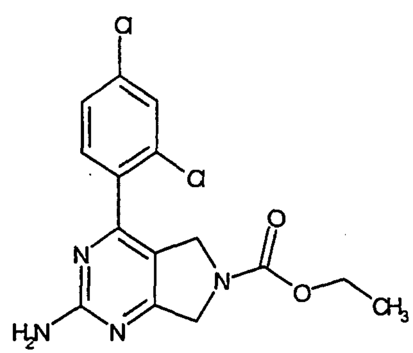 | ethyl 2-amino-4-(2,4-dichlorophenyl)-5,7-dihydro-6H-pyrrolo[3,4-d] pyrimidine-6-carboxylate | Example 1 | (300 MHz, DMSO-D6) (conformers) δ ppm 7.78 (d, J = 1.9 Hz, 1 H), 7.56 (dd, J = 8.3 and 1.9 Hz, 1H), 7.48 (d, J = 8.3 Hz, 1H), 6.92 (br s, 2H), 4.49 (s, 1 H), 4.47 (s, 1H), 4.34 (s, 1 H), 4.31 (s, 1 H), 4.13-4.03 (m, 2H), 1.24-1.15 (m, 3H). | 355.2, 353.2 |

(continued)

| Ex. No. | Structure | Name | Synthetic Method | $^1$H NMR | MS |
|---|---|---|---|---|---|
| 97 | 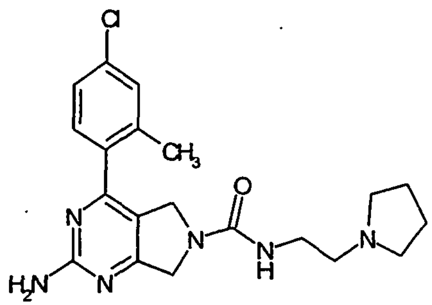 | 2-amino-4-(4-chloro-2-methylphenyl)-N-(2-pyrrolidin-1-ylethyl)-57-dihydro-6H-pyrrolo[34-d] pyrimidine-6-carboxamide | Example 7 | (300 MHz, DMSO-D6) δ ppm 7.43-7.42 (m, 1H), 7.36-7.30 (m, 2H), 6.77 (br s, 2H), 6.35 (t, J = 5.8 Hz, 1H), 4.40 (s, 2H), 4.24 (s, 2H), 3.17-3.10 (m, 2H), 2.46-2.39 (m, 6H), 2.22 (s, 3H), 1.66-1.62 (m, 4H). | 401.2 |
| 98 | 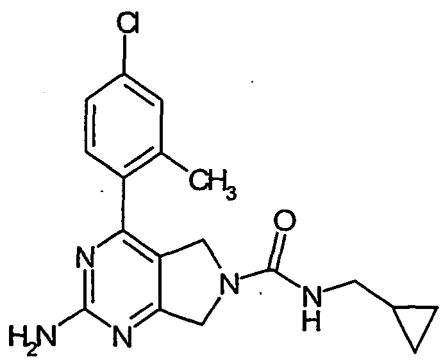 | 2-amino-4-(4-chloro-2-methylphenyl)-N-(cyclopropylmethyl)-57-dihydro-6H-pyrrolo[34-d] pyrimidine-6-carboxamide | Example 7 | (300 MHz, DMSO-D6) δ ppm 7.43-7.42 (m, 1H), 7.35-7.33 (m, 2H), 6.77 (br s, 2H), 6.46 (t, J = 5.6 Hz, 1H), 4.41 (s, 2H), 4.25 (s, 2H), 2.90 (dd, J = 6.0 and 6.0 Hz, 2H), 2.23 (s, 3H), 0.97-0.87 (m, 1H), 0.38-0.32 (m, 2H), 0.16-0.11 (m, 2H) | 358.2 |

**Example 100: N-allyl-2-amino-4-{2,4-dichloro-6-[2-(1H-pyrazol-1-yl)ethoxy]phenyl}-5,7-dihydro-6H-pyrrolo[3,4-d]pyrimidine-6-carboxamide**

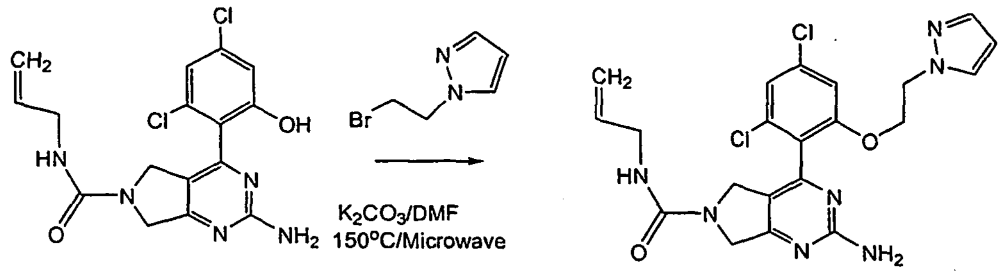


**100a** **100**

Compound **100a,** *N*-allyl-2-amino-4-(2,4-dichloro-6-hydroxyphenyl)-5,7-dihydra-6*H*-pyrrolo[3,4-*d*]pyrimidine-6-carboxamide, was prepared in a manner similar to Example **18a** except that allyl isocyanate was substituted for cyclobutylamine and CDI in Example **18b.** Compound **100a** (60mg, 0.16mmol), potassium carbonate (131mg, 0.95mmol.), 1-(2-bromoethyl)-1H-pyrazole (56mg, 0.316mmol) and DMF (2.0 mL) were microwaved at 120˚C for 30 min. Isolation by preparative HPLC gave compound 100 (39 mg, 0.08 mmol) as a white solid in 52% yield. 1 H NMR (400 MHz, DMSO-d6) δ ppm 3.60 (d, J=12.88 Hz, 2 H), 3.67 (t, J=5.05 Hz, 2 H), 3.94 (d, J=13.14 Hz, 2 H), 4.27 - 4.37 (m, 4 H), 4.41 (d, J=5.81 Hz, 2 H), 5.74 - 5.90 (m, 1 H), 6.00 (t, J=2.02 Hz, 1 H), 6.45 (br. s., 1 H), 6.78 (s, 2 H), 7.18 (d, J=2.02 Hz, 1 H), 7.25 (d, J=1.77 Hz, 1 H), 7.29 (d, J=1.52 Hz, 1 H), 7.32 (d, J=1.77 Hz, 1 H). LCMS $(M+H)^+$ 474.0, 476.0.

**Example 101: 2-amino-N-cyclopropyl-4-[2,4-dichloro-6-(2-morpholin-4-ylethoxy)phenyl]-5,7-dihydro-6H-pyrroto[3,4-d]pyrimidine-6-carboxamide**

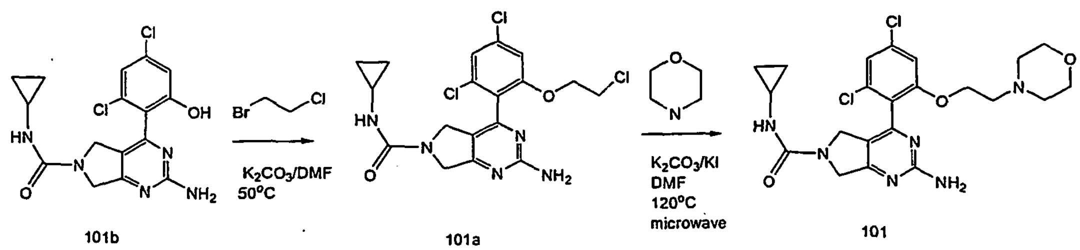


**101b** **101a** **101**

Compound **101b,** 2-amino-*N*-cyclopropyl-4-(2,4-dichloro-6-hydroxyphenyl)-5,7-dihydro-6*H*-pyrrolo[3,4-*d*]pyrimidine-6-carboxamide, was prepared in a manner similar to Example **18a** except that cyclopropylamine was substituted for cyclobutylamine in Example **18b.** Compound **101b** (800 mg, 2.1 mmol), potassium carbonate (872 mg, 6.31 mmol), 1-bromo-2-chloroethane (0.4 mL, 4.2 mmol) in DMF (8.0 mL) were heated at 50˚C for 12 h and the mixture was filtered. The collected insoluble material was washed with EtOAc. The combined filtrate/washings plus additional EtOAc (200 mL) were washed witch saturated aqueous $NaHCO_3$ (50 mL), brine (50ml), dried ($Na_2SO_4$), filtered and concentrated. Isolation by silica gel chromatography (gradient of 0-10% MeOH in $CH_2Cl_2$) gave compound **101a,** 2-amino-N-cyclopropyl-4-[2,4-dichloro-6-(2-chloroethoxy)phenyl]-5,7-dihydro-6H-pyrrolo[3,4-d]pyrimidine-6-carboxamide, (786 mg, 1.79 mmole) as brown grease in 85% yield. 1 H NMR (400 MHz, DMSO-d6) δ ppm 0.32 - 0.43 (m, 2 H), 0.47 - 0.58 (m, 2 H), 3.69 - 3.86 (m, 1 H), 4.00 - 4.12 (m, 2 H), 4.19 (d, J=13.14 Hz, 2 H), 4.23 - 4.31 (m, 2 H), 4.32 - 4.45 (m, 2 H), 6.42 (d, J=2.78 Hz, 1 H), 6.80 (s, 2 H), 7.38 (d, J=1.77 Hz, 1 H), 7.96 (s, 1 H). LCMS $(M+H)^+$ 444.0, 446.0. Compound **101a** (50 mg, 0.11 mmol), $K_2CO_3$ (94mg, 0.68 mmol), KI (38mg, 0.23mmol), morpholine (30mg, 0.34mmol) and DMF (2.0 mL) were then microwaved at 130˚C for 45min. Isolation by preparative HPLC gave compound **101** (32mg, 0.063mmole) as a white solid in 57% yield. 1 H NMR (400 MHz, DMSO-d6) δ ppm 0.32 - 0.45 (m, 2 H), 0.48 - 0.58 (m, 2 H), 2.07 - 2.37 (m, 4 H), 2.52 - 2.58 (m, 1 H), 3.39 (br. s., 4 H), 4.02 - 4.22 (m, 5 H), 4.34 - 4.46 (m, 3 H), 6.46 (d, J=2.27 Hz, 1 H), 6.81 (br. s., 2 H), 7.32 (d, J=15.66 Hz, 2 H). LCMS $(M+H)^+$ 493.1, 495.1.

**Example 102: 2-amino-N-(1-cyanocyclopropyl)-4-{2,4-dichloro-6-[2-(1H-pyrazol-1-yl)ethoxy]phenyl}-5,7-dihydro-6H-pyrrolo[3,4-d]pyrimidine-6-carboxamide**

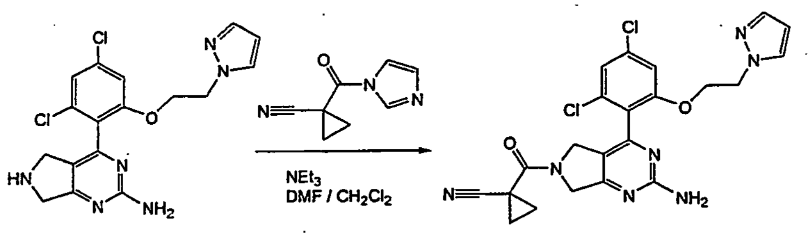


102a 102

Compound **102a** HCl (45 mg, 0.12 mmol) was treated with the preformed adduct of 1-amino-cyclopropanecarbonitrile hydrochloride (59mg, 0.5 mmol) and CDI (126mg, 0.775 mmol) in a manner similar to that described for Example **18b.** Following extractive work up from EtOAc, washing with saturated aqueous $NaHCO_3$ the product was isolated by preparative HPLC to give compound **102** (38mg, 0.08mmole) as a white solid in 66%yield. 1 H NMR (400 MHz, DMSO-d6) δ ppm 1.12 (d, J=7.33 Hz, 2 H), 1.41 (br. s., 2 H), 3.57 (s, 2 H), 3.87 (d, J=12.63 Hz, 1 H), 4.33 (s, 4 H), 4.41 (d, J=7.33 Hz, 2 H), 6.02 (t, J=1.89 Hz, 1 H), 6.82 (s, 2 H), 7.21 (d, J=2.02 Hz, 1 H),7.26 (d, J=1.52 Hz, 1 H), 7.32 (dd, J=3.66,1.64 Hz,2H). LCMS $(M+H)^+$ 499.2.

**Preparation of Compound 102a, 4-{2,4-dichloro-6-[2-(1*H*-pyrazol-1-yl)ethoxy]phenyl}-6,7-dihydro-5*H*-pyrrolo[3,4-*d*]pyrimidin-2-amine**

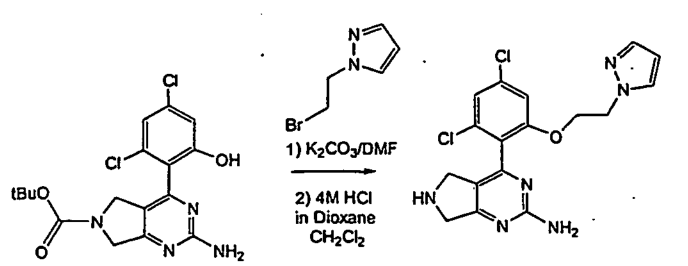


102b 102a

Compound **102b,** *tert*-butyl 2-amino-4-(2,4-dichloro-6-hydroxyphenyl)-5,7-dihydro-6*H*-pyrrolo[3,4-*d*]pyrimidine-6-carboxylate, was prepared from compound 9a (1090 mg, 3.0 mmol), compound **18c** (682mg, 3.3 mmol), Pd $(PPh_3)_4$ (347 mg, 0.3 mmol.), and 2M $Na_2CO_3$ (4.5 ml, 9.0 mmol) in 1,4-dioxane (25 mL) in a manner similar to that described for Example 1. 1 H NMR (400 MHz, DMSO-d6) d ppm 1.37 - 1.47 (m, J=12.88 Hz, 9 H) 4.07 - 4.24 (m, 2 H) 4.42 (d, J=7.58 Hz, 2 H) 6.82 (s, 2 H) 6.95 (d, J=1.77 Hz, 1 H) 7.08 - 7.21 (m, 1 H) 10.67 (s, 1 H). LCMS $(M+H)^+$ 397.1 399.1. Compound 102b (1.627g, 4.1mmol) was O-alkylated using potassium carbonate (3.4 g, 24.6 mmol.), 1-(2-bromoethyl)-1 H-pyrazole (1.43 g, 8.19 mmol) in DMF (10 mL) in a manner similar to that described for Example **100.** Boc deprotection was carried out using 4M HCl in 1,4-dioxane (10 mL) and MeOH (20mL)- stirring at ambient temperature for about 16h. Following extractive work up from EtOAc, washing with saturated aqueous $NaHCO_3$, the crude product was isolated by preparative HPLC to give compound **102a,** 4-{2,4-dichloro-6-[2-(1*H*-pyrazol-1-yl)ethoxy]phenyl}-6,7-dihydro-5*H*-pyrrolo[3,4-*d*]pyrimidin-2-amine, (405 mg, 0.95 mmol) as a white HCl salt in 26% yield. 1 H NMR (400 MHz, DMSO-d6) δ ppm 3.51 (d, J=13.39 Hz, 2 H), 3.93 - 4.07 (m, 2 H), 4.24 - 4.44 (m, 4 H), 6.14 (br. s., 1 H), 6.65 (br. s., 2 H), 7.19 (br. s., 1 H), 7.24 (br. s., 1 H), 7.29 (br. s., 1 H), 7.40 (s, 1 H), 8.24 (s, 1 H). LCMS $(M+H)^+$ 391.2, 393.2.

**Alternate Preparation of Compound 102a, 4-{2,4-dichloro-6-[2-(1*H*-pyrazol-1-yl)ethoxy]phenyl}-6,7-dihydro-5*H*-pyrrolo[3,4-*d*]pyrimidin-2-amine**

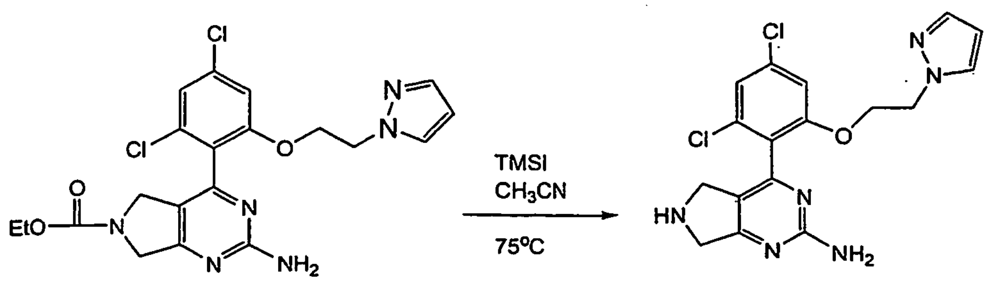


108

102a

Deprotection of the ethyl carbamate of compound **108** (5.06 g, 10.3 mmol) using iodotrimethylsilane (8.0 mL, 56 mmol) in $CH_3CN$ (80.0 mL) was carried out in a similar manner to Example **2**. Compound **102a** (8.46 g) was obtained as a crude HI salt. LCMS $(M+H)^+$: 393.2, 391.3.

**Example 103: 2-amino-*N*-cyclobutyl-4-[4-(hydroxymethyl)-2-methyl-6-(4,4,4-trifluorobutoxy) phenyl]-5,7-dihydro-6*H*-pyrrolo[3,4-*d*]pyrimidine-6-carboxamide**

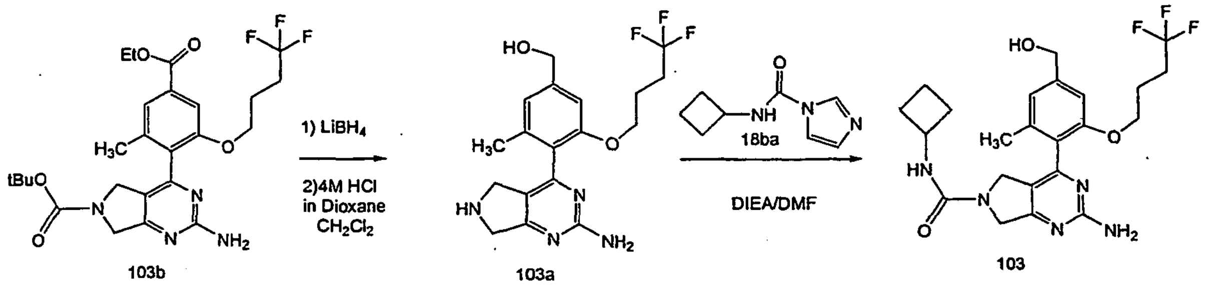


103b

103a

103

To compound **103b** (205 mg, 0.39 mmol) in THF (4 mL) at 0°C was added 2M $LiBH_4$ in THF (0.6 mL, 1.17 mmol) and MeOH (0.5 mL). The mixture was stirred at 0°C and allowed to warm to ambient temperature overnight. Following extractive work up from EtOAc (300 ml), washing with saturated aqueous $NaHCO_3$ (50ml) then brine (50ml), the EtOAc solution was dried ($Na_2SO_4$), filtered, and the volatile components removed to give the alcohol (185mg) as a clear grease which was carried on directly. LCMS $(M+H)^+$ 483.2, 484.2. For Boc deprotection, $CH_2Cl_2$ (5 mL) was added followed by 4M HCl in dioxane (1.0ml), 3.83mmol). After stirring 12 h, the mixture was concentrated and ether and hexane were added. The volatile components were removed to give compound **103a,** 4-(2-amino-6,7-dihydro-5*H*-pyrrolo[3,4-*d*]pyrimidin-4-yl)-3-methyl-5-(4,4,4-trifluorobutoxy)benzoic acid, (189 mg) as a yellow hydrochloride which was carried on without purification. LCMS $(M+H)^+$ 383.2, 384.2. Compound **103a** in DMF (2.0 mL) was reacted with compound **18ba** (76 mg, 0.46 mmol) and DIEA (0.4ml, 6.0mmol) in a manner similar to Example **18b**. Isolation by preparative HPLC gave compound **103** (33 mg, 0.083mmole) as a white solid in 21% yield. 1 H NMR (400 MHz, DMSO-d6) δ ppm 1.44 - 1.61 (m, 2 H), 1.69 - 1.81 (m, 2 H), 1.84 - 1.98 (m, 2 H), 2.00 - 2.16 (m, 7 H), 3.92 - 4.05 (m, 3 H), 4.06 - 4.20 (m, 2 H), 4.37 (d, J=5.31 Hz, 2 H), 4.49 (d, J=5.56 Hz, 2 H), 5.25 (t, J=5.68 Hz, 1 H), 6.51 (d, J=7.83 Hz, 1 H), 6.65 (s, 2 H), 6.85 (s, 1 H), 6.89 (s, 1 H). LCMS $(M+H)^+$ 480.4, 481.4.

**Preparation of Compound 103b, 2-amino-N-cyclobutyl-4-[4-(hydroxymethyl)-2-methyl-6-(4,4,4-trifluorobutoxy) phenyl]-5,7-dihydro-6H-pyrrolo[3,4-d]pyrimidine-6-carboxamide**

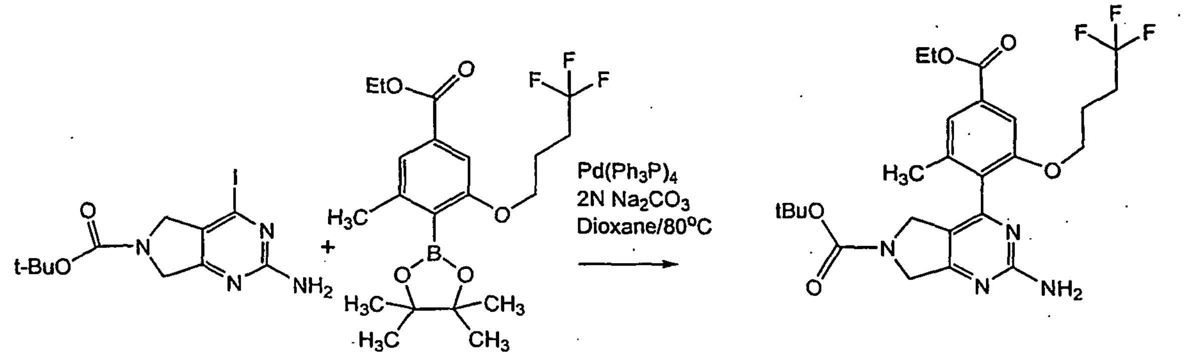


9a 103c 103b

Compound **9a** (200 mg, 0.552 mmol) and compound **103c** (253mg, 0.61 mmol), $Pd(PPh_3)_4$ (32 mg, 0.028 mmol) and 2M $Na_2CO_3$ solution (0.8 ml, 1.66 mmol) in1,4-dioxane (6mL) were reacted for 48 h in a manner similar to Example **1.** Monitoring (LCMS) indicated that the reaction was incomplete so the mixture was microwaved at 160 °C for 1 h whereupon the suspension was filtered and the solids washed with MeOH. The combined filtrate/washings were concentrated and subjected to EtOAc (300 ml) extractive work up washing with saturated aqueous $NaHCO_3$ (50ml) and brine (50ml). Isolation by silica gel chromatography (gradient of 0-60% EtOAc in $CH_2Cl_2$) gave compound **103b** (205mg, 0.433mmole) as a clear grease in 71% yield. 1H NMR (400 MHz, CHLOROFORM-d) δ ppm 1.21 - 1.28 (m, 9 H), 1.38 - 1.54 (m, 4 H), 1.85 - 1.98 (m, 2 H), 1.98 - 2.12 (m, 2 H), 2.20 (d, J=4.55 Hz, 3 H), 3.98 - 4.13 (m, 2 H), 4.41 (q, J=7.07 Hz, 2 H), 4.60 (d, J=22.48 Hz, 2 H), 7.39 - 7.52 (m, 2 H), 7.59 - 7.73 (m, 1H). LCMS $(M+H)^+$ 524.2, 526.2.

**Preparation of Compound 103c, ethyl 3-methyl-4-(4,4,5,5-tetramethyl-1,3,2-dioxaborolan-2-yl)-5-(4,4,4-trifluorobutoxy)benzoate**

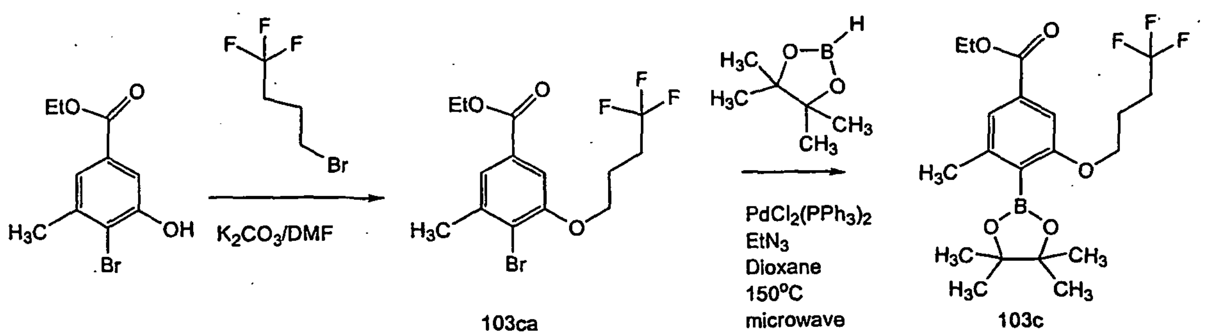


103ca 103c

Ethyl 4-bromo-3-hydroxy-5-methylbenzoate (650mg, 2.5mmol) was O-alkylated with 4-bromo-1,1,1-trifluorobutane (950mg, 5.0mmol) using potassium carbonate (1040mg, 7.5mmol) in DMF (5.0 mL) in a manner similar to that described for Example **100.** Isolation using silica gel chromatography (0-20% gradient of EtOAc in hexane) gave compound **103ca,** ethyl 4-bromo-3-methyl-5-(4,4,4-trifluorobutoxy)benzoate, (858 mg, 2.33mmole) as a white solid in 93% yield. 1 H NMR (400 MHz, CHLOROFORM-*d*) δ ppm 1.41 (t, *J*=7.20 Hz, 3 H), 2.04 - 2.21 (m, 2 H), 2.32 - 2.44 (m, 2 H), 2.44 - 2.50 (m, 3 H), 4.15 (t, *J*=5.94 Hz, 2 H), 4.38 (q, *J*=7.07 Hz, 2 H), 7.35 (d, *J*=1.77 Hz, 1 H), 7.57 (d, *J*=1.01 Hz, 1 H). Compound **103ca** (561mg, 1.52mmol), pinacolborane (0.7ml, 4.6 mmol), $Et_3N$ (0.9ml, 6.1 mmol) in 1,4-dioxane (6.0 mL) were purged with $N_2$ for 15 min, then $Pd(II)Cl_2(PPh_3)_2$ (107mg, 0.1 mmol) was added. The mixture was microwaved at 150°C for 1 h, cooled and filtered through celite washing with EtOAc. The combined filtrate/washings were washed with water (50ml), brine (50ml), dried ($Na_2SO_4$), filtered and concentrated. Isolation using silica gel chromatography (0-20% gradient of EtOAc in hexane) gave compound **103c** (252mg, 0.61 mmole) in 40% yield. 1H NMR (400 MHz, CHLOROFORM-d) δ ppm 1.37 - 1.41 (m, 15 H), 2.05 (dd, J=10.74, 5.68 Hz, 2 H), 2.31 - 2.39 (m, 2 H), 2.39 - 2.41 (m, 3 H), 4.06 (t, J=5.81 Hz, 2 H), 4.36 (q, J=7.24 Hz, 2 H), 7.25 (s, 1 H), 7.46 (s, 1 H).

**Example 104: 2-amino-4-(2-chloro-4-cyclopropyl-5-methoxyphenyl)-N-isopropyl-5,7-dihydro-6H-pyrrolo [3,4-d]pyrimidine-6-carboxamide**

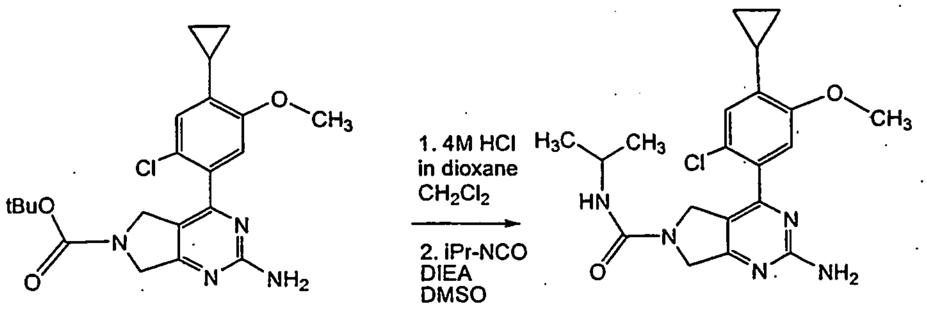


104a 104

To compound **104a** (67 mg, 0.16 mmol) in $CH_2Cl_2$ (4.0 mL) was added 4M HCl in 1,4-dioxane (0.2ml, 0.804mmol). After stirring at ambient temperature for 12 h, the mixture was concentrated and treated with ether and hexane. Removal of the volatile components gave the Boc-deprotected intermediate (65 mg) as a yellow hydrochloride which was carried on directly. LCMS $(M+H)^+$ 317.2, 319.2. Dimethylformamide (2.0 mL) and diisopropylethylamine (0.14, 0.8 mmol) were added followed by isopropyl isocyanate (14.0 mg, 0.16 mmol) to form the urea in a manner similar to Example **15a.** Isolation by preparative HPLC gave compound **104** (13 mg, 0.032mmol) as brown foam in 20% yield. 1 H NMR (400 MHz, DMSO-d6) δ ppm 0.69 - 0.78 (m, 2 H), 0.90 - 0.98 (m, 2 H), 1.05 (d, J=6.57 Hz, 6 H), 2.04 - 2.18 (m, 1 H),3.77 (t, J=6.95 Hz, 1 H), 3.81 (s, 3 H), 4.25 (s, 2 H), 4.40 (s, 2 H), 6.09 (d, J=7.83 Hz, 1 H), 6.82 (s, 2 H), 6.95 (s, 2 H). LCMS $(M+H)^+$ 402.2, 404.2.

**Preparation of Compound 104a, *tert*-butyl 2-amino-4-(2-chloro-4-cyclopropyl-5-methoxyphenyl)-5,7-dihydro-6H-pyrrolo[3,4-d]pyrimidine-6-carboxylate**

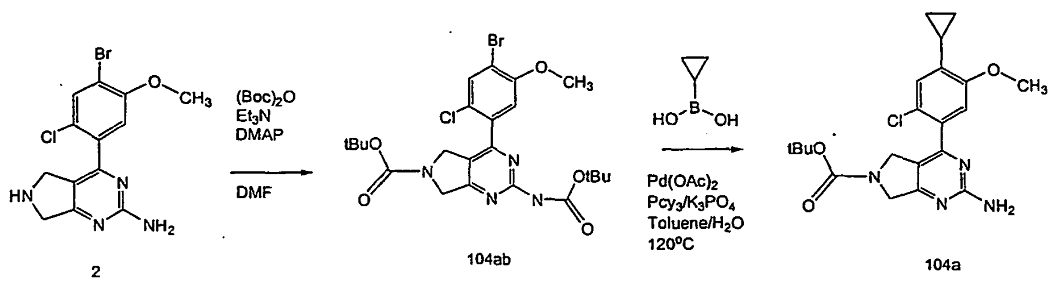


2 104ab 104a

To compound **2** (612mg, 1.0mmol) and di-tert-butyldicarbonate (655mg, 3.0mmol.) in DMF (5.0 mL) was added triethylamine (0.84ml, 6.0 mmol) and DMAP (25mg, 0.2 mmol). After stirring 4 h at ambient temperature, the mixture was subjected EtOAc (200ml) extractive work up washing with saturated aqueous $NaHCO_3$ (50ml) and brine (50ml). After drying ($Na_2SO_4$), filtering and concentrating, isolation using silica gel chromatography (0-50% gradient of EtOAc in $CH_2Cl_2$) gave compound **104ab,** *tert*-butyl 4-(4-bromo-2-chloro-5-methoxyphenyl)-2-[(*tert*-butoxycarbonyl) amino]-5,7-dihydro-6H-pyrrolo[3,4-*d*]pyrimidine-6-carboxylate, (320mg, 0.58mmole) as yellowish foam in 58% yield. 1 H NMR (400 MHz, DMF-d7) δ ppm 1.34 - 1.50 (m, 18 H), 3.84 - 3.90 (m, 3 H), 4.30 (d, J=7.33 Hz, 2 H), 4.45 (d, J=6.06 Hz, 2 H), 7.16 (d, J=3.79 Hz, 1 H), 7.88 (d, J=3.54 Hz, 1 H), 7.97 (s, 1 H). LCMS $(M+H)^+$ 556.4, 558.4. Compound **104ab** (167 mg, 0.3 mmol), cyclopropylboronic acid (52mg, 0.6 mmol), $K_3PO_4$ (255mg, 1.20 mmol) and tricyclohexylphosphine (9.0mg, 0.03 mmol) in toluene (4.0 mL) with $H_2O$ (0.1 mL) were purged with $N_2$ for 15 min then $Pd(OAc)_2$ (4.0 mg, 0.02mmol) was added. After heating at 120°C for 6 h, the mixture was filtered through celite washing with EtOAc. The filtrate/washings were subjected to EtOAc (500 ml) extractive work up washing with saturated aqueous $NaHCO_3$ (100ml) and brine (100ml). Following drying ($Na_2SO_4$), filtration and concentration, isolation using silica gel chromatography (0-50% gradient of EtOAc in $CH_2Cl_2$) gave compound **104a** (67mg, 0.324mmole) as a yellowish foam in 54% yield. 1

H NMR (400 MHz, CHLOROFORM-d) δ ppm 0.79 - 0.94 (m, 2 H), 0.94 - 1.06 (m, 2 H), 1.43 - 1.54 (m, 9 H), 2.08 - 2.25 (m, 1 H), 3.84 - 3.93 (m, 3 H), 4.48 (d, J=19.70 Hz, 2 H), 4.58 (d, J=23.75 Hz, 2 H), 5.28 (s, 2 H), 6.77 (d, J=10.11 Hz, 1 H), 6.87 (d, J=11.12 Hz, 1 H). LCMS $(M+H)^+$ 417.2, 419.2.

**Example 105: 4-(4-bromo-2-chloro-5-methoxyphenyl)-6-(2-methoxyethyl)-6,7-dihydro-5H-pyrrolo[3,4-*d*]pyrimidin-2-amine**

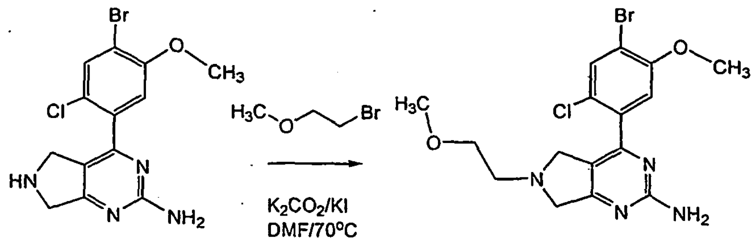


2

105

The hydrochloride of compound **2** (70 mg, 0.16 mmol), $K_2CO_3$ (225mg, 1.63 mmol), and 2-bromoethylmethyl ether (113mg, 0.82mmol) in DMF (3mL) were microwaved at 100˚C for 2 h. Isolation by preparative HPLC gave compound **105** (13mg, 0.03mmole) as a white solid in 19% yield. 1 H NMR (400 MHz, DMSO-d6) δ ppm 3.27 - 3.32 (m, 3 H), 3.47 - 3.60 (m, 2 H), 3.66 (br. s., 2 H), 3.84 - 3.93 (m, 3 H), 4.35 - 4.62 (m, 4 H), 7.05 - 7.12 (m, 1H), 7.14 - 7.25 (m, 2 H), 7.91 (s, 1 H). LCMS $(M+H)^+$ 415.0,417.0.

**Example 106: 2-amino-4-(2-chloro-4-ethenyl-5-methoxyphenyl)-*N*-(1-methylethyl)-5,7-dihydro-6H-pyrrolo [3,4-*d*]pyrimidine-6-carboxamide**

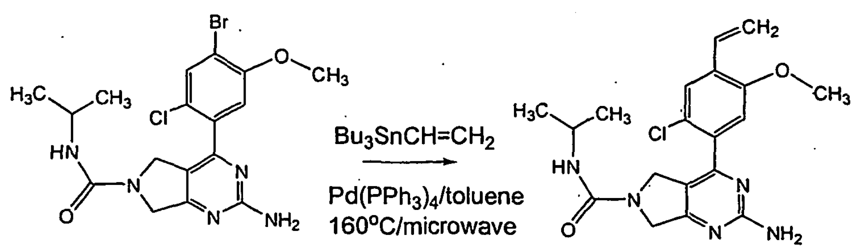


55

106

Compound **55** (66 mg, 0.15 mmol) and ethenyltributylstannane (62mg, 0.2 mmol) in toluene (1mL) and dioxane (1 mL) were purged with $N_2$ and $Pd(PPh_3)_4$ (9 mg, 0.008 mmol) was added and the mixture was microwaved at 160˚C for 30 min. The mixture was filtered through celite and washed with MeOH. The filtrate was concentrated and subjected to EtOAc (200 ml) extractive work up washing with saturated aqueous $NaHCO_3$ (50ml) and brine (50ml). Following drying ($Na_2SO_4$) and concentration, isolation by preparative HPLC gave compound **106** (11 mg, 0.029 mmole) as a white solid in 19% yield. 1H NMR (400 MHz, DMSO-d6) δ ppm 1.02 - 1.10 (m, 6 H), 3.74 - 3.80 (m, 1 H), 3.79 - 3.86 (m, 3 H), 4.28 (s, 2 H), 4.43 (s, 2 H), 5.40 (d, J=12.38 Hz, 1 H), 5.98 (dd, J=17.94, 1.01 Hz, 1 H), 6.09 (d, J=6.82 Hz, 2 H), 6.93 (dd, J=17.68, 11.37 Hz, 1 H), 7.06 (s, 1 H), 7.70 (s, 1 H). LCMS $(M+H)^+$ 386.2, 386.2.

**Example 107: 2-amino-4-(2-chloro-4,6-dimethoxyphenyl)-*N*-prop-2-en-1-yl-5,7-dihydro-6H-pyrrolo[3,4-*d*]pyrimidine-6-carboxamide**

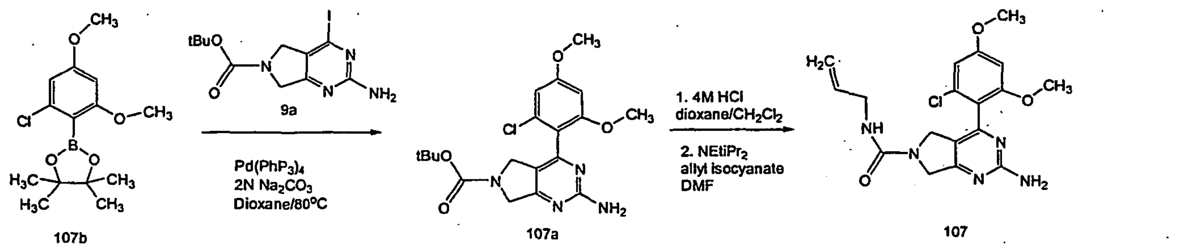


Compound **9a** (199 mg, 0.549 mmol), compound **107b** (164mg, 0.549 mmol), Pd($PPh_3$)$_4$ (32 mg, 0.027 mmol) and 2M aqueous $Na_2CO_3$ (0.8 ml, 1.65 mmol) in 1,4-dioxane (4 mL) were reacted in a manner similar to Example **9.** Isolation using silica gel chromatography (gradient of 0-10% MeOH in $CH_2Cl_2$) gave compound **107a,** *tert*-butyl 2-amino-4-(2-chloro-4,6-dimethoxyphenyl)-5,7-dihydro-6H-pyrrolo[3,4-*d*]pyrimidine-6-carboxylate, (204mg, 0.52mmole) as a yellowish solid. LCMS $(M+H)^+$ 407.4, 409.4. To Boc-deprotect, treatment of compound **107a** (204 mg, 0.5 mmol) in $CH_2Cl_2$ (5 mL) with 4M HCl in 1,4-dioxane (1.3mL) in a manner similar to Example **102a** gave the hydrochloride (200 mg) as a yellow grease which was carried on without purification. LCMS $(M+H)^+$ 307.2, 309.2. A portion of the Boc-deprotected hydrochloride (95mg, 0.25 mmol) in DMF (2.0 mL) was treated with diisopropylethylamine (0.3ml, 1.5 mmol) and allyl isocyanate (21 mg, 0.25 mmol) in a manner similar to Example **15a.** Isolation by preparative HPLC gave compound **107** (15mg, 0.038mmol) as a white solid in 15% yield. 1 H NMR (400 MHz, DMSO-d6) δ ppm 3.62 - 3.70 (m, 2 H), 3.73 (s, 3 H), 3.83 (s, 3 H), 4.09 - 4.18 (m, 2 H), 4.43 (s, 2 H), 5.00 (dd, J=10.36, 1.52 Hz, 1 H), 5.10 (dd, J=17.18, 1.52 Hz, 1 H), 5.72 - 5.88 (m, 1 H), 6.57 (t, J=5.56 Hz, 1 H), 6.67 (d, J=2.27 Hz, 1 H), 6.72 - 6.78 (m, 2 H). LCMS $(M+H)^+$ 390.1, 392.

**Preparation of Compound 107b, 2-(2-chloro-4,6-dimethoxyphenyl)-4,4,5,5-tetramethyl-1,3,2-dioxaborolane**

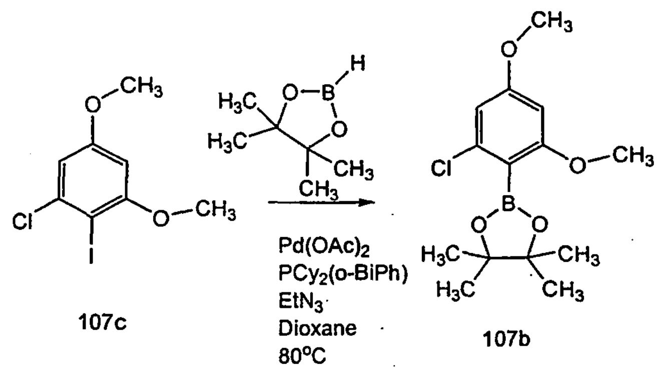


Compound **107c** (1.45g, 4.86mmol), pinacolborane (1.4ml, 9.72 mmol) and $Et_3N$ -(2.0ml, 14.6.0mmol), palladium (II) acetate (55mg, 0.243 mmol) and 2-(dicyclohexylphosphino)biphenyl (170mg, 0.7mmol.) in 1,4-dioxane (25 mL). were reacted in a manner similar to Example **18ca.** Isolation using silica gel chromatography (gradient of 0-20% EtOAc in hexane) gave compound **107b** (1.104g, 3.74mmole) as a yellowish solid in 77% yield. NMR data: 1 H NMR (400 MHz, CHLOROFORM-D) δ ppm 1.37 (s, 12 H), 3.73 (s, 3 H), 3.76 (s, 3 H), 6.25 (s, 1 H), 6.44 (s, 1 H).

**Preparation of Compound 107c, 1-chloro-2-iodo-3,5-dimethoxybenzene**

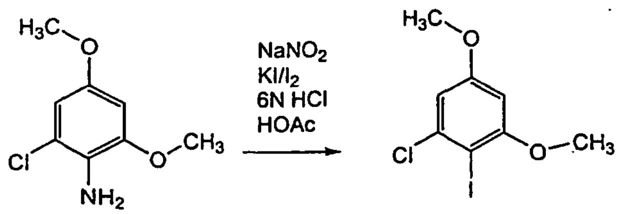


107c

To (2-chloro-4,6-dimethoxyphenyl)amine (1.9 g, 10.0 mmol) in acetic acid (40 mL) was added 6M HCl (10mL). With stirring & cooling in an ice salt bath, sodium nitrite (828 mg, 12.0 mmol) in water (6mL) was added slowly keeping the reaction temperature <5˚C. After addition, the mixture was stirred for about 30 min and a solution of potassium iodide (3320 mg, 20.0 mmol) and iodine (761 mg, 3.0 mmol) in water (35 mL) was added dropwise. The mixture was allowed to warm to ambient temperature over about 90 min and water (120 mL) was added. The mixture was subjected to EtOAc (2x300 mL) extractive work up and the combined extracts were washed with 10% aqueous $Na_2S_2O_3$ solution (2x100ml) and brine (100ml). After drying ($Na_2SO_4$), filtering and concentrating, isolation using silica gel chromatograpy (0-30% gradient of EtOAc in hexane) gave compound **107c** (1.45g, 4.9mmole) as a yellowish solid in 49% yield. 1 H NMR (400 MHz, CHLOROFORM-D) d ppm 3.79 (s, 3 H), 3.84 (s, 3 H), 6.30 (d, J=2.53 Hz, 1 H), 6.68 (d, J=2.53 Hz, 1 H).

**Example 108: ethyl 2-amino-4-{2,4-dichloro-6-[2-(1*H*-pyrazol-1-yl)ethoxy]phenyl}-5,7-dihydro-6*H*-pyrrolo [3,4-*d*]pyrimidine-6-carboxylate**

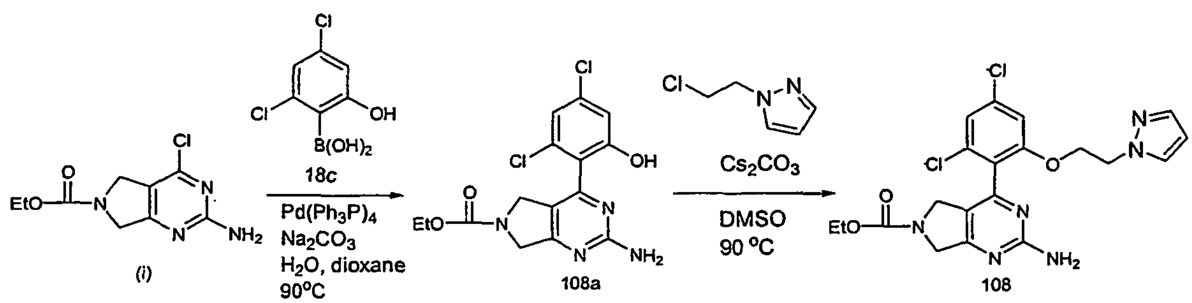


Compound (i) (4.04 g, 16.6 mmol), compound **18c** (4.82 g, 23.3 mmol), 2M aqueous sodium carbonate (21 ml, 42 mmol), and tetrakis(triphenylphosphino)palladium (0) (1.82 g, 1.58 mmol) in 1,4 dioxane (200 mL) were reacted in a manner similar to Example **1**. Extractive work up with EtOAc was carried out in a manner similar to Example **18a** first using 0.5N aq NaOH (300 mL) to extract the phenol product into the basic aqueous layer followed by acidification to pH 5 with 1 N $KH_2PO_4$ (75 mL) and extraction of phenol product into EtOAc (400 mL). The EtOAc layer was concentrated to give compound **108a,** ethyl 2-amino-4-(2,4-dichloro-6-hydroxyphenyl)-5,7-dihydro-6*H*-pyrrolo[3,4-*d*]pyrimidine-6-carboxylate (6.15 g) which was carried on without purification. 1 H NMR (400 MHz, DMSO-d6) d ppm 1.07 - 1.34 (m, 3 H) 3.99 - 4.26 (m, 4 H) 4.48 (d, J=11.87 Hz, 2 H) 6.84 (s, 2 H) 7.14 (d, J=1.77 Hz, 1 H) 7.48 - 7.79 (m, 1 H) 10.71 (s, 1 H). LCMS $(M+H)^+$: 369.0 371.0. Compound **108a** (5.11 g, 13.8 mmol), 1-(2-chloro-ethyl)-1H-pyrazole (5.02 g, 38.4 mmol) and cesium carbonate (15.8 g, 48.4 mmol) in DMSO (50.0 mL) were reacted at 90 ˚C in a manner similar to Example **18.** Following extractive work up from EtOAc (500 mL), washing with water (200 mL), the product was isolated using silica gel chrmoatography (100% EtOAc followed by a gradient of 0-13% MeOH in dichloromethane) to give compound **108** (5.06 g, 13.8 mmol) as a tan powder in 77% yield. 1 H NMR (300 MHz, DMSO-$d_6$) δ ppm 1.13 - 1.29 (m, 3 H) 3.49 - 3.64 (m, 1 H) 3.91 (d, *J*=13.19 Hz, 1 H) 4.02 - 4.19 (m, 2 H) 4.34 (s, 4 H) 4.40 - 4.48 (m, 2 H) 5.99 - 6.04 (m, 1 H) 6.83 (s, 2 H) 7.23 - 7.28 (m, 2 H) 7.28 - 7.33 (m, 2 H). LCMS $(M+H)^+$: 465.2, 463.2.

**Example 109: 2-amino-N-bicyclo[1.1.1]pent-1-yl-4-{2,4-dichloro-6-[2-(1H-pyrazol-1-yl)ethoxy]phenyl}-5,7-dihydro-6H-pyrrolo[3,4-*d*]pyrimidine-6-carboxamide**

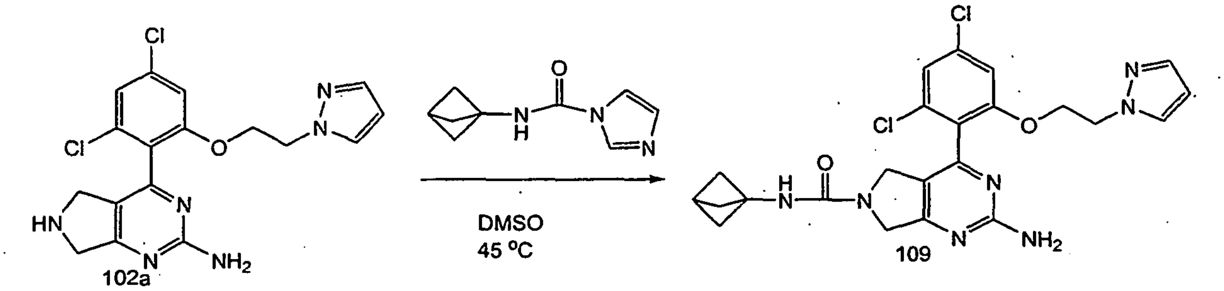


102a 109

Bicyclo[1.1.1]pentan-1-amine-HCl (637 mg, 5.33 mmol) followed by triethylamine (4.00 mL, 28.7 mmol) were added to 1,1-carbonyldiimidazole (870 mg, 5.37 mmol) in DMF (50 mL) at 0°C. The mixture was stirred at ambient temperature for 1h and crude compound **102a** HI salt (4.650 g, 5.150 mmol) was added. The mixture heated to 45°C for 0.5h. Following EtOAc extractive work up, washing with water, the crude product was subjected to silica gel chromatography (gradient of 10-15% MEOH in dichloromethane). After isolation, 10% $CH_3CN$ in water was added. Following lyophilization, compound **109** (1.54 g, 3.08 mmol) was obtained as a white powder in 58% yield. $^1H$ NMR (300 MHz, DMSO-d6) δ ppm 1.95 (s, 6 H) 2.36 (s, 1 H) 3.53 (d, J=13.19 Hz, 1 H) 3.89 (d, J=13.00 Hz, 1 H) 4.33 (s, 6 H) 5.99 - 6.02 (m, 1 H) 6.77 (br. s., 2 H) 6.85 (br. s., 1 H) 7.19 (d, J=2.07 Hz, 1 H) 7.25 (d, J=1.51 Hz, 1 H) 7.30 (d, J=1.70 Hz, 1 H) 7.32 (d, J=1.70 Hz, 1 H). LCMS $(M+H)^+$: 502.2, 500.2.

**Example 110: 2-amino-4-{2,4-dichloro-6-[2-(1*H*-pyrazol-1-yl)ethoxy]phenyl}-*N*-(2,2,2-trifluoroethyl)-5,7-dihydro-6*H*-pyrrolo[3,4-*d*]pyrimidine-6-carboxamide**

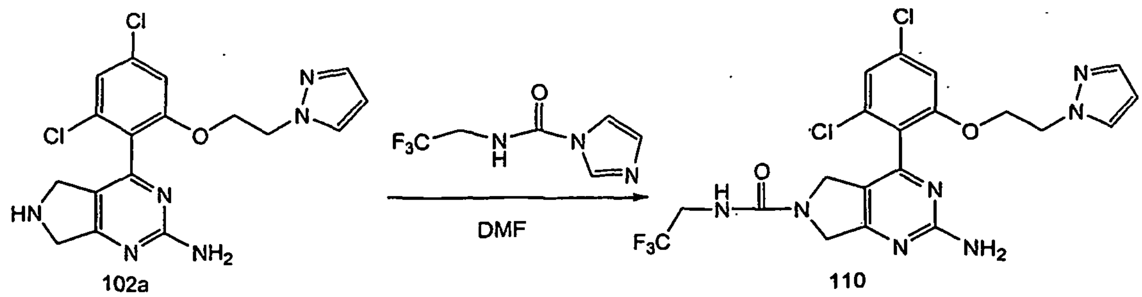


102a 110

2,2,2-Trifluoroethylamine (19.8 mg, 0.200 mmol), 1,1-carbonyldiimidazole (32.4 mg, 0.200 mmol), compound **102a** HCl (89.9 mg, 0.180 mmol) and triethylamine (0.080 mL, 0.57 mmol) in DMF (2 mL) were reacted in a manner similar to Example **18b.** Isolation using preparative HPLC gave compound **110** (37 mg, 0.72 mmol) as a white powder in 40% yield. 1 H NMR (400 MHz, DMSO-d6) δ ppm 3.54 (d, J=12.38 Hz, 1 H) 3.77 - 3.92 (m, 2 H) 3.94 (d, J=12.88 Hz, 1 H) 4.42 - 4.50 (m, 2 H) 5.98 (t, J=2.15 Hz, 1 H) 6.82 (s, 2 H) 7.21 (d, J=2.02 Hz, 1 H) 7.27 (s, 2 H) 7.33 (d, J=1.77 Hz, 1H). LCMS $(M+H)^+$: 518.0, 516.0.

**Example 111: 2-amino-*N*-bicyclo[1.1.1]pent-1yl-4-{4-bromo-2-chloro.5-[2.(1*H*-pyrazol-1-yl)ethoxy]phenyl}-5,7-dihydro-6*H*-pyrrolo[3,4-*d*]pyrimidine-6-carboxamide**

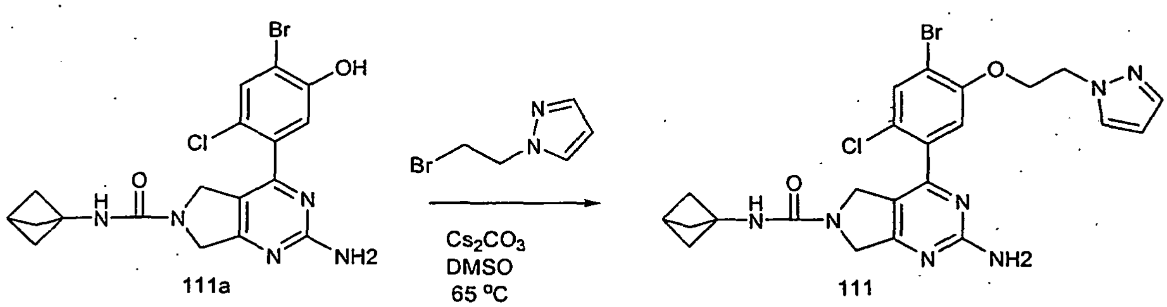

111a
111

Compound **111a** (44.0 mg, 0.0976 mmol) was alkylated with 1-(2-bromo-ethyl)-1H-pyrazole (32.4 mg, 0.185 mmol) and cesium carbonate (122 mg, 0.374 mmol) in DMSO (1.5 mL) in a manner similar to that described in Example **18.** Isolation using preparative HPLC gave compound **111** (29 mg, 0.053 mmol) as a white powder in 55% yield. 1 H NMR (300 MHz, DMSO-d6) d ppm 1.92 (s, 6 H) 2.34 (s, 1 H) 4.21 (s, 2 H) 4.36 - 4.42 (m, 4 H) 4.52 (t, J=4.71 Hz, 2 H) 6.24 (t, J=1.98 Hz, 1 H) 6.88 (s, 2 H) 6.99 (s, 1 H) 7.15 (s, 1 H) 7.44 (d, J=1.70 Hz, 1 H) 7.78 (d, J=2.26 Hz, 1 H) 7.84 (s, 1 H). LCMS $(M+H)^+$: 546.2, 544.2.

**Preparation of Compound 111a, 2-amino-*N*-bicyclo[1.1.1]pent-1-yl-4-(4-bromo-2-chloro-5-hydroxyphenyl)-5,7-dihydro-6*H*-pyrrolo[3,4-*d*]pyrimidine-6-carboxamide**

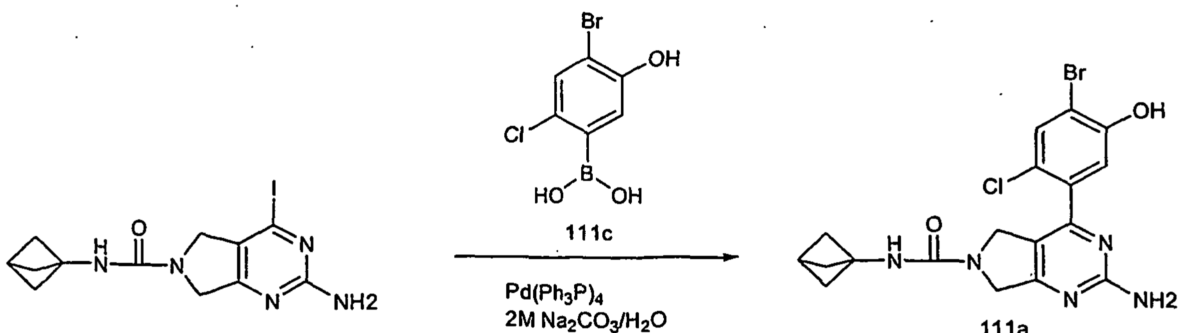

111b
111a

Compound **111b,** 2-amino-*N*-bicyclo[1.1.1]pent-1-yl-4-iodo-5,7-dihydro-6*H*-pyrrolo[3,4-*d*] pyrimidine-6-carboxamide, was prepared from compound **8b** in a manner similar to Example **18b** except that bicyclo[1.1.1]pentan-1-amine-HCl was subtituted for cyclobutylamine. The coupling of compound **111b** (0.2995 g, 0.8070 mmol) with compound **111c** (0.201 g, 0.800 mmol) was carried out using tetrakis(triphenylphosphino)palladium(0) (0.065 g, 0.070 mmol) and 2M aqueous sodium carbonate (0.80 mL, 1.6 mmol) in 1,4-dioxane (5.0 mL) in a manner similar to that described for Example **1.** Extractive work up involved aqueous base extraction followed by acidification and EtOAc extraction in a manner similar to Example **108a.** Isolation gave compound **111a** (0.316 g) as a tan solid which was carried on without purification. 1 H NMR (300 MHz, DMSO-$d_6$) δ ppm 1.93 (s, 6 H) 2.34 (s, 1 H) 4.22 (s, 2 H) 4.38 (s, 2 H) 6.87 (br. s., 2 H) 6.91 (s, 1 H) 7.01 (s, 1 H) 7.74 (s, 1 H) 10.83 (br. s., 1 H). LCMS $(M+H)^+$: 452.0, 450.0.

**Preparation of Compound 111c, (4-bromo-2-chloro-5-hydroxyphenyl)boronic acid**

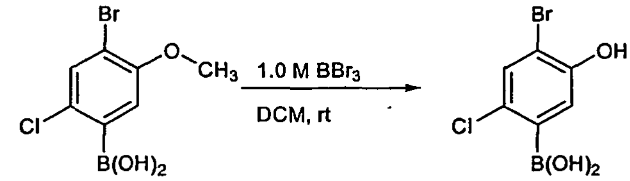


111c

To a suspension of (4-bromo-2-chloro-5-methoxyphenyl)boronic acid (4.96 g, 18.7 mmol) in dichloromethane (125 mL) at 0˚C was added 1 M boron tribromide (52 mL, 52 mmol) in dichloromethane. The reaction mixture was allowed to warm to ambient temperature and stir for 48h. The reaction mixture was then poured over ice, 3N NaOH (52 mL) was added, and the mixture was stirred vigorously for 30 minutes. The layers were separated, and the dichloromethane layer was discarded. The aqueous layer, a slurry, was acidified to pH 5 with concentrated HCl and EtOAc extractive work up gave compound **111 c** (4.614 g, 18.36 mmol) as a white powder which was carried on without purification.

**Example 112: 2-amino-*N*-bicyclo[1.1.1]pent-1-yl-4-[4-bromo-2-chloro-5-(difluoromethoxy) phenyl]-5,7-dihydro-6*H*-pyrrolo[3,4-*d*]pyrimidine-6-carboxamide**

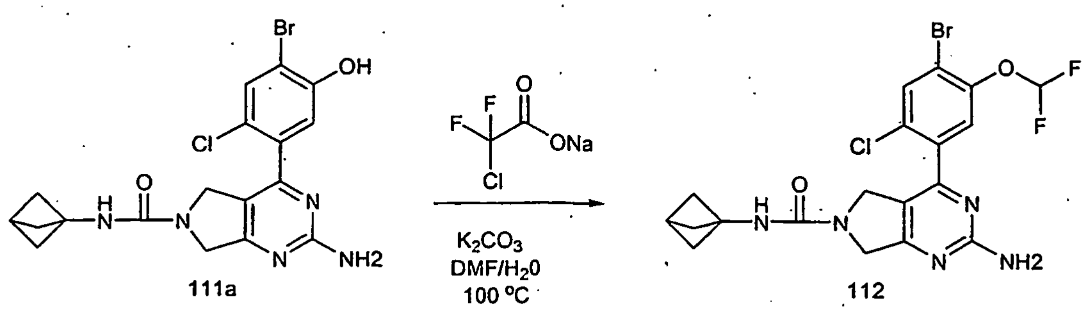


111a

112

Nitrogen was bubbled through a solution of compound **111a** (53 mg, 0.12 mmol) in DMF (1.0 mL) and $H_2O$ (0.13 mL) for 10 minutes. Potasium carbonate (0.092 g, 0.67 mmol) and sodium chlorodifluoroacetate (0.067 g, 0.44 mmol) were added. The reaction mixture was heated to 100˚C for 4h. Isolation using preparative HPLC gave compound **112** (0.019 g, 0.038 mmol) as a white powder in 32% yield. 1 H NMR (300 MHz, DMSO-d6) δ ppm 1.93 (s, 6 H) 2.35 (s, 1 H) 4.23 (s, 2 H) 4.39 (s, 2 H) 6.94 (s, 1 H) 6.99 - 7.03 (m, 1H) 7.36 (t, J=72.90 Hz, 1 H) 7.43 (s, 1 H) 8.10 (s, 1 H). LCMS $(M+H)^+$: 502.2, 500.0.

**Example 113: 2-amino-*N*-bicyclo[1.1.1]pent-1-yl-4-{2,4-dichloro-6-[2-(1,3-thiazol-2-yl) ethoxy]phenyl}-5,7-dihydro-6*H*-pyrrolo[3,4-*d*]pyrimidine-6-carboxamide**

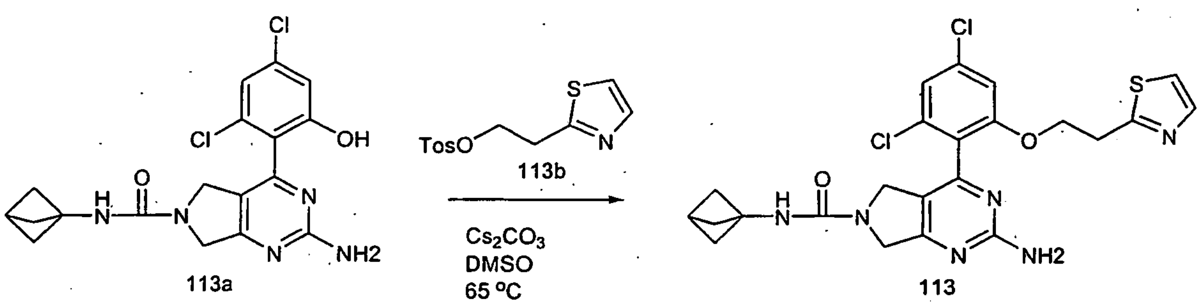


113a

113

compound **113a,** 2-amino-*N*-bicyclo[1.1.1]pent-1-yl-4-(2,4-dichloro-6-hydroxyphenyl)-5,7-dihydro-6*H*-pyrrolo[3,4-*d*]pyrimidine-6-carboxamide, was prepared in a similar manner as Example **18a** except that bicyclo[1.1.1]pentan-1-amine-HCl was subtituted for cyclobutylamine. Compound **113a** (65 mg, 0.16 mmol), cesium carbonate (201 mg, 0.617 mmol) and compound **113b** (73 mg, 0.26 mmol) in DMSO (1.5 mL) were heated to 65 °C. After 0.5 h, additional compound **113b** (196 mg ,0.629 mmol) was added in portions over 5 h with continuing heat. Extractive work up from EtOAc, washing with water, followed by isolation using preparative HPLC gave compound **113** (0.0101 g, 0.0195 mmol) as a white solid in 12% yield. 1H NMR (400 MHz, DMSO-d6) δ ppm 1.95 (s, 6 H) 2.36 (s, 1 H) 3.26 (t, J=5.81 Hz, 2 H) 3.57 (d, J=12.63 Hz, 1 H) 3.90 (d, J=13.14 Hz, 1 H) 4.31 - 4.39 (m, 4 H) 6.75 (s, 2 H) 6.87 (s, 1 H) 7.31 (d, J=1.77 Hz, 1 H) 7.33 (d, J=1.77 Hz, 1 H) 7.35 (d, J=3.28 Hz, 1H) 7.58 (d, J=3.28 Hz, 1 H). LCMS $(M+H)^+$: 519.2, 517.2.

**Preparation of Compound 113b, 2-(1,3-thiazol-2-yl)ethyl 4-methylbenzenesulfonate**

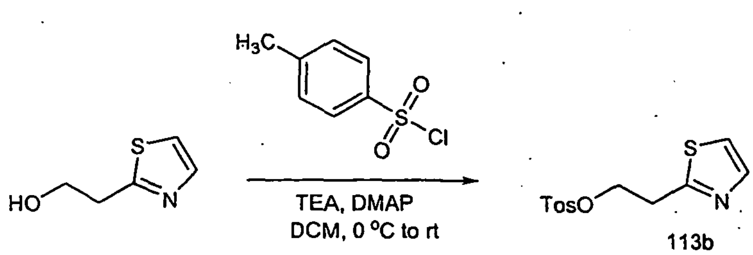

113b

To 2-(1,3-thiazol-2-yl)ethanol (123 mg, 0.952 mmol) (Young et. al. Eur. J. Med. Chem., 28: 201-211; (1993)) and triethylamine (0.15 mL, 1.1 mmol) in dichloromethane (5.0 mL) at 0°C was added 4-toluensulfonyl chloride (189 mg, 0.991 mmol) and an unmeasured amount of catalytic DMAP. The mixture was stirred overnight and allowed warm to warm to ambient temperature. Extractive work up from dichloromethane (30 mL), washing with saturated aqueous $NH_4Cl$, gave compound **113b** (0.255 g, 0.901 mmol) in 95% crude yield. 1H NMR (300 MHz, DMSO-$d_6$) δ ppm 2.42 (s, 3 H) 3.34 (t, *J*=6.03 Hz, 2 H) 4.36 (t, *J*=6.03 Hz, 2 H) 7.46 (d, *J*=8.48 Hz, 2 H) 7.60 (d, *J*=3.39 Hz, 1 H) 7.69 (d, *J*=3.39 Hz, 1 H) 7.72 (d, *J*=8.10 Hz, 2 H). LCMS $(M+H)^+$: 284.1.

**Example 114: 2-amino-*N*-bicyclo[1.1.1]pent-1-yl-4-{2,4-dichloro-6-[2-(4-methyl-1*H*-pyrazol-1-yl)ethoxy]phenyl}-5,7-dihydro-6*H*-pyrrolo[3,4-*d*]pyrimidine-6-carboxamide**

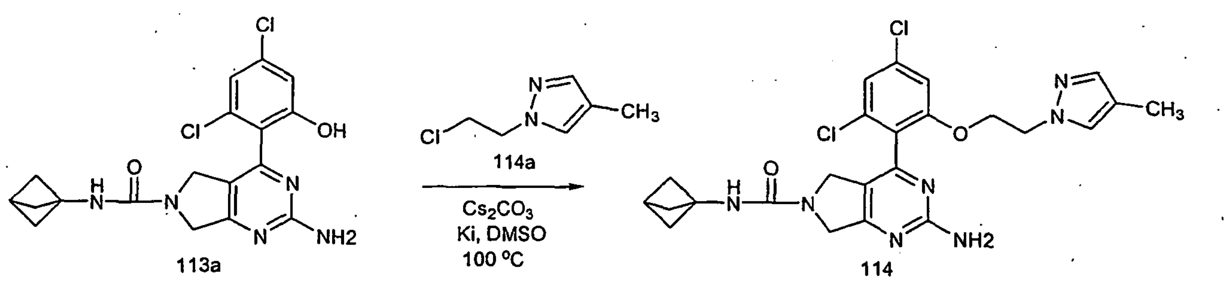

113a 114

Compound **113a** (59 mg, 0.15 mmol), compound **114a** (49 mg, 0.34 mmol), cesium carbonate (177 mg, 0.543 mmol), and potassium iodide (7 mg) in DMSO (1.5 mL) were heated at 100 °C for 6 h. Isolation by preparative HPLC gave compound **114** (0.0258 g, 0.0501 mmol) as a white solid in 35% yield. 1 H NMR (300 MHz, DMSO-d6) δ ppm 1.90 (s, 3 H) 1.94 (s, 6 H) 2.35 (s, 1 H) 3.69 (d, J=13.38 Hz, 1 H) 3.95 (d, J=12.24 Hz, 1 H) 4.20 - 4.39 (m, 6 H) 6.80 (s, 2 H) 6.89 - 6.93 (m, 1 H) 6.97 (s, 1 H) 7.10 (s, 1 H) 7.25 (d, J=1.51 Hz, 1 H) 7.33 (d, J=1.13 Hz, 1 H). LCMS $(M+H)^+$: 516.2, 514.2.

**Preparation of Compound 114a, 1-(2-chloroethyl)-4-methyl-1*H*-pyrazole**

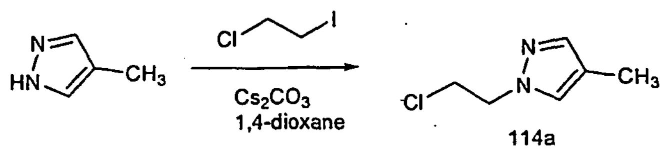

114a

4-Methyl pyrazole (0.177 g, 2.16 mmol), 1-chloro-2-iodoethane (1.20 g, 6.30 mmol) and cesium carbonate (1.10 g, 3.38 mmol) in 1,4-dioxane (5.0 mL) were heated at 95 °C for 24h. After cooling to ambient temperature, dichloromethane (10 mL) was added. Following filtration to remove solids, the filtrate was reduced and subjected to silica gel chromatography (gradient of 0-70% EtOAc in hexane) which gave compound **114a** (0.049 g, 0.34 mmol) as clear oil in 16% yield. 1 H NMR (300 MHz, CHLOROFORM-d) d ppm 2.12 (s, 3 H) 3.92 (t, *J*=5.84 Hz, 2 H) 4.49 (t, *J*=5.93 Hz, 2 H) 7.33 (s, 1 H) 7.46 (s, 1 H).

**Example 115: 2-amino-*N*-bicyclo[1.1.1]pent-1-yl-4-{2,4-dichloro-6-[3-(1*H*-pyrazol-1-yl)propoxy]phenyl}-5,7-dihydro-6*H*-pyrrolo[3,4-*d*]pyrimidine-6-carboxamide**

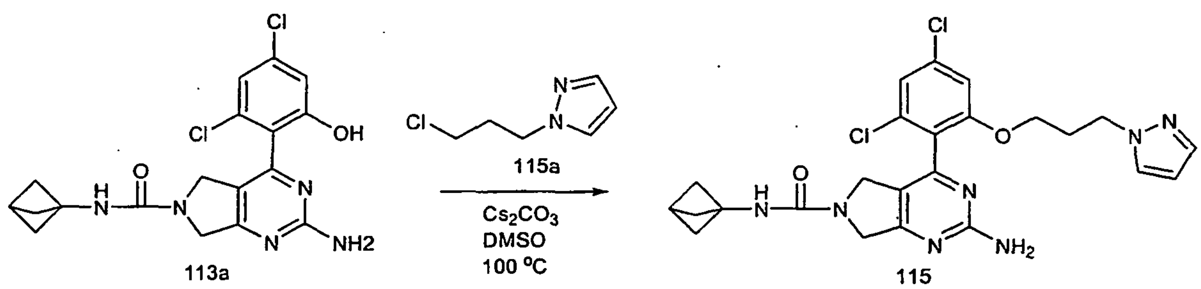

113a 115

Compound **115a,** 1-(3-chloropropyl)-1*H*-pyrazole, was prepared in a manner similar to compound **114a** except that pyrazole was substituted for 4-methylpyrazole and 1-bromo-3-chloropropane was substituted for 1-chloro-2-iodoethane. 1 H NMR (300 MHz, CHLOROFORM-d) δ ppm 2.33 - 2.42 (m, 2 H) 3.47 (t, *J*=6.03 Hz, 2 H) 4.41 (t, *J*=6.40 Hz, 2 H) 6.31 (t, *J*=2.17 Hz, 1 H) 7.49 (d, *J*=2.07 Hz, 1 H) 7.61 (d, *J*=1.51 Hz, 1 H). Compound **113a** (0.0442 g, 0.109 mmol) was then O-alkylated with compound **115a** (0.054 g, 0.37 mmol) and cesium carbonate (0.122g, 0.374 mmol) in DMSO (1.5 mL) at 100°C in a manner similar to Example **18.** Isolation using preparative HPLC gave compound **115** (0.0234, 0.0372 mmol) as a TFA salt in 34% yield. 1 H NMR (300 MHz, METHANOL-d4) d ppm 2.03 (s, 6 H) 2.12 - 2.22 (m, J=2.45 Hz, 2 H) 2.37 (s, 1 H) 3.86 - 3.94 (m, 1 H) 3.98 - 4.07 (m, 1 H) 4.12 (t, J=6.59 Hz, 2 H) 4.31 (s, 2 H) 4.57 (s, 2 H) 6.23 - 6.25 (m, 1 H) 7.14 (d, J=1.70 Hz, 1 H) 7.25 (d, J=1.70 Hz, 1 H) 7.45 (d, J=1.51 Hz, 1 H) 7.53 (d, J=2.07 Hz, 1 H). LCMS $(M+H)^+$: 516.2, 514.2.

**Example 116: 2-amino-*N*-cyclobutyl-4-[2,4-dichloro-6-(2-pyridin-2-ylethoxy)phenyl]-5,7-dihydro-6*H*-pyrrolo [3,4-*d*]pyrimidine-6-carboxamide**

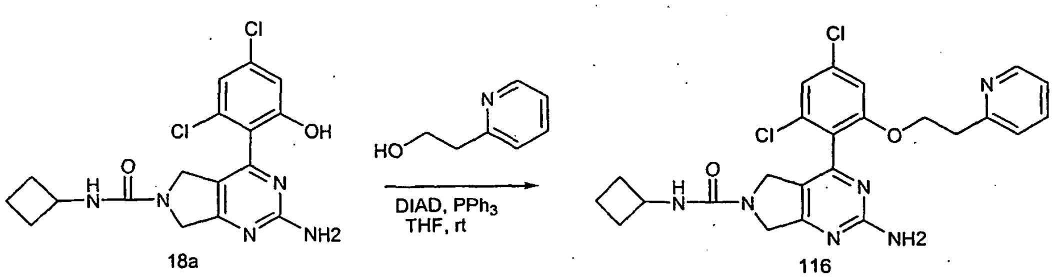

18a 116

To compound **18a** (0.032 g, 0.081 mmol), 2-(2-hydroxyethyl)pyridine, and triphenylphosphine (0.053 g, 0.20

mmol) in THF (1.5 mL) was added diisopropyl azodicarboxylate (40 μL, 0.207 mmol). After 3h, DMSO (1.5 mL) was added and the volatile components removed under vacuum. Isolation using preparative HPLC gave compound **116** (0.0208 g, 0.0416 mmol) as a tan powder in 51% yield. 1H NMR (300 MHz, DMSO-d6) δ ppm 1.49 - 1.63 (m, 2 H) 1.88 - 2.00 (m, 2 H) 2.05 - 2.21 (m, 2 H) 2.93 - 3.02 (m, 2 H) 3.38 (d, 1 H, overlaps with water peak) 3.86 (d, J=13.00 Hz, 1 H) 4.08 - 4.23 (m, 1 H) 4.28 - 4.45 (m, 4 H) 6.37 (d, J=8.10 Hz, 1 H) 6.77 (s, 2 H) 6.91 (d, J=7.72 Hz, 1 H) 6.99 - 7.06 (m, 1 H) 7.28 - 7.32 (m, 2 H) 7.50 (dt, J=7.58, 1.79 Hz, 1 H) 8.36 (d, J=4.14 Hz, 1 H). LCMS $(M+H)^+$: 501.2, 499.2.

**Example 117: 2-amino-*N*-cyclobutyl-4-(4,6-dichloro-2,3-dimethoxyphenyl)-5,7-dihydro-6*H*-pyrrolo[3,4-*d*]pyrimidine-6-carboxamide**

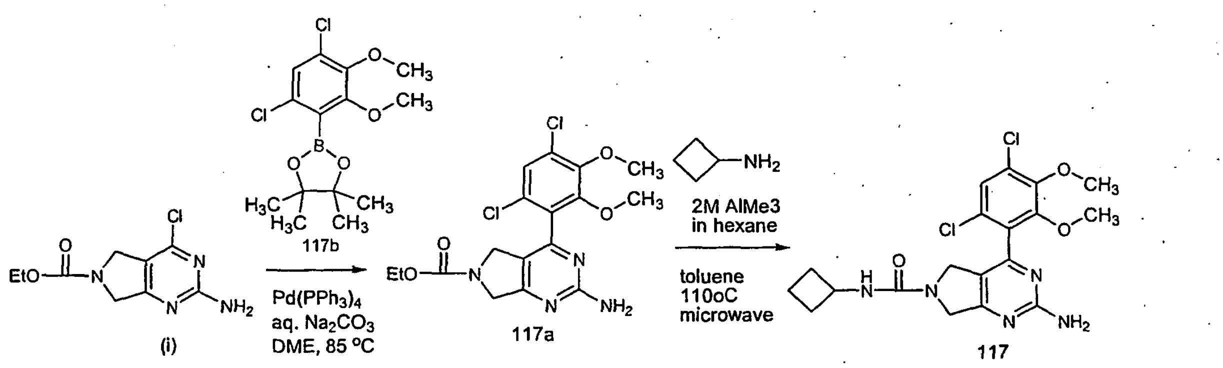


(i) 117a 117

Compound (i) (0.263 g, 1.08 mmol), compound **117b** (0.525 g, 1.58 mmol), 2M aqueous sodium carbonate (0.80 mL, 1.6 mmol) and tetrakis(triphenylphosphino)palladium(0) (0.075 g, 0.065 mmol) in DME (10 mL) were reacted in a manner similar to Example **1.** Extractive work up with EtOAc (30 mL), washing with water (30 mL) followed by isolation using silica gel chromatography (gradient of 0-25% MeOH in dichloromethane) gave compound **117a,** ethyl 2-amino-4-(4,6-dichloro-2,3-dimethoxyphenyl)-5,7-dihydro-6*H*-pyrrolo[3,4-d]pyrimidine-6-carboxylate, (0.034 mg, 0.082 mmol) in 8% yield. Compound **117a** (0.034 g, 0.082 mmol), cyclobutylamine (0.040 g, 0.60 mmol) and 2M trimethyl aluminum in hexane (0.25 mL, 0.50 mmol) were reacted in a manner similar to Example **7.** Isolation using preparative HPLC gave compound **117** (0.010 g, 0.023 mmol) in 28% yield. 1 H NMR (300 MHz, DMSO-d6) δ ppm 1.49 - 1.61 (m, 2 H) 1.85 - 2.00 (m, 2 H) 2.03 - 2,15 (m, 2 H) 3.69 (s, 3 H) 3.85 (s, 3 H) 4.07 - 4.24 (m, 3 H) 4.43 (s, 2 H) 6.51 (d, J=7.91 Hz, 1 H) 6.89 (br. s., 2 H) 7.60 (s, 1 H). LCMS $(M+H)^+$: 440.2, 438.2.

**Preparation of Compound 117b, 2-(4,6-dichloro-2,3-dimethoxyphenyl)-4,4,5,5-tetramethyl-1,3,2-dioxaborolane**

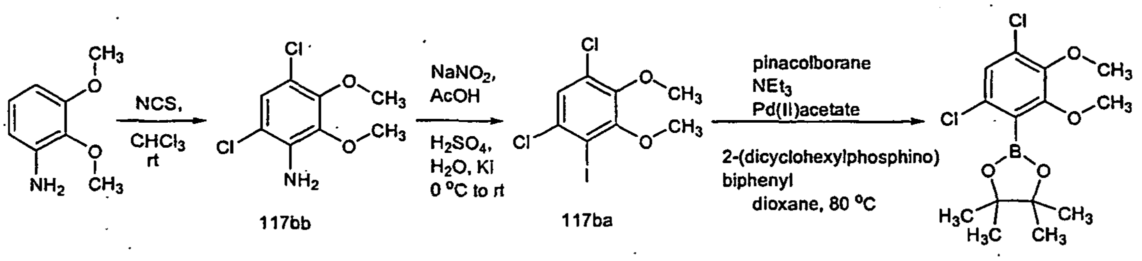


117bb 117ba 117b

N-chlorosuccinimide (6.87 g, 50.4 mmol) was added to 2,3-dimethoxyaniline (3.86 g, 25.2 mmol) in chloroform (100 mL) at 0°C. After allowing to warm to ambient temperature and stir for an additional 4h, isolation by silica gel chromatography (dichloromethane) gave compound **117bb,** 4,6-dichloro-2,3-dimethoxyaniline, (1.97 g, 8.87 mmol) as an orange oil in 35% yield. 1H NMR (300 MHz, DMSO-$d_6$) δ ppm 3.74 (s, 3 H) 3.77 (s, 3 H) 5.27 (br. s., 2 H) 7.12 (s, 1 H). With stirring, sodium nitrite (799 mg, 11.6 mmol) in $H_2O$ (4.0 mL) was added dropwise to compound **117bb** (1.97 g, 8.87 mmol) in acetic acid (20.0 mL), $H_2O$ (6.0 mL) and concentrtaed $H_2SO_4$ (6.0 mL) at 0°C. After 3 h at 0°C, the mixture was poured into a potassium iodide (10.4 g, 62.7 mmol) solution in $H_2O$ (20.0 mL) at 0°C rinsing with additional $H_2O$

(20 mL). The mixture was allowed to slowly warm to ambient temperature and stirred for an additional 16 h. The mixture was then extracted with diethyl ether (100mL) and the $Et_2O$ layer washed with saturated aqueous $Na_2S_2O_3$ (100 mL), 3N NaOH (100 mL) and water (100mL). Isolation using silica gel chromatography (gradient of 0-20% dichloromethane in hexane) gave compound **117ba,** 1,5-dichloro-2-iodo-3,4-dimethoxybenzene, (2.46 g, 7.39 mmol) in 83% yield. 1 H NMR (300 MHz, DMSO-$d_6$) δ ppm 3.81 (s, 3 H) 3.82 (s, 3 H) 7.62 (s, 1 H). Compound **117ba** (2.45 g, 7.36 mmol), pinacolborane (2.1 mL, 14.5 mmol), and triethyl amine (3.00 mL, 21.5 mmol) in 1,4-dioxane (40 mL) were purged with $N_2$ for 15 min and palladium (II) acetate (86 mg, 0.38 mmol) and 2-(dicyclohexylphosphino)biphenyl (263 mg, 0.750 mmol) were added. The mixture was heated to 80˚C for 2.5 h, cooled, and subjected to EtOAc extractive work up, washing with saturated aqueous $NH_4Cl$. Isolation using silica gel chromatography (gradient of 0-50% dichloromethane in hexane) gave compound **117b** (1.155 g, 3.468 mmol) in 47% yield. 1H NMR (300 MHz, DMSO-$d_6$) δ ppm 1.32 (s, 12 H) 3.79 (s, 6 H) 7.36 (s, 1 H).

**Example 118: 2-amino-4-(4-bromo-2-chloro-5-methoxyphenyl)-*N*-(6-cyanopyridin-3-yl)-5,7-dihydro-6*H*-pyrrolo [3,4-*d*]pyrimidine-6-carboxamide**

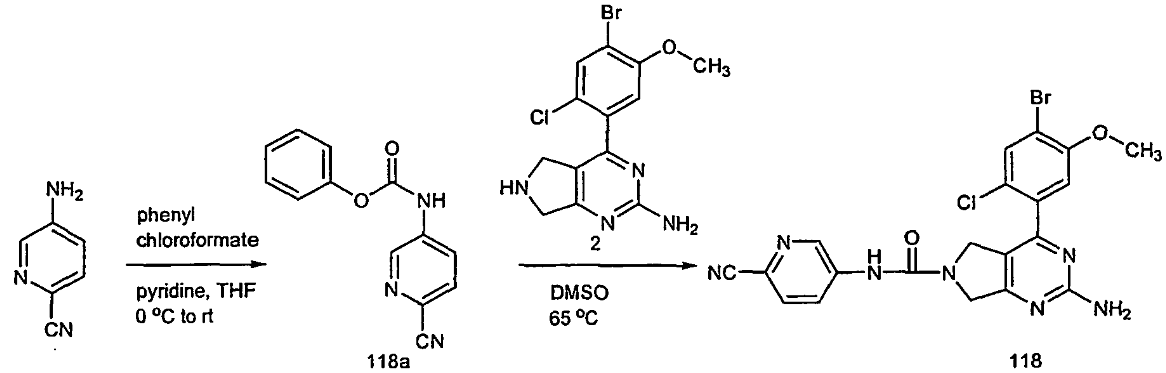


118a 118

Phenylchloroformate (0.70 mL, 5.6 mmol) in THF (10 mL) was added dropwise to 5-amino-2-cyanopyridine (655 mg. 5.50 mmol) and pyridine (0.50 mL, 6.2 mmol) in THF (10.0 mL) at 0˚C. After allowing to warm to ambient temperature and stirring for 1h, the mixture was again cooled to 0˚C, and the precipitate removed by filtration. Removal of the volatile components from the filtrate gave compound **118a,** phenyl (6-cyanopyridin-3-yl)carbamate, (0.921, 3.85 mmol) as a pale orange solid in 70% crude yield. 1H NMR (300 MHz, DMSO-$d_6$) δ ppm 7.24 - 7.33 (m, 3 H) 7.45 (t, *J*=7.82 Hz, 2 H) 8.01 (d, *J*=8.48 Hz, 1 H) 8.13 (dd, *J*=8.48, 2.55 Hz, 1 H) 8.81 (d, *J*=2.45 Hz, 1 H) 11.01 (s, 1 H). Compound **2** (0.093 g, 0.152 mmol), compound **118a** (0.038 g, 0.160 mmol), and cesium carbonate (0.154 g, 0.473 mmol) in DMSO (1.5 mL) were heated to 65 ˚C for 20 min. Isolation using preparative HPLC gave compound **118** (0.013 g, 0.026 mmol) as an orange solid in 17% yield. 1 H NMR (300 MHz, DMSO-d6) δ ppm 3.87 (s, 3 H) 4.52 (br. s., 2 H) 4.64 (br. s., 2 H) 6.97 (br. s., 2 H) 7.19 (s, 1 H) 7.87 - 7.94 (m, 2 H) 8.20 (dd, J=8.57, 2.17 Hz, 1 H) 8.87 (d, J=2.45 Hz, 1 H) 9.04 (br. s., 1 H). LCMS $(M+H)^+$: 502.2, 500.2.

**Example 119: 2-amino-*N*-cyclobutyl-4-[(*E*)-2-phenylvinyl]-5,7-dihydro-6*H*-pyrrolo[3,4-*d*]pyrimidine-6-carboxamide**

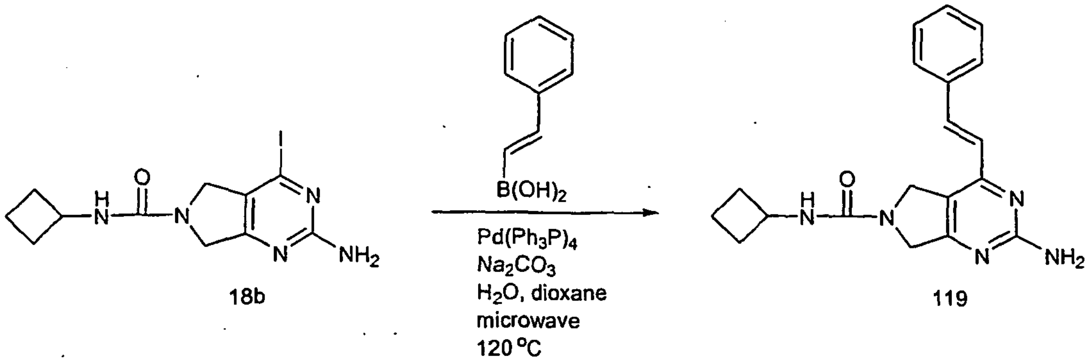


Compound **18b** (0.103 g, 0.287 mmol), trans-2-phenylvinylboronic acid (0.0602 mg, 0.407 mmol) and 2M aqueous sodium carbonate (0.30 mL, 0.60 mmol) in 1,4-dioxane (3.0 mL) were purged with $N_2$. Tetrakis(triphenylphosphino)palladium(0) (0.024 g, 0.021 mmol) was added and the mixture microwaved at 120˚C for 1.5 h. After EtOAc (20 mL) extractive work up, washing with water (20 mL), isolation using preparative HPLC gave compound **119** (0.022 g, 0.066 mmol) in 23% yield. 1 H NMR (300 MHz, DMSO-d6) δ ppm 1.52 - 1.68 (m, 2 H) 1.92 - 2.07 (m, 2 H) 2.09 - 2.23 (m, 2 H) 4.11 - 4.28 (m, 1 H) 4.37 (s, 2 H) 4.60 (s, 2 H) 6.52 (d, J=7.72 Hz, 1 H) 6.59 (br. s., 2 H) 7.02 (d, J=16.01 Hz, 1 H) 7.34 - 7.48 (m, 3 H) 7.63 - 7.77 (m, 3 H). LCMS $(M+H)^+$: 336.4.

**Example 120: 2-{[2-amino-4-(4-bromo-2-chloro-5-methoxyphenyl)-5,7-dihydro-6*H*-pyrrolo [3,4-*d*]pyrimidin. 6-yl]methyl}benzonitrile**

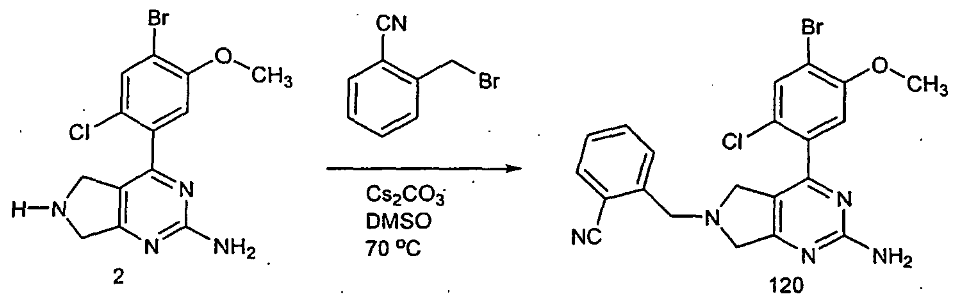


Compound **2** (0.067 g, 0.110 mmol), 2-(bromomethyl)-benzonitrile (0.0215 mg; 0.110 mmol) and cesium carbonate (0.1189 g, 0.365 mmol) in DMSO (1.5 mL) were heated at 70 ˚C for 10 min. Isolation using preparative HPLC gave compound **120** (0.007 g, 0.01 mmol) in 10% yield. 1 H NMR (300 MHz, DMSO-d6) δ ppm 3.67 (s, 2 H) 3.83 (s, 2 H) 3.84 (s, 3 H) 4.03 (s, 2 H) 6.79 (br. s., 2 H) 7.10 (s, 1 H) 7.47 (t, J=7.44 Hz, 1 H) 7.60 - 7.71 (m, 2 H) 7.79 - 7.84 (m, 2 H). LCMS $(M+H)^+$: 472.2, 470.2.

**Example 121: 2-amino-4-(2-chloro-4-cyano-5-methoxyphenyl)-*N*-isopropyl-5,7-dihydro.6*H*-pyrrolo[3,4-*d*]pyrimidine-6-carboxamide**

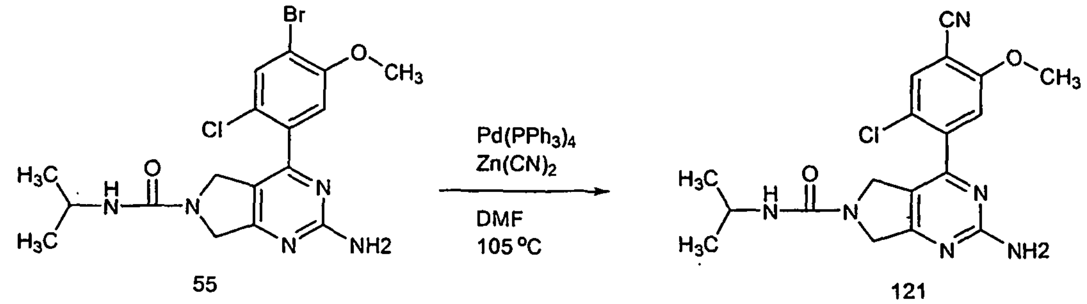

55
121

Compound **55** (0.0247 g, 0.056 mmol) and zinc cyanide (0.0212 g, 0.181 mmol) in DMF (1.5 mL) were purged with $N_2$. Tetrakis(triphenylphosphino)palladium(0) (0.0102 mg, 0.00883 mmol) was added and the mixture was heated at 105 °C for 5h. Ethyl acetate (50 mL) extractive work up, washing with water (50 mL) followed by isolation using preparative HPLC gave compound **121** (0.008 g) as the hydrochloride in 30% yield. 1 H NMR (300 MHz, DMSO-d6) d ppm 1.06 (d, J=6.59 Hz, 6 H) 3.71 - 3.85 (m, 1 H) 3.93 (s, 3 H) 4.25 (s, 2 H) 4.43 (s, 2 H) 6.07 (d, J=7.54 Hz, 1 H) 6.95 (br. s., 2 H) 7.34 (s, 1 H) 8.12 (s, 1 H). LCMS $(M+H)^+$: 387.2.

**Example 122: 2-amino-4-(4-bromo-2-chloro-5-methoxyphenyl)-5,7-dihydro-6*H*-pyrrolo[3,4-*d*]pyrimidine-6-carboximidamide**

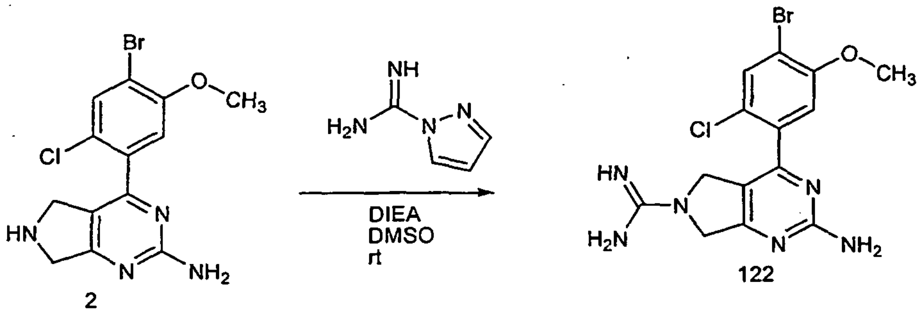

2
122

To compound **2** (0.0779 g, 0.105 mmol) in DMSO (1.0 mL) at ambient temperature was added diisopropyl ethylamine (0.10 mL, 0.57 mmol) and (1H)-pyrazole-1-carboxamidine hydrochloride (0.0201 g, 0.137 mmol). After stirring for 16h, isolation using preparative HPLC gave compound **122** (0.016 g, 0.040 mmol) as a white solid in 38% yield. 1 H NMR (300 MHz, DMSO-d6) δ ppm 3.88 (s, 3 H) 4.45 (s, 2 H) 4.60 (s, 2 H) 7.07 (br. s., 2 H) 7.17 (s, 1 H) 7.31 - 7.47 (br. m, 3 H) 7.90 (s, 1 H). LCMS $(M+H)^+$: 399.0, 397.2.

**Example 123: ethyl 2-amino-4-(2,4-dichloro-5-ethylphenyl)-5,7-dihydro-6*H*-pyrrolo[3,4-*d*]pyrimidine-6-carboxylate**

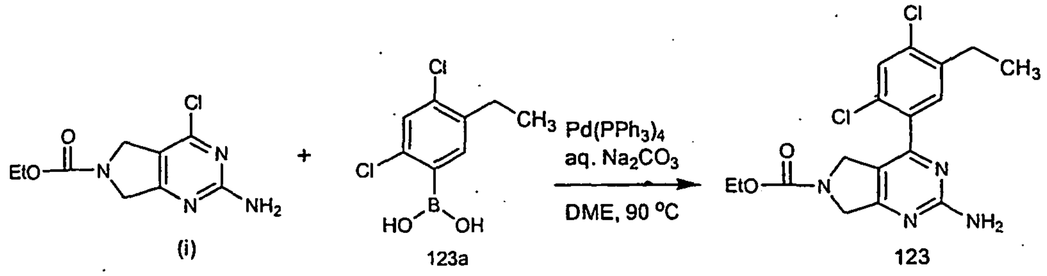

(i)
123a
123

Compound (i) (0.0953 g, 0.393 mmol), compound **123a** (0.086 mg, 0.39 mmol), 2M aqueous sodium carbonate (0.4 mL, 0.8 mmol), and tetrakis(triphenylphosphino)palladium(0) (0.0227 mg, 0.0196 mmol) in DME (4 mL) were reacted in a manner similar to Example 1. Isolation using preparative HPLC gave compound **123** (0.047 g, 0.12 mmol) as a white powder in 31% yield. 1H NMR (300 MHz, DMSO-d6) δ ppm 1.13 - 1.24 (m, 6 H) 2.73 (q, J=7.41 Hz, 2 H) 4.01 - 4.14 (m, 2 H) 4.30 - 4.36 (m, 2 H) 4.44 - 4.51 (m, 2 H) 6.94 (br. s., 2 H) 7.42 (s, 1 H) 7.73 (s, 1 H). LCMS $(M+H)^+$: 383.2, 381.2.

**Preparation of Compound 123a, (2,4-dichloro-5-ethylphenyl)boronic acid**

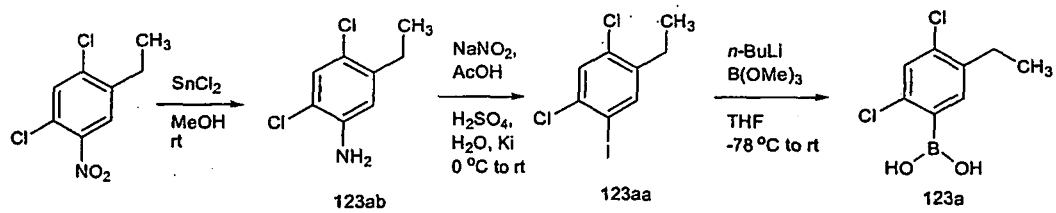

123ab 123aa 123a

1,5-Dichloro-2-ethyl-4-nitrobenzene (2.1 g, 9.5 mmol) and $SnCl_2$ (8.1 g, 42.8 mmol) were refluxed in anhydrous methanol until the reduction was complete. Aqueous 10% NaOH and EtOAc were added, and the resulting suspended solids were allowed to settle and most of the EtOAc was decanted. This procedure was repeated several times and the EtOAc batches were combine and filtered through celite, dried ($Na_2SO_4$), filtered, and the volatile components removed. The residue was dissolved in $Et_2O$ and excess 4M HCl in 1,4-dioxane was added. Evaporation of the volatile components gave compound **123ab,** 2,4-dichloro-5-ethylaniline, (2.1 g, 9.27 mmol) as a yellow hydrochloride in 97% yield. Compound **123ab** was converted to compound **123aa,** 1,5-dichloro-2-ethyl-4-iodobenzene, (0.577 g, 1.92 mmol) in 36% yield in a manner similar to Example **117ba** except that compound 123ab was substituted for compound **117bb.** 1H NMR (300 MHz, CHLOROFORM-d) δ ppm 1.22 (t, *J*=7.54 Hz, 3 H) 2.69 (q, *J*=7.54 Hz, 2 H) 7.44 (s, 1 H) 7.70 (s, 1 H). To compound **123aa** (0.576 g, 1.91 mmol) in THF (15.0 mL) at -78 °C was added 2M n-BuLi in cyclohexane (1.00 mL, 2.00 mmol) dropwise. After 1h, trimethyl borate (0.30 mL, 2.7 mmol) was added dropwise, and the reaction was allowed to warm to ambient temperature. Water (50 mL) followed by ether/hexanes (25mL/25 mL) were added, and the layers separated. The organic layer was then extracted with 0.5N NaOH (30 mL), and the aqueous extraction was brought to pH 6 with 1 N $KH_2PO_4$ (15 mL) and extracted with EtOAc (50 mL). Removal of the volatile components of the EtOAc layer gave compound **123a** (0.088 g) as white waxy solid which was carried on without purification.

**Example 124: ethyl 2-amino-4-[2-chloro-6-(4,4,4-trifluorobutoxy)phenyl]-5,7-dihydro-6*H*-pyrrolo[3,4-*d*]pyrimidine-6-carboxylate**

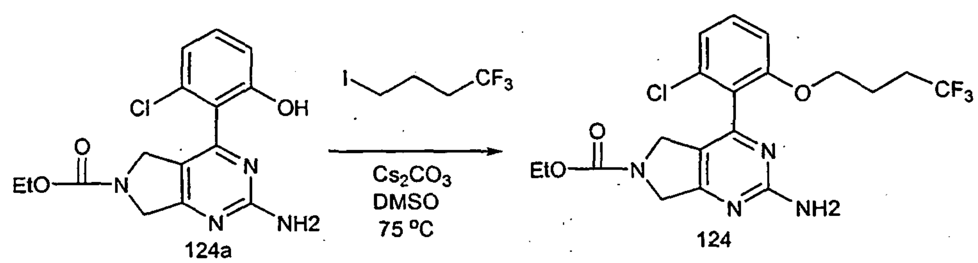

124a 124

Compound **124a** (0.0763 g, 0.228 mmol) was O-alkylated with 1-iodo-4,4,4-trifluorobutane (0.0801 g, 0.337 mmol) and cesium carbonate (0.0992 mg, 0.304 mmol) in DMSO (1.0 mL) in a manner similar to that described for Example **18.** Isolation using preparative HPLC gave compound **124** (0.0353 g, 0.0794 mmol) as a white powder in 35% yield. 1 H NMR (300 MHz, DMSO-d6) δ ppm 1.11 - 1.25 (m, 3 H) 1.70 - 1.82 (m, 2 H) 2.03 - 2.23 (m, 2 H) 3.99 - 4.21 (m, 6 H) 4.37 - 4.57 (m, 2 H) 6.83 (br. s., 2 H) 7.11 - 7.20 (m, 2 H) 7.44 (t, J=8.19 Hz, 1 H). LCMS $(M+H)^+$: 445.2.

**Preparation of Compound 124a, ethyl 2-amino-4-(2-chloro-6-hydroxyphenyl)-5,7-dihydro-6*H*-pyrrolo[3,4-*d*]pyrimidine-6-carboxylate**

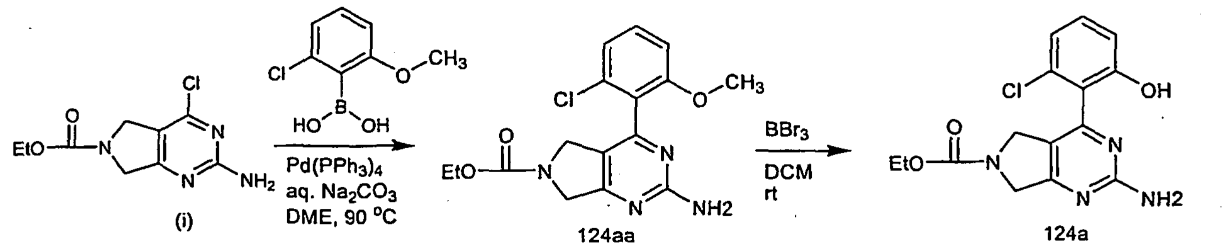


Compound (i) (0.300 g, 1.24 mmol), 2-chloro-6-methoxyphenylboronic acid (0.351 g, 1.88 mmol), 2M aqueous sodium carbonate (1.85 mL, 3.70 mmol) and tetrakis(triphenylphosphino) palladium(0) (0.0789 g, 0.0683 mmol) in DME (10.0 mL) were reacted in a manner similar to that described for Example 1 to give crude compound **124aa,** ethyl 2-amino-4-(2-chloro-6-methoxyphenyl)-5,7-dihydro-6*H*-pyrrolo[3,4-*d*]pyrimidine-6-carboxylate, (0.481 g) which was carried on without purification. LCMS $(M+H)^+$: 349.2. To compound **124aa** (0.407 g) in dichloromethane

(10.0 mL) at 0˚C was added dropwise 1 M boron tribromide (0.20 mL, 2.1 mmol) in dichloromethane. The mixture was allowed to warm to ambient temperature and stir for 2h. Additional boron tribromide (0.20 mL, 2.1 mmol) in dichloromethane was added and the mixture stirred for an additional 4h. The mixture was then poured into cold saturated aqueous $NaHCO_3$ and extracted with dichloromethane/MeOH. Removal of the volatile components gave compound **124a** (0.249 g) as an orange residue which was carried on without purification. LCMS $(M+H)^+$: 335.2. **Example 125: ethyl 2-amino-4-(4-bromo-2-ethylphenyl)-5,7-dihydro-6*H*-pyrrolo[3,4-*d*]pyrimidine-6-carboxylate**

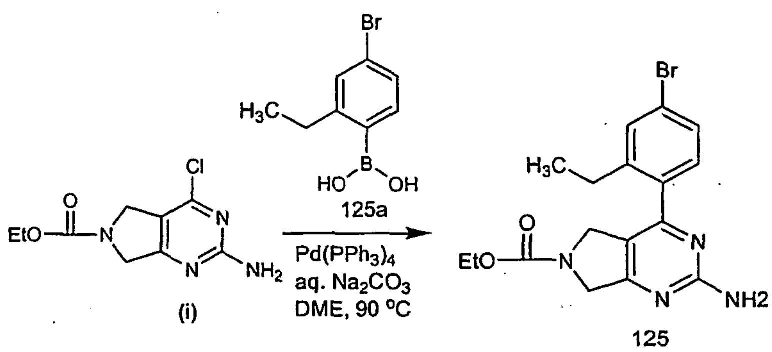


Compound **125a,** 4-bromo-2-ethylphenylboronic acid, was prepared in a similar manner to Example **123a** except that 4-bromo-2-ethyl-1-iodobenzene was substituted for compound **123aa.** 1 H NMR (300 MHz, DMSO-$d_6$) δ ppm 1.19 (t, *J*=7.35 Hz, 3 H) 3.02 (q, *J*=7.41 Hz, 2 H) 7.33 - 7.41 (m, 2 H) 7.74 (d, *J*=7.91 Hz, 1 H). Compound (i) (0.114 g, 0.470 mmol), compound **125a** (0.0852 g, 0.372 mmol), 2M aqueous sodium carbonate (0.50 mL, 1.0 mmol) and tetrakis (triphenylphosphino)palladium(0) (0.031 g, 0.027 mmol) in DME (3.5 mL) were reacted in a manner similar to that described for Example **1.** Isolation using preparative HPLC gave compound **125** (0.029 g, 0.074 mmol) in 15% yield. 1H NMR (300 MHz, DMSO-d6) δ ppm 1.02 (t, J=7.54 Hz, 3 H) 1.13 - 1.25 (m, 3 H) 2.55 - 2.63 (m, 2 H) 4.00 - 4.13 (m, 2 H) 4.26 - 4.32 (m, 2 H) 4.44 - 4.50 (m, 2 H) 6.82 (br. s., 2 H) 7.24 (d, J=7.91 Hz, 1 H) 7.48 (dd, J=8.01, 1.60 Hz, 1 H) 7.57 (d, J=1.70 Hz, 1 H). LCMS $(M+H)^+$: 393.2, 391.2.

**Example 126: 2-amino-*N*-cyclobutyl-4-(2,5-dimethoxy-4-methylphenyl)-5,7-dihydro-6*H*-pyrrolo[3,4-*d*]pyrimidine-6-carboxamide**

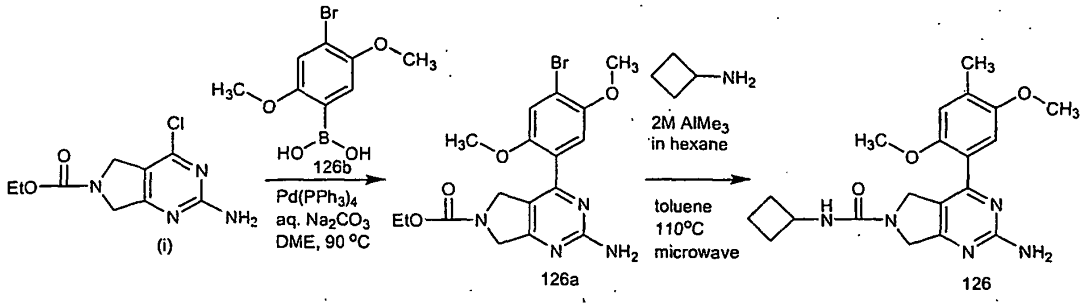


Compound **126b,** 4-dibromo-2,5-dimethoxyphenylboronic acid, was prepared in a similar manner as Example **123a** except that 1,4-dibromo-2,5-dimethoxybenzene was substituted for compound **123aa.** 1 H NMR (300 MHz, DMSO-$d_6$) δ ppm 3.77 (s, 3 H) 3.78 (s, 3 H) 7.20 (s, 1 H) 7.22 (s, 1 H) 7.83 (s, 2 H). Compound (i) (0.114 g, 0.470 mmol), compound **126b** (0.112 g, 0.429 mmol), 2M aqueous sodium carbonate (0.50 mL, 1.0 mmol) and tetrakis(triphenylphosphino)palladium(0) (0.030 g, 0.026 mmol) in DME (3.5 mL) were reacted in a manner similar to Example **1** to give compound **126a,** ethyl 2-amino-4-(4-bromo-2,5-dimethoxyphenyl)-5,7-dihydro-6*H*-pyrrolo[3,4-d]pyrimidine-6-carboxylate, (0.065 g, 0.154 mmol) in 36% yield which was carried on without purification. 1 H NMR (300 MHz, DMSO-$d_6$) δ ppm 1.14 - 1.25 (m, 3 H) 3.78 (s, 3 H) 3.80 (s, 3 H) 4.02 - 4.14 (m, 2 H) 4.27 - 4.33 (m, 2 H) 4.42 - 4.48 (m, 2 H) 6.82 (br. s., 2 H) 7.05 (s, 1 H) 7.39 (s, 1 H). LCMS $(M+H)^+$: 425.2, 423.2. Cyclobutylamine (0.10 mL, 1.2 mmol), 2.0 M trimethylaluminum (0.30 mL, 0.6 mmol) in hexane, and compound **126a** (49.2 mg, 0.116 mmol) in toluene (2.0 mL) were microwaved for 1 h in a manner similar to Example 7. Work up, also in a similar manner, and isolation using preparative HPLC gave compound **126** (0.0174 g, 0.0454 mmol) in 30%yield as a TFA salt (conversion of the bromine in compound **126a** to the methyl group of compound **126** was unanticipated). 1 H NMR (300 MHz, DMSO-d6) δ ppm 1.50 - 1.63 (m, 2 H) 1.88 - 2.03 (m, 2 H) 2.06 - 2.17 (m, 2 H) 2.24 (s, 3 H) 3.77 (s, 6 H) 4.10 - 4.20 (m, 1 H) 4.28 (s, 2 H) 4.42 (s, 2 H) 6.53 (d, J=7.72 Hz, 1 H) 6.91 (s, 1 H) 7.04 (s, 1 H). LCMS $(M+H)^+$: 384.4.

**Example 127: 2.amino-N-cyclopropyl-4-{2,4-dichloro-5-[2-(1H-imidazol-1-yl)ethoxy]phenyl}-5,7-dihydro-6H-pyrrolo[3,4-*d*]pyrimidine-6-carboxamide**

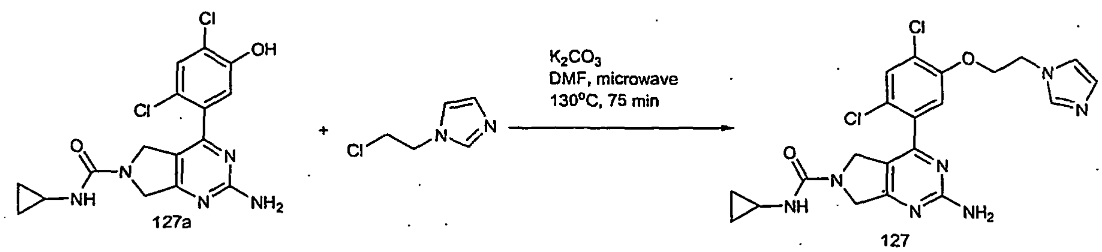


Compound **127a** (70 mg, 0.184 mmol), 1-(2-chloroethyl)-1*H*-imidazole HCl (84 mg, 0.55 mmol), and potassium carbonate (127 mg, 0.92 mmol) in DMF (2 mL) were reacted in a manner similar to Example **100.** Isolation using preparative HPLC gave compound **127** (9.0 mg, 0.02 mmol) as a powder in 11% yield. $^1$H-NMR (DMSO-d6, 300 MHz): δ 7.71 (s, 1 H), 7.63 (s, 1H), 7.19 (s, 1 H), 7.16 (s, 1 H), 6.85 (m, 3H), 6.43 (s, 1 H) 4.37 (s, 4H), 4.31 (m, 2H), 4,18 (s, 2H), 2.69 (bs, 1 H), 0.50 (m, 2H), 0.36 (m, 2H).

**Preparation of Compound 127a, 2-amino-*N*-cyclopropyl-4-(2,4-dichloro-5-hydroxyphenyl)-5,7-dihydro-6*H*-pyrrolo[3,4-*d*]pyrimidine-6-carboxamide**

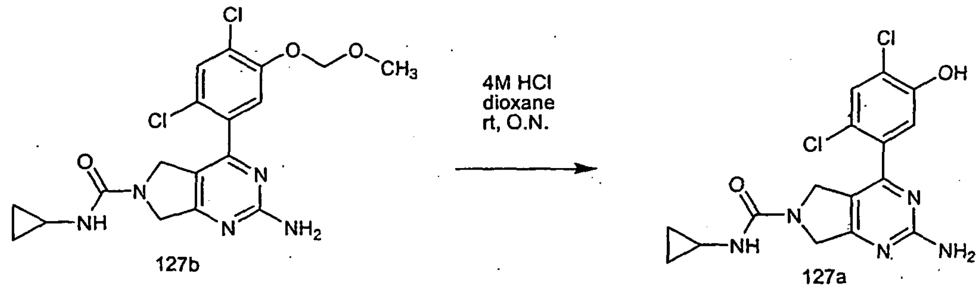


127b

127a

To a suspension of compound **127b** (640 mg, 1.51 mol) in MeOH (4 mL) was added 4 M HCl in 1,4-dioxane (4 mL), 16.0 mmol). After stirring overnight, the volatile components were removed and the residue subjected to EtOAc extractive work up, washing with saturated aqueous $NaHCO_3$ Drying ($Na_2SO_4$), filtration, and evaporation of the volatile components gave compound **127a** (426 mg, 1.12 mmol) as a powder in 74% yield which was carried on without purification. 1H NMR (400 MHz, DMSO-d6) d ppm: 10.77 (s, 1 H), 7.62 (s, 1 H), 6.94 (s, 1 H), 6.87 (s, 2 H), 6.47 (d, J=2.78 Hz, 1 H), 4.39 (s, 2 H), 4.23 (s, 2 H), 2.51 - 2.56 (m, 1 H), 0.50 - 0.57 (m, 2 H), 0.36 - 0.43 (m, 2 H). LCMS $(M+H)^+$: 380.2, 382.2

**Preparation of Compound 127b, 2-amino-*N*-cyclopropyl-4-[2,4-dichloro-5-(methoxymethoxy)phenyl]-5,7-dihydro-6*H*-pyrrolo[3,4-*d*]pyrimidine-6-carboxamide**

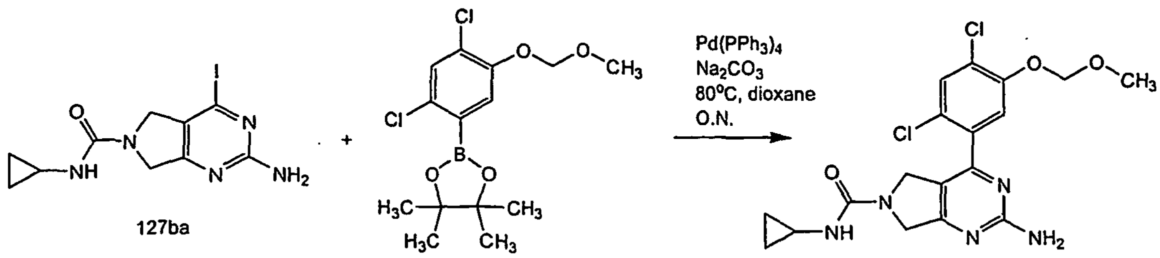


127ba

127c

127b

Compound **127ba,** 2-amino-*N*-cyclopropyl-4-iodo-5,7-dihydro-6*H*-pyrrolo[3,4-d]pyrimidine-6-carboxamide, was prepared from compound **8b** in a manner similar to example **18b** except that cyclopropylamine was substituted for cyclobutylamine. Compound **127ba** (825 mg, 2.39 mmol), compound **127c** (876 mg, 2.63 mmol), 2M aqueous sodium carbonate (3.6 mL, 7.18 mmol) and tetrakis(triphenylphosphine) Palladium(0) (276 mg, 0.239 mmol) in 30 mL 1,4-dioxane were reacted in a manner similar to Example **18a.** After filtering and washing the collected insoluble material with EtOAc, the combined filtrate/washings were concentrated and subjected to EtOAc (100mL) extractive work up, washing with water (200mL), brine (2x100mL), and drying ($Na_2SO_4$). Isolation using silica gel chromatography (gradient 0-5% MeOH in EtOAc) gave compound **127b** (656 mg, 1.55 mmol) as a solid in 65% yield. 1 H NMR (400 MHz, DMSO-d6) d ppm: 7.78 (s, 1 H), 7.28 (s, 1 H), 6.90 (s, 2 H), 6.46 (d, J=2.78 Hz, 1 H), 5.32 (s, 2 H), 4.40 (s, 2 H), 4.23 (s, 2 H), 3.41 (s, 3 H), 0.49 - 0.60 (m, 2 H), 0.36 - 0.44 (m, 2 H). LCMS $(M+H)^+$: 424.2, 426.2.

**Preparation of Compound 127c, 2-(2,4-dchloro-5-methoxymethoxy-phenyl)-4,4,5,5-tetramethyl-[1,3,2]dioxaborolane**

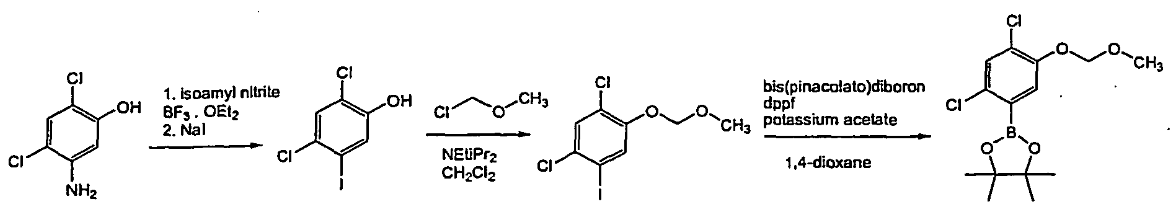


127cb 127ca 127c

$BF_3$ $OEt_2$ (70.6 mL, 560 mmol) was cooled to 0˚C and 5-amino-2,4-dichlorophenol (50.0 g, 280 mmol) in THF (700 mL) was added over 45 min. Then isoamyl nitrite (48.8 mL, 365 mmol) in THF (150 mL) was added over 15 min. After stirring for 30 min at 0˚C a yellow precipitate formed, more $Et_2O$ was added and the yellow solid was collected by filtration, washed with $Et_2O$, and air dried. The collected solid was added in portions to a solution of NaI (54.7 g, 365 mmol) in acetone (1.2 L) and stirred at ambient temperature overnight. The volatile components were reduced, water was added and the mixture was subjected to EtOAc extractive work up, washing with saturated aqueous $NaHSO_3$ and dried ($Na_2SO_4$). Isolation after silica gel chromatography plug ($CH_2Cl_2$) gave a mixture contaminated by the des-iodo impurity. Recrystallization from hexanes (3 crops) gave compound **127cb,** 2,4-dichloro-5-iodophenol, (43.4 g, 150 mmol) as a yellow solid in 54% yield. 1H NMR (400 MHz, CHLOROFORM-d) d ppm 3.77 (q, *J*=7.07 Hz, 3 H) 5.27 (s, 2 H) 7.45 (s, 1 H) 7.66 (s, 1 H). Compound **127cb** in $CH_2Cl_2$ (400 mL) was cooled to 0˚C and methoxymethyl chloride (13.7 mL, 180 mmol) followed by diisopropylethyl amine (31.5 mL, 23.3 g, 180 mmol) were added. After warming to ambient temperature overnight, the $CH_2Cl_2$ solution was washed with saturated aqueous $NaHCO_3$ and dried over $Na_2SO_4$. Filtration and evaporation of the volatile components gave compound **127ca,** 1,5-dichloro-2-iodo-4-(methoxymethoxy) benzene, (46.8 g, 141 mmol) as a yellow solid in 94% yield. Compound **127ca** (41.8 g, 125 mmol), bis(pinacolato)diboron (35.1 g, 138 mmol), potassium acetate (24.6 g, 250 mmol), bis(diphenylphosphino)ferrocene-dichloropalladium dichloromethane adduct (6.15 g, 7.5 mmol), and dry 1,4-dioxane (375 mL) were purged with $N_2$ for 1 h and heated to 90 ˚C. After 5 days, the mixture was cooled to ambient temperature, filtered, and the collected solid washed with $Et_2O$. The combined filtrate/washings were subjected to silica gel chromatography (stepwise gradient of 0 to 17% EtOAc in hexane) and isolation gave compound **127c** (30.2 g, 91 mmol) as a yellow solid in 73% yield (>94% purity by GCMS).

**Example 128: tert-butyl 3-[(2-{2-amino-6-[(cyclopropylamino)carbonyl]-6,7-dihydro-5H-pyrrolo[3,4-*d*]pyrimidin-4-yl}-3,5-dichlorophenoxy)methyl]azetidine-1-carboxylate**

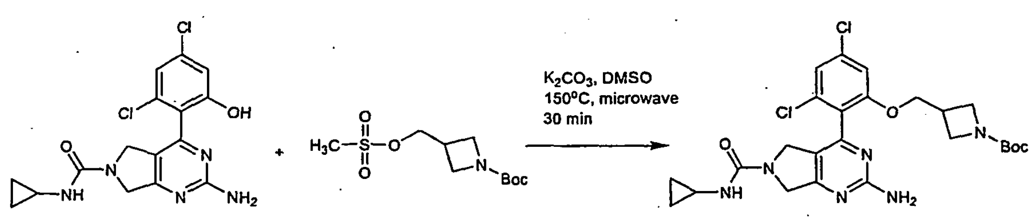


101b 128

Compound **101b** (300 mg, 0.789 mmol), *tert*-butyl 3-{[(methylsulfonyl)oxy]methyl}azetidine-1-carboxylate (419 mg, 1.58 mmol), and potassium carbonate (327 mg, 2.37 mmol) in DMF (10 mL) were microwaved at 150˚C for 30 min. Following EtOAc extractive work up, washing with water and brine, and drying ($Na_2SO_4$), isolation using silica gel chromatography (10% MeOH in EtOAc) gave compound **128** (272 mg, 0.495 mmol) in 63% yield. 1 H NMR (400 MHz, CHLOROFORM-d) d ppm: 7.15 (d, J=1.77 Hz, 1 H), 6.90 (d, J=1.77 Hz, 1 H), 5.15 (s, 2 H), 4.55 (s, 3 H), 4.35 - 4.42 (m, 1 H), 4.21 - 4.29 (m, 1 H), 4.01 - 4.13 (m, 2 H), 3.86 - 3.96 (m, 2 H), 3.53 - 3.62 (m, 2 H), 2.74 - 2.86 (m, 1 H), 2.64 - 2.74 (m, 1 H), 1.43 (s, 9 H), 0.71 - 0.79 (m, 2 H), 0.48 - 0.56 (m, 2 H). LCMS (M+H)+: 549.2, 550.2.

**Example 129: 2-amino-4-[2-(azetidin-3-ylmethoxy)-4,6-dichlorophenyl]-N-cyclopropyl-5,7-dihydro-6H-pyrrolo [3,4-*d*]pyrimidine-6-carboxamide**

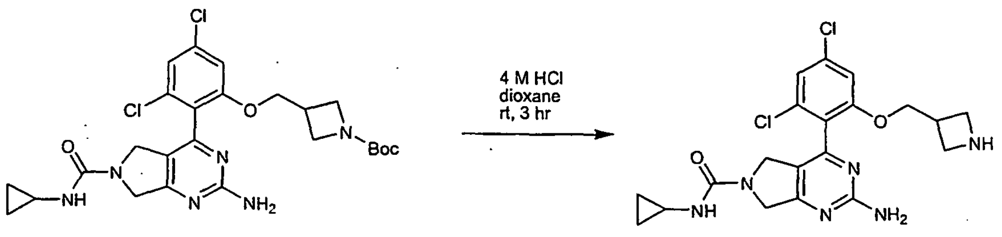


128 129

To compound **128** (490 mg, 0.892 mmol, 1.0 eq) in 1,4-dioxane (5 mL) was added 4 M HCl in 1,4-dioxane (5 mL). After stirring 3 h, isolation by preparative HPLC gave compound **129** (25 mg, 0.056 mmol) as a solid in 6% yield. 1 H NMR (400 MHz, *MeOD*) ppm 8.55 (s, 1 H), 7.15 - 7.35 (m, 2 H), 4.44 - 4.62 (m, 2 H), 4.15 - 4.40 (m, 4 H), 3.85 - 4.03 (m, 2 H), 3.66 - 3.83(m, 2 H), 3.13 - 3.27 (m, 1 H), 2.47 - 2.63 (m, 1 H), 0.58 - 0.75 (m, 2 H), 0.49 (d, *J*=2.02 Hz, 2 H). LCMS (M+H)+: 449.2,450.2.

**Example 130: 2-amino-4-(2,4-dichloro-6-{[1-(cyanomethyl)azetidin-3-yl]methoxy}phenyl)-*N*-ethyl-5,7-dihydro-6*H*-pyrrolo[3,4-*d*]pyrimidine-6-carboxamide**

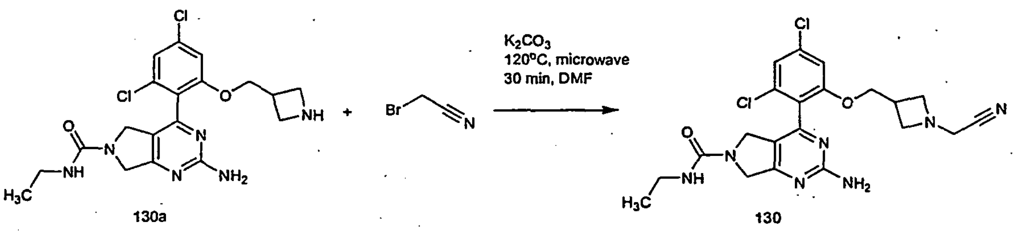


130a 130

Compound **130a,** 2-amino-4-[2-(azetidin-3-ylmethoxy)-4,6-dichlorophenyl]-*N*-ethyl-5,7-dihydro-6*H*-pyrrolo [3,4-d]pyrimidine-6-carboxamide was prepared from compound **131** in a similar manner to Example **129.** A mixture of compound **130a,** (78 mg, 0.180 mmol), bromoacetonitrile (26 mg, 0.216 mmol), and potassium carbonate (124 mg, 0.899 mmol) in DMF (2.5 mL) were microwaved at 120˚C for 30 min. Isolation by preparative HPLC gave compound **130** (20.0 mg, 0.042 mmol) as a powder in 23% yield. 1 H NMR (400 MHz, DMSO-d6) d ppm: 7.35 (d, J=1.26 Hz, 1 H), 7.32 (d, J=1.77 Hz, 1 H), 6.81 (br. s., 2 H), 6.34 (t, J=5.31 Hz, 1 H), 4.42 (s, 2 H), 4.04 - 4.20 (m, 4 H), 3.43 - 3.48 (m, 2 H), 3.25 - 3.34 (m, 2 H), 3.00 - 3.10 (m, 2 H), 2.87 - 2.99 (m, 2 H), 2.62 - 2.75 (m, 1 H), 1.01 (t, J=7.07 Hz, 3 H). LCMS $(M+H)^+$: 476.0, 478.0.

Compouds of Examples 131-2xx were prepared following the methods of Examples 1-18 and 100-130, as shown in the following Table 2.

**Table 2**

| Ex. No. | Structure | Name | Synthetic Method | $^1$H NMR | MS |
|---|---|---|---|---|---|
| 131 | 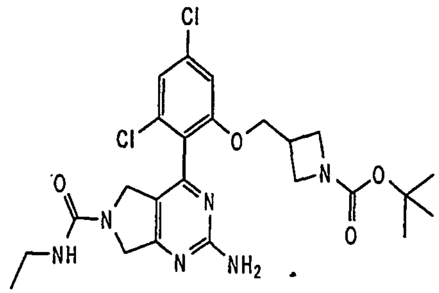 | tert-butyl 3-[(2-{2-amino-6-[(ethylamino) carbonyl]-6,7-dihydro-5H-pyrrolo[3,4-d] pyrimidin-4-yl}-3,5-dichlorophen oxy)methyl]a zetidine-1-carboxylate | Ex.128 | $^1$H NMR (400 MHz, CHLOROFORM-d) d ppm: 7.15 (d, J=1.77 Hz, 1 H), 6.90 (d, J=1.77 Hz, 1 H), 5.17 (s, 2 H), 4.58 (s, 2 H), 4.37 - 4.45 (m, 1 H), 4.20 - 4.32 (m, 2 H), 4.01 - 4.13 (m, 2 H), 3.87-3.96 (m, 2 H), 3.53 - 3.62 (m, 2 H), 3.27 - 3.38 (m, 2 H), 2.73 - 2.87 (m, 1 H), 1.42 (s, 9 H), 1.17 (t, J=7.20 Hz, 3 H). | 537.1, 539.1 |
| 132 | 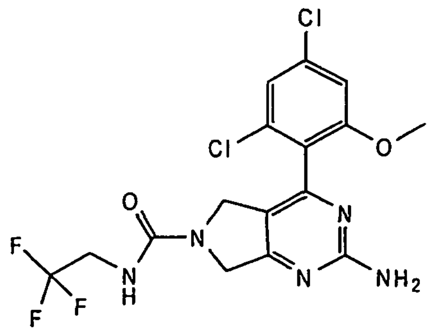 | 2-amino-4-(2,4-dichloro-6-methoxyphe nyl)-N-(2,2,2-trifluoroethyl) -5,7-dihydro-6H-pyrrolo[3,4-d]pyrimidine-6-carboxamide | Ex. 102 | 1H NMR (400 MHz, DMSO-d6) d ppm 3.78 (s, 3 H), 3.82 (dd, J=9.60, 6.57 Hz, 2 H), 4.16 (s, 2 H), 4.48 (s, 2 H), 6.87 (s, 2 H), 7.07 (br. s., 1 H), 7.28 (d, J=1.52 Hz, 1 H), 7.34 (d, J=1.77 Hz, 1 H) | 436.0, 438.0 |
| 133 | 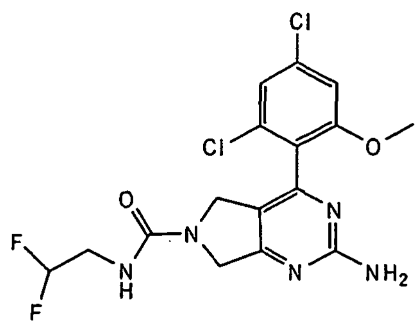 | 2-amino-4-(2,4-dichloro-6-methoxyphe nyl)-N-(2,2-difluoroethyl) -5,7-dihydro-6H-pyrrolo[3,4-d]pyrimidine-6-carboxamide | Ex. 102 | 1H NMR (400 MHz, DMSO-d6) d ppm 3.38 - 3.48 (m, 2 H), 3.72 - 3.82 (m, 3 H), 4.11 - 4.18 (m, 2 H), 4.46 (s, 2 H), 5.74 - 6.15 (m, 1 H), 6.82 (t, J=5.68 Hz, 1 H), 6.86 (s, 2 H), 7.28 (d, J=1.77 Hz, 1 H), 7.34 (d, J=1.77 Hz, 1 H) | 418.0, 420.0 |

(continued)

| Ex. No. | Structure | Name | Synthetic Method | $^1$H NMR | MS |
|---|---|---|---|---|---|
| 134 | 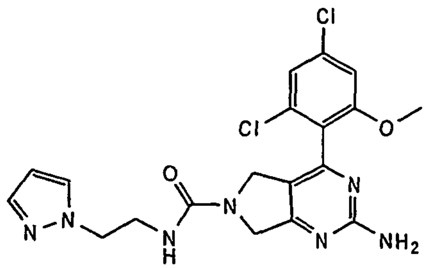 | 2-amino-4-(2,4-dichloro-6-methoxyphe nyl)-N-[2-(1H-pyrazol-1-yl)ethyl]-5,7-dihydro-6H-pyrrolo[3,4-d]pyrimidine-6-carboxamide | Ex. 102 | 1 H NMR (400 MHz, DMSO-d6) d ppm 3.35 - 3.40 (m, 2 H), 3.78 (s, 3 H), 4.09 (s, 2 H), 4.15 (t, J=6.57 Hz, 2 H), 4.41 (s, 2 H), 6.20 (t, J=2.02 Hz, 1 H), 6.54 (t, J=5.05 Hz, 1 H), 6.86 (br. s., 2 H), 7.28 (d, J=1.52 Hz, 1 H), 7.34 (d, J=1.77 Hz, 1 H), 7.42 (d, J=1.26 Hz, 1 H), 7.67 (d, J=2.02 Hz, 1 H) | 448.2, 450.2 |
| 135 | 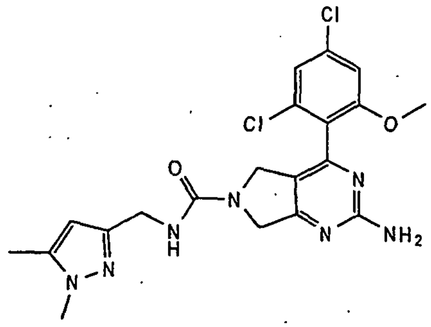 | 2-amino-4-(2,4-dichloro-6-methoxyphe nyl)-N-[(1,5-dimethyl-1H-pyrazol-3-yl) methyl]-5,7-dihydro-6H-pyrrolo [3,4-d]pyrimidine-6-carboxamide | Ex. 102 | 1H NMR (400 MHz, DMSO-d6) d ppm 2.17 (s, 3 H), 3.62 (s, 3 H), 3.77 (s, 3 H), 4.08 (d, J=5.31 Hz, 2 H), 4.12 (s, 2 H), 4.44 (s, 2 H), 5.88 (s, 1 H), 6.73 (t, J=5.68 Hz, 1 H), 6.84 (s, 2 H), 7.28 (d, J=1.52 Hz, 1 H), 7.34 (d, J=1.77 Hz, 1 H) | 462.2, 464.2 |
| 136 | 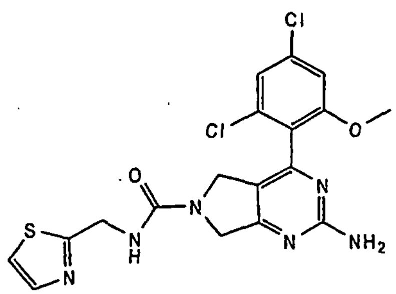 | 2-amino-4-(2,4-dichloro-6-methoxyphe nyl)-N-(1,3-thiazol-2-ylmethyl)-5,7-dihydro-6H-pyrrolo[3,4-d]pyrimidine-6-carboxamide | Ex.102 | 1H NMR (400 MHz, DMSO-d6) d ppm 3.78 (s, 3 H), 4.18 (s, 2 H), 4.43 - 4.56 (m, 4 H), 6.87 (s, 2 H), 7.28 (s, 1 H), 7.35 (s, 1 H), 7.36 - 7.42 (m, 1 H), 7.55 - 7.60 (m, 1 H), 7.66 - 7.71 (m, 1 H) | 451.0, 453.0 |

(continued)

| Ex. No. | Structure | Name | Synthetic Method | $^1$H NMR | MS |
|---|---|---|---|---|---|
| 137 | 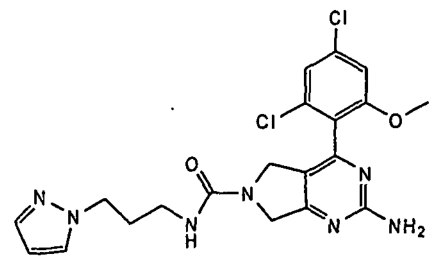 | 2-amino-4-(2,4-dichloro-6-methoxyphe nyl)-N-[3-(1H-pyrazol-1-yl)propyl]-5,7-dihydro-6H-pyrrolo[3,4-d]pyrimidine-6-carboxamide | Ex. 102 | 1 H NMR (400 MHz, DMSO-d6) d ppm 1.89 (t, J=6.95 Hz, 2 H), 3.02 (q, J=6.40 Hz, 2 H), 3.77 (s, 3 H), 4.06 - 4.15 (m, 4 H), 4.42 (s, 2 H), 6.19 (t, J=2.02 Hz, 1 H), 6.40 (t, J=5.43 Hz, 1 H), 6.84 (s, 2 H), 7.28 (d, J=2.02 Hz, 1 H), 7.34 (d, J=1.77 Hz, 1 H), 7.39 (d, J=1.26 Hz, 1 H), 7.72 (d, J=2.27 Hz, 1 H) | 462.2, 464.2 |
| 138 | 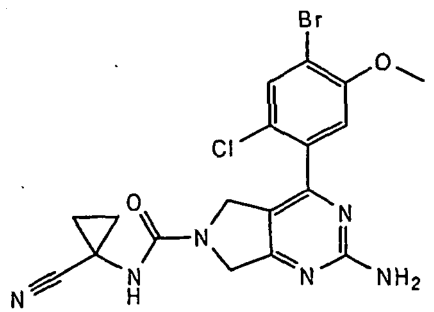 | 2-amino-4-(4-bromo-2-chloro-5-methoxyphe nyl)-N-(1-cyanocyclopr opyl)-5,7-dihydro-6H-pyrrolo[3,4-d]pyrimidine-6-carboxamide | Ex. 102 | 1 H NMR (400 MHz, DMSO-d6) d ppm 1.06 - 1.17 (m, 2 H), 1.35 - 1.47 (m, 2 H), 3.86 (s, 3 H), 4.27 (br. s., 2 H), 4.45 (s, 2 H), 6.93 (s, 2 H), 7.15 (s, 1 H), 7.46 (br. s., 1 H), 7.87 (s, 1 H) | 463.0, 465.0 |
| 139 | 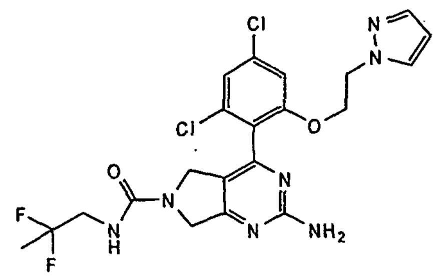 | 2-amino-4-{2,4-dichloro-6-[2-(1H-pyrazol-1-yl)ethoxy]ph enyl}-N-(2,2-difluoropropy l)-5,7-dihydro-6H-pyrrolo[3,4-d] pyrimidine-6-carboxamide | Ex. 102 | 1H NMR (400 MHz, DMSO-d6) d ppm 1.54 - 1.66 (m, 3 H), 3.48 (td, J=13.89, 8.08 Hz, 2 H), 3.58 (d, J=12.88 Hz, 2 H), 3.97 (d, J=12.88 Hz, 1 H), 4.33 (s, 4 H), 4.44 (br. s., 2 H), 6.00 (t, J=2.02 Hz, 1 H), 6.66 (br. s., 1 H), 6.80 (s, 2 H), 7.20 (d, J=2.02 Hz, 1 H), 7.26 (d, J=1.52 Hz, 1 H), 7.29 (d, J=1.52 Hz, 0 H), 7.33 (d, J=1.52 Hz, 1 H) | 512.0, 514.0 |

(continued)

| Ex. No. | Structure | Name | Synthetic Method | $^1$H NMR | MS |
|---|---|---|---|---|---|
| 140 | 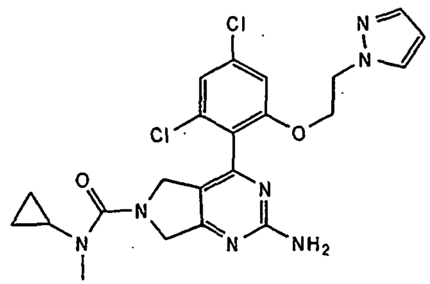 | 2-amino-N-cyclopropyl-4-{2,4-dichloro-6-[2-(1H-pyrazol-1-yl) ethoxy]ph enyl}-N-methyl-5,7-dihydro-6H-pyrrolo[3,4-d] pyrimidine-6-carboxamide | Ex. 102 | 1H NMR (400 MHz, DMSO-d6) d ppm 0.50 - 0.63 (m, 2 H), 0.68 (dd, J=6.57, 2.27 Hz, 2 H), 2.63 (ddd, J=6.82, 3.28, 3.03 Hz, 1 H), 2.74 (s, 3 H), 3.82 (d, J=13.39 Hz, 1 H), 4.18 (dd, J=13.39, 1.52 Hz, 1 H), 4.33 (s, 4 H), 4.49 - 4.69 (m, 2 H), 6.06 (t, J=2.02 Hz, 1 H), 6.76 (s, 2 H), 7.19 (d, J=2.02 Hz, 1 H), 7.23 (d, J=1.77 Hz, 1 H), 7.31 (d, J=1.77 Hz, 1 H), 7.33 (d, J=1.52 Hz, 1 H) | 489.0, 491.0 . |
| 141 | 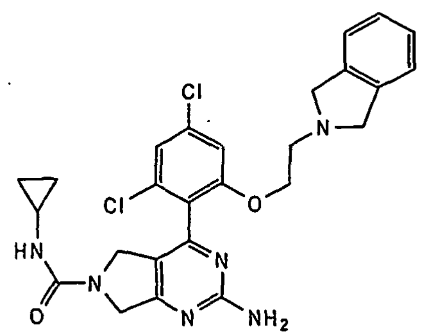 | 2-amino-N-cyclopropyl-4-{2,4-dichloro-6-[2-(1,3-dihydro-2H-isoindol-2-yl)ethoxy]ph enyl}-5,7-dihydro-6H-pyrrolo[3,4-d] pyrimidine-6-carboxamide | Ex. 101 | 1 H NMR (400 MHz, DMSO-d6) H), 0.45 0.32 - 0.41 (m, 2 H), 0.45 - 0.57 (m, 2 H), 1.91 (s, 1 H), 2.92 (br. s., 2 H), 3.69 (br. s., 4 H), 4.10 - 4.25 (m, 4 H), 4.26 - 4.36 (m, 1 H), 4.38 - 4.48 (m, 1 H), 6.45 (d, J=2.78 Hz, 1 H), 6.90 (s, 2 H), 7.15 (s, 4 H), 7.31 (d, J=1.77 Hz, 1 H), 7.35 (d, J=1.52 Hz, 1 H) | 525.2, 527.2 |
| 142 | 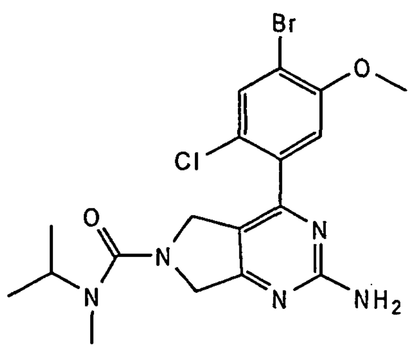 | 2-amino-4-(4-bromo-2-chloro-5-methoxyphe nyl)-N-isopropyl-N-methyl-5,7-dihydro-6H-pyrrolo [3,4-d]pyrimidine-6-carboxamide | Ex. 102 | 1 H NMR (400 MHz, DMSO-d6) d ppm 1.07 (d, J=6.57 Hz, 6 H), 2.64 (s, 3 H), 3.82 - 3.89 (m, 3 H), 3.94 - 4.08 (m, 1 H), 4.40 (s, 2 H), 4.53 (s, 2 H), 6.87 (s, 2 H), 7.16 (s, 1 H), 7.85 (s, 1 H) | 456.0, 458.0 |

(continued)

| Ex. No. | Structure | Name | Synthetic Method | $^1$H NMR | MS |
|---|---|---|---|---|---|
| 143 | 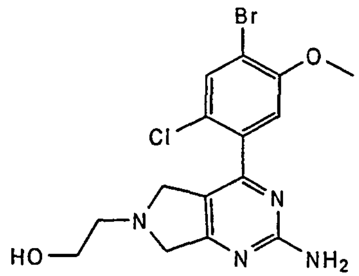 | 2-[2-amino-4-(4-bromo-2-chloro-5-methoxyphe nyl)-5,7-dihydro-6H-pyrrolo[3,4-d]pyrimidin-6-yl] ethanol | Ex. 105 | 1 H NMR (400 MHz, DMSO-d6) d ppm 3.44 (d, J=10.36 Hz, 2 H), 3.71 - 3.79 (m, 2 H), 3.85 - 3.91 (m, 3 H), 4.46 (br. s., 2 H), 4.59 (s, 2 H), 5.36 (br. s., 1 H), 7.09 (s, 1 H), 7.17 - 7.24 (m, 2 H), 7.91 (s, 1 H) | 401.0, 403.0 |
| 144 | 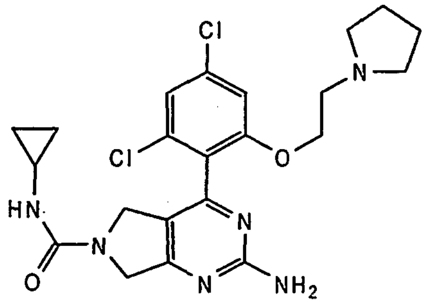 | 2-amino-N-cyclopropyl-4-[2,4-dichloro-6-(2-pyrrolidin-1-ylethoxy)phe nyl]-5,7-dihydro-6H-pyrrolo[3,4-d]pyrimidine-6-carboxamide | Ex. 101 | 1H NMR (400 MHz, DMSO-d6) d ppm 0.32 - 0.43 (m, 2 H), 0.46 - 0.58 (m, 2 H), 1.59 (br. s., 4 H), 2.25 - 2.45 (m, 3 H), 2.54 (s, 1 H), 2.62 - 2.84 (m, 2 H), 4.03 - 4.20 (m, 5 H), 4.29 - 4.48 (m, 2 H), 6.54 (s, 1 H), 6.80 (s, 2 H), 7.29 (s, 1 H), 7.34 (d, J=1.52 Hz, 1 H) | 477.2, 479.2 |
| 145 | 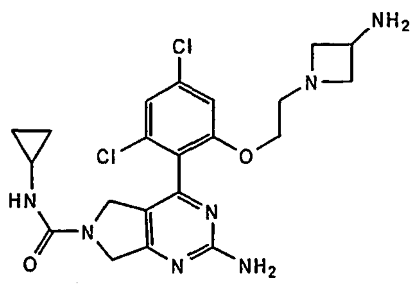 | 2-amino-4-{2-[2-(3-aminoazetidi n-1-yl)ethoxy]-4,6-dichlorophen yl}-N-cyclopropyl-5,7-dihydro-6H-pyrrolo[3,4-d]pyrimidine-6-carboxamide | Ex. 101 | 1 H NMR (400 MHz, DMSO-d6) d ppm 0.34 - 0.46 (m, 2 H), 0.47 - 0.60 (m, 2 H), 1.91 (s, 2 H), 2.59 (dd, J=4.93, 3.16 Hz, 2 H), 2.62 - 2.75 (m, 4 H), 3.98 (t, J=5.05 Hz, 2 H), 4.03 - 4.14 (m, 2 H), 4.14 - 4.25 (m, 2 H), 4.41 (s, 2 H), 6.46 (d, J=2.53 Hz, 1 H), 6.77 (s, 2 H), 7.25 (d, J=1.77 Hz, 1 H), 7.32 (d, J=1.77 Hz, 1 H) | 478.2, 480.2 |

(continued)

| Ex. No. | Structure | Name | Synthetic Method | $^1$H NMR | MS |
|---|---|---|---|---|---|
| 146 | 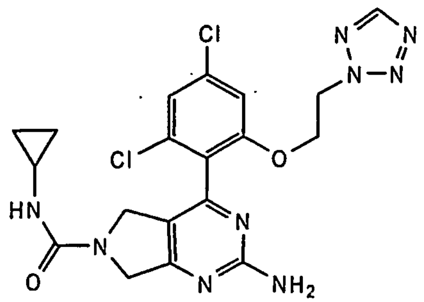 | 2-amino-N-cyclopropyl-4-{2,4-dichloro-6-[2-(2H-tetrazol-2-yl) ethoxy]ph enyl}-5,7-dihydro-6H-pyrrolo[3,4-d]pyrimidine-6-carboxamide | Ex. 101 | 1H NMR (400 MHz, DMSO-d6) d ppm 0.34 - 0.48 (m, 2 H), 0.47 - 0.59 (m, 2 H), 2.51 - 2.57 (m, 1 H), 3.62 (d, J=13.14 Hz, 1 H), 3.95 (dd, J=13.01, 1.89 Hz, 1 H), 4.28 -4.45 (m, 3 H), 4.44 - 4.57 (m, 1 H), 4.75 (t, J=5.05 Hz, 2 H), 6.34 (br. s., 1 H), 6.77 (br. s., 2 H), 7.33 (d, J=1.77 Hz, 1 H), 7.36 (d, J=1.77 Hz, 1 H), 8.82 (s, 1 H) | 476.1, 478.1 |
| 147 | 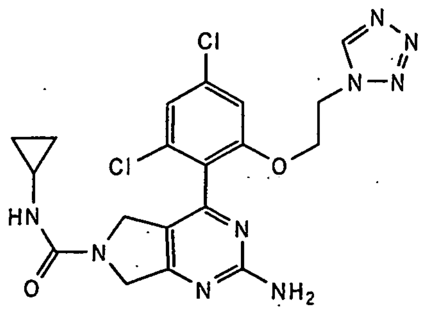 | 2-amino-N-cyclopropyl-4-{2,4-dichloro-6-[2-(1H-tetrazol-1-yl) ethoxy]ph enyl}-5,7-dihydro-6H-pyrrolo[3,4-d]pyrimidine-6-carboxamide | Ex.101 | 1H NMR (400 MHz, DMSO-d6) d ppm 0.36 - 0.48 (m, 2 H), 0.50 - 0.61 (m, 2 H), 2.53 - 2.58 (m, 1 H), 3.36 - 3.42 (m, 1 H), 3.83 (dd, J=13.01, 2.15 Hz, 1 H), 4.25 - 4.47 (m, 2 H), 4.46 - 4.63 (m, 2 H), 4.91 - 5.04 (m, 2 H), 6.34 (br. s., 1 H), 6.72 (s, 2 H), 7.34 (s, 2 H), 8.61 (s, 1 H) | 476.1, 477.1 |
| 148 | 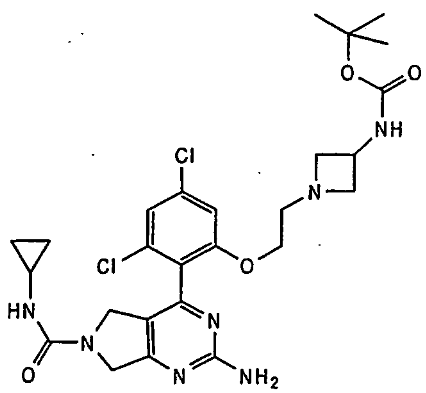 | tert-butyl {1-[2-(2-{2-amino-6-[(cyclopropyl-amino)carbonyl]-6,7-dihydro-5H-pyrrolo[3,4-d] pyrimidin-4-yl}-3,5-dichlorophen oxy)ethyl]aze tidin-3-yl} carbamate | Ex. 101 | 1H NMR (400 MHz, CHLOROFORM-d) d ppm 1.44 (s, 9 H), 1.66 (br. s., 3 H), 2.70 (br. s., 4 H), 2.75 - 2.89 (m, 2 H), 3.37 - 3.47 (m, 1 H), 3.50 (s, 1 H), 3.86 - 4.03 (m, 2 H), 4.19 (br. s., 1 H), 4.34 (br. s., 2 H), 4.57 (s, 2 H), 4.62 (s, 1 H), 5.08 (br. s., 1 H), 5.30 (br. s., 2 H), 6.85 (d, J=1.52 Hz, 1 H), 7.12 (d, J=1.77 Hz, 1 H) | 578.2, 580.2 |

(continued)

| Ex. No. | Structure | Name | Synthetic Method | $^1$H NMR | MS |
|---|---|---|---|---|---|
| 149 | 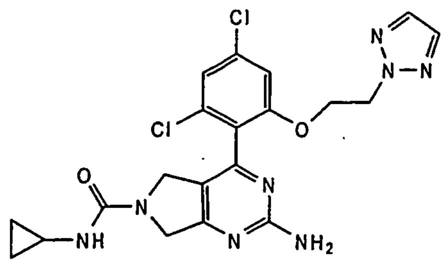 | 2-amino-N-cyclopropyl-4-{2,4-dichloro-6-[2-(2H-1,2,3-triazol-2-yl)ethoxy]ph enyl}-5,7-dihydro-6H-pyrrolo[3,4-d]pyrimidine-6-carboxamide | Ex. 101 | 1H NMR (400 MHz, DMSO-d6) d ppm 0.35 - 0.47 (m, 2 H), 0.56 (d, J=7.07 Hz, 2 H), 2.52 - 2.60 (m, 1 H), 3.38 - 3.47 (m, 1 H), 3.84 (d, J=13.14 Hz, 1 H), 4.35 (d, J=4.80 Hz, 2 H), 4.42 - 4.56 (m, 2 H), 4.65 (t, J=4.93 Hz, 2 H), 6.33 (br. s., 1 H), 6.53 (s, 1 H), 6.71 (s, 2 H), 7.26 - 7.37 (m, 1 H), 7.53 (s, 2 H) | 475.1, 477.1 |
| 150 | 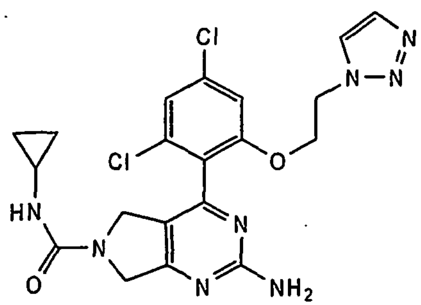 | 2-amino-N-cyclopropyl-4-{2,4-dichloro-6-[2-(1H-1,2,3-triazol-1-yl)ethoxy]ph enyl}-5,7-dihydro-6H-pyrrolo[3,4- d]pyrimidine-6-carboxamide | Ex.101 | 1H NMR (400 MHz, DMSO-d6) d ppm 0.35 - 0.48 (m, 2 H), 0.49 - 0.60 (m, 2 H), 2.52 - 2.58 (m, 1 H), 3.60 (d, J=13.14 Hz, 1 H), 3.94 (dd, J=12.88, 2.02 Hz, 1 H), 4.30 - 4.51 (m, 4 H), 4.59 - 4.71 (m, 2 H), 6.35 (br. s., 1 H), 6.79 (br. s., 2 H), 7.29 (d, J=1.77 Hz, 1 H), 7.35 (d, J=1.52 Hz, 1 H), 7.48 (s, 1 H), 7.56 (s, 1 H) | 475.1, 477.1 |
| 151 | 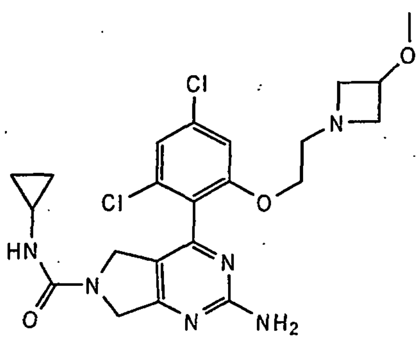 | 2-amino-N- cyclopropyl-4-{2,4-dichloro-6-[2-(3-methoxyazeti din-1-yl)ethoxy]ph enyl}-5,7-dihydro-6H-pyrrolo[3,4-d] pyrimidine-6-carboxamide | Ex.101 | 1H NMR (400 MHz, DMSO-d6) d ppm 0.34 - 0.46 (m, 2 H), 0.49 - 0.59 (m, 2 H), 2.52 - 2.57 (m, 1 H), 2.59 - 2.75 (m, 4 H), 3.07 (s, 3 H), 3.23 - 3.28 (m, 2 H), 3.79 (t, J=5.68 Hz, 1 H), 3.93 - 4.05 (m, 2 H), 4.04 - 4.23 (m, 2 H), 4.29 - 4.48 (m, 2 H), 6.46 (d, J=2.78 Hz, 1 H), 6.82 (s, 2 H), 7.23 (d, J=1.52 Hz, 1 H), 7.32 (d, J=1.77 Hz, 1 H) | 493.1, 495.1 |

(continued)

| Ex. No. | Structure | Name | Synthetic Method | $^1$H NMR | MS |
|---|---|---|---|---|---|
| 152 | 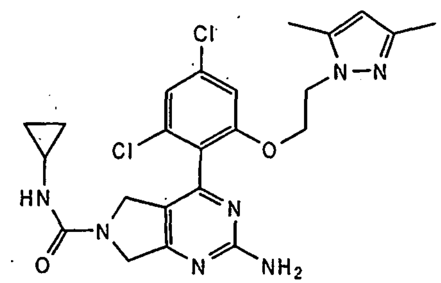 | 2-amino-N-cyclopropyl-4-{2,4-dichloro-6-[2-(3,5-dimethyl-1H-pyrazol-1-yl)ethoxy]ph enyl}-5,7-dihydro-6H-pyrrolo[3,4-d] pyrimidine-6-carboxamide | Ex.101 | 1H NMR (400 MHz, DMSO-d6) d ppm 0.32 - 0.47 (m, 2 H), 0.54 (dd, J=7.07, 2.27 Hz, 2 H), 1.66 (s, 3 H) 2.00 (s, 3 H), 2.54 - 2.58 (m, 1 H), 3.84 (d, J=12.88 Hz, 1 H), 4.00 - 4.17 (m, 2 H), 4.16 - 4.27 (m, 2 H), 4.31 - 4.43 (m, 2 H), 5.49 (s, 1 H), 6.33 (br. s., 1 H), 6.53 (s, 1 H), 6.78 (s, 2 H), 7:25 (d, J=1.77 Hz, 1 H), 7.31 (d, J=1.77 Hz, 1 H) | 502.1, 504.1 |
| 153 | 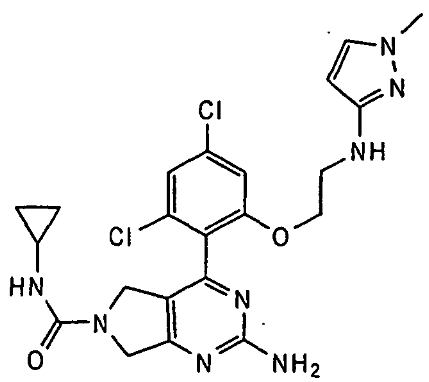 | 2-amino-N-cyclopropyl-4-(2,4-dichloro-6-{2-[(1-methyl-1H-pyrazol-3-yl)amino]eth oxy} phenyl)-5,7-dihydro-6H-pyrrolo [3,4-d]pyrimidine-6-carboxamide | Ex.101 | 1H NMR (400 MHz, DMSO-d6) d ppm 0.35 - 0.45 (m, 2 H), 0.49 - 0.61 (m, 2 H), 2.53 - 2.58 (m, 1 H), 3.25 (t, J=5.81 Hz, 2 H), 3.60 - 3.67 (m, 3 H), 4.04 - 4.12 (m, 2 H), 4.12 - 4.23 (m, 3 H), 4.40 (s, 2 H), 5.39 (d, J=2.27 Hz, 1 H), 6.41 (br. s., 1 H), 6.62 - 7.04 (m, 2 H), 7.34 (d, J=1.77 Hz, 1 H), 7.38 (d, J=2.02 Hz, 1 H), 7.45 (d, J=1.77 Hz, 1 H) | 503.1, 505.1 |
| 154 | 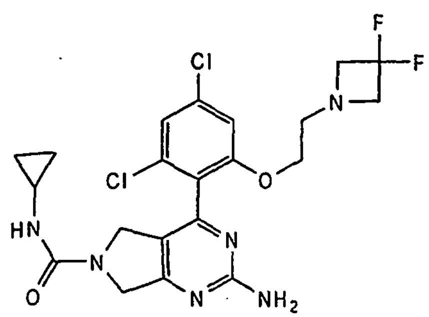 | 2-amino-N-cyclopropyl-4-{2,4-dichloro-6-[2-(3,3-difluoroazeti din-1-yl)ethoxy]ph enyl}-5,7-dihydro-6H-pyrrolo[3,4-d] pyrimidine-6-carboxamide | Ex. 101 | 1 H NMR (400 MHz, DMSO-d6) d ppm 0.34 - 0.44 (m, 2 H), 0.47 - 0.59 (m, 2 H), 2.74 (d, J=3.54 Hz, 2 H), 3.22 - 3.35 (m, 4 H), 3.57 (s, 1 H), 3.97 - 4.09 (m, 2 H), 4.08 - 4.17 (m, 2 H), 4.26 - 4.51 (m, 2 H), 6.46 (d, J=2.53 Hz, 1 H), 6.85 (s, 2 H), 7.25 (d, J=1.77 Hz, 1 H), 7.30 - 7.38 (m, 1 H) | 499.1, 500.1 |

(continued)

| Ex. No. | Structure | Name | Synthetic Method | $^1$H NMR | MS |
|---|---|---|---|---|---|
| 155 | 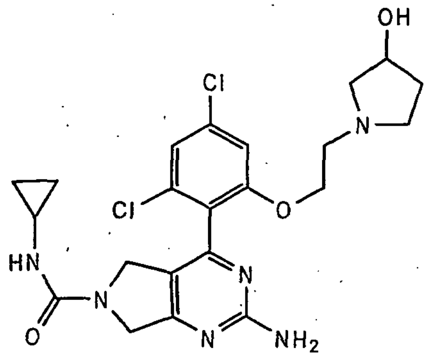 | 2-amino-N-cyclopropyl-4-{2,4-dichloro-6-[2-(3-hydroxypyrrolidin-1-yl)ethoxy]ph enyl}-5,7-dihydro-6H-pyrrolo[3,4-d] pyrimidine-6-carboxamide | Ex.101 | 1H NMR (400 MHz, DMSO-d6) d ppm 0.34 - 0.45 (m, 2 H), 0.47 - 0.58 (m, 2 H), 1.34 - 1.49 (m, 2 H), 1.74 - 1.88 (m, 0 H), 2.17 - 2.34 (m, 2 H), 2.34 - 2.45 (m, 1 H), 2.55 - 2.73 (m, 3 H), 3.17 (d, J=4.80 Hz, 2 H), 3.99 - 4.20 (m, 4 H), 4.38 (br. s., 2 H), 4.59 (br. s., 1 H), 6.38 - 6.48 (m, 1 H), 6.78 (s, 2 H), 7.27 (s, 1 H), 7.32 (d, J=1.52 Hz, 1 H) | 493.1, 495.1 |
| 156 | 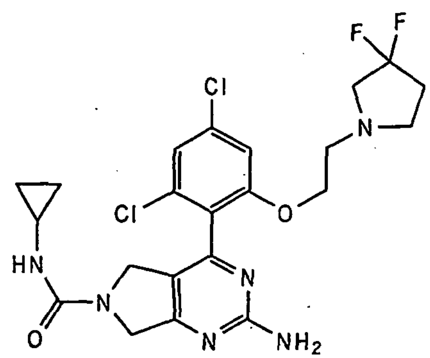 | 2-amino-N-cyclopropyl-4-{2,4-dichloro-6-[2-(3,3-difluoropyrrolidin-1-yl)ethoxy]ph enyl}-5,7-dihydro-6H-pyrrolo[3,4-d] pyrimidine-6-carboxamide | Ex.101 | 1H NMR (400 MHz, DMSO-d6) d ppm 0.33 - 0.43 (m, 2 H), 0.47 - 0.57 (m, 2 H), 2.09 - 2.32 (m, 2 H), 2.52 - 2.58 (m, 1 H), 3.00 (d, J=3.79 Hz, 3 H), 3.13 - 3.21 (m, 2 H), 4.11 (s, 2 H), 4.16 - 4.26 (m, 2 H), 4.29 - 4.47 (m, 3 H), 6.44 (d, J=2.27 Hz, 1 H), 6.82 (br. s., 2 H), 7.32 (s, 1 H), 7.37 (s, 1 H) | 513.1, 514.1 |

(continued)

| Ex. No. | Structure | Name | Synthetic Method | $^1$H NMR | MS |
|---|---|---|---|---|---|
| 157 | 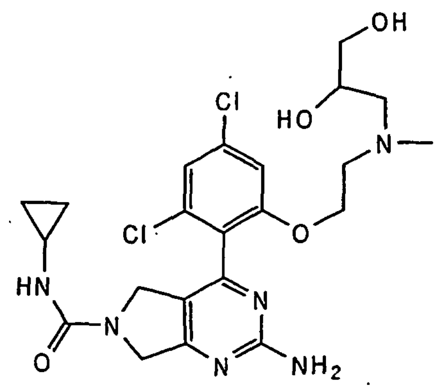 | 2-amino-N-cyclopropyl-4-(2,4-dichloro-6-{2-[(2,3-dihydroxypropyl)(methyl)a mino]ethoxy}phenyl)-5,7-dihydro-6H-pyrrolo[3,4-d]pyrimidine-6-carboxamide | Ex. 101 | 1H NMR (400 MHz, DMSO-d6) d ppm 1.90 (s, 1 H), 2.06 - 2.12 (m, 3 H), 2.11 - 2.14 (m, 2 H), 2.20 (dd, J=12.88, 7.07 Hz, 2 H), 2.29 - 2.40 (m, 2 H), 2.56 - 2.63 (m, 2 H), 3.17 - 3.27 (m, 2 H), 3.39 - 3.49 (m, 2 H), 4.02 - 4.13 (m, 3 H), 4.13 - 4.30 (m, 2 H), 4.39 (s, 2 H), 6.43 (d, J=2.53 Hz, 1 H), 6.77 (s, 2 H), 7.28 (d, J=1.77 Hz, 1 H), 7.32 (d, J=1.77 Hz, 1 H) | 512.2, 513.2 |
| 158 | 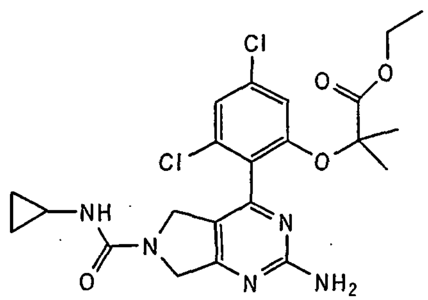 | ethyl 2-(2-{2-amino-6-[(cyclopropyl amino)carbo nyl]-6,7-dihydro-5H-pyrrolo[3,4-d]pyrimidin-4-yl}-3,5-dichlorophenoxy)-2-methylpropa noate | Ex.100 | 1H NMR (400 MHz, DMSO-d6) d ppm 0.34 - 0.45 (m, 2 H), 0.48 - 0.58 (m, 2 H), 1.17 (t, J=7.07 Hz, 3 H), 1.24 (s, 1 H), 1.35 (s, 3 H), 1.43 (s, 3 H), 4.10 - 4.23 (m, 4 H), 4.40 (s, 2 H), 6.45 (d, J=2.78 Hz, 1 H), 6.79 - 6.88 (m, 3 H), 7.45 (d, J=2.02 Hz, 1 H) | 494.1, 496.1 |

(continued)

| Ex. No. | Structure | Name | Synthetic Method | $^1$H NMR | MS |
|---|---|---|---|---|---|
| 159 | 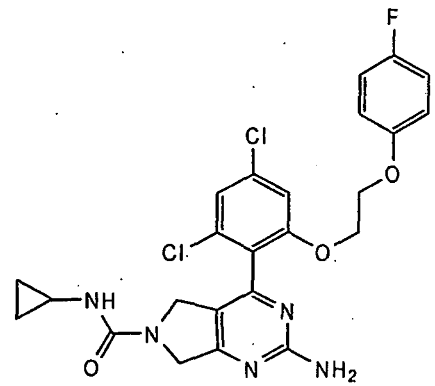 | 2-amino-N-cyclopropyl-4-{2,4-dichloro-6-[2-(4-fluorophenox y)ethoxy]phe nyl}-5,7-dihydro-6H-pyrrolo[3,4-d]pyrimidine-6-carboxamide | Ex.100 | 1H NMR (400 MHz, DMSO-d6) d ppm 0.32 - 0.43 (m, 2 H), 0.49 - 0.61 (m, 2 H), 1.23 (s, 1 H), 3.96 - 4.28 (m, 5 H), 4.29 - 4.42 (m, 3 H), 6.41 (d, J=2.02 Hz, 1 H), 6.70 - 6.83 (m, 3 H), 6.95 - 7.06 (m, 2 H), 7.31 - 7.41 (m, 2 H), 7.50 - 7: 68 (m, 1 H) - | 518.1, 520.1 |
| 160 | 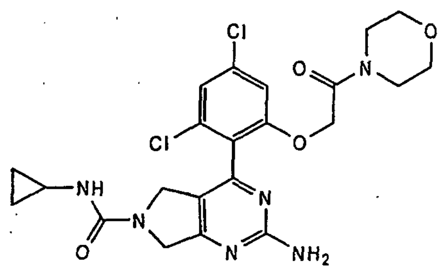 | 2-amino-N- cyclopropyl-4-[2,4-dichloro-6-(2-morpholin-4-yl-2-oxoethoxy)p henyl]-5,7-dihydro-6H-pyrrolo[3,4-d]pyrimidine-6-carboxamide | Ex.100 | 1H NMR (400 MHz, DMSO-d6) d ppm 0.32 - 0.44 (m, 2 H), 0.47 - 0.59 (m, 2 H), 2.54 (s, 1 H), 3.46 (s, 4 H), 3.47 - 3.60 (m, 4 H), 4.10 (d, J=13.39 Hz, 1 H), 4.19 - 4.32 (m, 1 H), 4.39 (s, 2 H), 4.81 - 4.96 (m, 1 H), 4.96 - 5.09 (m, 1 H), 6.45 (d, J=2.53 Hz, 1 H), 6.79 (br. s., 2 H), 7.18 (d, J=1.26 Hz, 1 H), 7.31 (d, J=1.52 Hz, 1 H) | 507.1, 509.1 |
| 161 | 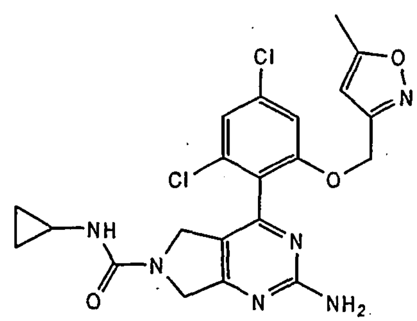 | 2-amino-N-cyclopropyl-4-{2,4-dichloro-6-[(5-methylisoxaz ol-3-yl)methoxy]p henyl}-5,7-dihydro-6H-pyrrolo[3,4-d]pyrimidine-6-carboxamide | Ex. 100 | 1 H NMR (400 MHz, DMSO-d6) d ppm 0.34 - 0.45 (m, 2 H), 0.48 - 0:59 (m, 2 H), 2.33 - 2.41 (m, 3 H), 2.52 - 2.56 (m, 1 H), 3.96 - 4.16 (m, 2 H), 4.40 (s, 2 H), 5.19 - 5.33 (m, 2 H), 6.04 (s, 1 H), 6.44 (d, J=2.53 Hz, 1 H), 6.84 (s, 2 H), 7.39 (d, J=1.77 Hz, 1 H), 7.42 (d, J=1.77 Hz, 1 H) | 475.1, 477.1 |

(continued)

| Ex. No. | Structure | Name | Synthetic Method | $^1$H NMR | MS |
|---|---|---|---|---|---|
| 162 | 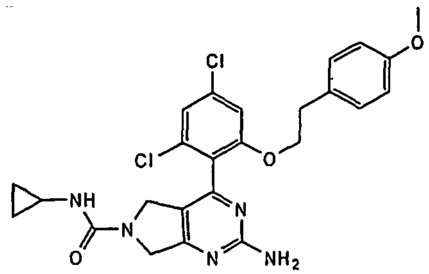 | 2-amino-N-cyclopropyl-4-{2,4-dichloro-6-[2-(4-methoxyphe nyl) ethoxy]p henyl}-5,7-dihydro-6H-pyrrolo[3,4-d]pyrimidine-6-carboxamide | Ex. 100 | 1H NMR (400 MHz, DMSO-d6) d ppm 0.34 - 0.44 (m, 2 H), 0.47 - 0.58 (m, 2 H), 2.65 - 2.84 (m, 2 H), 3.69 (s, 3 H), 3.74 (d, J=13.14 Hz, 1 H), 3.97 (d, J=12.88 Hz, 1 H), 4.06 - 4.23 (m, 3 H), 4.41 (s, 2 H), 6.36 (br. s., 1 H), 6.69 (d, J=8.59 Hz, 2 H), 6.83 (s, 2 H), 6.86 (s, 1 H), 6.88 (s, 1 H), 7.24 (d, J=1.77 Hz, 1 H), 7.29 (d, J=1.77 Hz, 1 H) | 514.1, 515.1 |
| 163 | 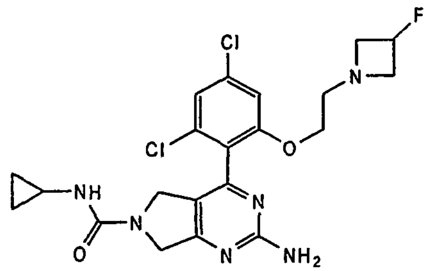 | 2-amino-N-cyclopropyl-4-{2,4-dichloro-6-[2-(3-fluoroazetidi n-1-yl)ethoxy]ph enyl}-5,7-dihydro-6H-pyrrolo[3,4-d]pyrimidine-6-carboxamide | Ex.101 | 1H NMR (400 MHz, DMSO-d6) d ppm 0.32 - 0.44 (m, 2 H), 0.47 - 0.59 (m, 2 H), 2.52 - 2.57 (m, 1 H), 2.66 (br. s., 2 H), 2.90 (d, J=7.83 Hz, 2 H), 4.00 (t, J=4.93 Hz, 2 H), 4.07 - 4.20 (m, 4 H), 4.33 - 4.46 (m, 2 H), 4.84 - 5.11 (m, 1 H), 6.46 (d, J=2.53 Hz, 1 H), 6.81 (s, 2 H), 7.24 (d, J=1.52 Hz, 1 H), 7.29 - 7.36 (m, 1 H) | 481.1, 483.1 |
| 164 | 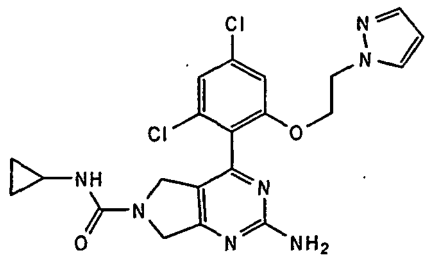 | 2-amino-N-cyclopropyl-4-{2,4-dichloro-6-[2-(1 H-pyrazol-1-yl) ethoxy]ph enyl}-5,7-dihydro-6H-pyrrolo[3,4-d]pyrimidine-6-carboxamide | Ex.100 | 1H NMR (400 MHz, DMSO-d6) d ppm 0.32 - 0.45 (m, 2 H), 0.50 - 0.59 (m, 2 H), 2.52 - 2.57 (m, 1 H), 3.55 (d, J=12.88 Hz, 1 H), 3.82 - 3.97 (m, 1 H), 4.32 (s, 4 H), 4.35 - 4.42 (m, 2 H), 6.00 (t, J=2.02 Hz, 1 H), 6.33 (br. s., 1 H), 6.78 (s, 2 H), 7.18 (d, J=2.02 Hz, 1 H), 7.25 (d, J=1.77 Hz, 1 H), 7.29 (d, J=1.77 Hz, 1 H), 7.32 (d, J=1.52 Hz, 1 H) | 474.1, 476.1 |

(continued)

| Ex. No. | Structure | Name | Synthetic Method | $^1$H NMR | MS |
|---|---|---|---|---|---|
| 165 | 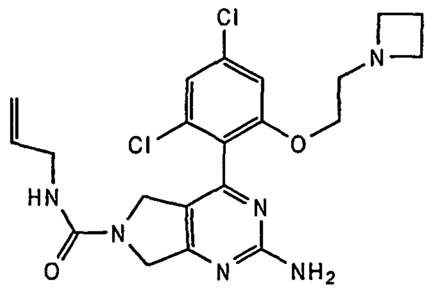 | N-allyl-2-amino-4-[2-(2-azetidin-1-ylethoxy)-4,6-dichlorophen yl]-5,7-dihydro-6H-pyrrolo[3,4-d] pyrimidine-6-carboxamide | Ex. 100 | 1H NMR (400 MHz, DMSO-d6) d ppm 1.85 (t, J=7.07 Hz, 2 H), 2.60 (t, 2 H), 3.01 (br. s., 2 H), 3.67 (t, J=5.31 Hz, 2 H), 3.98 (t, J=4.93 Hz, 2 H), 4.08 - 4.28 (m, 4 H), 4.37 - 4.48 (m, 2 H), 5.00 (dd, J=10.23, 1.64 Hz, 1 H), 5.10 (dd, J=17.18, 1.77 Hz, 1 H), 5.68 - 5.90 (m, 1 H), 6.57 (t, J=5.68 Hz, 1 H), 6.81 (s, 2 H), 7.24 (d, J=1.77 Hz, 1 H), 7.29 - 7.36 (m, 1 H) | 463.2, 464.2 |
| 166 | 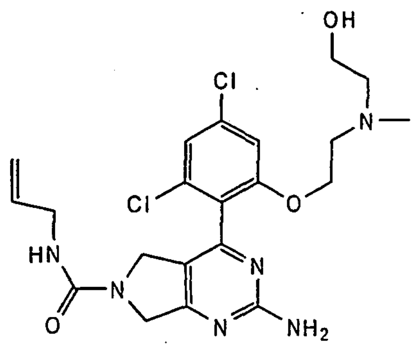 | N-allyl-2-amino-4-(2,4-dichloro-6-{2-[(2-hydroxyethyl )(methyl) ami no]ethoxy}ph enyl)-5,7-dihydro-6H-pyrrolo[3,4-d] pyrimidine-6-carboxamide | Ex. 100 | 1H NMR (400 MHz, DMSO-d6) d ppm 2.08 (s, 3 H), 2.33 (t, J=6.19 Hz, 2 H), 2.56 - 2.63 (m, 2 H), 3.67 (t, J=5.31 Hz, 3 H), 3.97 - 4.17 (m, 4 H), 4.25 (d, J=13.14 Hz, 2 H), 4.43 (br. s., 2 H), 5.00 (dd, J=10.23, 1.64 Hz, 1 H), 5.10 (dd, J=17.31, 1.64 Hz, 1 H), 5.73 - 5.88 (m, 1 H), 6.55 (t, J=5.56 Hz, 1 H), 6.79 (s, 2 H), 7.29 (d, J=1.77 Hz, 1 H), 7.32 (d, J=1.77 Hz, 1 H) | 481.2, 483.2 |
| 167 | 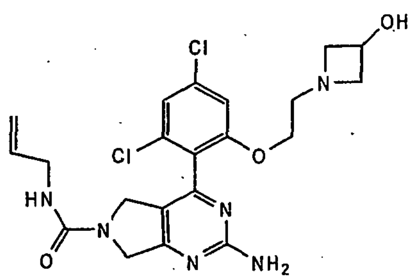 | N-allyl-2-amino-4-{2,4-dichloro-6-[2-(3-hydroxyazeti din-1-yl) ethoxy]ph enyl}-5,7-dihydro-6H-pyrrolo[3,4-d]pyrimidine-6-carboxamide | Ex. 101 | 1H NMR (400 MHz, DMSO-d6) d ppm 2.56 (d, J=6.57 Hz, 2 H), 3: 63 - 3.72 (m, 2 H), 3.93 - 4.04 (m, 4 H), 4.06 - 4,18 (m, 2 H), 4.18 - 4.29 (m, 2 H), 4.39 - 4.49 (m, 2 H), 5.01 (dd, J=10.23, 1.64 Hz, 1 H), 5.06 - 5.16 (m, 1 H), 5.20 (br. s., 1 H), 5.73 - 5.89 (m, 1 H), 6.51 - 6.61 (m, 2 H), 6.81 (s, 2 H), 7.25 (d, J=1.77 Hz, 1 H), 7.31 - 7.36 (m, 1 H) | 479.2, 481.2 |

(continued)

| Ex. No. | Structure | Name | Synthetic Method | $^1$H NMR | MS |
|---|---|---|---|---|---|
| 168 | 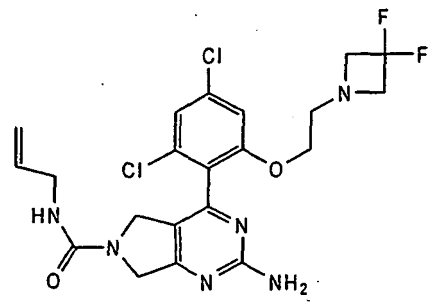 | N-allyl-2-amino-4-{2,4-dichloro-6-[2-(3,3-difluoroazeti din-1-yl) ethoxy]ph enyl}-5,7-dihydro-6H-pyrrolo[3,4-d]pyrimidine-6-carboxamide | Ex. 101 | 1H NMR (400 MHz, DMSO-d6) d ppm 2.73 (d, J=4.04 Hz, 2 H), 3.20 - 3.29 (m, 2 H), 3.66 (t, J=5.31 Hz, 2 H), 3.99 - 4.09 (m, 2 H), 4.15 (s, 2 H), 4.29 - 4.51 (m, 4 H), 4.99 (dd, J=10.23, 1.64 Hz, 1 H), 5.09 (dd, J=17.43, 1.77 Hz, 1 H), 5.73 - 5.85 (m, 1 H), 6.56 (t, J=5.56 Hz, 1 H), 6.85 (s, 2 H), 7.24 (d, J=1.77 Hz, 1 H), 7.33 (d, J=1.77 Hz, 1 H) | 499.0, 501.0 |
| 169 | 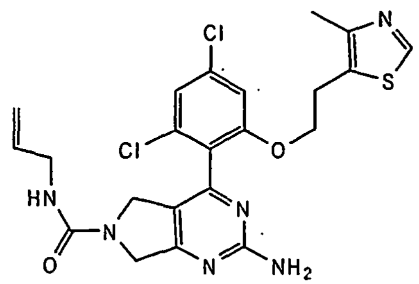 | N-allyl-2-amino-4-(2,4-dichloro-6-[2-(4-methyl-1,3-thiazol-5-yl) ethoxy]ph enyl)-5,7-dihydro-6H-pyrrolo[3,4-d]pyrimidine-6-carboxamide | Ex. 100 | 1H NMR (400 MHz, DMSO-d6) d ppm 2.02 (s, 3 H), 2.99 - 3.12 (m, 2 H), 3.55 - 3.65 (m, 1 H), 3.68 (t, J=5.18 Hz, 2 H), 3.87 - 4.00 (m, 1 H), 4.01 - 4.12 (m, 1 H), 4.23 - 4.34 (m, 1 H), 4.33 - 4.54 (m, 2 H), 5.02 (dd, J=10.36, 1.52 Hz, 1 H), 5.13 (dd, J=17.18, 1.52 Hz, 1 H), 5.74 - 5.93 (m, 1 H), 6.46 (br. s., 1 H), 6.80 (s, 2 H), 7.26 (d, J=1.52 Hz, 1 H), 7.32 (d, J=1.77 Hz, 1 H), 8.56 (s, 1H) | 505.0, 507.0 |
| 170 | 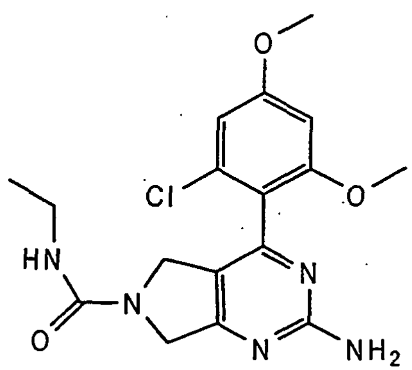 | 2-amino-4-(2-chloro-4,6-dimethoxyph enyl)-N-ethyl-5,7-dihydro-6H-pyrrolo[3,4-d] pyrimidine-6-carboxamide | Ex.107 | 1H NMR (400 MHz, DMSO-d6) d ppm 1.01 (t, J=7.20 Hz, 3 H), 2.97 - 3.12 (m, 2 H), 3.73 (s, 3 H), 3.80 - 3.87 (m, 3 H), 4.05 4.17 (m, 2 H), 4.37 - 4.53 (m, 2 H), 6.35 (t, J=5.43 Hz, 1 H), 6.68 (d, J=2.27 Hz, 1 H), 6.75 (d, J=2.27 Hz, 2 H), 7.21 (s, 1 H) | 378.1, 380.1 |

(continued)

| Ex. No. | Structure | Name | Synthetic Method | $^1$H NMR | MS |
|---|---|---|---|---|---|
| 171 | 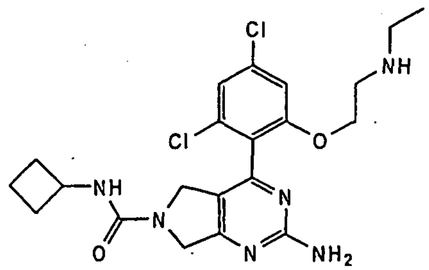 | 2-amino-N-cyclobutyl-4-{2,4-dichloro-6-[2-(ethylamino) ethoxy]phen yl}-5,7-dihydro-6H-pyrrolo[3,4-d]pyrimidine-6-carboxamide | Ex. 100 | 1 H NMR (400 MHz, DMSO-d6) d ppm 0.87 (t, J=7.07 Hz, 3 H), 1.44 - 1.61 (m, 2 H), 1.83 - 1.96 (m, 4 H), 2.02 - 2.14 (m, 2 H), 2.38 - 2.45 (m, 2 H), 2.73 (t, J=4.93 Hz, 2 H), 4.01-4.19 (m, 4 H), 4.40 (br. s., 2 H), 6.51 (d, J=7.83 Hz, 1 H), 6.82 (s, 2 H), 7.31 (s, 1 H), 7.34 (s, 1 H) | 465.1, 467.1 |
| 172 | 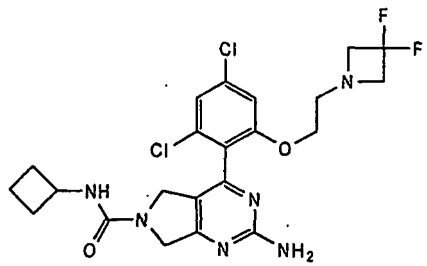 | 2-amino-N-cyclobutyl-4-(2,4-dichloro-6-[2-(3,3-difluoroazeti din-1-yl)ethoxy]ph enyl)-5,7-dihydro-6H-pyrrolo[3,4-d] pyrimidine-6-carboxamide | Ex.100 | 1H NMR (400 MHz, DMSO-d6) d ppm 1.54 (dd, J=9.85, 7.33 Hz, 2 H), 1.85 - 1.97 (m, 2 H), 2.08 (dd, J=6.82, 3.54 Hz, 2 H), 2.74 (br. s., 2 H), 3.36 - 3.47 (m, 2 H), 3.98 - 4.08 (m, 2 H), 4.09 - 4.17 (m, 3 H), 4.26 - 4.48 (m, 2 H), 6.47 - 6.57 (m, 2 H), 6.85 (s, 2 H), 7.25 (d, J=1.77 Hz, 1 H), 7.34 (d, J=1.52 Hz, 1 H) | 513.1, 515.1 |
| 173 | 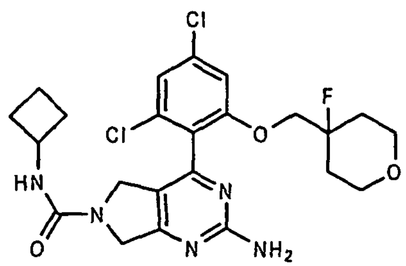 | -amino-N-cyclobutyl-4-{2,4-dichloro-6-[(4-fluorotetrahy dro-2H-pyran-4-yl)methoxy]p henyl}-5,7-dihydro-6H-pyrrolo[3,4-d] pyrimidine-6-carboxamide | Ex. 100 . | 1H NMR (400 MHz, DMSO-d6) d ppm 1.46 - 1.60 (m, 4 H), 1.91 (br. s., 2 H), 2.09 (dd, J=6.82, 3.28 Hz, 2 H), 3.28 - 3.40 (m, 4 H), 3.42 - 3.52 (m, 2 H), 3.56 - 3.68 (m, 2 H), 4.09 - 4.16 (m, 3 H), 4.36 - 4.44 (m, 2 H), 6.52 - 6.59 (m, 2 H), 6.82 (s, 1 H), 7.37 (dd, J=8.08, 1.77 Hz, 2 H) | |

(continued)

| Ex. No. | Structure | Name | Synthetic Method | $^1$H NMR | MS |
|---|---|---|---|---|---|
| 174 | 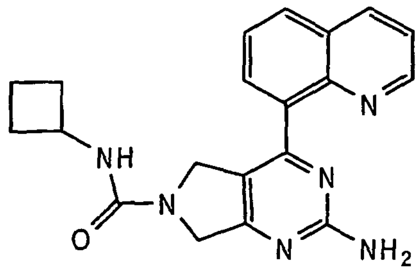 | 2-amino-N-cyclobutyl-4-quinolin-8-yl-5,7-dihydro-6H-pyrrolo[3,4-d]pyrimidine-6-carboxamide | Ex.18a | 1H NMR (400 MHz, DMSO-d6) d ppm 1.44 - 1.62 (m, 2 H), 1.81-1.94 (m, 2 H), 1.99 - 2.18 (m, 2 H), 3.95 - 4.19 (m, 3 H), 4.42 (d, J=18.44 Hz, 2 H), 6.42 (d, J=7.83 Hz, 1 H), 6.75 (s, 2 H), 7.59 - 7.66 (m, 1 H), 7.69 - 7.77 (m, 1 H), 7.78 - 7.84 (m, 1 H), 8.08 - 8.17 (m, 1 H), 8.43-8.55 (m, 1 H), 8.93 (dd, J=4.17, 1.64 Hz, 1 H) | 361.4 |
| 175 | 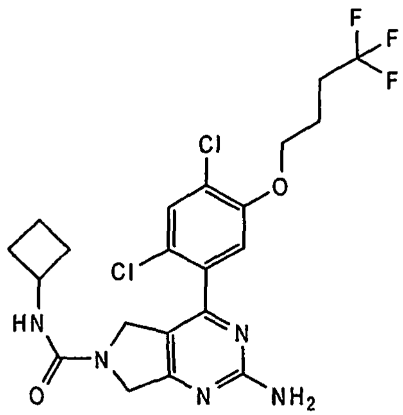 | 2-amino-N-cyclobutyl-4-[2,4-dichloro-5-(4,4,4-trifluorobutox y) phenyl]-5,7-dihydro-6H-pyrrolo [3,4-d]pyrimidine-6-carboxamide | Ex. 100 | 1 H NMR (400 MHz, DMSO-d6) d ppm 1.45 - 1.65 (m, 2 H) 1.86 - 2.01 (m, 4 H) 2.03-2.15 (m, 2 H) 2.37 - 2.46 (m, 2 H) 4.06 - 4.21 (m, 3 H) 4.26 - (s, 2 H) 4.41 (s, 2 H) 6.53 (d, J=8.08 Hz, 1 H) 6.89 (s, 2H) 7.23 (s, 1 H) 7.77 (s, 1 H) | 504.2, 506.2 |
| 177 | 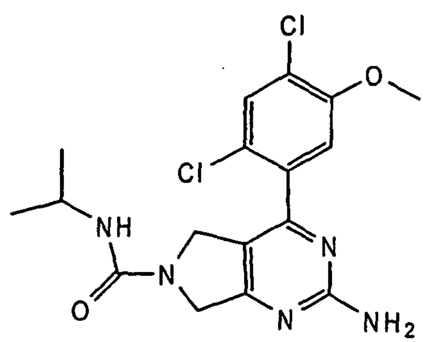 | 2-amino-4-(2,4-dichloro-5-methoxyphe nyl)-N-isopropyl-5,7-dihydro-6H-pyrrolo[3,4-d] pyrimidine-6-carboxamide | Ex.102 | 1H NMR (400 MHz, DMSO-d6) d ppm 1.05 (d, J=6.57 Hz, 6 H) 3.77 (d, J=7.07 Hz, 1 H) 3.87 (s, 3 H) 4.25 (s, 2 H) 4.41 (s, 2 H) 6.07 (d, J=7.58 Hz, 1 H) 6.89 (s, 2 H) 7.20 (s, 1 H) 7.74 (s, 1H) | 396.0, 397.0 |

(continued)

| Ex. No. | Structure | Name | Synthetic Method | $^1$H NMR | MS |
|---|---|---|---|---|---|
| 178 | 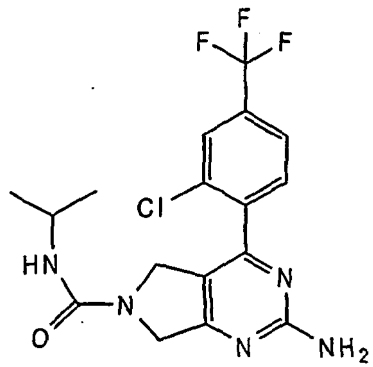 | 2-amino-4-[2-chloro-4-(trifluorometh yl)phenyl]-N-isopropyl-5,7-dihydro-6H-pyrrolo [3,4-d]pyrimidine-6-carboxamide | Ex. 18a | 1 H NMR (400 MHz, DMSO-d6) d ppm 1.05 (d, J=6.32 Hz, 6 H) 3.77 (d, J=6.82 Hz, 1 H) 4.25 (s, 2 H) 4.43-(s, 2 H) 6.08 (d, J=7.83 Hz, 1 H) 6.93 (s, 2 H) 7.70 (d, J=7.83 Hz, 1 H) 7.86 (d, J=7.83 Hz, 1 H) 8.06 (s, 1 H) | 400.1; 402.1 |
| 179 | 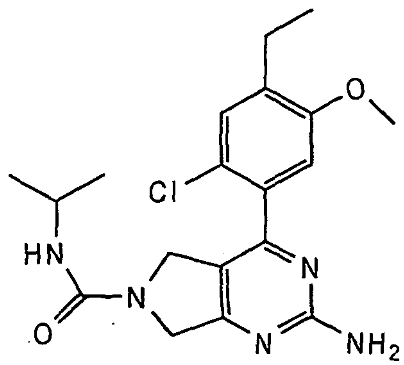 | 2-amino-4-(2-chloro-4-ethyl-5-methoxyphe nyl)-N-isopropyl-5,7-dihydro-6H-pyrrolo[3,4-d] pyrimidine-6-carboxamide | Ex.104 | 1 H NMR (400 MHz, DMSO-d6) d ppm 1.00 - 1.11 (m, 6 H) 1.10 - 1.21 (m, 3 H) 2.54 - 2.67 (m, 2 H) 3.76-3.83 (m, 3 H) 4.27 (s, 2 H) 4.37 - 4.44 (m, 2 H) 6.10 (d, J=7.83 Hz, 1 H) 6.82 (s, 2 H) 6.96 (s, 1 H) 7.27 - 7.35 (m, 1 H) | 390.4, 392.4 |

(continued)

| Ex. No. | Structure | Name | Synthetic Method | $^1$H NMR | MS |
|---|---|---|---|---|---|
| 180 | 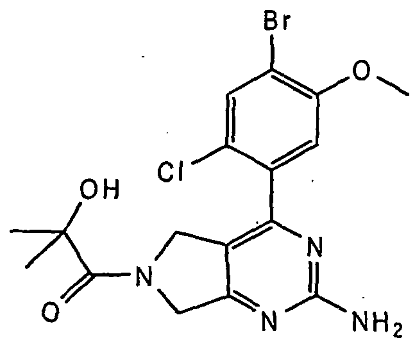 | 1-[2-amino-4-(4-bromo-2-chloro-5-methoxyphenyl)-5,7-dihydro-6H-pyrrolo[3,4-d]pyrimidin-6-yl]-2-methyl-1-oxopropan-2-ol | Ex.6 | 1H NMR (400 MHz, DMSO-d6) d ppm 1.31 (d, J=13.89 Hz, 6 H) 3.85 (s, 3 H) 4.44 (d, J=61.39 Hz, 2 H) 4.91 -(d, J=48.25 Hz, 2 H) 5.35 (d, J=25.52 Hz, 1 H) 6.92 (s, 2 H) 7.16 (d, J=14.65 Hz, 1 H) 7.87 (d, J=5.05 Hz, 1 H) | |
| 181 | 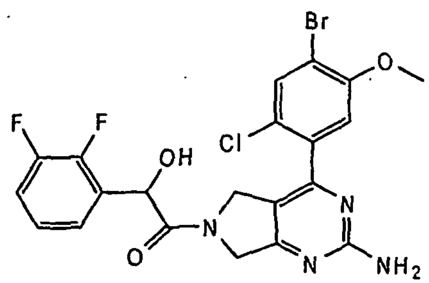 | 2-[2-amino-4-(4-bromo-2-chloro-5-methoxyphenyl)-5,7-dihydro-6H-pyrrolo[3,4-d]pyrimidin-6-yl]-1-(2,3-difluorophen yl)-2-oxoethanol | Ex. 6 | 1 H NMR (400 MHz, DMSO-d6) d ppm 3.82 - 3.89 (m, 3 H), 4.40 (d, J=6.06 Hz, 2 H), 4.49 - 4.58 (m, 2 H), 4.76 - 5.02 (m, 1 H), 5.60 (d, J=24.25 Hz, 1 H), 6.86 - 7.05 (m, 2 H), 7.11 - 7.21 (m, 1 H), 7.22 - 7.31 (m, 2 H), 7.33 - 7.44 (m, 1 H), 7.86 (d, J=1.01 Hz, 1 H) | |
| 182 | 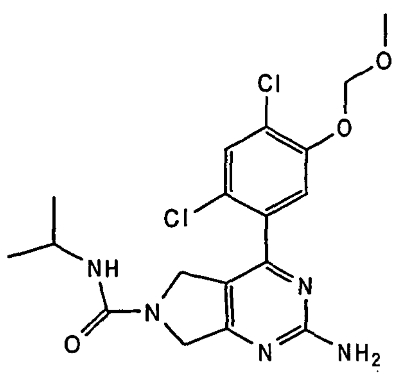 | 2-amino-4-[2,4-dichloro-5-(methoxymet hoxy)phenyl] -N-isopropyl-5,7-dihydro-6H-pyrrolo [3,4-d]pyrimidine-6-carboxamide | Ex. 100 | 1H NMR (400 MHz, DMSO-d6) d ppm 1.06 (d, J=6.57 Hz, 6 H), 3.39 - 3.45 (m, 3 H), 3.78 (dd, J=14.15, 6.57 Hz, 1 H), 4.25 (s, 2 H), 4.42 (s, 2 H), 5.33 (s, 2 H), 6.08 (d, J=7.58 Hz, 1 H), 6.92 (s, 2 H), 7.29 (s, 1 H), 7.80 (s, 1 H) | 427.2, 429.2 |

(continued)

| Ex. No. | Structure | Name | Synthetic Method | $^1$H NMR | MS |
|---|---|---|---|---|---|
| 183 | 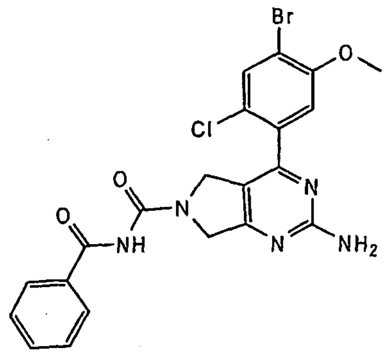 | 2-amino-N-benzoyl-4-(4-bromo-2-chloro-5-methoxyphe nyl)-5,7-dihydro-6H-pyrrolo[3,4-d] pyrimidine-6-carboxamide | Ex. 15a | 1 H NMR (400 MHz, DMSO-d6) d ppm 3.85 (d, J=10.61 Hz, 3 H), 4.45 (s, 2 H), 4.60 (s, 2 H), 4.75 (s, 1 H), 6.97 (s, 2 H), 7.16 (s, 1 H), 7.44 - 7.57 (m, 2 H), 7.61 (s, 1 H), 7.78-7.85 (m, 1 H), 7.85 - 7.93 (m, 2 H) | 505.2, 506.2 |
| 184 | 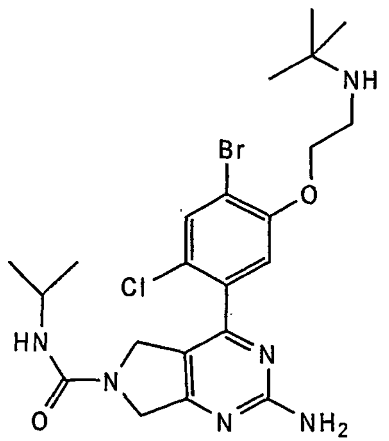 | 2-amino-4-{4-bromo-5-[2-(tert-butylamino)e thoxy]-2-chlorophenyl }-N-isopropyl-5,7-dihydro-6H-pyrrolo[3,4-d] pyrimidine-6-carboxamide | Ex.15 | 1H NMR (400 MHz, DMSO-d6) d ppm 1.05 (t, J=3.16 Hz, 15 H), 1.90 (s, 1 H), 2.87 (t, J=5.43 Hz, 2 H), 3.78 (dd, J=14.02, 6.69 Hz, 1 H), 4.09 (t, J=5.68 Hz, 2 H), 4.25 (s, 2 H), 4.41 (s, 2 H), 6.07 (d, J=7.58 Hz, 1 H), 6.88 (s, 2 H), 7.19 (s, 1 H), 7.86 (s, 1 H) | 527.1, 528.1 |

(continued)

| Ex. No. | Structure | Name | Synthetic Method | $^1$H NMR | MS |
|---|---|---|---|---|---|
| 185 | 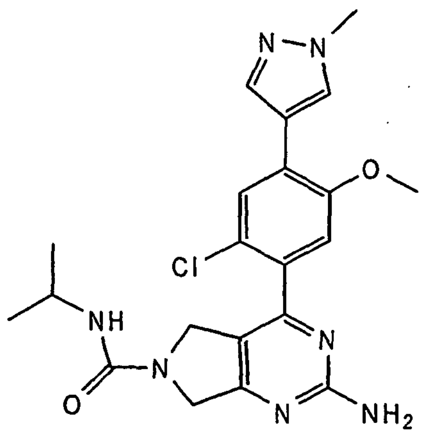 | 2-amino-4-[2-chloro-5-methoxy-4-(1-methyl-1 H-pyrazol-4-yl) phenyl]-N-isopropyl-5,7-dihydro-6H-pyrrolo[3,4-d]pyrimidine-6-carboxamide | Ex. 104 | 1H NMR (400 MHz, DMSO-d6) d ppm 1.05 (t, J=5.81 Hz, 6 H), 3.78 (s, 1 H), 3.86 - 3.92 (m, 6 H), 4.30 (s, 2 H), 4.43 (s, 2 H), 6.05 - 6.16 (m, 1 H), 6.85 (s, 2 H), 7.08 (s, 1 H), 7.80 (s, 1 H), 8.03 (s, 1 H), 8.25 (s, 1 H) | 442.2, 444.2 |
| 186 | 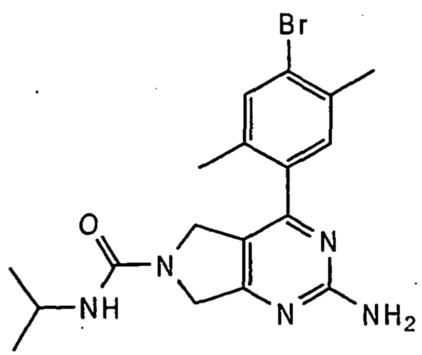 | 2-amino-4-(4-bromo-2,5-dimethylphe nyl)-N-isopropyl-5,7-dihydro-6H-pyrrolo[3,4-d] pyrimidine-6-carboxamide | Ex. 15a | 1 H NMR (400 MHz, DMSO-d6) d ppm 1.05 (d, J=6.57 Hz, 6 H), 2.17 (s, 3 H), 2.33 (s, 3 H), 3.72 - 3.84 (m, 1 H), 4.24 (s, 2 H), 4.41 (s, 2 H), 6.06 (d, J=7.33 Hz, 1 H), 7.28 (s, 1 H), 7.56 (s, 1 H) | 405.1, 406.1 |

(continued)

| Ex. No. | Structure | Name | Synthetic Method | $^1$H NMR | MS |
|---|---|---|---|---|---|
| 187 | 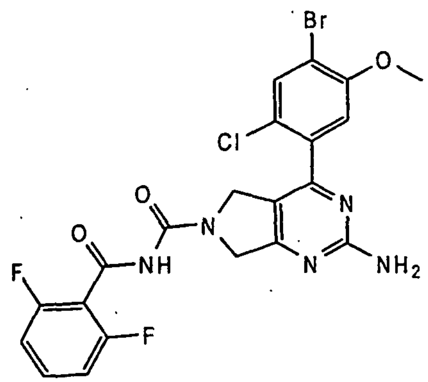 | 2-amino-4- (4-bromo-2-chloro-5-methoxyphe nyl)-N-(2,6-difluorobenzoyl)-5,7-dihydro-6H-pyrrolo[3,4-d]pyrimidine-6-carboxamide | Ex. 15a | 1 H NMR (400 MHz, DMSO-d6) d ppm 3.86 (d, J=10.86 H, 3 H), 4.34 (s, 1 H), 4.48 (s, 1 H), 4.59 (s, 1 H), 4.74 (s, 1 H), 6.97 (s, 2 H), 7.03 - 7.11 (m, 1 H), 7.11-7.23 (m, 2 H), 7.46 - 7.59 (m, 1 H), 7.58 - 7.68 (m, 1 H), 7.86 (d, J=13.64 Hz, 1 H) | 540.2, 542.2 |
| 188 | 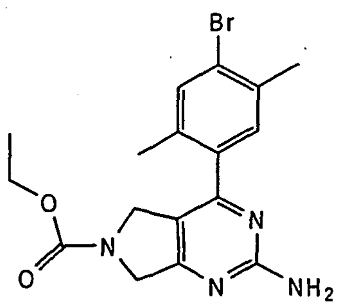 | ethyl 2-amino-4-(4-bromo-2,5-dimethylphenyl)-5,7-dihydro-6H-pyrrolo[3,4-d]pyrimidine-6-carboxylate | Ex.1 | 1H NMR (400 MHz, DMSO-d6) d ppm 1.09 - 1.30 (m, 3 H), 2.18 (d, J=7.83 Hz, 3 H), 2.33 (s, 3 H), 4.08 (dd, J=14.02, 6.95 Hz, 2 H), 4.32 (d, J=5.05 Hz, 2 H), 4.46 (d, J=10.36 Hz, 2 H), 6.81 (s, 2 H), 7.29 (d, J=5.05 Hz, 1 H),7.55 (s, 1 H) | 391.1, 392.1 |
| 189 | 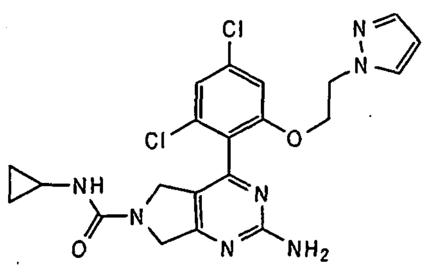 | 2-amino-N-cyclopropyl-4-{2,4-dichloro-6-[2-(1 H-pyrazol-1-yl) ethoxy]phenyl}-5,7-dihydro-6H-pyrrolo[3,4-d]pyrimidine-6-carboxamide | Ex. 100 | 1 H NMR (400 MHz, DMSO-d6) d ppm 0.33 - 0.44 (m, 2 H), 0.49 - 0.58 (m, 2 H), 2.51 - 2.56 (m, 1. H), 3.55 (d, J=12.88 Hz, 1 H), 3.82 - 3.94 (m, 1 H), 4.29 - 4.35 (m, 4 H), 4.36 - 4.42 (m, 2 H), 6.00 (t, J=2.02 Hz, 1 H), 6.32 (br. s., 1 H), 6.77 (br. s., 2 H), 7.18 (d, J=2.27 Hz, 1 H), 7.24 (d, J=1.77 Hz, 1 H), 7.29 (d, J=1.52 Hz, 1 H), 7.31 (d, J=1.52 Hz, 1 H) | 474.2, 476.2 |

(continued)

| Ex. No. | Structure | Name | Synthetic Method | $^1$H NMR | MS |
|---|---|---|---|---|---|
| 190 | 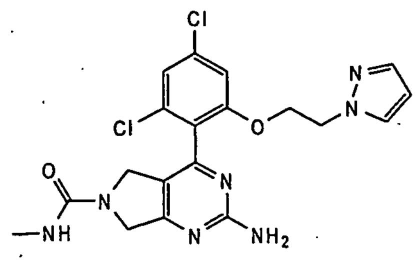 | 2-amino-4-{2,4-dichloro-6-[2-(1H-pyrazol-1-yl)ethoxy]phenyl}-N-methyl-5,7-dihydro-6H-pyrrolo[3,4-d]pyrimidine-6-carboxamide | Ex. 106 | 1 H NMR (400 MHz, DMSO-d6) d ppm 2.59 (d, J=4.29 Hz, 3 H) 3.60 (d, J=12.63 Hz, 1 H) 3.92 (dd, 1 H) 4.29 - 4.36 (m, 4 H) 4.36 - 4.46 (m, 2 H) 6.00 (t, J=2.02 Hz, 1 H) 6.18 (d, J=4.29 Hz, 1 H) 6.78 (s, 2 H) 7.19 (d, J=2.02 Hz, 1 H) 7.25 (d, J=1.77 Hz, 1 H) 7.29 (d, J=1.52 Hz, 1 H) 7.32 (d, J=1.77Hz, 1 H) | 450.2, 448.2 |
| 191 | 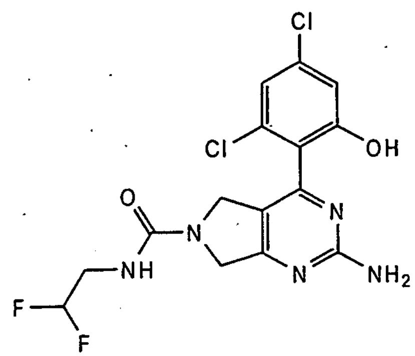 | 2-amino-4-(2,4-dichloro-6-hydroxyphen yl)-N-(2,2-difluoroethyl)-5,7-dihydro-6H-pyrrolo[3,4-d]pyrimidine-6-carboxamide | Ex. 18 | 1H NMR (400 MHz, DMSO-d6) d ppm 3.35 - 3.49 (m, 2 H) 4.19 (s, 2 H) 4.45 (s, 2 H) 5.96 (tt, 1 H) 6.76 - 6.89 (m, 3 H) 6.96 (d, J=1.77 Hz, 1 H) 7.15 (d, J=2.02 Hz, 1 H) 10.66-(s, 1 H) | 406.0, 404.2 |
| 192 | 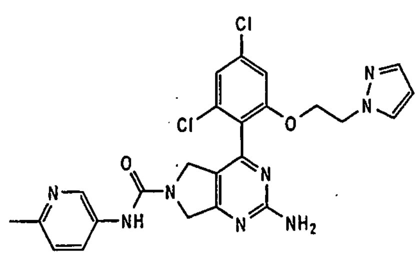 | 2-amino-4-{2,4-dichloro-6-[2-(1H-pyrazol-1-yl)ethoxy]phenyl}-N-(6-methylpyridin-3-yl)-5,7-dihydro-6H-pyrrolo[3,4-d]pyrimidine-6-carboxamide | Ex. 109 | 1 H NMR (400 MHz, DMSO-d6) d ppm 2.42 (s, 3 H) 3.69 (d, J=12.63 Hz, 1 H) 4.13 (d, J=12.63 Hz, 1 H) 4.31 - 4.40 (m, 4 H) 4.49 - 4.63 (m, 2 H) 6.01 (t, J=1.89 Hz, 1 H) 6.84 (s, 2 H) 7.22 - 7.27 (m, 3 H) 7.29 (d, J=1.52 Hz, 1 H) 7.34 (d, J=1.77 Hz, 1 H) 7.91 (dd, J=8.46, 2.40 Hz, 1 H) 8.43 (s, 1 H) 8.61 (s, 1 H) | 527.2, 525.2 |

(continued)

| Ex. No. | Structure | Name | Synthetic Method | $^1$H NMR | MS |
|---|---|---|---|---|---|
| 193 | 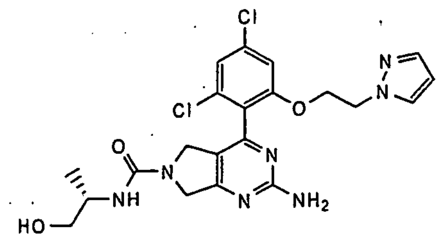 | 2-amino-4-(2,4-dichloro-6-[2-(1 H-pyrazol-1-yl)ethoxy] phenyl)-N-[(1S)-2-hydroxy-1-methylethyl]-5,7-dihydro-6H-pyrrolo[3,4-d]pyrimidine-6-carboxamide | Ex. 110 | | 494.2, 492.2 |
| 194 | 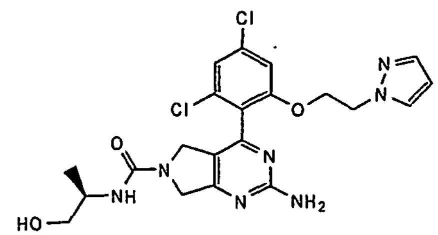 | 2-amino-4-(2,4-dichloro-6-[2-(1H-pyrazol-1-yl)ethoxy]phenyl)-N-[(1R)-2-hydroxy-1-methylethyl]-5,7-dihydro-6H-pyrrolo[3,4-d]pyrimidine-6-carboxamide | Ex.110 | | 494.2, 492.2 |
| 195 | 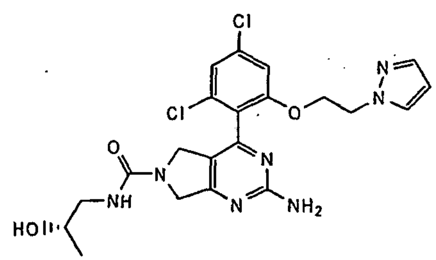 | 2-amino-4-{2,4-dichloro-6-[2-(1H-pyrazol-1-yl)ethoxy]phenyl}-N-[(2S)-2-hydroxyprop yl]-5,7-dihydro-6H-pyrrolo[3,4-d] pyrimidine-6-carboxamide | Ex. 110 | 1 H NMR (400 MHz, DMSO-d6) d ppm 1,01 (dd, J=6.19, 1.39 Hz, 3 H) 2.96 - 3.03 (m, J=5.56, 5.56 Hz, 2 H) 3.58 - 3.70 (m, 2 H) 3.95 (d, J=12.63 Hz, 1 H) 4.30 - 4.37 (m, 4 H) 4.39 - 4.47 (m, 2 H) 6.01 (q, J=1.94 Hz, 1 H) 6.21 - 6.30 (m, 1 H) 6.78 (s, 2 H) 7.19 (d, J=2.27 Hz, 1 H) 7.25 (d, J=1.77 Hz, 1 H) 7.30-7.32 (m, 1 H) 7.32 (d, J=1.77 Hz, 1 H) | 494.2, 492.2 |

(continued)

| Ex. No. | Structure | Name | Synthetic Method | $^1$H NMR | MS |
|---|---|---|---|---|---|
| 196 | 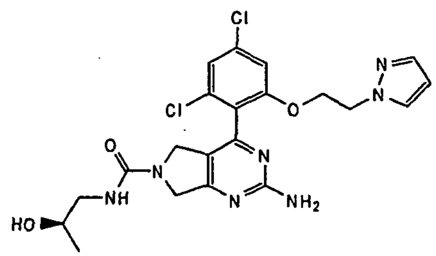 | 2-amino-4-(2,4-dichloro-6-[2-(1H-pyrazol-1-yl)ethoxy]phenyl}-N-[(2R)-2-hydroxyprop yl]-5,7-dihydro-6H-pyrrolo[3,4-d] pyrimidine-6-carboxamide | Ex. 110 | 1 H NMR (400 MHz, DMSO-d6) d ppm 1.01 (dd, J=6.32, 1.26 Hz, 3 H) 2.95 -3.02 (m, J=5.56, 5.56 Hz, 2 H) 3.58 - 3.70 (m, 2 H) 3.95 (d, J=12.63 Hz, 1 H) 4.30 - 4.38 (m, 4 H) 4.39 - 4.47 (m, 2 H) 6.01 (q, J=1.85 Hz, 1 H) 6.22 - 6.30 (m, 1 H) 6.78 (s, 2 H) 7.19 (d, J=2.27 Hz, 1 H) 7.25 (d, J=1.77 Hz, 1 H) 7.29 - 7.31 (m, 1 H) 7.32 (d, J=1.52 Hz, 1 H) | 494.2, 492.2 |
| 197 | 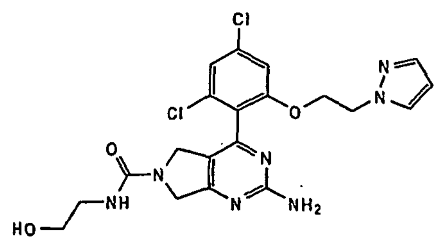 | 2-amino-4-{2,4-dichloro-6-[2-(1H-pyrazol-1-yl)ethoxy]phenyl}-N-(2-hydroxyethyl)-5,7-dihydro-6H-pyrrolo(3,4-d] pyrimidine-6-carboxamide | Ex. 110 | 1 H NMR (300 MHz, DMSO-d6) d ppm 3.06 - 3.15 (m, 2 H) 3.37 - 3.43 (m, 2 H) 3.59 (d, J=13.00 Hz, 1 H) 3.93 (d, J=12.62 Hz, 1 H) 4.33 (s, 4 H) 4.38 - 4.48 (m, 2 H) 6.01 (t, J=1.98 Hz, 1 H) 6.22 - 6.28 (m, 1 H) 6.79 (s, 2 H) 7.19 (d, J=2.26 Hz, 1 H) 7.25 (d, J=1.70 Hz, 1 H) 7.30 - 7.33 (m, 1.70 Hz, 2 H) | 480.2, 478.2 |
| 198 | 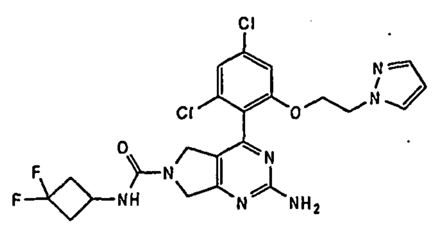 | 2-amino-4-{2,4-dichloro-6-[2-(1H-pyrazol-1-yl)ethoxy]phenyl}-N-(3,3-difluorocyclobutyl)-5,7-dihydro-6H-pyrrolo[3,4-d] pyrimidine-6-carboxamide | Ex. 110 | 1 H NMR (400 MHz, DMSO-d6) d ppm 2.57 - 2.70 (m, 2 H) 2.77 - 2.90 (m, 2 H) 3.60 (d, J=12.88 Hz, 1 H) 3.95 (d, J=12.88 Hz, 1 H) 3.99 - 4.08 (m, 1 H) 4.31 - 4.37 (m, 4 H) 4.39 - 4.47 (m, 2 H) 6.00 (t, J=2.02 Hz, 1 H) 6.60 (d, J=6.32 Hz, 1 H) 6.77 (s, 2 H) 7.20 (d, J=2.02 Hz, 1 H) 7.26 (d, J=1.52 Hz, 1 H) 7.29 (d, J=1.52 Hz, 1 H) 7.32 (d, J=1.77 Hz, 1 H) | 526.0, 524.0 |

(continued)

| Ex. No. | Structure | Name | Synthetic Method | $^1$H NMR | MS |
|---|---|---|---|---|---|
| 199 | 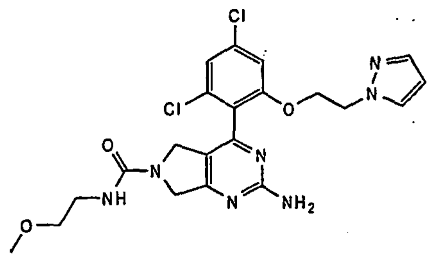 | 2-amino-4{2,4-dichloro-6-[2-(1H-pyrazol-1-yl)ethoxy]ph enyl}-N-(2-methoxyethyl)-5,7-dihydro-6H--pyrrolo[3,4-d] pyrimidine-6-carboxamide | Ex. 110 | 1 H NMR (400 MHz, DMSO-d6) d ppm 3.16 - 3.22 (m, 2 H) 3.24 (s, 3 H) 3.31 - 3.37 (m, 2 H) 3.57 (d, J=12.88 Hz, 1 H) 3.92 (d, J=13.14 Hz, 1 H) 4.30 - 4.36 (m, 4 H) 4.37 - 4.45 (m, 2 H) 6.00 (t, J=1.89 Hz, 1 H) 6.28 - 6.35 (m, 1 H) 6.79 (s, 2 H) 7.19 (d, J=1.77 Hz, 1 H) 7.25 (d, J=1.77 Hz, 1 H) 7.30 (d, J=1.26 Hz, 1 H) 7.32 (d, J=1.77 Hz, 1 H) | 494.2, 492.2 |
| 200 | 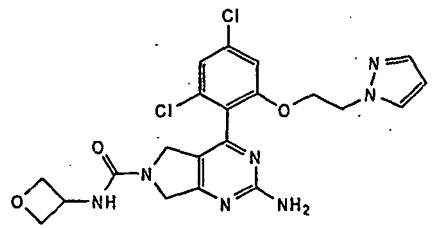 | 2-amino-4{2,4-dichloro-6-[2-(1H-pyrazol-1-yl)ethoxy]ph enyl)-N-oxetan-3-yl-5,7-dihydro-6H-pyrrolo[3,4-d]pyrimidine-6-carboxamide | Ex. 110 | 1 H NMR (300 MHz, DMSO-d6) d ppm 3.60 (d, J=13.00 Hz, 1 H) 3.97 (d, J=12.81 Hz, 1 H) 4.33 (s, 4 H) 4.38 - 4.52 (m, 4 H) 4.61 - 4.81 (m, 3 H) 5.97 - 6.02 (m, 1 H) 6.80 (s, 2 H) 6.89 (d, J=5.65 Hz, 1 H) 7.20 (d, J=1.88 Hz, 1 H) 7.25 - 7.30 (m, 2 H) 7.33 (d, J=1.51 Hz, 1 H) | 492.2, 490.2 |
| 201 | 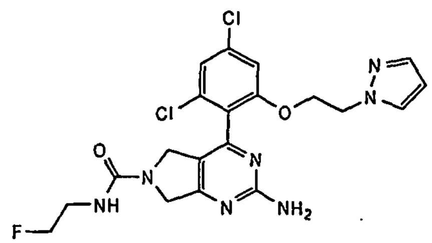 | 2-amino-4{2,4-dichloro-6-[2-(1H-pyrazol-1-yl)ethoxy]ph enyl}-N-(2-fluoroethyl)-5,7-dihydro-6H-pyrrolo[3,4-d] pyrimidine-6-carboxamide | Ex. 110 | 1 H NMR (300 MHz, DMSO-d6) d ppm 3.26 - 3.41 (m, 2 H) 3.57 (d, J=13.00 Hz, 1 H) 3.93 (d, J=12.81 Hz, 1 H) 4.30 - 4.54 (m, 8 H) 6.00 (t, J=1.88 Hz, 1 H) 6.50 (s, 1 H) 6.79 (s, 2 H) 7.20 (d, J=2.07 Hz, 1 H) 7.25 (d, J=1.70 Hz, 1 H) 7.30 (d, J=1.51 Hz, 1 H) 7.32 (d, J=1.51 Hz, 1 H) | 482.2, 480.2 |

(continued)

| Ex. No. | Structure | Name | Synthetic Method | $^1$H NMR | MS |
|---|---|---|---|---|---|
| 202 | 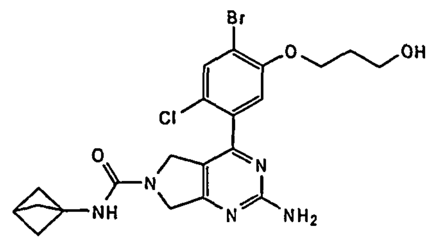 | 2-amino-N-bicyclo[1.1.1] pent-1-yl-4-[4-bromo-2-chloro-5-(3-hydroxypropoxy)phenyl]-5,7-dihydro-6H-pyrrolo[3,4-d] pyrimidine-6-carboxamide | Ex. 111 | 1H NMR (300 MHz, DMSO-d6) d ppm 1.82 - 1.90 (m, 2 H) 1.93 (s, 6 H) 2.34 (s, 1 H) 3.57 (t, J=6.22 Hz, 2 H) 4.12 (t, J=6.12 Hz, 2 H) 4.23 (s, 2 H) 4.39 (s, 2 H) 6.90 (s, 2 H) 7.01 (s, 1 H) 7.15 (s, 1 H) 7.85 (s, 1 H) | 510.2, 508.2 |
| 203 | 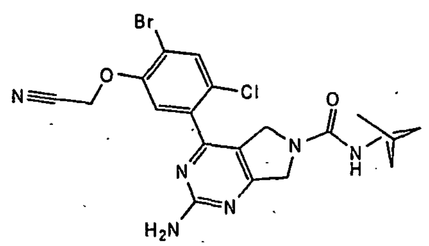 | 2-amino-N-bicyclo[1.1.1] pent-1-yl-4-[4-bromo-2-chloro-5-(cyanometho xy)phenyl]-5,7-dihydro-6H-pyrrolo[3,4-d] pyrimidine-6-carboxamide | Ex. 111 | 1 H NMR (300 MHz, DMSO-d6) d ppm 1.93 (s, 6H) 2.34 (s, 1H) 4.23 (s, 2H) 4.40 (s, 2H) 5.31 (s, 2H) 6.93 (s, 2H) 6.98 (s, 1H) 7.36 (s, 1H) 7.98 (s, 1H) | 491.2, 489.2 |
| 204 | 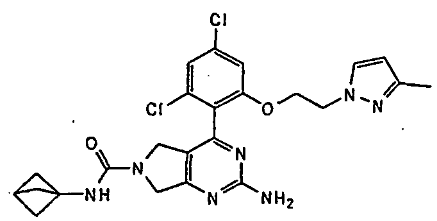 | 2-amino-N-bicyclo[1.1.1] pent-1-yl-4-{2,4-dichloro-6-[2-(3-methyl-1H-pyrazol-1-yl)ethoxy]phenyl)-5,7-dihydro-6H-pyrrolo[3,4-d] pyrimidine-6-carboxamide | Ex. 18 | 1H NMR (300 MHz, METHANOL-d4) d ppm 2.05 (s, 6 H) 2.19 (s, 3 H) 2.38 (s, 1 H) 3.65 (d, J=12.81 Hz, 1 H) 4.06 (d, J=12.81 Hz, 1 H) 4.32 (s, 4 H) 4.53 (s, 2 H) 5.89 (d, J=2.07 Hz, 1 H) 7.08 (d, J=2.26 Hz, 1 H) 7.18 (d, J=1.70 Hz, 1 H) 7.24 (d, J=1.70 Hz, 1 H) | 516.2, 514.2 |
| 205 | 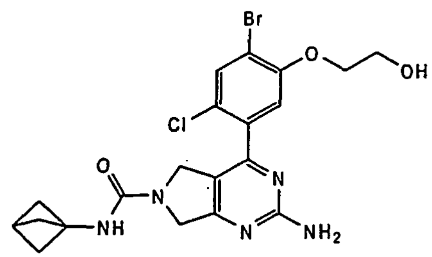 | 2-amino-N-bicyclo[1.1.1] pent-1-yl-4-[4-bromo-2-chloro-5-(2-hydroxyetho xy)phenyl]-5,7-dihydro-6H-pyrrolo[3,4-d] pyrimidine-6-carboxamide | Ex.111 | 1H NMR (300 MHz, DMSO-d6) d ppm 1.93 (s, 6 H) 2.34 (s, 1 H) 3.72 (t, J=4.71 Hz, 2 H) 4.09 (t, J=4.80 Hz, 2 H) 4.23 (s, 2 H) 4.39 (s, 2 H) 6.89 (s, 2 H) 7.01 (s, 1 H) 7.18 (s, 1 H) 7.85 (s, 1 H) | 496.2, 494.2 |

(continued)

| Ex. No. | Structure | Name | Synthetic Method | $^1$H NMR | MS |
|---|---|---|---|---|---|
| 206 | 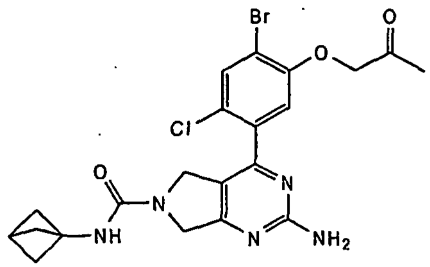 | 2-amino-N-bicyclo[1.1.1] pent-1-yl-4-[4-bromo-2-chloro-5-(2-oxopropoxy)phenyl]-5,7-dihydro-6H-pyrrolo[3,4-d]pyrimidine-6-carboxamide | Ex. 111 | 1 H NMR (300 MHz, DMSO-d6) d ppm 1.93 (s, 6 H) 2.14 (s, 3 H) 2.34 (s, 1 H) 4.18 (s, 2 H) 4.38 (s, 2 H) 4.99 (s, 2 H) 6.88 (s, 2 H) 7.00 (s, 1 H) 7.07 (s, 1 H) 7.87 (s, 1 H) | 508.2, 506.2 |
| 207 | 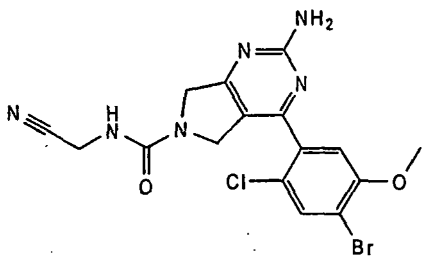 | 2-amino-4-(4-bromo-2-chloro-5-methoxyphe nyl)-N-(cyanomethyl)-5,7-dihydro-6H-pyrrolo[3,4-d] pyrimidine-6-carboxamide | Ex. 2 Ex. 109 | 1 H NMR (300 MHz, DMSO-d6) d ppm 3.87 (s, 3 H) 4.06 (d, J=5.09 Hz, 2 H) 4.31 (s, 2 H) 4.47 (s, 2 H) 6:93 (s, 2 H) 7.17 (s, 1 H) 7.18 (t, J=5.36 Hz, 1 H) 7.87 (s, 1 H) | 439.0, 437.0 |
| 208 | 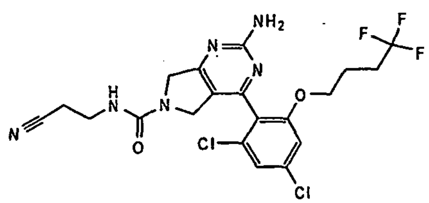 | 2-amino-N-(2-cyanoethyl)-4-[2,4-dichloro-6-(4,4,4-trifluorobutoxy) phenyl]-5,7-dihydro-6H-pyrrolo [3,4-d]pyrimidine-6-carboxamide | Ex. 18 | 1 H NMR (300 MHz, DMSO-d6) d ppm 1.70 - 1.82 (m, 2 H) 2.02 - 2.20 (m, 2 H) 2.62 (t, J=6.31 Hz, 2 H) 3.25 (q, J=6.09 Hz, 2 H) 4.04 - 4.20 (m, 4 H) 4.35 - 4.50 (m, 2 H) 6.81 (t, J=5.46 Hz, 1 H) 6.85 (s, 2 H) 7.31 (d, J=1.51 Hz, 1 H) 7.36 (d, J=1.32 Hz, 1 H) | 505.2, 503.2 |

(continued)

| Ex. No. | Structure | Name | Synthetic Method | $^1$H NMR | MS |
|---|---|---|---|---|---|
| 209 | 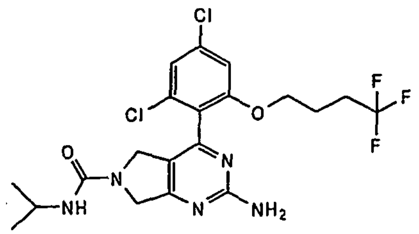 | 2-amino-4-[2,4-dichloro-6-(4,4,4-trifluorobutoxy)phenyl]-N-isopropyl-5,7-dihydro-6H-pyrrolo [3,4-d]pyrimidine-6-carboxamide | Ex. 8a, Ex. 18 | 1 H NMR (300 MHz, DMSO-d6) d ppm 1.05 (d, J=5.27 Hz, 6 H) 1.69 - 1.83 (m, 2 H) 2.01 - 2.20 (m, 2 H) 3.70 - 3.85 (m, 1 H) 4.04 - 4.18 (m, J=7.54 Hz, 4 H) 4.37 - 4.44 (m, 2 H) 6.04 (d, J=7.72 Hz, 1 H) 6.81 (br. s., 2 H) 7.31 (d, J=1.32 Hz, 1 H) 7.36 (d, J=1.51 Hz, 1 H) | 494.2, 492.2 |
| 210 | 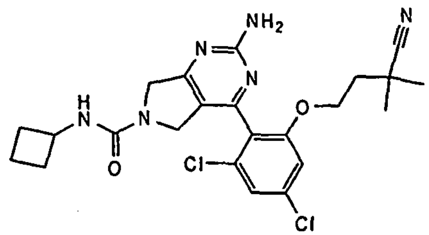 | 2-amino-N-cyclobutyl-4-[2,4-dichloro-6-(3-cyano-3-methylbutoxy )phenyl]-5,7-dihydro-6H-pyrrolo[3,4-d] pyrimidine-6-carboxamide | Ex. 18 | 1H NMR (300 MHz, DMSO-d6) d ppm 1.19 (d, J=3.01 Hz, 6 H) 1.46 - 1.60 (m, 2 H) 1.81 - 1.99 (m, 4 H) 2.02 - 2.15 (m, 2 H) 4.07 - 4.23 (m, 5 H) 4.30 - 4.45 (m, 2 H) 6.51 (d, J=7.72 Hz, 1 H) 6.80 (s, 2 H) 7.36 - 7.39 (m, J=3.20 Hz, 2 H) | 491.2, 489.2 |
| 211 | 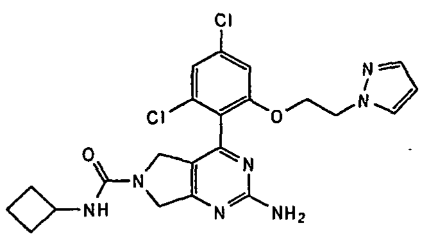 | 2-amino-N-cyclobutyl-4-(2,4-dichloro-6-[2-(1 H-pyrazol-1-yl) ethoxy]phenyl)-5,7-dihydro-6H-pyrrolo[3,4-d]pyrimidine-6-carboxamide | Ex. 18 | 1H NMR (300 MHz, DMSO-d6) d ppm 1.48 - 1.62 (m, 2 H) 1.85 - 2.01 (m, 2 H) 2.04 - 2.18 (m, 2 H) 3.57 (d, J=13.00 Hz, 1 H) 3.93 (d, J=12.24 Hz, 1 H) 4.08 - 4.20 (m, 1 H) 4.29 - 4.46 (m, 6 H) 5.98 - 6.00 (m, 1 H) 6.40 (d, J=7.72 Hz, 1 H) 6.79 (s, 2 H) 7.19 (d, J=2.26 Hz, 1 H) 7.26 (d, J=1.32 Hz, 1 H) 7.28 (d, J=1.51 Hz, 1 H) 7.33 (d, J=1.32 Hz, 1 H) | 490.2, 488.2 |

(continued)

| Ex. No. | Structure | Name | Synthetic Method | $^1$H NMR | MS |
|---|---|---|---|---|---|
| 212 | 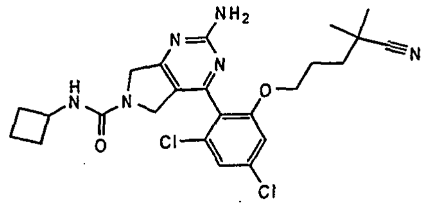 | 2-amino-N-cyclobutyl-4-{2,4-dichloro-6-[(4-cyano-4-methylpentyl )oxy]phenyl}-5,7-dihydro-6H-pyrrolo[3,4-d] pyrimidine-6-carboxamide | Ex. 18 | 1H NMR (300 MHz, DMSO-d6) d ppm 1.17 (s, 6 H) 1.32 - 1.42 (m, 2 H) 1.48 - 1.72 (m, 4 H) 1.83 - 1.99 (m, 2 H) 2.02 - 2.15 (m, 2 H) 4.02 - 4.20 (m, 5 H) 4.33 - 4.47 (m, 2 H) 6.52 (d, J=7.72 Hz, 1 H) 6.83 (s, 2 H) 7.29 (d, J=1.32 Hz, 1 H) 7.34 (d, J=1.70 Hz, 1 H) | 505.2, 503.2 |
| 213 | 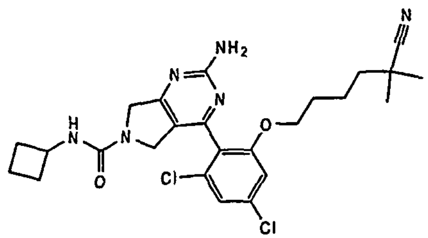 | 2-amino-N-cyclobutyl-4-{2,4-dichloro-6-[(5-cyano-5-methylhexyl) oxy]phenyl}-5,7-dihydro-6H-pyrrolo[3,4-d] pyrimidine-6-carboxamide | Ex. 18 | 1H NMR (300 MHz, DMSO-d6) d ppm 1.18 (d, J=2.07 Hz, 6 H) 1.25 - 1.64 (m, 8 H) 1.83 - 1.99 (m, 2 H) 2.02 - 2.15 (m, 2 H) 4.00 - 4.18 (m, 5 H) 4.40 (s, 2 H) 6.50 (d, J=7.72 Hz, 1 H) 6.81 (s, 2 H) 7.29 (d, J=1.51 Hz, 1 H) 7.33 (d, J=1.70 Hz, 1 H) | 519.2, 517.2 |
| 214 | 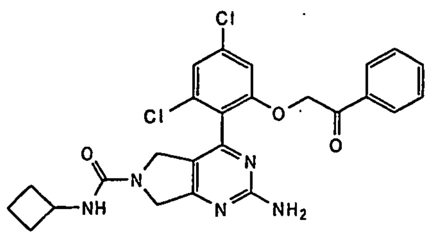 | 2-amino-N-cyclobutyl-4-[2,4-dichloro-6-(2-oxo-2-phenylethoxy)phenyl]-5,7-dihydro-6H-pyrrolo[3,4-d] pyrimidine-6-carboxamide | Ex. 18 | 1H NMR (400 MHz, DMSO-d6) d ppm 1.47 - 1.61 (m, 2 H) 1.87 - 1.99 (m, 2 H) 2.05 - 2.14 (m, 2 H) 4.07 - 4.21 (m, 2 H) 4.29 (d, J=13.14 Hz, 1 H) 4.41 (s, 2 H) 5.66 (d, J=17.94 Hz, 1 H) 5.79 (d, J=17.68 Hz, 1 H) 6.54 (d, J=7.83 Hz, 1 H) 6.83 (s, 2 H) 7.29 (d, J=1.77 Hz, 1 H) 7.34 (d, J=1.77 Hz, 1 H) 7.54 (t, J=7.71 Hz, 2 H) 7.67 (t, J=7.45 Hz, 1 H) 7.93 (d, J=7.33 Hz, 2 H) | 514.2, 512.2 |

(continued)

| Ex. No. | Structure | Name | Synthetic Method | $^1$H NMR | MS |
|---|---|---|---|---|---|
| 215 | 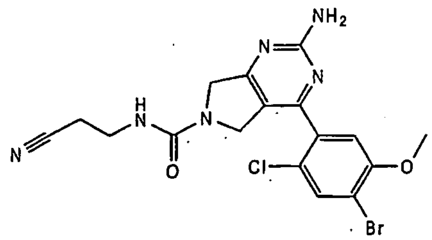 | 2-amino-4-(4-bromo-2-chloro-5-methoxyphenyl)-N-(2-cyanoethyl)-5,7-dihydro-6H-pyrrolo[3,4-d]pyrimidine-6-carboxamide | Ex. 2, Ex. 109 | 1H NMR (300 MHz, DMSO-d6) d ppm 2.63 (t, J=6.40 Hz, 2 H) 3.21 - 3.30 (m, 2 H) 3.86 (s, 3 H) 4.28 (s, 2 H) 4.44 (s, 2 H) 6.83 (t, J=4.52 Hz, 1 H) 6.93 (s, 2 H) 7.18 (s, 1 H) 7.87 (s, 1 H) | 453.0, 451.0 |
| 216 | 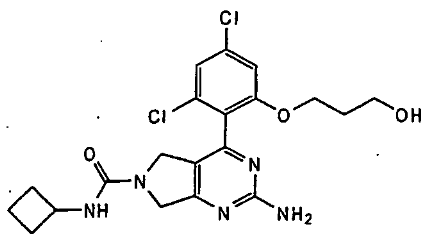 | 2-amino-N-cyclobutyl-4-[2,4-dichloro-6-(3-hydroxyprop oxy) phenyl]-5,7-dihydro-6H-pyrrolo [3,4-d]pyrimidine-6-carboxamide | Ex. 18 | 1H NMR (300 MHz, DMSO-d6) d ppm 1.47 - 1.60 (m, 2 H) 1.62 - 1.73 (m, 2H) 1.84 - 1.99 (m, 2 H) 2.02 - 2.15 (m, 2 H) 3.31 (t, J=5.56 Hz, 2 H) 4.04 - 4.18 (m, 5 H) 4.41 (s, 2 H) 6.51 (d, J=7.54 Hz, 1 H) 6.82 (s, 2 H) 7.28 (s, 1H) 7.32 (s, 1 H) | 454.2, 452.2 |
| 217 | 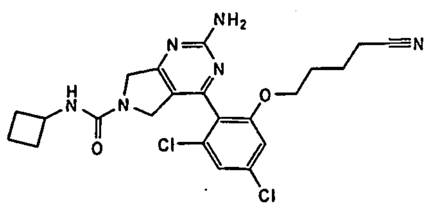 | 2-amino-N-cyclobutyl-4-[2,4-dichloro 6-(4-cyanobutoxy) phenyl]-5,7-dihydro-6H-pyrrolo [3,4-d]pyrimidine-6-carboxamide | Ex. 18 | 1H NMR (300 MHz, DMSO-d6) d ppm 1:43 - 1.72 (m, 6 H) 1.84 - 2.00 (m, 2 H) 2.03 - 2.17 (m, 2 H) 2.40 (t, J=7.06 Hz, 2 H) 4.02 - 4.21 (m, 5 H) 4.41 (s, 2 H) 6.50 (d, J=7.91 Hz, 1 H) 6.81 (s, 2 H) 7.29 (d, J=1.70 Hz, 1 H) 7.34 (d, J=1.51 Hz, 1H) | 477.2, 475.2 |

(continued)

| Ex. No. | Structure | Name | Synthetic Method | $^1$H NMR | MS |
|---|---|---|---|---|---|
| 218 | 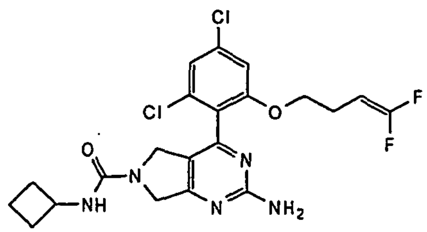 | 2-amino-N-cyclobutyl-4-12,4-dichloro-6-[(4,4-difluorobut-3-en-1-yl)oxy]phenyl }-5,7-dihydro-6H-pyrrolo[3,4-d]pyrimidine-6-carboxamide | Ex. 18 | 1H NMR (300 MHz, DMSO-d6) d ppm 1.46 - 1.61 (m, 2 H) 1.82 - 1.99 (m, 2 H) 2.02 - 2.14 (m, 2 H) 2.15 - 2.28 (m, 2 H) 3.94 - 4.46 (m, 8 H) 6.48 (d, J=8.48 Hz, 1 H) 6.79 (s, 2 H) 7.28 (d, J=1.32 Hz, 1 H) 7.34 (d, 1 H) | 486.2, 484.2 |
| 219 | 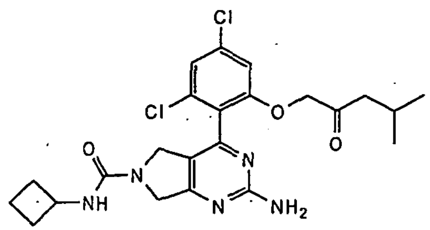 | 2-amino-N-cyclobutyl-4-{2,4-dichloro-6-[(4-methyl-2-oxopentyl)oxy]phenyl)-5,7-dihydro-6H-pyrrolo[3,4-d] pyrimidine-6-carboxamide | Ex. 18 | 1H NMR (300 MHz, DMSO-d6) d ppm 0.80 (dd, J=6.59, 2.45 Hz, 6 H) 1.48 - 1.60 (m, 2 H) 1.86-2.15 (m, 5H)2.24 (dd, J=6.78, 2.07 Hz, 2 H) 4.06 - 4.29 (m, 3 H) 4.41 (s, 2 H) 4.81 (d, J=17.52 Hz, 1 H) 4.93 (d, J=17.33 Hz, 1 H) 6.52 (d, J=7.72 Hz, 1 H) 6.83 (s, 2 H) 7.13 (d, J=1.32 Hz, 1 H) 7.35 (d, J=1.70 Hz, 1 H) | 494.0, 492.0 |
| 220 | 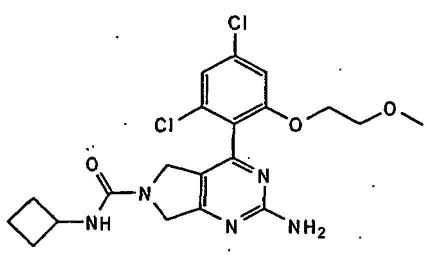 | 2-amino-N-cyclobutyl-4-[2,4-dichloro-6-(2-methoxyethoxy) phenyl]-5,7-dihydro-6H-pyrrolo [3,4-d]pyrimidine-6-carboxamide | Ex. 18 | 1H NMR (300 MHz, DMSO-d6) d ppm 1.48 - 1.60 (m, 2 H) 1.83 - 1.99 (m, 2 H) 2.02 - 2.14 (m, 2 H) 3.12 (s, 3 H) 3.45 - 3.52 (m, 2 H) 4.06 - 4.22 (m, 5 H) 4.40 (br. s., 2 H) 6.49 (d, J=8.10 Hz, 1 H) 6.81 (br. s., 2 H) 7.31 (d, J=1.51 Hz, 1 H) 7.34 (d, J=1.70 Hz, 1 H) | 454.0, 452.0 |

(continued)

| Ex. No. | Structure | Name | Synthetic Method | $^1$H NMR | MS |
|---|---|---|---|---|---|
| 221 | 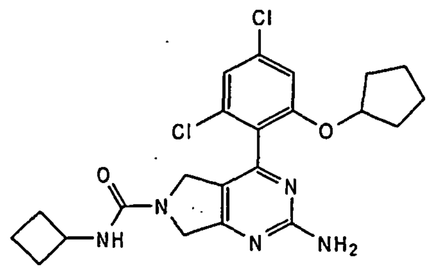 | 2-amino-N-cyclobutyl-4-[2,4-dichloro-6-(cyclopentylo xy) phenyl]-5,7-dihydro-6H-pyrrolo [3,4-d]pyrimidine-6-carboxamide | Ex. 18 | 1H NMR (300 MHz, DMSO-d6) d ppm 1.39 - 1.98 (m, 1-2 H) 2.02 - 2.15 (m, 2 H) 4.03 - 4.18 (m, 3 H) 4.40 (s, 2 H) 4.89 - 4.97 (m, 1 H) 6.52 (d, J=7.91 Hz, 1 H) 6.80 (s, 2 H) 7.26 (d, J=1.70 Hz, 1 H) 7.30 (d, J=1.70 Hz, 1 H) | 464.0, 462.0 |
| 222 | 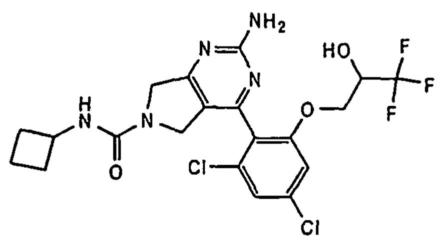 | 2-amino-N-cyclobutyl-4-[2,4-dichloro-6-(3,3,3-trifluoro-2-hydroxyprop oxy)phenyl]-5,7-dihydro-6H-pyrrolo[3,4-d] pyrimidine-6-carboxamide | Ex. 18 | 1H NMR (300 MHz, DMSO-d6) d ppm 1.48 - 1.59 (m, 2 H) 1.84 - 1.99 (m, 2 H) 2.03 - 2.14 (m, 2 H) 4.04 - 4.25 (m, 6 H) 4.39 (s, 2 H) 6.48 (d, J=7.35 Hz, 1 H) 6.63 (br. s., 1 H) 6.78 (s, 2 H) 7.38 (s, 1 H) 7.41 (s, 1 H) | 508.0, 506.0 |
| 223 | 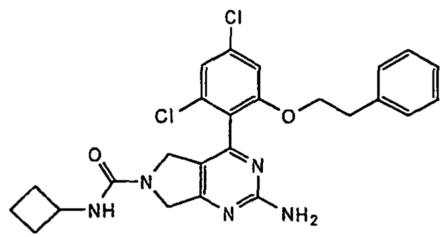 | 2-amino-N-cyclobutyl-4-[2,4-dichloro-6-(2-phenylethoxy ) phenyl]-5,7-dihydro-6H-pyrrolo [3,4-d)pyrimidine-6-carboxamide | Ex. 18 | 1H NMR (300 MHz, DMSO-d6) d ppm 1.48 - 1.62 (m, 2 H) 1.84 - 2.01 (m, 2 H) 2.04 - 2.19 (m, 2 H) 2.70 - 2.92 (m, 2 H) 3.60 (d, J=12.62 Hz, 1 H) 3.94 (d, J=13.19 Hz, 1 H) 4.07 - 4.46 (m, 5 H) 6.39 (d, J=7.72 Hz, 1 H) 6.83 (br. s., 2 H) 6.98 (d, J=6.41 Hz, 2 H) 7.04 - 7.19 (m, 3 H) 7.26 (s, 1 H) 7.30 (s, 1 H) | 500.0, 498.0 |

(continued)

| Ex. No. | Structure | Name | Synthetic Method | $^1$H NMR | MS |
|---|---|---|---|---|---|
| 224 | 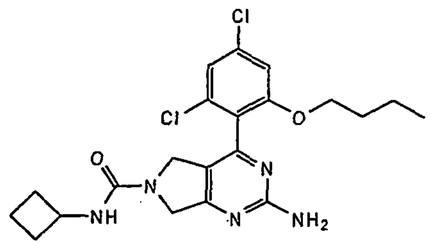 | 2-amino-4-(2-butoxy-4,6-dichlorophenyl)-N-cyclobutyl-5,7-dihydro-6H-pyrrolo[3,4-d] pyrimidine-6-carboxamide | Ex. 18 | 1H NMR (400 MHz, DMSO-d6) d ppm 0.78 (t, J=7.45 Hz, 3 H) 1.14 - 1.25 (m, 2 H) 1.46 - 1.58 (m, 4 H) 1.86 - 1.97 (m, 2 H) 2.04 - 2.13 (m, 2 H) 3.98 - 4.05 (m, 2 H) 4.08 - 4.16 (m, 3 H) 4.40 (s, 2 H) 6.52 (d, J=7.83 Hz, 1 H) 6.82 (s, 2 H) 7.28 (d, J=1.77 Hz, 1 H) 7.32 (d, J=1.77 Hz, 1 H) | 452.0, 450.0 |
| 225 | 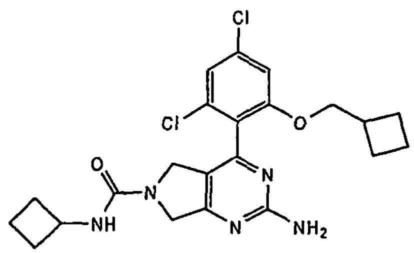 | 2-amino-N-cyclobutyl-4-[2,4-dichloro-6-(cyclobutylmethoxy) phenyl]-5,7-dihydro-6H-pyrrolo [3,4-d]pyrimidine-6-carboxamide | Ex. 18 | 1H NMR (300 MHz, DMSO-d6) d ppm 1.45 - 2.00 (m, 11 H) 2.02 - 2.16 (m, 2 H) 3.98 (d, J=5.84 Hz, 2 H) 4.08 - 4.20 (m, 3 H) 4.36 - 4.48 (m, 2 H) 6.51 (d, J=7.91 Hz, 1 H) 6.81 (s, 2 H) 7.28 (s, 1 H) 7.33 (s, 1 H) | 464.2, 462.2 |
| 226 | 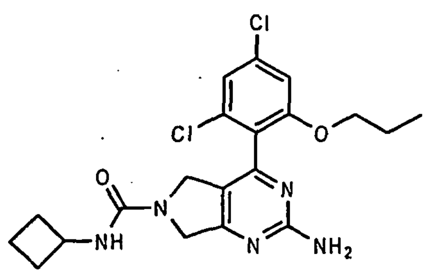 | 2-amino-N-cyclobutyl-4-(2,4-dichloro-6-propoxyphenyl)-5,7-dihydro-6H-pyrrolo[3,4-d] pyrimidine-6-carboxamide | Ex. 18 | 1H NMR (300 MHz, DMSO-d6) d ppm 0.76 (t, J=7.35 Hz, 3 H) 1.47 - 1.62 (m, 4 H) 1.83-2.01 (m, 2 H) 2.02 - 2.15 (m, 2 H) 3.93 - 4.05 (m, 2 H) 4.07 - 4.17 (m, 3 H) 4.40 (s, 2 H) 6.51 (d, J=8.29 Hz, 1 H) 6.82 (br. s., 2 H) 7.27 (d, J=1.32 Hz, 1 H) 7.32 (d, J=1.32Hz, 1 H) | 438.2, 436.2 |

(continued)

| Ex. No. | Structure | Name | Synthetic Method | $^1$H NMR | MS |
|---|---|---|---|---|---|
| 227 | 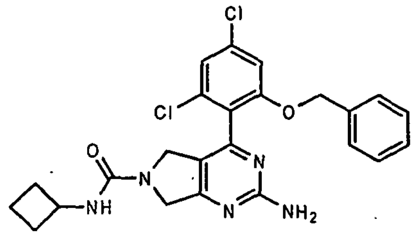 | 2-amino-4-[2-(benzyloxy)-4,6-dichlorophenyl]-N-cyclobutyl-5,7-dihydro-6H-pyrrolo[3,4-d] pyrimidine-6-carboxamide | Ex. 18 | 1H NMR (300 MHz, DMSO-d6) d ppm 1.48 - 1.60 (m, 2 H) 1.84 - 2.01 (m, 2 H) 2.03 - 2.15 (m, 2 H) 4.07 - 4.20 (m, 3 H) 4.40 (s, 2 H) 5.20 (s, 2 H) 6.51 (d, J=7.91 Hz, 1 H) 6.85 (br. s., 2 H) 7.19 - 7.25 (m, 2 H) 7.26 - 7.34 (m, 3 H) 7.36 (s, 2 H) | 486.2, 484.2 |
| 228 | 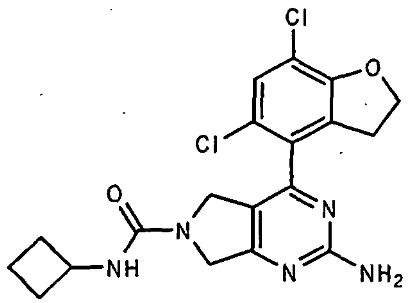 | 2-amino-N-cyclobutyl-4-(5,7-dichloro-2,3-dihydro-1-benzofuran-4-yl)-5,7-dihydro-6H-pyrrolo[3,4-d]pyrimidine-6-carboxamide | Ex. 7 | 1H NMR (400 MHz, DMSO-d6) d ppm 1.47 - 1.61 (m, 2 H) 1.87 - 1.99 (m, 2 H) 2.05 - 2.13 (m, 2 H) 2.99 - 3.21 (m, 2 H) 4.07 - 4.21 (m, 2 H) 4.27 (d, J=13.14 Hz, 1 H) 4.36 - 4.48 (m, 2 H) 4.61 - 4.74 (m, 2 H) 6.54 (d, J=7.83 Hz, 1 H) 6.90 (br. s., 2 H) 7.53 (s, 1 H) | 422.2, 420.2 |
| 229 | 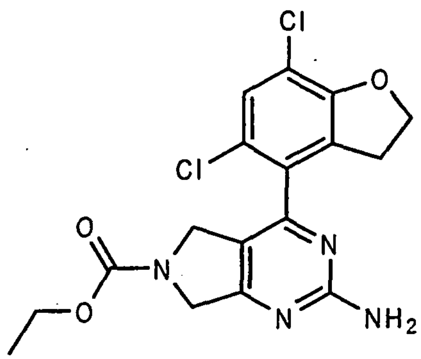 | ethyl 2-amino-4-(5,7-dichloro-2,3-dihydro-1-benzofuran-4-yl)-5.7-dihydro-6H-pyrrolo[3,4-d] pyrimidine-6-carboxylate | Ex. 1 | 1H NMR (300 MHz, DMSO-d6) d ppm 1.13 - 1.27 (m, 3 H) 2.95 - 3.26 (m, 2 H) 3.98 - 4.16 (m, 2 H) 4.22 - 4.39 (m, 2 H) 4.41 - 4.58 (m, 2 H) 4.61 - 4.76 (m, 2 H) 6.94 (br. s., 2 H) 7.51 (s, 1 H) | 397.2, 395.2 |

(continued)

| Ex. No. | Structure | Name | Synthetic Method | $^1$H NMR | MS |
|---|---|---|---|---|---|
| 230 | 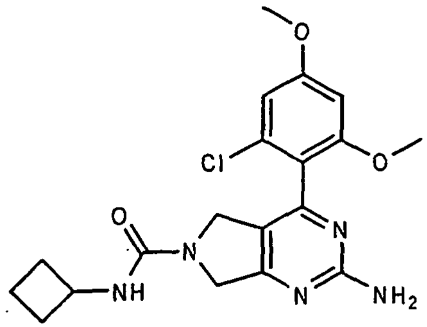 | 2-amino-4-(2-chloro-4,6-dimethoxyphenyl)-N-cyclobutyl-5,7-dihydro-6H-pyrrolo[3,4-d] pyrimidine-6-carboxamide | Ex.117 | 1H NMR (300 MHz, DMSO-d6) d ppm 1.48 - 1.60 (m, 2 H) 1.84 - 2.00 (m, 2 H) 2.03 - 2.15 (m, 2 H) 3.72 (s, 3 H) 3.83 (s, 3 H) 4.04 - 4.19 (m, 3 H) 4.40 (s, 2 H) 6.51 (d, J=7.91 Hz, 1 H) 6.67 (d, J=2.07 Hz, 1 H) 6.71 - 6.77 (m, 3 H) | 406.0, 404.0 |
| 231 | 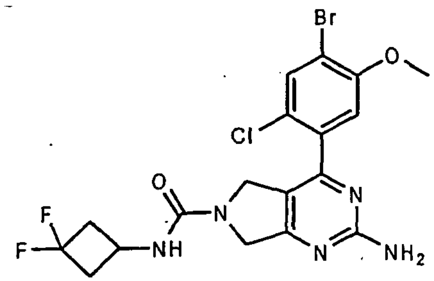 | 2-amino-4-(4-bromo-2-chloro-5-methoxyphe nyl)-N-(3,3-difluorocyclobutyl)-5,7-dihydro-6H-pyrrolo[3,4-d]pyrimidine-6-carboxamide | Ex. 2, Ex. 109 | 1H NMR (300 MHz, DMSO-d6) d ppm 2.53 - 2.89 (m, 4 H) 3.87 (s, 3 H) 3.95 - 4.08 (m, 1 H) 4.28 (s, 2 H) 4.44 (s, 2 H) 6.75 (d, J=6.78 Hz, 1 H) 6.91 (br. s., 2 H) 7.17 (s, 1 H) 7.87 (s, 1 H) | 489.8, 487.8 |
| 232 | 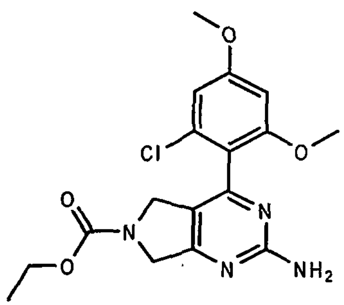 | ethyl 2-amino-4-(2-chloro-4,6-dimethoxyphenyl)-5,7-dihydro-6H-pyrrolo[3,4-d]pyrimidine-6-carboxylate | Ex.117a | 1H NMR (300 MHz, DMSO-d6) d ppm 1.13 - 1.24 (m, 3 H) 3.72 (s, 3 H) 3.83 (s, 3 H) 4.00 - 4.19 (m, 4 H) 4.47 (d, J=8.48 Hz, 2 H) 6.66 (d, J=2.26 Hz, 1 H) 6.74 (d, J=2.07 Hz, 1 H) 6.79 (br. s., 2 H) | 379.2 |

(continued)

| Ex. No. | Structure | Name | Synthetic Method | $^{1}$H NMR | MS |
|---|---|---|---|---|---|
| 233 | 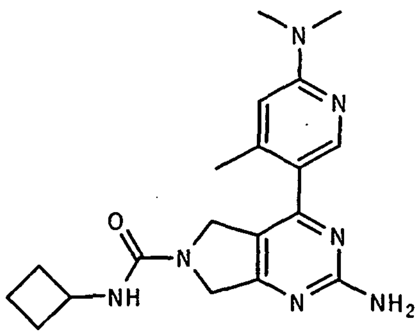 | 2-amino-N-cyclobutyl-4-[6-(dimethylami no)-4-methylpyridin -3-yl]-5,7-dihydro-6H-pyrrolo[3,4-d]pyrimidine-6-carboxamide | Ex. 117 | 1H NMR (300 MHz, DMSO-d6) d ppm 1.48 - 1.63 (m, 2 H) 1.87 - 2.02 (m, 2 H) 2.04 - 2.17 (m, 2 H) 2.28 (s, 3 H) 3.06 (s, 6 H) 4.06 - 4.22 (m, 1 H) 4.34 - 4.48 (m, 4 H) 6.52 - 6.69 (m, 4 H) 8.05 (s, 1 H) | 343.4 |
| 234 | 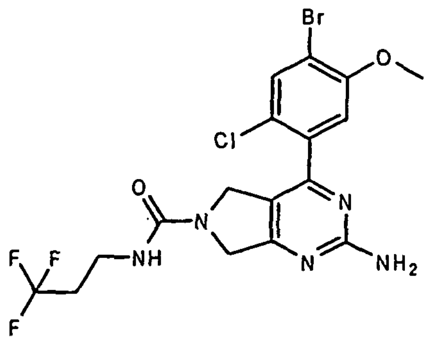 | 2-amine-4-(4-bromo-2-chloro-5-methoxyphe nyl)-N-(3,3,3-trifluoropropy l)-5,7-dihydro-6H-pyrrolo[3,4-d]pyrimidine-6-carboxamide | Ex. 2, Ex. 109 | 1H NMR (300 MHz, DMSO-d6) d ppm 2.32 - 2.47 (m, 2 H) 3.22 - 3.30 (m, 2 H) 3.86 (s, 3 H) 4.27 (s, 2 H) 4.42 (s, 2 H) 6.63 (t, J=5.27 Hz, 1 H) 6.90 (br. s., 2 H) 7.17 (s, 1 H) 7.86 (s, 1 H) | 496.0, 493.9 |
| 235 | 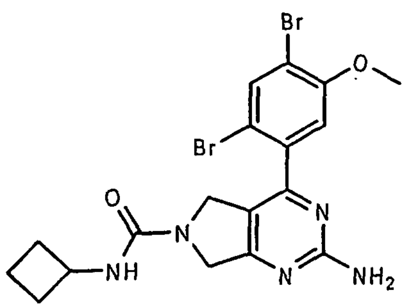 | 2-amino-N-cyclobutyl-4-(2,4-dibromo-5-methoxyphe nyl)-5,7-dihydro-6H-pyrrolo[3,4-d) pyrimidine-6-carboxamide | Ex. 117 | 1H NMR (300 MHz, DMSO-d6) d ppm 1.47 - 1.63 (m, 2 H) 1.84 - 2.01 (m, 2 H) 2.02 - 2.16 (m, 2 H) 3.85 (s, 3 H) 4.05 - 4.20 (m, 1 H) 4.24 (s, 2 H) 4.41 (s, 2 H) 6.52 (d, J=7.91 Hz, 1 H) 6.88 (br s., 2 H) 7.15 (s, 1 H) 7.96 (s, 1 H) | 498.2 |

(continued)

| Ex. No. | Structure | Name | Synthetic Method | $^1$H NMR | MS |
|---|---|---|---|---|---|
| 236 | 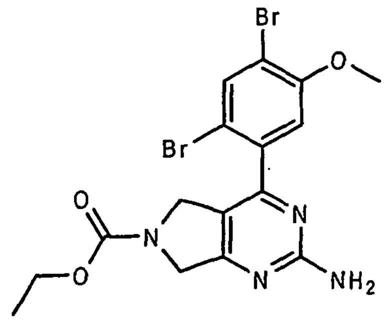 | ethyl 2-amino-4-(2,4-dibromo-5-methoxyphenyl)-5,7-dihydro-6H-pyrrolo[3,4-d]pyrimidine-6-carboxylate | Ex. 117a | 1H NMR (300 MHz, DMSO-d6) d ppm 1.13 - 1.27 (m, 3 H) 3.85 (s, 3 H) 4.02 - 4.16 (m, 2 H) 4.29 - 4.35 (m, 2 H) 4.45 - 4.52 (m, 2 H) 6.94 (br. s., 2 H) 7.12 (s, 1 H) 7.96 (s, 1 H) | 473.2 |
| 237 | 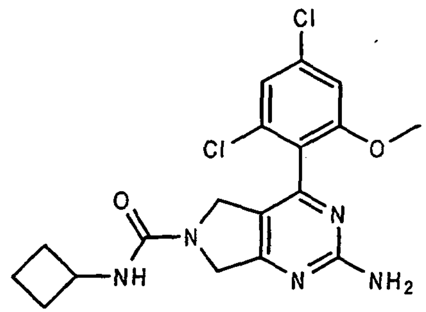 | 2-amino-N-cyclobutyl-4-(2,4-dichloro-6-methoxyphenyl)-5,7-dihydro-6H-pyrrolo[3,4-d] pyrimidine-6-carboxamide | Ex. 117 | 1H NMR (300 MHz, DMSO-d6) d ppm 1.48 - 1.61 (m, 2 H) 1.85 - 2.00 (m, 2 H) 2.03 - 2.15 (m, 2 H) 3.77 (s, 3 H) 4.04 - 4.19 (m, 3 H) 4.41 (s, 2 H) 6.50 (d, J=7.91 Hz, 1 H) 6.82 (br. s., 2 H) 7.28 (d, J=1.70 Hz, 1 H) 7.34 (d, J=1.70 Hz, 1 H) | 408.2 |
| 238 | 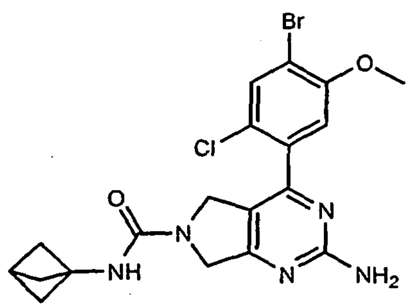 | 2-amino-N-bicyclo[1.1.1] pent-1-yl-4-(4-bromo-2-chloro-5-methoxyphe nyl)-5,7-dihydro-6H-pyrrolo[3,4-d]pyrimidine-6-carboxamide | Ex. 2, Ex. 109 | 1H NMR (300 MHz, DMSO-d6) d ppm 1.93 (s, 6 H) 2.34 (s, 1 H) 3.86 (s, 3 H) 4.23 (s, 2 H) 4.39 (s, 2 H) 6.87 (br. s., 2 H) 6.99 (s, 1 H) 7.15 (s, 1 H) 7.85 (s, 1 H) | 466.0, 464.0 |

(continued)

| Ex. No. | Structure | Name | Synthetic Method | $^1$H NMR | MS |
|---|---|---|---|---|---|
| 239 | 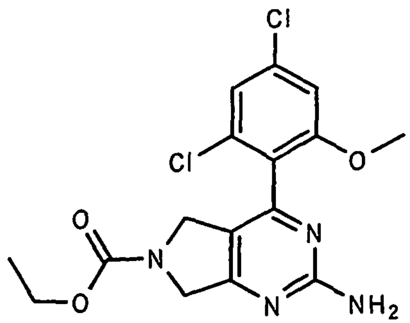 | ethyl 2-amino-4-(2,4-dichloro-6-methoxyphenyl)-5,7-dihydro-6H-pyrrolo[3,4-d]pyrimidine-6-carboxylate | Ex. 117a | 1H NMR (300 MHz, DMSO-d6) d ppm 1.13 - 1.24 (m, 3 H) 3.77 (s, 3 H) 4.00 - 4.12 (m, 2 H) 4.14 (s, 1H) 4.17 (s, 1 H) 4.47 (s, 1 H) 4.49 (s, 1H) 6.87 (br. s., 2 H) 7.25 (d, J=1.51 Hz, 1 H) 7.32 (d, J=1.51 Hz, 1 H) | 385.2, 383.2 |
| 240 | 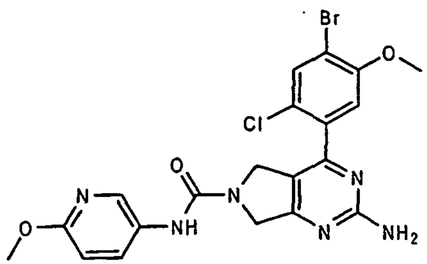 | 2-amino-4-(4-bromo-2-chloro-5-methoxyphe nyl)-N-(6-methoxypyri din-3-yl)-5,7-dihydro-6H-pyrrolo[3,4-d] pyrimidine-6-carboxamide | Ex. 118 | 1H NMR. (300 MHz, DMSO-d6) d ppm 3.79 (s, 3 H) 3.87 (s, 3 H) 4.46 (s, 2 H) 4.58 (s, 2 H) 6.73 (d, J=8.85 Hz, 1 H) 6.94 (s, 2 H) 7.19 (s, 1 H) 7.79 (dd, J=8.95, 2.54 Hz, 1 H) 7.88 (s, 1 H) 8.21 (d, J=2.64 Hz, 1 H) 8.37 (s, 1 H) | 507.2, 505.2 |
| 241 | 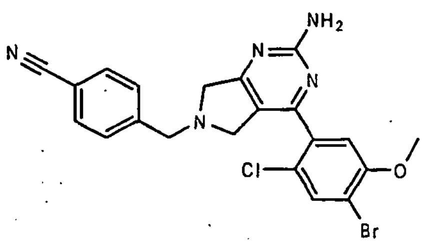 | 4-{[2-amino-4-(4-bromo-2-chloro-5-methoxyphenyl)-5,7-dihydro-6H-pyrrolo[3,4-d] pyrimidin-6-y1]methyl} benzonitrile | Ex. 120 | 1H NMR (300 MHz, DMSO-d6) d ppm 3.63 (s, 2 H) 3.78 (s, 2 H) 3.84 (s, 3 H) 3.94 (s, 2 H) 6.76 (br. s., 2 H) 7.09 (s, 1 H) 7.54 (d, J=8.10 Hz, 2 H) 7.76 - 7.83 (m, 3 H) | 472.2, 470.2 |

(continued)

| Ex. No. | Structure | Name | Synthetic Method | $^1$H NMR | MS |
|---|---|---|---|---|---|
| 242 | 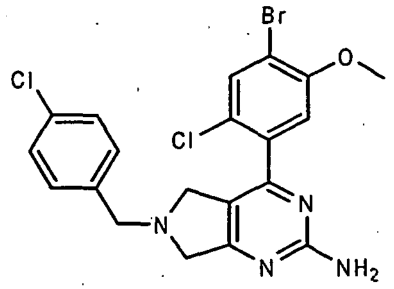 | 4-(4-bromo-2-chloro-5-methoxyphenyl)-6-(4-chlorobenzyl )-6,7-dihydro-5H-pyrrolo[3,4-d]pyrimidin-2-amine | Ex. 120 | 1H NMR (300 MHz, DMSO-d6) d ppm 3.59 (s, 2 H) 3.75 (s, 2 H) 3.82 - 3.86 (m, 5 H) 6.77 (br. s., 2 H) 7.09 (s, 1 H) 7.35 - 7.39 (m, 4 H) 7.79 (s, 1 H) | 481.0, 479.0 |
| 243 | 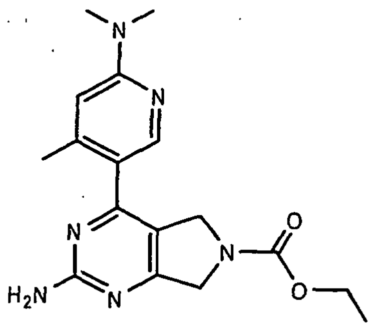 | ethyl2-amino-4-[6-(dimethylamino)-4-methylpyridin-3-yl]-5,7-dihydro-6H-pyrrolo[3,4-d] pyrimidine-6-carboxylate | Ex. 117a | 1H NMR (300 MHz, DMSO-d6) d ppm 1.15 - 1.26 (m, 3 H) 2.28 (s, 3 H) 3.06 (s, 6 H) 4.02 - 4.15 (m, 2 H) 4.40 - 4.50 (m, 4 H) 6.56 (s, 1 H) 6.67 (s, 2 H) 8.06. (d, J=3.58 Hz, 1 H) | 343.4 |
| 244 | 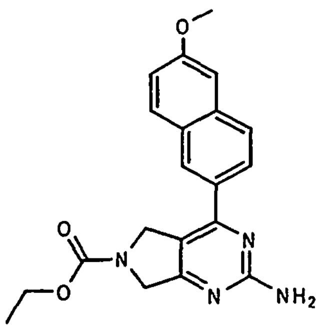 | ethyl 2-amino-4-(6-methoxy-2-naphthyl)-5,7-dihydro-6H-pyrrolo [3,4-d]pyrimidine-6-carboxylate | Ex: 1 | 1H NMR (300 MHz, DMSO-d6) d ppm 1.24 (t, J=7.06 Hz, 3 H) 3.91 (s, 3 H) 4.14 (q, J=6.78 Hz, 2 H) 4.44 - 4.51 (m, 2 H) 4.86 (s, 2 H) 7.24 (dd, J=8.95, 2.35 Hz, 1 H) 7.40 (d, J=1.88 Hz, 1 H) 7.93 - 8.04 (m, 3 H) 8.32 (d, J=3.20 Hz, 1 H) | 365.4 |

(continued)

| Ex. No. | Structure | Name | Synthetic Method | $^1$H NMR | MS |
|---|---|---|---|---|---|
| 245 | 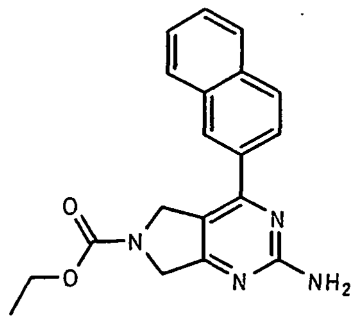 | ethyl 2-amino-4-(2-naphthyl)-5,7-dihydro-6H-pyrrolo[3,4-d] pyrimidine-6-carboxylate | Ex. 1 | 1 H NMR (300 MHz, DMSO-d6) d ppm 1.20 - 1.28 (m, 3 H) 4.14 (q, J=7.16 Hz, 2 H) 4.45 - 4.51 (m, 2 H) 4.87 (s, 2 H) 6.86 (br. s., 2 H) 7.57 - 7.67 (m, 2 H) 7.95 - 8.14 (m, 4 H) 8.38 (d, J=3.58 Hz, 1 H) | 335.2 |
| 246 | 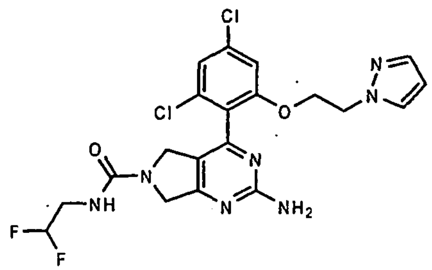 | 2-amino-4-{2,4-dichloro-6-[2-(1 H-pyrazol-1-yl)ethoxy] phenyl}-N-(2,2-difluoroethyl)-5,7-dihydro-6H-pyrrolo[3,4-d] pyrimidine-6-carboxamide | Ex. 110 | 1 H NMR (300 MHz, DMSO-d6) d ppm 3.33 - 3.51 (m, 2 H) 3.56 (d, J=12.81 Hz, 1 H) 3.94 (d, J=12.62 Hz, 1 H) 4.29 - 4.38 (m, 4 H) 4.40 - 4.50 (m, 2 H) 5.77 - 6.20 (m, 2 H) 6.70 (br. s., 1 H) 6.80 (br. s., 2 H) 7.20 (d, J=2.07 Hz, 1 H) 7.26 (d, J=1.51 Hz, 1 H) 7.30 (d, J=1.51 Hz, 1 H) 7.32 (d, J=1.70 Hz, 1 H) | 500.2, 498.2 |
| 247 | 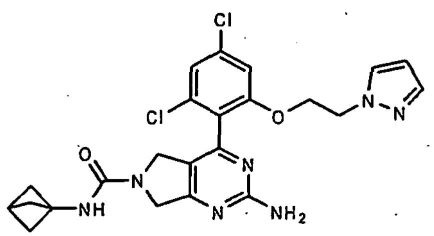 | 2-amino-N-bicyclo[1.1.1]pent-1-yl-4-{2,4-dichloro-6-[2-(1 H-pyrazol-1-yl)ethoxy]phenyl}-5,7-dihydro-6H-pyrrolo[3,4-d] pyrimidine-6-carboxamide | Ex. 110 | 1 H NMR (300 MHz, DMSO-d6) d ppm 1.95 (s, 6 H) 2.36 (s, 1 H) 3.53 (d, J=13.19 Hz, 1 H) 3.89 (d, J=13.00 Hz, 1 H) 4.33 (s, 6 H) 5.99 - 6.02 (m, 1 H) 6.77 (br. s., 2 H) 6.85 (br. s., 1 H) 7.19 (d, J=2.07 Hz, 1 H) 7.25 (d, J=1.51 Hz, 1 H) 7.30 (d, J=1.70 Hz, 1 H) 7.32 (d, J=1.70 Hz, 1 H) | 502.2, 500.2 |

(continued)

| Ex. No. | Structure | Name | Synthetic Method | $^1$H NMR | MS |
|---|---|---|---|---|---|
| 248 | 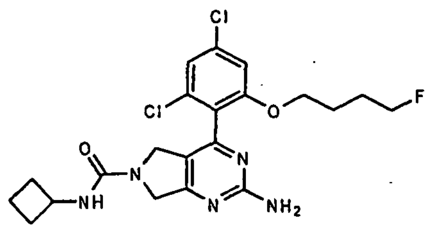 | 2-amino-N-cyclobutyl-4-[2,4-dichloro-6-(4-fluorobutoxy) phenyl]-5,7-dihydro-6H-pyrrolo [3,4-d]pyrimidine-6-carboxamide | Ex. 18 | 1 H NMR (300 MHz, DMSO-d6) d ppm 1.61 (d, J=5.27 Hz, 6 H) 1.84 - 1.98 (m, 2 H) 2.03 - 2.15 (m, 2 H) 4.03 - 4.28 (m, 6 H) 4.37 - 4.43 (m, 3 H) 6.51 (d, J=7.91 Hz, 1 H) 6.82 (br. s., 2 H) 7.29. (d, J=1.32 Hz, 1 H) 7.34 (d, J=1.13 Hz, 1 H) | 470.2, 468.2 |
| 249 | 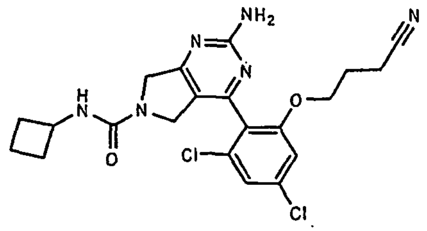 | 2-amino-N-cyclobutyl-4-[2,4-dichloro-6-(3-cyanopropoxy) phenyl]-5,7-dihydro-6H-pyrrolo [3,4-d]pyrimidine-6-carboxamide | Ex. 18 | 1H NMR (400 MHz, DMSO-d6) d ppm 1.47 - 1.59 (m, 2 H) 1.82 - 1.98 (m, 4 H) 2.04 - 2.13 (m, 2 H) 2.38 (t, J=7.20 Hz, 2 H) 4.06 - 4.18 (m, 5 H) 4.40 (br, s., 2 H) 6.50 (d, J=7.83 Hz, 1 H) 6.82 (br. s., 2 H) 7.33 (d, J=1.77 Hz, 1 H) 7.37 (d, J=1.77 Hz, 1 H) | 463.2, 461.2 |
| 250 | 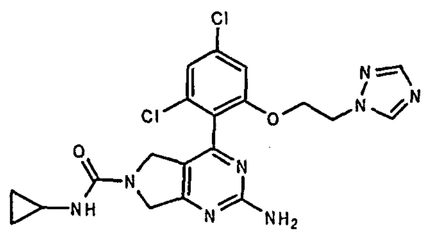 | 2-amino-N-cyclopropyl-4-{2,4-dichloro-6-[2-(1H-1,2,4-triazol-1-yl)ethoxy]ph enyl}-5,7-dihydro-6H-pyrrolo[3,4-d]pyrimidine-6-carboxamide | Ex. 15 | 1 H NMR (400 MHz, DMSO-d6) d ppm: 7.96 (s, 1 H), 7.73 (s, 1 H), 7.33 (d, J=1.77 Hz, 1 H), 7.29 (d, J=1.77 Hz, 1 H), 6.75 (s, 2 H), 6.35 (br. s., 1 H), 4.37 - 4.51 (m, 4 H), 4.25 - 4.36 (m, 2 H), 3.87 (dd, J=13.01, 2.15 Hz, 1 H), 3.44 - 3.52 (m, 1 H), 2.52 - 2.57 (m, 1 H), -0.50 - 0.58 (m, 2 H), 0.42 (d, J=3.28 Hz, 2 H). | 475.0, 477.0 |

(continued)

| Ex. No. | Structure | Name | Synthetic Method | $^1$H NMR | MS |
|---|---|---|---|---|---|
| 251 | 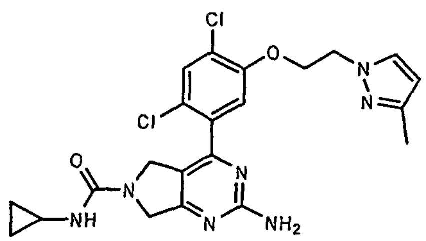 | 2-amino-N-cyclopropyl-4-{2,4-dichloro-5-[2-(3-methyl-1H-pyrazol-1-yl)ethoxy]phenyl)-5,7-dihydro-6H-pyrrolo[3,4-d] pyrimidine-6-carboxamide | Ex. 127 | 1 H-NMR (CDCl3, 300 MHz): d 7.46 (s, 2H), 6.72 (s, 1 H), 5.97 (s, 1 H), 5.19 (s, 2H), 4.52 (s, 2H), 4.51 (s, 1 H), 4.46 (t, 2H, J = 5 Hz), 4.38 (s, 2H), 4.31 (t, 2H, J = 5 Hz), 2.67 (bs, 1 H), 2.20 (s, 3H), 0.70 (q, 2H, J = 5 Hz), 0.51 (m, 2H). | 488.0, 490.0 |
| 252 | 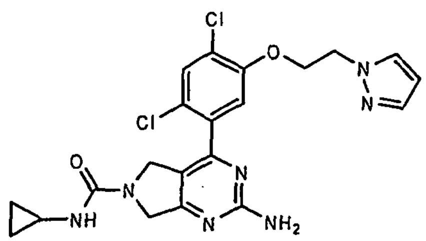 | 2-amino-N-cyclopropyl-4-{2,4-dichloro-5-[2-(1H-pyrazol-1-yl) ethoxy]phenyl}-5,7-dihydro-6H-pyrrolo[3,4-d]pyrimidine-6-carboxamide | Ex. 127 | 1 H-NMR (CDCl3, 300 MHz): d 7.60 (s, 1 H), 7.49 (s, 1 H), 7.44 (s, 1 H), 6.72 (s, 1 H), 6.24 (s, 1 H), 5.18 (s, 2H), 4.54 (m, 5H), 4.38 (s, 2H), 4.32 (t, .2H, J = 5 Hz), 2.66 (bs, 1 H), 0.73 (q, 2H, J = 6 Hz), 0.51 (m, 2H). | 474.0, 476.0 |
| 253 | 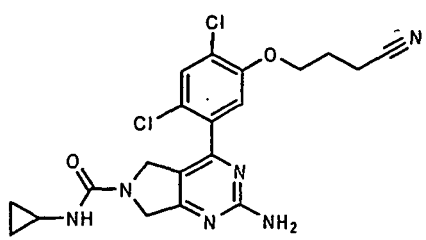 | 2-amino-N-cyclopropyl-4-[2,4-dichloro-5-(3-cyanopropoxy) phenyl]-5,7-dihydro-6H-pyrrolo [3,4-d]pyrimidine-6-carboxamide | Ex. 127 | 1H-NMR (CDCl3, 300 MHz): d 7.48 (s, 1 H), 6.87 (s, 1 H), 5.18 (s, 2H), 4.56 (s, 3H), 4.48 (s, 2H), 4.12 (t, 2H, J = 6 Hz), 2.68 (bs, 2H), 2.65 (t, 2H, J = 6 Hz), 2.19 (q, 2H, J = 6 Hz), 0.72 (m, 2H), 0.50 (m, 2H). | 446.0, 448.0 |

(continued)

| Ex. No. | Structure | Name | Synthetic Method | $^1$H NMR | MS |
|---|---|---|---|---|---|
| 254 | 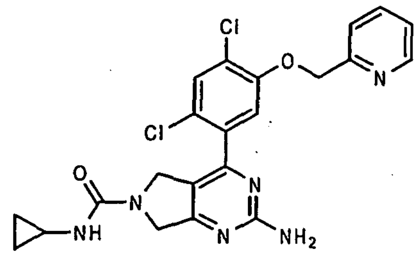 | 2-amino-N-cyclopropyl-4-[2,4-dichloro-5-(pyridin-2-ylmethoxy)p henyl]-5,7-dihydro-6H-pyrrolo [3,4-d]pyrimidine-6-carboxamide | Ex. 127 | 1H-NMR (CDCl3, 300 MHz): d 8.55 (d, 1 H, J = 6 Hz), 7.72 (t, 1 H, J = 6 Hz), 7.60 (d, 1 H, J = 6 Hz), 7.51 (s, 1 H), 7.25 (m, 1 H), 6.95 (s, 1 H), 5.24 (s, 2H), 5.14 (s, 2H), 4.55 (s, 2H), 4.49 (s, 1 H), 4.32 (s, 2H), 2.68 (bs, 1 H), 0.73 (q, 2H, J = 6 Hz), 0.50 (m, 2H). | 471.0, 473.0 |
| 255 | 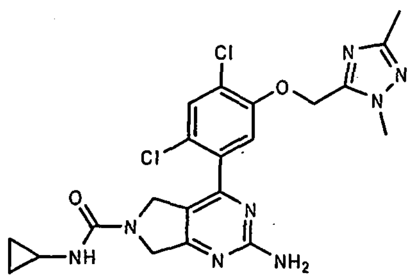 | 2-amino-N-cyclopropyl-4-{2,4-dichloro-5-[(1,3-dimethyl-1H-1,2,4-triazol-5-yl)methoxy]p henyl}-5,7-dihydro-6H-pyrrolo [3,4-d]pyrimidine-6-carboxamide | Ex 127 | 1 H-NMR (CDCl3, 300 MHz): d 7.49 (s, 1 H), 7.16 (s, 1 H), 5.23 (s, 2H), 5.20 (s, 2H), 4.57 {s, 1 H), 4.56 (s, 2H), 4.38 (s, 2H), 3.93 (s, 3H), 2.69 (bs, 1 H), 2.33 (s, 3H), 0.74 (q, 2H, J = 6 Hz), 0.51 (m, 2H). | 489.0, 491.0 |
| 256 | 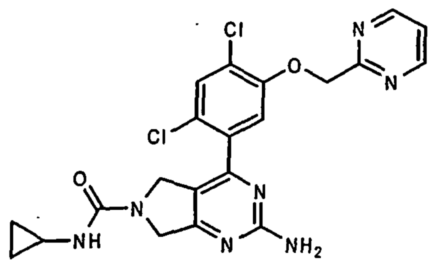 | 2-amino-N-cyclopropyl-4-[2,4-dichloro-5-(pyrimidin-2-ylmethoxy)p henyl]-5,7-dihydro-6H-pyrrolo[3,4-d]pyrimidine-6-carboxamide | Ex. 127 | 1H-NMR (CDCl3, 300 MHz): d 8.79 (d, 2H, J = 6 Hz), 7.51 (s, 1H), 7.27 (t, 1 H), 6.97 (s, 1H, J = 6 Hz), 5.37 (s, 2H), 5.17 (s, 2H), 4.52 (s, 2H), 5.41 (s, 1 H)., 4.32 (s, 2H), 2.68 (bs, 1 H), 0.73 (m, 2H), 0.50 (s, 2H). | 473.0, 475.0 |

(continued)

| Ex. No. | Structure | Name | Synthetic Method | $^1$H NMR | MS |
|---|---|---|---|---|---|
| 257 | 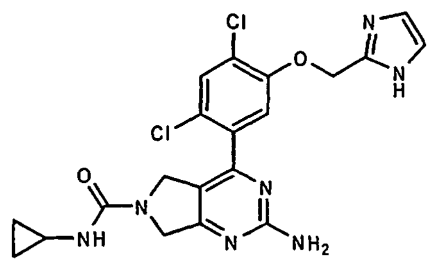 | 2-amino-N-cyclopropyl-4-[2,4-dichloro-5-(1H-imidazol-2-ylmethoxy)p henyl]-5,7-dihydro-6H-pyrrolo[3,4-d]pyrimidine-6-carboxamide | Ex. 127 | 1 H-NMR (DMSO-d6, 300 MHz): d 7.59 (s, 1 H), 7.46 (s, 1 H), 6.85 (bs, 2H), 6.77 (s, 1 H), 6.44 (s, 1H), 4.50 (d, 1 H, J = 3 Hz), 4.39 (m, 4H), 4.14 (s, 1 H), 2.06 (2H), 0.50 (m, 2H), 0.39 (m, 2H). | 460.0, 462.0 |
| 258 | 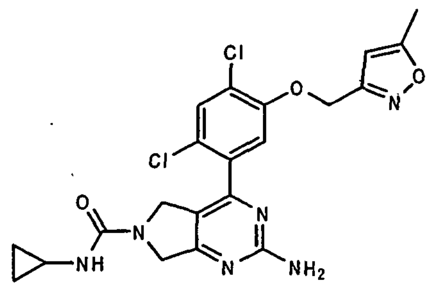 | 2-amino-N-cyclopropyl-4-{2,4-dichloro-5-[(5-methylisoxazol-3-yl)methoxy]phenyl}-5,7-dihydro-6H-pyrrolo[3,4-d]pyrimidine-6-carboxamide | Ex. 127 | 1H-NMR (CDCl3, 300 MHz): d 7.50 (s, 1 H), 7.01 (s, 1 H), 6.16 (s, 1 H), 5.21 (s, 2H), 5.16 (s, 2H), 4.66 (s, 1 H), 4.59 (s, 2H), 4.42 (s, 2H), 2.70 (bs, 1 H), 2.44 (s, 3H), 0.76 (m, 2H), 0.54 (m, 2H). | 477.0, 479.0 |
| 259 | 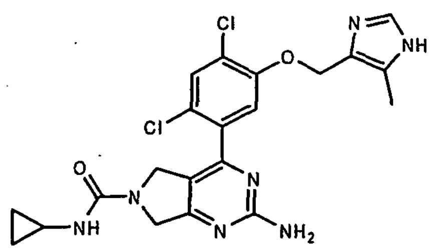 | 2-amino-N-cyclopropyl-4-{2,4-dichloro-5-[(5-methyl-1H-imidazol-4-yl)methoxy]phenyl}-5,7-dihydro-6H-pyrrolo[3,4-d] pyrimidine-6-carboxamide | Ex. | 1 H-NMR (DMSO-d6, 300 MHz): d 7.42 (s, 1H), 7.36 (s, 1 H), 6.77 (s, 1 H), 6.42 (s, 1 H), 4.68 (s, 2H), 4.30 (d, 2H, J = 3 Hz), 4.21 (s, 2H), 2.10 (bs, 2H) 2.06 (s, 3H) 0.51 (m, 2H), 0.39 (m, 2H). | 476.0, 478.0 |

(continued)

| Ex. No. | Structure | Name | Synthetic Method | $^1$H NMR | MS |
|---|---|---|---|---|---|
| 260 | 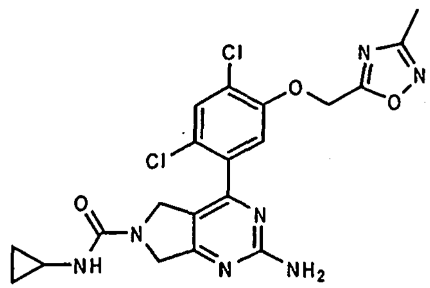 | 2-amino-N-cyclopropyl-4-{2,4-dichloro-5-[(3-methyl-1,2,4-oxadiazol-5-yl)methoxy]p henyl}-5,7-dihydro-6H-pyrrolo[3,4-d] pyrimidine-6-carboxamide | Ex. 127 | 1 H-NMR (CDCl3, 300 MHz): d 7.56 (s, 1 H), 7.01 (s, 1 H), 5.34 (s, 2H), 5.16 (s, 1 H), 4.59 (s, 2H), 4.42 (s, 2H), 2.65 (bs, 1H), 2.44 (s, 3H), 0.74 (m, 2H), 0.52 (m, 2H). | 475.0, 477.0 |
| 261 | 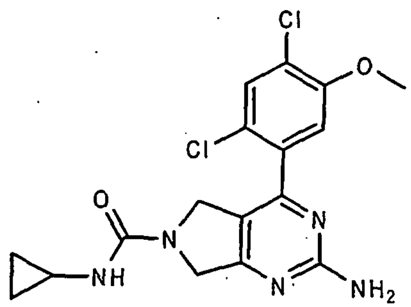 | 2-amino-N-cyclopropyl-4-(2,4-dichloro-5-methoxyphenyl)-5,7-dihydro-6H-pyrrolo[3,4-djpyrimidine-6-carboxamide | Ex. 14 | 1H NMR (400 MHz, DMSO-d6) d ppm: 7.74 (s, 1 H), 7.20 (s, 1 H), 6.89 (s, 2 H), 6.47 (d, J=2.78 Hz, 1 H), 4.41 (s, 2 H), 4.24 (s, 2 H), 3.87 (s, 3 H), 2.52 - 2.55 (m, 1 H), 0:50 - 0.57 (m, 2 H), 0.35 - 0.42 (m, 2 H). | 394.1, 396.1 |
| 262 | 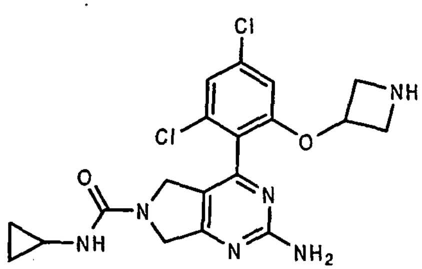 | 2-amino-4-[2-(azetidin-3-yloxy)-4,6-dichlorophenyl]-N-cyclopropyl-5,7-dihydro-6H-pyrrolo[3,4-d]pyrimidine-6-carboxamide | Ex. 129 | 1 H NMR (400 MHz, DMSO-d6) d ppm: 7.39 (d, J=1.77 Hz, 1 H), 7.01 (d, J=1.77 Hz, 1 H), 6.84 (s, 2 H), 6.45 (d, J=2.53 Hz, 1 H), 5.02 - 5.13 (m, 1 H), 4.42 (s, 2 H), 4.05- 4.19 (m, 2 H); 3.89 - 4.04 (m, 2 H), 3.45 - 3.55 cm, 2 H), 3.17 - 3.45 (m, 2 H), 0.47 - 0.58 (m, 2 H), 0.33 - 0.44 (m, 2 H). | 435.2, 437.2 |

(continued)

| Ex. No. | Structure | Name | Synthetic Method | $^1$H NMR | MS |
|---|---|---|---|---|---|
| 263 | 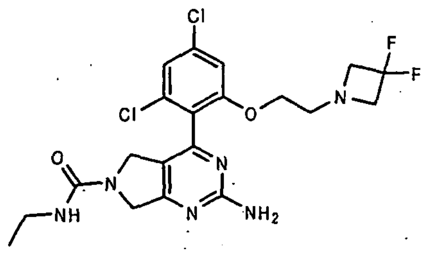 | 2-amino-4-(2,4-dichloro-6-[2-(3,3-difluoroazetidin-1-yl) ethoxy]ph enyl)-N-ethyl-5,7-dihydro-6H-pyrrolo[3,4-d] pyrimidine-6-carboxamide | Ex. 15 | 1 H NMR (400 MHz, DMSO-d6) d ppm: 7.34 (d, J=1.77 Hz, 1 H), 7.25 (d, J=1.77 Hz, 1 H), 6.85 (s, 2 H), 6.35 (t, J=5.31 Hz, 1 H), 4.30 - 4.47 (m, 2 H), 4.13 (s, 2 H), 4.00 - 4.08 (m, 2 H), 3.36 - 3.42 (m, 2 H), 3.21 - 3.29 (m, 2 H), 3.01 - 3.10 (m, 2 H), 2.70 - 2.77 (m, 2 H), 1.01 (t, J=7.20 Hz, 3 H). | 487.0, 489.0 |
| 264 | 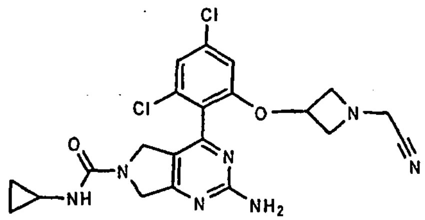 | 2-amino-N-cyclopropyl-4-(2,4-dichloro-6-{[1-(cyanomethyl) azetidin-3-yl]oxy}phenyl)-5,7-dihydro-6H-pyrrolo[3,4-d] pyrimidine-6-carboxamide | Ex. 130 | 1H NMR (400 MHz, CHLOROFORM-d) d ppm: 7.16 (d, J=1.77 Hz, 1 H), 6.59 (d, J=1.77 Hz, 1 H), 5.24 (s, 2 H), 4.75 - 4.84 (m, 1 H), 4.53 - 4.66 (m, 3 H), 4.33 - 4.43 (m, 2 H), 3.76 - 3.90 (m, 2 H), 3.51 (s, 2 H), 3.27 - 3.38 (m, 2 H), 2.64 - 2.75 (m, 1 H), 0.67 - 0.80 (m, 2 H), 0.46 - 0.56 (m, 2 H). | 474.0, 476.0 |
| 265 | 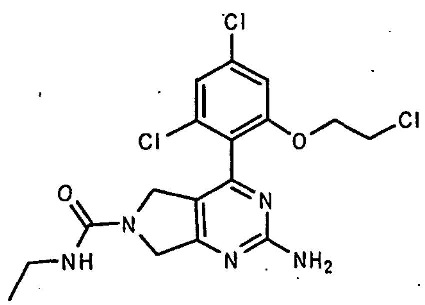 | 2-amino-4-[2,4-dichloro-6-(2-chloroethoxy )phenyl]-N-ethyl-5,7-dihydro-6H-pyrrolo[3,4-d] pyrimidine-6-carboxamide | Ex. 14 | 1 H NMR (400 MHz, CHLOROFORM-d) d ppm: 7.17 (d, J=1.77 Hz, 1 H), 6.89 (d, J=1.77 Hz, 1 H), 5.18 (s, 2 H), 4.61 (s, 2 H), 4.31 - 4.43 (m, 2 H), 4.18 - 4.26 (m, 3 H), 3.62 - 3:69 (m, 2 H), 3.27 - 3.37 (m, 2 H), 1.17 (t, J=7.20 Hz, 3 H). | 430.0, 432.0 |

(continued)

| Ex. No. | Structure | Name | Synthetic Method | $^1$H NMR | MS |
|---|---|---|---|---|---|
| 266 | 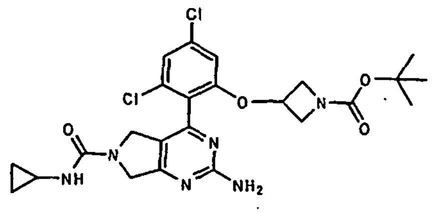 | tert-butyl 3-(2-{2-amino-6-[(cyclopropyl amino)carbonyl]-6,7-dihydro-5H-pyrrolo[3,4-d]pyrimidin-4-yl}-3,5-dichlorophenoxy)azetidine -1-carboxylate | Ex. 128 | 1 H NMR (400 MHz, CHLOROFORM-d) d ppm: 7.15 (d, J=1.26 Hz, 1 H), 6.52 (s, 1 H), 5.24 - 5.38 (m, 2 H), 4.78 - 4.90 (m, 1 H), 4.65 (br. s., 1 H), 4.56 (br. s., 2 H), 4.32 - 4.43 (m, 1 H), 4.19 - 4.32 (m, 3 H), 3.76 - 3.93 (m, 2 H), 2.80 - 2.75 (m, 1 H), 1.42 (s, 9 H), 0.74 (t, J=5.94 Hz, 2 H), 0.46 - 0.59 (m, 2 H). | 534.1, 536.1 |
| 267 | 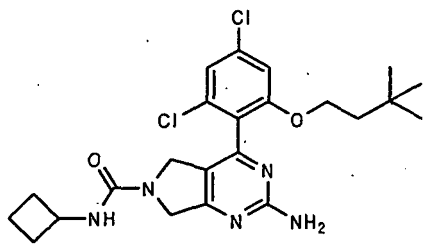 | 2-amino-N-cyclobutyl-4-[2,4-dichloro-6-(3,3-dimethylbutoxy) phenyl]-5,7-dihydro-6H-pyrrolo [3,4-d]pyrimidine-6-carboxamide | Ex. 127 | 1 H NMR (400 MHz, DMSO-d6) d ppm: 7.32 (s, 2 H), 6.80 (br. s., 2 H), 6.52 (d, J=7.83 Hz, 1 H), 4.39 (d, J=4.04 Hz, 2 H), 4.09 - 4.17 (m, 3 H), 4.00 - 4.08 (m, 2 H), 2.03 - 2.15 (m, 2 H), 1.84 - 1.99 (m, 2 H), 1.49 - 1.62 (m, 2 H), 1.45 (t, J=6.69 Hz, 2 H), 0.80 (s, 9 H). | 478.0, 480.0 |
| 268 | 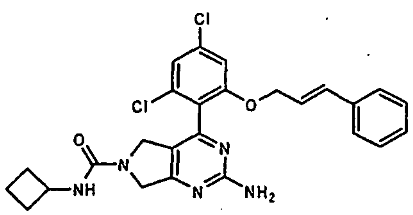 | 2-amino-N-cyclobutyl-4-(2,4-dichloro-6-{[(2E)-3-phenylprop-2-en-1-yl]oxy}phenyl)-5,7-dihydro-6H-pyrrolo[3,4-d] pyrimidine-6-carboxamide | Ex. 127 | 1 H NMR (400 MHz, DMSO-d6) d ppm: 7.22 - 7.42 (m, 7 H), 6.87 (s, 2 H), 6.46 - 6.57 (m, 2 H), 6.28 - 6.40 (m, 1 H), 4.81 (d, J=4.80 Hz, 2 H), 4.42 (s, 2 H), 4.17 (s, 2 H), 4.06 - 4.15 (m, 1 H), 2.02 - 2.14 (m, 2 H), 1.82 - 1.98 (m, 2 H), 1.46 - 1.60 (m, 2 H). | 510.0, 512.0 |

(continued)

| Ex. No. | Structure | Name | Synthetic Method | $^1$H NMR | MS |
|---|---|---|---|---|---|
| 269 | 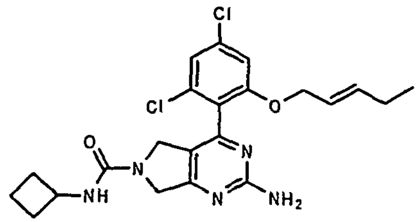 | 2-amino-N-cyclobutyl-4-{2,4-dichloro-6-[(2E)-pent-2-en-1-yloxy]phenyl}-5,7-dihydro-6H-pyrrolo[3,4-d]pyrimidine-6-carboxamide | Ex. 127 | 1H NMR (400 MHz, DMSO-d6) d ppm: 7.33 (d, J=1.77 Hz, 1 H), 7.28 (d, J=1.52 Hz, 1 H), 6.83 (br. s., 2 H), 6.48 - 6.55 (m, 1 H), 5.64 - 5.76 (m, 1 H), 5.42 - 5.53 (m, 1 H), 4.57 (d, J=5.56 Hz, 2 H), 4.40 (s, 2 H), 4.07 - 4.17 (m, 3 H), 2.04 - 2.13 (m, 2 H), 1.85 - 2.02 (m, 4 H), 1.46 - 1.59 (m, 2 H), 0.88 (t, J=7.45 Hz, 3 H). | 462.0, 464.0 |
| 270 | 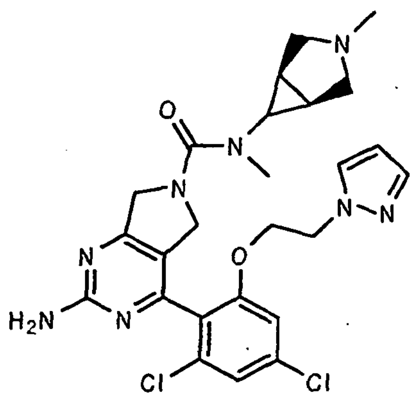 | 2-amino-4-{2,4-dichloro-6-[2-(1 H-pyrazol-1-yl)ethoxy]phenyl}-N-methyl-N-[(1R,5S)-3-methyl-3-azabicyclo[31.0]hex-6-yl]-5,7-dihydro-6H-pyrrolo[3,4-d] pyrimidine-6-carboxamide | Ex. 102 | 1 H NMR (400 MHz, DMSO-d6) d ppm 1.66 (s, 1H) 1.85 (s, 1 H) 2.19 (s, 3 H) 2.26 - 2.35 (m, 2 H) 2.85 - 2.91 (m, 1 H) 2.97 - 3.12 (m, 2 H) 3.56 - 3.65 (m, 2 H) 4.04 - 4.11 (m, 2 H) 4.28 - 4.42 (m, 4 H) 5.86 - 5.91 (m, 1 H) 7.17 (d, J=2.02 Hz, H) 7.19 (d, J=1.52 Hz, 1 H) 7.29 (s, 1 H) 7.35 (s, 1 H) | 543.2, 544.2 |

(continued)

| Ex. No. | Structure | Name | Synthetic Method | $^1$H NMR | MS |
|---|---|---|---|---|---|
| 271 | 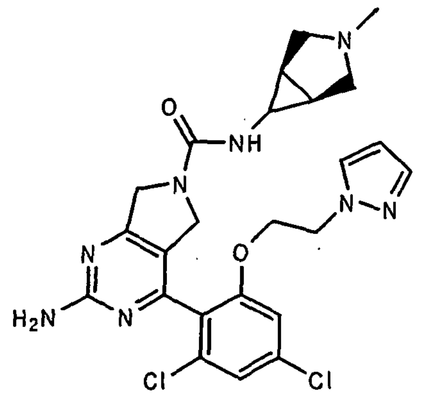 | 2-amino-4-{2,4-dichloro-6-[2-(1 H-pyrazol-1-yl)ethoxy] phenyl}-N-[(1R,5S)-3-methyl-3-azabicyclo[3.1.0]hex-6-yl]-5,7-dihydro-6H-pyrrolo[3,4-d] pyrimidine-6-carboxamide | Ex. 102 | 1 H NMR (400 MHz, DMSO-d6) d ppm 1.81 - 1.97 (m, 2 H) 2.54 (s, 3 H) 2.60 - 2.72 (m, J=1.52 Hz, 2 H) 3.03 (s, 2 H) 3.11 - 3.20 (m, 1 H) 3.60 - 3.70 (m, J=19.45 Hz, 1 H) 3.85 (d, J=12.63 Hz, 2 H) 4.29 - 4.33 (m, 4 H) 4.37 (d, J=7.58 Hz, 2 H) 5.94 - 6.02 (m, 1 H) 7.17 (d, J=1.77 Hz, 1 H) 7.23 (d, J=1.52 Hz, 1 H) 7.29 (d, J=1.52 Hz, 1 H) 7.30 (d, J=1.52 Hz, 1 H) | 464.0, 466.0 |
| 272 | 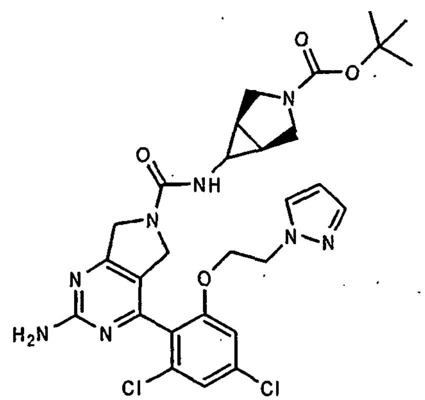 | tert-butyl (1R,5S)-6-{[(2-amino-4-{2,4-dichloro-6-[2-(1H-pyrazol-1-yl)ethoxy]ph enyl}-5,7-dihydro-6H-pyrrolo[3,4-d]pyrimidin-6-yl) carbonyl]amino}-3-azabicyclo [3.1.0]hexane-3-carboxylate | Ex. 102 | 1H NMR (400 MHz, DMSO-d6) d ppm 1.20 - 1.26 (m, 2 H) 1.54 - 1.67 (m, J=23.49 Hz, 2 H) 2.13 - 2.23 (m, J=2.02 Hz, 1 H) 2.82 - 2.94 (m, 2 H) 3.30 - 3.36 (m, 1 H) 3.45 (dd, J=10.74, 4.17 Hz, 2 H) 3.52 - 3.59 (m, 1 H) 3.84 - 3.96 (m, 1 H). 4.32 (s, 2 H) 4.38 (d, J=6.82 Hz; 1 H) 6.00 (t, J=1.89 Hz, 1 H) 6.42 (s, 1 H) 6.79 (s, 1 H) 7.22 (dd, J=26.78, 1.77 Hz, 1 H) 7.31 (dd, J=8.34, 1.52 Hz, 1 H) | 615.2, 617.2 |

(continued)

| Ex. No. | Structure | Name | Synthetic Method | $^1$H NMR | MS |
|---|---|---|---|---|---|
| 273 | 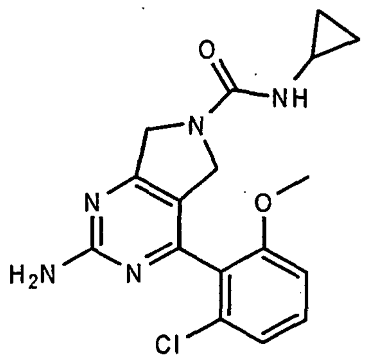 | 2-amino-4-(2-chloro-6-methoxyphenyl)-N-cyclopropyl-5,7-dihydro-6H-pyrrolo[3,4-d] pyrimidine-6-carboxamide | Ex. 18 | 1H NMR (400 MHz, DMSO-d6) d ppm 0.33 - 0.43 (m, 2 H) 0.47 - 0.60 (m, 2 H) 2.49 - 2.58 (m, 1 H) 3.73 (s, 3 H) 4.08 (s, 2 H) 4.40 (s, 2 H) 6.46 (d, J=2.78 Hz, 1 H) 6.78 (s, 2 H) 7.14 (dd, J=8.08, 5.56 Hz, 2 H) 7.44 (t, J=8.21 Hz, 1 H) | 360.2, 362.2 |
| 274 | 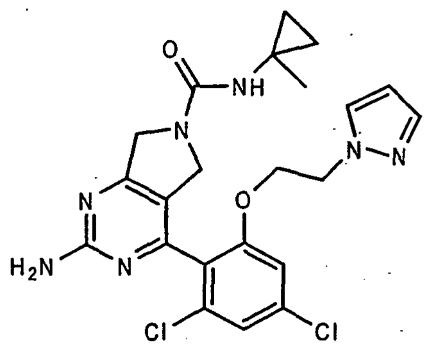 | 2-amino-4-{2,4-dichloro-6-[2-(1H-pyrazol-1-yl)ethoxy] phenyl}-N-(1-methylcyclopropyl)-5,7-dihydro-6H-pyrrolo[3,4-d] pyrimidine-6-carboxamide | Ex. 102 | 1H NMR (400 MHz, DMSO-d6) d ppm 0.44 - 0.48 (m, 2H) 0.55 - 0.68 (m, 2 H) 1.29 (s, 3H) 3.50 (d, J=13.39 Hz, 1H) 3.86 (dd, J=13.01, 1.89 Hz, 1 H) 4.26 - 4.34 (m, 4 H) 4.34 - 4.44 (m, 2H) 5.99 (t, J=2.02 Hz, 1H) 6.46 - 6.55 (m, 1 H) 6.77 (s, 2 H) 7.18 (d, J=2.02 Hz, 1 H) 7.25 (d, J=1.777 Hz, 1 H) 7.28 (d, J=1.26 Hz, 1 H) 7.32. (d, J=1.77 Hz, 1 H) | 488.2, 490.2 |

(continued)

| Ex. No. | Structure | Name | Synthetic Method | $^1$H NMR | MS |
|---|---|---|---|---|---|
| 275 | 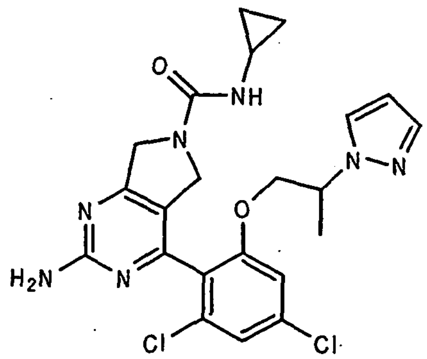 | 2-amino-N-cyclopropyl-4-{2,4-dichloro-6-[2-(1H-pyrazol-1-yl) propoxy]phenyl}-5,7-dihydro-6H-pyrrolo[3,4-d]pyrimidine-6-carboxamide | Ex.18 | 1H NMR (400 MHz, DMSO-d6) d ppm 0.35 - 0.45 (m, 2 H) 0.49 - 0.64 (m, 2 H) 1.34 (d, J=2.02 Hz, 3 H) 2.52 - 2.58 (m, 1 H) 3.35 - 3.40 (m, 1 H) 3.75 - 3.88 (m, 1 H) 4.14 - 4.44 (m, 4 H) 4.51 - 4.65 (m, 1 H) 6.01 (t, J=2.02 Hz, 1 H) 6.27 - 6.44 (m, 1 H) 6.74 - 6.79 (m, 2 H) 7.21 - 7.27 (m, 2 H) 7.29 - 7.36 (m, 2 H) | 488.1, 490.1 |
| 276 | 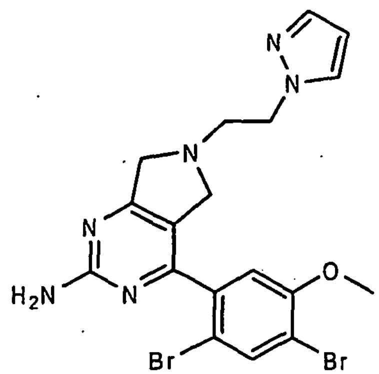 | 4-(2,4-dibromo-5-methoxyphenyl)-6-[2-(1H-pyraial-1-yl)ethyl]-6,7-dihydro-5H-pyrrolo[3,4-d]pyrimidin-2-amine | Ex. 105 | 1H NMR (400 MHz, DMSO-d6) d ppm 3.80 - 3.91 (m, J=3.79 Hz, 3 H) 4.25 (s, 1 H) 4.27 - 4.41 (m, 6 H) 4.45 (s, 1 H) 6.24 (d, J=2.02 Hz, 1 H) 6.94 (s, 2 H) 7.13 (d, J=2.78 Hz, 1 H) 7.45 (s, 1 H) 7.79 (d, J=1.77 Hz, 1 H) 7.86 (d, J=8.84 Hz, 1 H) | 494.0, 496.1 |
| 277 | 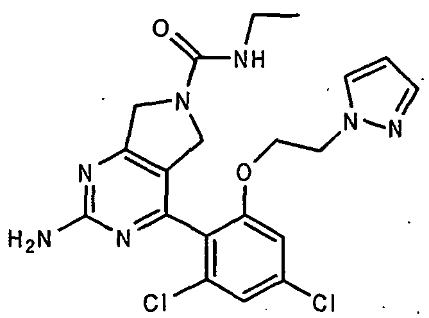 | 2-amino-4-{2,4-dichloro-6-[2-(1H-pyrazol-1-yl)ethoxy]phenyl}-N-ethyl-5,7-dihydro-6H-pyrrolo[3,4-d]pyrimidine-6-carboxamide | Ex.18 | 1H NMR (400 MHz, DMSO-d6) d ppm 1.02 (t, J=7.20 Hz, 3 H) 2.98 - 3.14 (m, 2 H) 3.59 (d, J=12.88 Hz, 1 H) 3.87 - 3.97 (m, 1 H) 4.33 (s, 4 H) 4.36 - 4.47 (m, 2 H) 6.00 (t, J=2.02 Hz, 1 H) 6.23 (t, J=4.80 Hz, 1 H) 6.78 (s, 2 H) 7.19 (d, J=1.77 Hz, 1 H) 7.25 (d, J=1.77 Hz, 1 H) 7.29 (d, J=1.26 Hz, 1 H) 7.32 (d, J=1.77 Hz, 1 H) | 462.1, 464.1 |

(continued)

| Ex. No. | Structure | Name | Synthetic Method | $^1$H NMR | MS |
|---|---|---|---|---|---|
| 278 | 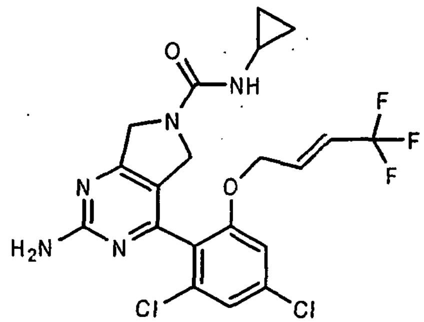 | 2-amino-N-cyclopropyl-4-(2,4-dichloro-6-{[(2E)-4,4,4-trifluorobut-2-en-1-yl] oxylphenyl)-5,7-dihydro-6H-pyrrolo[3,4-d]pyrimidine-6-carboxamide | Ex. 18 | 1 H NMR (400 MHz, DMSO-d6) d ppm 0.33 - 0.43 (m, 2 H) 0.47 - 0.59 (m, 2 H) 4.02 - 4.18 (m, 2 H) 4.39 (d, J=7.33 Hz, 2 H) 4.74 - 4.91 (m, 2 H) 5.61 - 5.74 (m, 1 H) 6.43 (d, J=2.53 Hz, 1 H) 6.57 - 6.67 (m, 1 H) 7.32 (d, J=1.77 Hz, 1 H) 7.40 (d, J=1.77 Hz, 1 H) | 488.0, 490.1 |
| 279 | 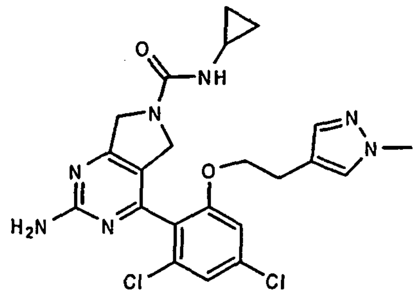 | 2-amino-N-cyclopropyl-4-{2,4-dichloro-6-[2-(1-methyl-1H-pyrazol-4-yl)ethoxy]ph enyl}-5,7-dihydro-6H-pyrrolo[3,4-d] pyrimidine-6-carboxamide | Ex. 18 | 1 H NMR (400 MHz, DMSO-d6) d ppm 0.34 - 0.44 (m, 2 H) 0.53 (dd, J=6.82, 2.27 Hz, 2 H) 2.53 - 2.56 (m, 1 H) 2.61 - 2.70 (m, 2 H) 3.70 (s, 3 H) 3.85 (d, J=13.14 Hz, 1 H) 3.96 - 4.05 (m, 1 H) 4.06 - 4.16 (m, 2 H) 4.35 - 4.50 (m, 2 H) 6.41 (d, J=2.02 Hz, 1 H) 6.85 (s, 2 H) 7.00 (s, 1 H) 7.17 (s, 1 H) 7.27 (d, J=1.77 Hz, 1 H) 7.31 (d. J=1.77 Hz, 1 H) | 488.1, 490.1 |
| 280 | 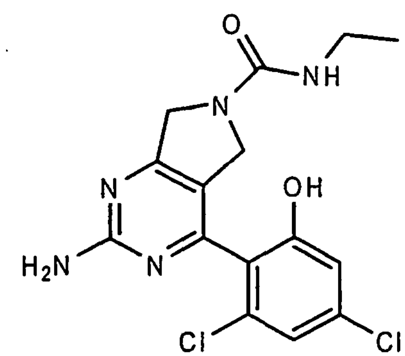 | 2-amino-4-(2,4-dichloro-6-hydroxyphenyl)-N-ethyl-5,7-dihydro-6H-pyrrolo[3,4-d] pyrimidine-6-carboxamide | Ex.18 | 1H NMR (400 MHz, DMSO-d6) d ppm 1.00 (t, J=7.20 Hz, 3 H) 2.97 - 3.13 (m, 2 H) 4.14 (s, 2 H) 4.38 - 4.48 (m, 2 H) 6.35 (t, J=5.43 Hz, 1 H) 6.76 - 6.83 (m, 2 H) 6.95 (d, J=1.77 Hz, 1 H) 7.14 (d, J=1.77 Hz, 1 H) 10:68 (s, 1 H) | 368.1, 370.0 |

(continued)

| Ex. No. | Structure | Name | Synthetic Method | $^1$H NMR | MS |
|---|---|---|---|---|---|
| 281 | 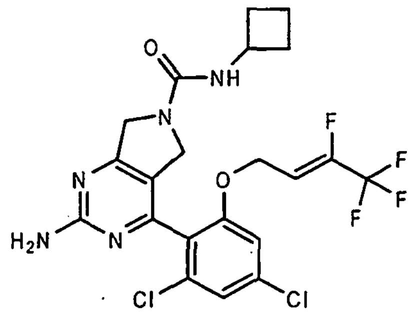 | 2-amino-N-cyclobutyl-4-(2,4-dichloro-6-{[(2Z)-3,4,4,4-tetrafluorobut-2-en-1-yl]oxy}phenyl)-5,7-dihydro-6H-pyrrolo[3,4-d]pyrimidine-6-carboxamide | Ex. 18 | 1H NMR (400 MHz, DMSO-d6) d ppm 1.46 - 1.65 (m, 2 H) 1.85 - 2.00 (m, 2 H) 2.02 - 2.14 (m, 2 H) 4.01 - 4.24 {m, 3 H) 4.35 - 4.44 (m, 2 H) 5.63 - 5.84 (m, 1 H) 6.49 (d, J=7.83 Hz, 1 H) 6.86 (d, J=2.53 Hz, 2 H) 7.42 - 7.49 (m, 1 H) 7.53 - 7.69 (m, 1 H) | 520.0, 522.0 |
| 282 | 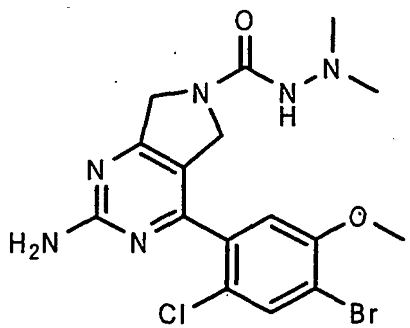 | 2-amino-4-(4-bromo-2-chloro-5-methoxyphenyl)-N',N'-dimethyl-5,7-dihydro-6H-pyrrolo[3,4-d]pyrimidine-6-carbohydrazide | Ex.102 | 1H NMR (400 MHz, DMSO-d6) d ppm 2.46 - 2.57 (m, 6 H) 3.86 (s, 3 H) 4.20 - 4.34 (m, 2 H) 4.37 - 4.57 (m, 1 H) 6.91 (s, 2 H) 7.16 , (s, 1 H) 7.56 (s, 1 H) 7.87 (s, 1 H) | 441.9, 442.9 |
| 283 | 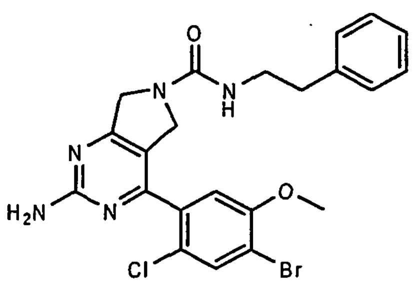 | 2-amino-4-(4-bromo-2-chloro-5-methoxyphenyl)-N-(2-phenylethyl)-5,7-dihydro-6H-pyrrolo[3,4-d]pyrimidine-6-carboxamide | Ex. 102 | 1 H NMR (400 MHz, DMSO-d6) d ppm 2.65 - 2.82 (m, 2 H) 3.16 - 3.30 (m, 2 H) 3.87 (s, 3 H) 4.27 (s, 2 H) 4.43 (s, 2 H) 6.53 (t, J=5.43 Hz, 1 H) 6.92 (s, 2 H) 7.10 - 7.22 (m, 4 H) 7.24 - 7.37 (m, 2 H) 7.88 (s, 2 H) | 502.9, 503.9 |

(continued)

| Ex. No. | Structure | Name | Synthetic Method | 1H NMR | MS |
|---|---|---|---|---|---|
| 284 | 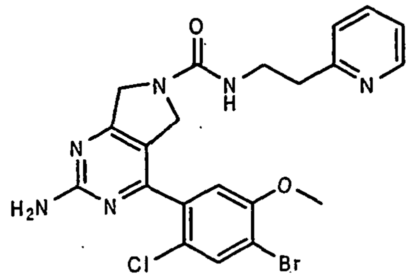 | 2-amino-4-(4-bromo-2-chloro-5-methoxyphenyl)-N-(2-pyridin-2-ylethyl)-5,7-dihydro-6H-pyrrolo [3,4-d]pyrimidine-6-carboxamide | Ex. 102 | 1H NMR (400 MHz, DMSO-d6) d ppm 2.81 - 2.93 (m, 2 H) 3.35 - 3.45 (m, 2 H) 4.19 - 4.29 (m, 2 H) 4.36 - 4:50 (m, 2 H) 6.54 (t, J=5.68 Hz, 1 H) 6.91 (s, 2 H) 7.12 - 7.27 (m, 3 H) 7.61 - 7.74 (m, 1 H) 7.87 (s, 1 H) 8.47 (d, J=1.01 Hz, 1 H) | 503.8, 504.9 |
| 285 | 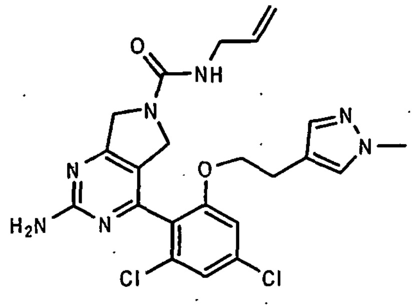 | N-allyl-2-amino-4-{2,4-dichloro-6-[2-(1-methyl-1H-pyrazol-4-yl) ethoxy]phenyl}-5,7-dihydro-6H-pyrrolo[3,4-d]pyrimidine-6-carboxamide | Ex. 18 | 1H NMR (400 MHz, MeOD) d ppm 2.77 (t, J=5.43 Hz, 2 H) 3.77 (s, 3 H) 3.79 (d, J=5.31 Hz, 2 H) 3.96 - 4.12 (m, 2 H) 4.14 - 4.25 (m, 2 H) 4.49 - 4.69 (m, 2 H) 5.06 (dd, J=10.11, 1.26 Hz, 1 H) 5.16 (dd, J=17.18, Hz, 1H) 5.81 - 5.96 (m, 1 H) 7.04 (s, 1 H) 7.12 - 7.17 (m, 2 H) 7.19 (d, J=1.77 Hz, 1 H) | 488.1, 489.1 1.52 |
| 286 | 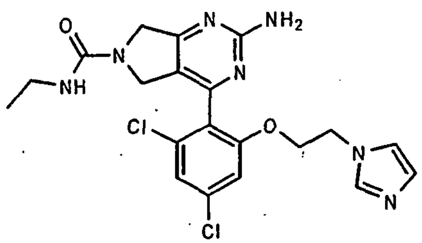 | 2-amino-4-{2,4-dichloro-6-[2-(1H-imidazol-1-yl)ethoxy] phenyl)-N-ethyl-5,7-dihydro-6H-pyrrolo[3,4-d]pyrimidine-6-carboxamide | Ex. 111 | 1H NMR (300 MHz, DMSO-d6) d ppm 1.04 (t, J = 7.1 Hz, 3H), 3.04-3.13 (m, 2H), 3.75 (d, J=13.2 Hz, 1H), 4.01 (d, J =13.2 Hz, 1H), 4.19-4.56 (m, 6H), 6.27 (brs, 1H), 6.71 (s, 1H), 6.75- 6.86 (m, 3H), 7.28 (s, 2H), 7.37 (s, 1H). | 462.2, 464.2 |

(continued)

| Ex. No. | Structure | Name | Synthetic Method | $^1$H NMR | MS |
|---|---|---|---|---|---|
| 287 | 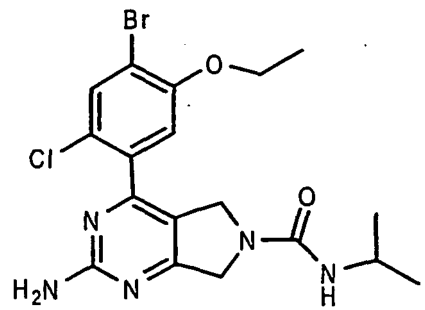 | 2-amino-4-(4-bromo-2-chloro-5-ethoxyphenyl)-N-isopropyl-5,7-dihydro-6H-pyrrolo[3,4-d] pyrimidine-6-carboxamide | Ex. 111 | 1H NMR (400 MHz, DMSO-d6) d ppm 1.06 (d, J = 6.5 Hz, 6H), 1.35 (t, J = 6.9 Hz, 3H), 3.75 (m, 1H), 4.09-4.17 (m, 2H), 4.25 (s, 2H), 4.42 (s, 2H), 6.07 (d, J = 8.6 Hz, 1 H), 6.88 (s, 2H), 7.15 (s, 1 H), 7.86 (s, 1 H). | 454.2, 456.2 |
| 288 | 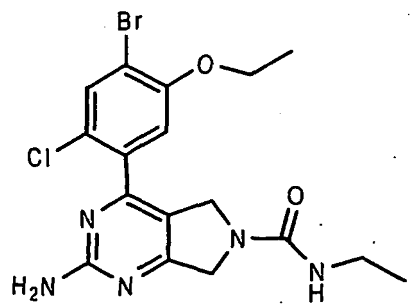 | 2-amino-4-(4-bromo-2-chloro-5-ethoxyphenyl)-N-ethyl-5,7-dihydro-6H-pyrrolo[3,4-d] pyrimidine-6-carboxamide | Ex. 111 | 1 H NMR (300 MHz, DMSO-d6) d ppm 0.98- 1.07 (m, 3H), 1.30-1.41 (m, 3H), 3.01-3.14 (m, 2H), 4.08-4.20 (m, 2H), 4.26 (s, 2H), 4.43 (s, 2H), 6.39 (br s, 1H), 6.92 (br s, 2H), 7.17 (s, 1H), 7.87 (s, 1H) | 440.2, 442.2 |
| 289 | 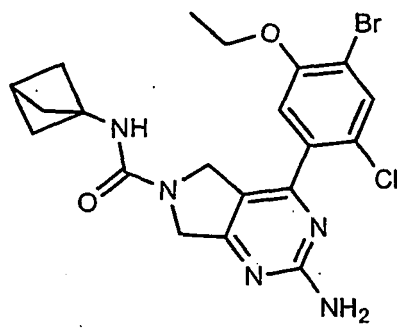 | 2-amino-N-bicyclo[1.1.1]pent-1-yl-4-(4-bromo-2-chloro-5-ethoxyphenyl)-5,7-dihydro-6H-pyrrolo[3,4-d]pyrimidine-6-carboxamide | Ex. 111 | 1 H NMR (300 MHz, DMSO-d6) d ppm 1.36 (t, J = 7.2 Hz, 3H), 1.95 (s, 6H), 2.36 (s, 1H), 4.10-4.18 (m, 2H, partially obscured), 4.25 (s, 2H), 4.40 (s, 2H), 6.90 (br s, 2H), 7.02 9br s, 1H), 7.16 (s, 1 H), 7.87 (s, 1 H) | 478.2, 480.2 |

(continued)

| Ex. No. | Structure | Name | Synthetic Method | $^1$H NMR | MS |
|---|---|---|---|---|---|
| 290 | 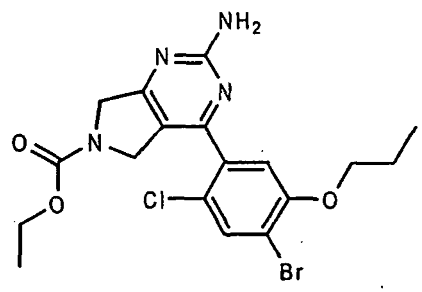 | ethyl 2-amino-4-(4-bromo-2-chloro-5-propoxyphen yl)-5,7-dihydro-6H-pyrrolo[3,4-d] pyrimidine-6-carboxylate | Ex. 108 | 1H NMR (300 MHz, DMSO-d6) d ppm 0.96-1.04 (m, 3H), 1.14-1.29 (m, 3H), 1.69-1.82 (m, 2H), 3.98-4.16 (m, 4H), 4.32-4.39 (m, 2H), 4.46-4.56 (m, 2H), 6.97 (br s, 2H), 7.15 (s, 1H), 7.86 (s, 1 H) | 455.0, 457.0 |
| 291 | 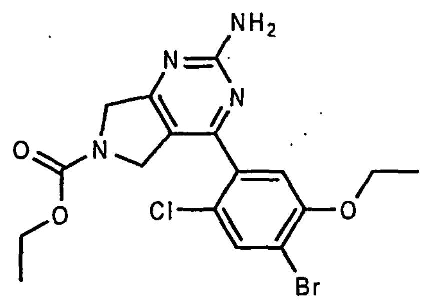 | ethyl 2-amino-4-(4-bromo-2-chloro-5-ethoxyphenyl )-5,7-dihydro-6H-pyrrolo[3,4-d] pyrimidine-6-carboxylate | Ex. 108 | 1 H NMR (300 MHz, DMSO-d6) d ppm 1.13- 1.26 (m, 3H), 1.31-1.40 (m, 3H), 4.04-4.17 9m, 4H), 4.31-4.38 (m, 2H), 4.47-4.54 (m, 2H), 6.96 (br s, 2H), 7.15 | 441.0, 443.0 |
| 292 | 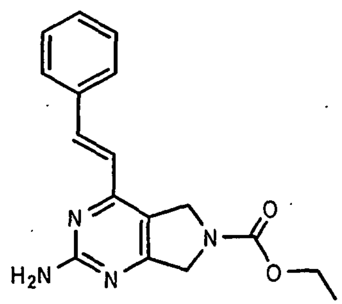 | ethyl 2-amino-4-[(E)-2-phenylvinyl]-5,7-dihydro-6H-pyrrolo[3,4-d]pyrimidine-6-carboxylate | Ex. 1 | 1H NMR (300 MHz, DMSO-d6) d ppm 1.22- 1.30 (m, 3H), 4.09-4.16 (m, 2H), 4.42 (d, J = 7.6 Hz, 2H), 4.67 (d, J = 7.5 Hz), 6.62 (s, 2H), 7.11 (dd, J = 6.9, 15.8 Hz, 1H), 7.36-7.48 (m, 3H), 7.71-7.79 (m, 3H). | 311.4 |

### HSP-90 Biochemical Assay

Compounds of the present invention were evaluated for potency against HSP-90 using a SPA (scintillation proximity assay) competition binding assay. Briefly, either full length or N-terminal HSP-90 that contains a 6-His tag on its C-terminus binds to copper on Yttrium-silicate scintillant beads via the His-tag. Tritiated propyl-Geldanamycin (pGA), whose structure is shown below, is an analog of a natural inhibitor of HSP-90 called Geldanamycin. Tritiated pGA, which contains a tritiated propyl-amine group added at the #17 position, binds HSP-90 and brings the isotope into proximity with the beads. 17-n-propylamino-Geldanamycin can be prepared as described in U.S. Patent No. 4,261,989, which is incorporated herein by reference. A second tritiated compound that can also be used in this assay is shown below and is designated as Compound A.

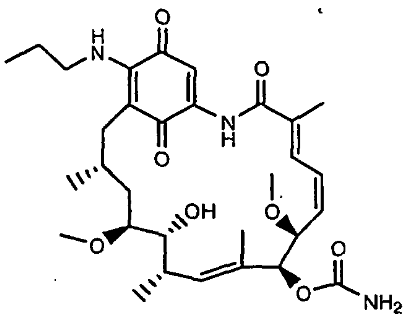

pGA

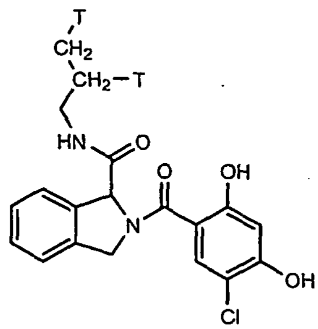

Compound A

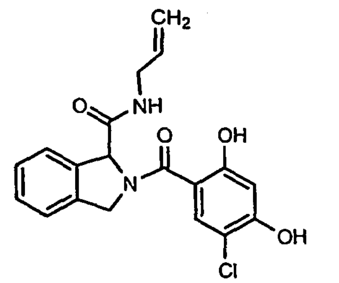

parent compound of compound A

The "T" in the structure of Compound A above indicates the position of the labeled tritiated hydrogen atoms. This compound has a $K_d$ of 40 nM and can be prepared as follows. Compound A can be prepare from the parent compound of Compound A, (N-allyl-2-(5-chloro-2,4-dihydroxybenzoyl)isoindoline-1-carboxamide) as described in the following. Allylamine (2.5 mL, 5 mmol, 2M in THF) was added to a solution of Boc(R,S)-1,3-dihydro-2H-isoindole carboxylic acid (263 mg, 1 mmole), diisopropylethyl amine (0.9 mL, 5 mmol), and O-(7-azabenzotriazol-1-yl)-N,N,N',N'-tetramethyluronium phosphorus pentafloride (HATU) (420 mg, 1.1 mmol) in 5 mL of DMF under a nitrogen atmosphere. The reaction was allowed to stir at room temperature for 12 hours. Saturated $NaHCO_3$ (30 mL) was added to the reaction mixture to quench the reaction. EtOAc (2x50 mL) was then added to extract the aqueous solution. Dry EtOAc layer over $Na_2SO_4$. The $Na_2SO_4$ was filtered off and the filtrate was evaporated to give a brown oil residue. The residue was purified by silica gel chromatography (gradient elution 40→ 50% EtOAc in hexanes) to give the desired intermediate product (321 mg, qunatitative yield) tert-butyl 1-[(allylamino)carbonyl]-1,3-dihydro-2*H*-isoindole-2-carboxylate.

Hydrogen chloride (3 mL, 12 mmol; 4 M in dioxane) was added to a solution of tert-butyl 1-[(allylamino)carbonyl]-1,3-dihydro-2H-isoindole-2-carboxylate (1 mmol) in DCM (5 mL) at room temperature. The reaction was heated and stirred at room temperature for 12 hours. The reaction mixture was evaporated to give an oil residue. The residue (N-allylisoindoline-1-carboxamide) was used for the next step reaction without further purification.

N-allylisoindoline-1-carboxamide (1 mmol) was then added to a solution of 5-chloro-2,4-bis(methoxymethoxy) benzoic acid (which can be prepared as shown in WO 2006/117669) (340 mg, 1.2 mmol), 4-methylmorpholine (2.2 mL, 20 mmol), N-(3-dimethylaminopropyl)-N'-ethylcarbodiimide hydrochloride (460 mg, 2.4 mmol), and 1-hydroxy benzotriazole (330 mg, 2.4 mmol) in 12 mL of DMF under a nitrogen atmosphere. The reaction was allowed to stir at room temperature for 12 hours. $H_2O$ (50 mL) was added to the reaction mixture to quench the reaction. EtOAc (2 x 100 mL) was then added to extract the aqueous solution. Dry EtOAc layer over $Na_2SO_4$. The $Na_2SO_4$ was filtered off and the filtrate was evaporated to give a brown oil residue. The residue was purified by silica gel chromatography (gradient elution 50→ 60% EtOAc in hexanes) to give the desired intermediate product (423 mg, 91.8% yield) N-allyl-2-[5-chloro-2,4-bis(methoxymethoxy)benzoyl]isoindoline-1-carboxamide.

Hydrogen chloride (4 mL, 16 mmol; 4 M in dioxane) was added to a solution of N-allyl-2-[5-chloro-2,4-bis (methoxymethoxy)benzoyl]isoindoline-1-carboxamide (392 mg, 0.85 mmol) in DCM (5 mL). The reaction was stirred at room temperature for 12 hours. The reaction mixture was neutralized with saturated $NaHCO_3$ (aq) and then extracted with EtOAc (2 x 50 mL). The combined organic layers were dried, filtered, and evaporated to give the desired final product as the parent compound (N-allyl-2-(5-chloro-2,4-dihydroxybenzoyl)isoindoline-1-carboxamide) as a white solid (221 mg, 69.7% yield). $^1H$ NMR (400 MHz, DMSO-D6) δ ppm 3.57 (d, J=79.33 Hz, 2 H) 4.65 - 4.93 (m, 1 H) 4.97 - 5.19 (m, 1 H) 5.42 - 5.70 (m, 1 H) 5.68 - 5.95 (m, 1 H) 6.40 - 6.71 (m, 1 H) 6.92 (s, 1 H) 7.15 - 7.67 (m, 4 H) 8.28 (s, 1 H) 10.06 (s, 1 H) 10.40 (s, 1 H). Anal. Calcd for $C_{19}H_{17}ClN_2O_4$: C, 61.21; H, 4.60; N, 7.51. Found: C, 61.02; H, 4.63; N, 7.36.

Once the parent compound was made, Compound A was prepared using standard hydrogenation methods

using tritium gas.

The beta signal emitted from the isotope excites the scintillant, which creates a. measurable signal. As competitive compounds are added to the assay mixture, they compete with bound tritiated pGA or Compound A at the ATP-binding site on the N-terminal of HSP-90. When a compound displaces the labeled pGA or Compond A, the signal is reduced (the beta-particles are no longer in proximity with the bead). This reduction in signal is used to quantify the extent to which the inhibitor/compound is competitive with pGA or Compound A.

The SPA assay for $^3$H-pGA (designated G1) and Compound A (designated G2) binding to HSP-90 was performed in 96-well flat bottom white plates (Corning #3604). For G1, typical reaction solutions contained 30 nM HSP-90 and 200 nM $^3$H-pGA in binding buffer (100 mM Hepes, pH 7.5 and 150 mM KCl). For G2, typical reaction solutions contained 5 nM HSP-90 and 50 nM of Compound A. For G1, the $^3$H-pGA was first diluted to 33% label with unlabeled pGA that was synthesized and purified to give a final concentration of 200 nM. For G2, labeled Compound A was diluted with unlabeled Compound A to provide a ratio of labeled:unlabeled of 1:2 for a final concentration of 50 nM. Inhibitors were added to the HSP-90/$^3$H-pGA (or HSP-90/Compound A) solutions at eleven different concentrations for $K_i$ determinations. The range of inhibitor concentrations were 100 μM, or an appropriate range, for solid samples and 10 μM for targeted library compounds and 4 mM liquid stocks. To determine percent inhibition, the compound was tested at 1 and 10 μM. The final DMSO in the samples was 4%. Copper-Ysi beads (Amersham, #RPNQ0096) that have been diluted in binding buffer were added to each well to give a final concentration of 100 μg/well. The plates were sealed, covered with a foil-covered lid and shaken for 30 minutes at room temperature. The beads were allowed to settle for 30 minutes after which the plates were counted using a Packard TopCount NXT instrument. This procedure has also been adapted for medium throughput using a Beckman Biomek FX. Samples were run in duplicate and on two separate days to assure an accurate value of $K_i$.

For $K_i$ determinations, the corrected cpm's (actual cpm's minus background) were plotted vs. inhibitor concentration using GraphPad Prism software. The data were fit to a generic $IC_{50}$ equation, Y = YI / (1 + [X]/$IC_{50}$), where YI = Y-intercept and [X] is the competing ligand/inhibitor. The $IC_{50}$ was the used to calculate the Ki by using the Cheng-Prusoff equation:

$$Ki\{cl\} = \frac{IC_{50}\{cl\}}{1 + ([hl]/Kd\{hl\})}$$

Where cl = cold ligand concentration (varies), [hl] = concentration of hot ligand (200 nM or 50 nM) and Kd{hl} = 240 nM (for $^3$H-pGA) or 40 nM (for Compound A). Error was calculated as follows: $IC_{50}$ error / $IC_{50}$ value = fractional error and fractional error * $K_i$ value = $K_i$ error.

In the cases in which inhibitor binds to HSP-90 so tightly that the population of free inhibitor molecules is significantly depleted by formation of the enzyme-inhibitor complex, the above equation is no longer valid. This is normally true when the observed $IC_{50}$ is about the same as the HSP-90 concentration. For a tight binding inhibitor, the following equation can be applied:

$$\frac{EL}{EL_o} = \frac{-(K_I^{app} + I_o - E_o) + \sqrt{(K_I^{app} + I_o - E_o)^2 + 4 \times E_o \times K_I^{app}}}{2 \times E_o}$$

Where

$$K_I^{app} = K_I \times (1 + \frac{L_o}{K_L})$$

EL and $EL_o$ are the radioligand-HSP-90 complexes in the presence and absence of inhibitor, respectively. EL/$EL_o$ represents the fractional signal in the presence of inhibitor. Io, $E_o$, and $L_o$ are the inhibitor, HSP-90, and radioligand concentrations, respectively. $K_i$ is the inhibition constant for the ligand, while $K_L$ is the binding affinity constant between the enzyme (HSP-90) and the ligand.

The Ki assay data of the compound of Examples 1-292 are listed in the following Table 3.

**Table 3**

| Example No. | (G1):Ki (μM) | (G2):Ki (μM) |
|---|---|---|
| 1 | 0.54 | 0.112 |
| 2 | 2.77 | |
| 3 | 0.052 | |
| 4 | 1.7 | 0.475 |
| 5 | 0.244 | |
| 6 | 4.6 | |
| 7 | 0.952 | 0.12 |
| 8 | 0.369 | |
| 9 | 4 | |
| 10 | | 19.2 |
| 11 | | 2.4 |
| 12 | | 16 |
| 13 | 0.388 | |
| 14 | 1.74 | |
| 15 | 0.57 | |
| 17 | | 17.5 |
| 18 | 0.008 | |
| 20 | | 27 |
| 22 | | 21.5 |
| 23 | | 0% inhibition at 10μM |
| 24 | | 38 |
| 25 | | 0% inhibition at 10μM |
| 26 | 44 | |
| 27 | | 0% inhibition at 10μM |
| 29 | | 0.304 |
| 30 | | 0.56 |
| 31 | | 0.36 |
| 32 | 1.9 | 0.499 |
| 33 | 1.3 | 0.268 |
| 34 | 2.6 | 0.74 |
| 355 | 1.5 | 0.437 |
| 36 | 7.3 | 2.86 |
| 37 | 4 | 1.54 |
| 38 | 2.5 | 0.822 |
| 39 | 1.66 | 0.300 |
| 40 | 4 | 0.831 |
| 41 | 2.5 | 0.56 |

(continued)

| Example No. | (G1):Ki (μM) | (G2):Ki (μM) |
|---|---|---|
| 42 | 0.56 | 0.082 |
| 43 | 0.47 | 0.105 |
| 44 | 0.72 | 0.17 |
| 45 | 0.78 | 0.167 |
| 46 | | 0% inhibition |
| | | at 10μM |
| 47 | | 32 |
| 48 | | 20 |
| 49 | | 3.4 |
| 50 | | 11.4 |
| 51 | 2.71 | |
| 52 | 0.626 | |
| 53 | 0.289 | |
| 54 | 0.4 | |
| 55 | 0.143 | |
| 56 | 0.104 | |
| 57 | 0.304 | |
| 58 | 0.648 | |
| 59 | 9.78 | |
| 60 | 0.343 | |
| 61 | 0.154 | |
| 62 | 0.058 | |
| 63 | 3.32 | |
| 64 | 0.134 | |
| 65 | 0.409 | |
| 66 | 0.3 | |
| 67 | 0.169 | |
| 68 | 0.249 | |
| 69 | 0.089 | |
| 70 | 0.209 | |
| 71 | 0.209 | |
| 72 | 0.535 | |
| 73 | 0.314 | |
| 74 | 0.631 | |
| 75 | 1.59 | |
| 76 | 0.431 | |
| 77 | 0.155 | |
| 78 | 0.157 | |

(continued)

| Example No. | (G1):Ki (μM) | (G2):Ki (μM) |
|---|---|---|
| 79 | 0.111 | |
| 80 | 0.081 | |
| 81 | 36 | |
| 82 | 2.4 | |
| 83 | 2.95 | |
| 84 | 4.34 | |
| 85 | 24 | |
| 86 | 1.65 | |
| 87 | 1.95 | |
| 88 | 3.78 | |
| 89 | 2.38 | |
| 90 | | 1.93 |
| 91 | | 0.745 |
| 92 | 93 0.349 | 0.619 |
| 94 | 0.82 | 0.216 |
| 95 | 12 | 3.71 |
| 96 | 1.66 | 0.3 |
| 97 | | 1.5 |
| 98 | 1.5 | 0.245 |
| 100 | | 0.014 |
| 101 | | 0.115 |
| 101a | | 0.149 |
| 102 | | 0.00918 |
| 102b | | 12 |
| 103 | | 0.0265 |
| 104 | | 0.765 |
| 105 | | 1.22 |
| 106 | | 0.319 |
| 107 | | 0.99 |
| 108 | | 0.0202 |
| 108a | | 1.9 |
| 109 | | 0.00825 |
| 110 | | 0.0101 |
| 111 | | 0.064 |
| 112 | | 0.17 |
| 113 | | 0.0245 |
| 114 | | 0.0165 |
| 115 | | 0.0665 |

(continued)

| Example No. | (G1):Ki (μM) | (G2):Ki (μM) |
|---|---|---|
| 116 | | 0.0274 |
| 117 | | 0.547 |
| 118 | | 0.19 |
| 119 | | 8.77 |
| 120 | | 0.324 |
| 121 | | 0.354 |
| 122 | | 1.05 |
| 123 | | 36 |
| 124 | | 0.844 |
| 125 | | 9.38 |
| 126 | | 2.35 |
| 127 | | 0.0688 |
| 127a | | 0.0628 |
| 127b | | 0.0839 |
| 128 | | 0.648 |
| 129 | | 0.449 |
| 130 | | 0.154 |
| 131 | | 0.775 |
| 132 | | 0.268 |
| 133 | | 0.273 |
| 134 | | 1.63 |
| 135 | | 1.14 |
| 136 | | 0.267 |
| 137 | | 0.332 |
| 138 | | 0.132 |
| 139 | | 0.0128 |
| 140 | | 0.132 |
| 141 | | 0.154 |
| 142 | | 0.303 |
| 143 | | 1.3 |
| 144 | | 0.473 |
| 145 | | 0.434 |
| 146 | | 0.00792 |
| 147 | | 0.0517 |
| 148 | | 2.26 |
| 149 | | 0.329 |
| 150 | | 0.0528 |
| 151 | | 0.463 |

(continued)

| Example No. | (G1):Ki (μM) | (G2):Ki (μM) |
|---|---|---|
| 152 | | 0.332 |
| 153 | | 0.811 |
| 154 | | 0.029 |
| 155 | | 0.546 |
| 156 | | 0.0267 |
| 157 | | 1.2 |
| 158 | | 4.72 |
| 159 | | 0.062 |
| 160 | | 0.685 |
| 161 | | 0.592 |
| 162 | | 0.03 |
| 163 | | 0.104 |
| 164 | | 0.00838 |
| 165 | | 0.392 |
| 166 | | 2.99 |
| 167 | | 2.81 |
| 168 | | 0.067 |
| 169 | | 0.14 |
| 170 | | 1.3 |
| 171 | | 0.247 |
| 172 | | 0.0162 |
| 173 | | 1.02 |
| 174 | | 17.1 |
| 175 | | 0.378 |
| 176 | | 0.319 |
| 177 | | 0.235 |
| 178 | | 2.14 |
| 179 | | 1.15 |
| 180 | | 2.28 |
| 181 | | 0.735 |
| 182 | | 0.24 |
| 183 | | 0.504 |
| 184 | | 0.658 |
| 185 | | 2.88 |
| 186 | | 0.802 |
| 187 | | 0.577 |
| 188 | | 1.77 |
| 189 | | 0.0116 |

(continued)

| Example No. | (G1):Ki (μM) | (G2):Ki (μM) |
|---|---|---|
| 190 | | 0.0201 |
| 191 | | 0.396 |
| 192 | | 0.014 |
| 193 | | 0.0296 |
| 194 | | 0.0466 |
| 195 | | 0.0467 |
| 196 | | 0.0355 |
| 197 | | 0.0139 |
| 198 | | 0.0091 |
| 199 | | 0.0427 |
| 200 | | 0.0144 |
| 201 | | 0.0117 |
| 202 | | 0.0631 |
| 203 | | 0.02 |
| 204 | | 0.00862 |
| 205 | | 0.0487 |
| 206 | | 0.0353 |
| 207 | | 0.07 |
| 208 | | 0.0379 |
| 209 | | 0.0214 |
| 210 | | 0.306 |
| 211 | | 0.00665 |
| 212 | | 0.224 |
| 213 | | 0.03 |
| 214 | | 0.255 |
| 215 | | 1.05 |
| 216 | | 0.185 |
| 217 | | 0.0135 |
| 218 | | 0.00593 |
| 219 | | 0.0676 |
| 220 | | 0.121 |
| 221 | | 0.243 |
| 222 | | 0.0568 |
| 223 | | 0.0073 |
| 224 | | 0.0549 |
| 225 | | 0.0794 |
| 226 | | 0.105 |
| 227 | | 0.48 |

(continued)

| Example No. | (G1):Ki (μM) | (G2):Ki (μM) |
|---|---|---|
| 228 | | 0.179 |
| 229 | | 1.25 |
| 230 | | 0.225 |
| 231 | | 0.069 |
| 232 | | 3.18 |
| 233 | | 2.26 |
| 234 | | 0.184 |
| 235 | | 0.0318 |
| 236 | | 0.21 |
| 237 | | 0.205 |
| 238 | | 0.0455 |
| 239 | | 0.699 |
| 240 | | 0.13 |
| 241 | | 0.472 |
| 242 | | 1.08 |
| 243 | | 3.82 |
| 244 | | 39 |
| 245 | | 14.9 |
| 246 | | 0.00853 |
| 247 | | 0.00661 |
| 248 | | 0.016 |
| 249 | | 0.039 |
| 250 | | 0.0092 |
| 251 | | 0.144 |
| 252 | | 0.104 |
| 253 | | 0.0255 |
| 254 | | 0.145 |
| 255 | | 0.275 |
| 256 | | 0.164 |
| 257 | | 6.29 |
| 258 | | 0.114 |
| 259 | | 5.85 |
| 260 | | 0.213 |
| 261 | | 0.0831 |
| 262 | | 0.267 |
| 263 | | 0.0355 |
| 264 | | 0.195 |
| 265 | | 0.098 |

(continued)

| Example No. | (G1):Ki (μM) | (G2):Ki (μM) |
|---|---|---|
| 266 | | 0.504 |
| 267 | | 0.0642 |
| 268 | | 0.46 |
| 269 | | 0.0911 |
| 270 | | 0.00352 |
| 271 | | 0.0148 |
| 272 | | 0.0649 |
| 273 | | 1.32 |
| 274 | | 0.0149 |
| 275 | | 0.0141 |
| 276 | | 0.775 |
| 277 | | 0.0125 |
| 278 | | 0.0352 |
| 279 | | 0.0108 |
| 280 | | 0.565 |
| 281 | | 0.098 |
| 282 | | 0.22 |
| 283 | | 0.53 |
| 284 | | 0.635 |
| 285 | | 0.023 |
| 286 | | 0.00693 |
| 287 | | 0.155 |
| 288 | | 0.0814 |
| 289 | | 0.0557 |
| 290 | | 0.807 |
| 291 | | 0.224 |
| 292 | | 2.51 |

## Claims

1. A compound of formula (I)

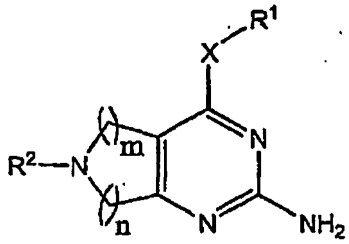

(I)

wherein:

m is 1 or 2, n is 1 or 2, when m is 2, n is 1;

X is a bond or a diradical selected from the group consisting of -O-, -S-, -($C_1$-$C_3$ alkylene)-, -O-($C_1$-$C_3$ alkylene)-, -NH-($C_1$-$C_3$ alkylene)-, -S-($C_1$-$C_3$ alkylene)-, -C(O)-, - C(O)-O-, -C(O)-NH-, -OC(O)-NH-, -NH-C(O)-NH-, -S(O)-, -$S(O)_2$-, -$S(O)_2$-O- and -$S(O)_2$-NH-, wherein each end of the diradical may be connected to $R^1$ or to the aminopyrimidine ring of formula I;

where permissible, each nitrogen or carbon atom of X is optionally further substituted by one group selected from -($C_1$-$C_3$ alkylene)$_t$-CN, -($C_1$-$C_3$ alkylene)$_t$-F, -($C_1$-$C_3$ alkylene)$_t$-($C_1$-$C_3$ perfluoroalkyl), -($C_1$-$C_3$ alkylene)$_t$-O-($C_1$-$C_6$ alkyl), -($C_1$-$C_3$ alkylene)$_t$-OH, -($C_1$-$C_3$ alkylene)$_t$-$NH_2$, -($C_1$-$C_3$ alkylene)$_t$-NH($C_1$-$C_3$ alkyl) and -($C_1$-$C_3$ alkylene)$_t$-N($C_1$-$C_3$ alkyl)($C_1$-$C_3$ alkyl), and t is 0 or 1;

$R^1$ is selected from the group consisting of $C_6$-$C_{12}$ aryl, 5 to 12 member heteroaryl, $C_3$-$C_{12}$ cycloalkyl, 3-12 member heterocyclyl and $C_5$-$C_{12}$ unsaturated nonaromatic carbacyclyl, and each $R^1$ is optionally further substituted with 1-5 R, provided that when $R^1$ is phenyl, then $R^1$ is further substituted with at least two R and at least one of the R is not a halogen;

R is selected from the group consisting of $R^x$, -($C_1$-$C_6$ alkylene)$_p$-O($C_1$-$C_6$ alkylene)$_p$-($C_6$-$C_{10}$ aryl), -($C_1$-$C_6$ alkylene)$_p$-O-($C_1$-$C_6$ alkylene)$_p$-($C_7$-$C_{10}$ cycloalkyl), -($C_1$-$C_6$ alkylene)$_p$-O-($C_1$-$C_6$ alkylene)$_p$-(7-10 member heteroaryl), -($C_1$-$C_6$ alkylene)$_p$-O-($C_1$-$C_6$ alkylene)$_p$-(7-10 member heterocyclyl), -($C_1$-$C_6$ alkylene)p-O-($C_2$-$C_6$ alkenyl), -($C_1$-$C_6$ alkylene)p-O-($C_2$-$C_6$ alkenylene)$_p$-($C_6$-$C_{10}$ aryl), -($C_1$-$C_6$ alkylene)$_p$-O-($C_2$-$C_6$ alkenylene)$_p$-($C_3$-$C_{10}$ cycloalkyl), -($C_1$-$C_6$ alkylene)$_p$-O-($C_2$-$C_6$ alkenylene)$_p$-(5-10 member heteroaryl), -($C_1$-$C_6$ alkylene)$_p$-O-($C_2$-$C_6$ alkenylene)$_p$-(3-10 member heterocyclyl), -($C_1$-$C_6$ alkylene)$_p$-O-($C_2$-$C_6$ alkynyl), -($C_1$-$C_6$ alkylene)p-O-($C_2$-$C_6$ alkynylene)$_p$-($C_6$-$C_{10}$ aryl), - ($C_1$-$C_6$ alkylene)$_p$-O-($C_2$-$C_6$ alkynylene)$_p$-($C_3$-$C_{10}$ cycloalkyl), -($C_1$-$C_6$ alkylene)$_p$-O-($C_2$-$C_6$ alkynylene)$_p$-(5-10 member heteroaryl) and -($C_1$-$C_6$ alkylene)$_p$-O-($C_2$-$C_6$ alkynylene)$_p$-(3-10 member heterocyclyl);

$R^2$ is selected from the group consisting of -($C_1$-$C_6$ alkylene)$_p$-C(O)-$R^b$,-($C_1$-$C_6$ alkylene)$_p$-C(O)-O-$R^a$, -($C_1$-$C_6$ alkylene)$_p$-C(O)-N($R^a$)$_2$, -($C_1$-$C_6$ alkylene)$_p$-S(O)-$R^a$, -($C_1$-$C_6$ alkylene)$_p$-$S(O)_2$-$R^a$, -($C_1$-$C_6$ alkylene)$_p$-$S(O)_2$-N($R^a$)$_2$, -($C_1$-$C_6$ alkylene)$_p$-$S(O)_2$-O-$R^a$, and $R^3$, wherein $R^3$ is selected from -($C_1$-$C_6$ alkylene)-($C_1$-$C_3$ perfluoroalkyl), $C_2$-$C_8$ alkenyl, $C_2$-$C_8$ alkynyl, -($C_1$-$C_6$ alkylene)$_p$-($C_3$-$C_{12}$ cycloalkyl), -($C_1$-$C_6$ alkylene)$_p$-(3-12 member heterocyclyl), -($C_1$-$C_6$ alkylene)$_p$-(5-12 member heteroaryl) and -($C_1$-$C_6$ alkylene)p-($C_5$-$C_{12}$ unsaturated nonaromatic carbocyclyl);

each $R^a$ is independently selected from the group consisting of H, $C_1$-$C_8$ alkyl, $C_2$-$C_8$ alkenyl, $C_2$-$C_8$ alkynyl, $C_1$-$C_8$ perfluoroalkyl, -($C_1$-$C_6$ alkylene)$_p$-($C_6$-$C_{12}$ aryl), -($C_1$-$C_6$ alkylene)$_p$-(5 to 12 member heteroaryl), -($C_1$-$C_6$ alkylene)$_p$-($C_3$-$C_{12}$ cycloalkyl), -($C_1$-$C_6$ alkylene)$_p$-(3-12 member heterocyclyl), -($C_1$-$C_6$ alkylene)$_p$-($C_5$-$C_{12}$ unsaturated nonaromatic carbocyclyl);

two $R^a$ attached to the same nitrogen atom, together with the nitrogen atom, may optionally form a 3-12 member heterocyclyl or a 5-12 member heteroaryl; the said 3-12 member heterocyclyl and the said 5-12 member heteroaryl is optionally further substituted by 1-5 $R^x$;

$R^b$ is selected from the group consisting of -$NR^a$N($R^a$)$_2$, -$NR^a$O$R^a$, $C_2$-$C_8$ alkenyl, $C_2$-$C_8$ alkynyl, $C_1$-$C_8$ perfluoroalkyl, -($C_3$-$C_6$ alkylene)-($C_1$-$C_3$ perfluoroalkyl), -($C_1$-$C_6$ alkylene)$_p$-($C_6$-$C_{12}$ aryl), -($C_1$-$C_6$ alkylene)$_p$-($C_3$-$C_{12}$ cycloalkyl), -($C_1$-$C_6$ alkylene)$_p$-(3-12 member heterocyclyl), -($C_1$-$C_6$ alkylene)$_p$-(5-12 member heteroaryl), -($C_1$-$C_6$ alkylene)$_p$-($C_5$-$C_{12}$ unsaturated nonaromatic carbocyclyl);

p is 0 or 1;

each R, $R^a$, $R^b$ and $R^3$ is optionally further substituted with 1-5 $R^x$;

each $R^x$ is independently selected from the group consisting of -oxo-, -($C_1$-$C_4$ alkylene)-, halogen, -CN, -OH, $C_1$-$C_6$ alkyl, $C_2$-$C_6$ alkenyl, $C_2$-$C_6$ alkynyl, $C_1$-$C_6$ perfluoroalkyl, -($C_1$-$C_6$ alkylene)-halogen, -($C_1$-$C_6$ alkylene)-OH, -($C_1$-$C_6$ alkylene)-CN, - ($C_1$-$C_6$ alkylene)$_q$-($C_3$-$C_6$ cycloalkyl), -($C_1$-$C_6$ alkylene)$_q$-(3-6 member heterocyclyl), -($C_1$-$C_6$ alkylene)$_q$-(5-6 member heteroaryl), -($C_1$-$C_6$ alkylene)$_q$-C(O)-($C_1$-$C_6$ alkyl), -($C_1$-$C_6$ alkylene)$_q$-C(O)-($C_3$-$C_6$ cycloalkyl), -($C_1$-$C_6$ alkylene)q-C(O)-($C_1$-$C_6$ alkylene)-($C_3$-$C_6$ cycloalkyl), -($C_1$-$C_6$ alkylene)$_q$-C(O)-O-($C_1$-$C_6$ alkyl), -($C_1$-$C_6$ alkylene)$_q$-C(O)-NH-($C_1$-$C_6$ alkyl), -($C_1$-$C_6$ alkylene)$_q$-C(O)-N($C_1$-$C_6$ alkyl)($C_1$-$C_6$ alkyl), -($C_1$-$C_6$ alkylene)$_q$-O-($C_1$-$C_6$ alkyl), -($C_1$-$C_6$ alkylene)q-O-($C_1$-$C_6$ alkylene)-halogen, -($C_1$-$C_6$ alkylene)$_q$-O-($C_1$-$C_6$ alkylene)-($C_1$-$C_3$ perfluoroalkyl), -($C_1$-$C_6$ alkylene)q-O-($C_1$-$C_6$ alkylene)$_q$-($C_3$-$C_6$ cycloalkyl), -($C_1$-$C_6$ alkylene)$_q$-O-($C_1$-$C_6$ alkylene)$_q$-(3-6 member heterocyclyl), -($C_1$-$C_6$ alkylene)q-O-($C_1$-$C_6$ alkylene)$_q$-(5-6 member heteroaryl), -($C_1$-$C_6$ alkylene)$_q$-O-($C_1$-$C_6$ alkylene)-$NH_2$, -($C_1$-$C_6$ alkylene)$_q$-O-($C_1$-$C_6$ alkylene)-NH-($C_1$-$C_6$ alkyl), -($C_1$-$C_6$ alkylene)$_q$-O-($C_1$-$C_6$ alkylene)-NH-($C_3$-$C_6$ cycloalkyl), -($C_1$-$C_6$ alkylene)$_q$-O-($C_1$-$C_6$ alkylene)-N($C_1$-$C_6$ alkyl)$_2$, -($C_1$-$C_6$ alkylene)$_q$-$NH_2$, -($C_1$-$C_6$ alkylene)$_q$-NH-($C_1$-$C_6$ alkyl), -($C_1$-$C_6$ alkylene)$_q$-NH-($C_3$-$C_6$ cycloalkyl), -($C_1$-$C_6$ alkylene)$_q$-N($C_1$-$C_6$ alkyl)($C_1$-$C_6$ alkyl), - ($C_1$-$C_6$ alkylene)$_q$-NHC(O)-($C_1$-$C_6$ alkyl), -($C_1$-$C_6$ alkylene)$_q$-NH-$SO_2$-($C_1$-$C_6$ alkyl), -($C_1$-$C_6$ alkylene)$_q$-$SO_2$-($C_1$-$C_6$ alkyl), -($C_1$-$C_6$ alkylene)$_q$-$SO_2$-($C_1$-$C_3$ alkylene)$_q$-($C_3$-$C_6$ cycloalkyl), -($C_1$-$C_6$ alkylene)$_q$-$SO_2$-$NH_2$, -($C_1$-$C_6$ alkylene)$_q$-$SO_2$-NH

($C_1$-$C_3$ alkyl), -($C_1$-$C_6$ alkylene)q-$SO_2$-NH-($C_1$-$C_3$ alkylene)$_q$-($C_3$-$C_6$ cycloalkyl) and -($C_1$-$C_6$ alkylene)$_q$-$SO_2$-N($C_1$-$C_3$ alkyl)$_2$; each q is independently 0 or 1; where permissible, each carbon atom of $R^x$ is optionally further substituted by 1-3 fluorine;

or a pharmaceutically acceptable salt thereof.

**2.** The compound of claim 1, or a pharmaceutically acceptable salt thereof, wherein m is 1 and n is 1, and the compound is of formula II,

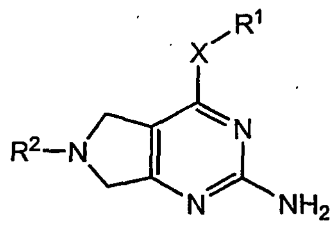

II

or a pharmaceutically acceptable salt thereof.

**3.** The compound of any of claims 1-2, or a pharmaceutically acceptable salt thereof, wherein X is a bond or -O-, $R^1$ is $C_6$-$C_{12}$ aryl, 5 to 12 member heteroaryl, or 3-12 member heterocyclyl, and $R^1$ is further substituted with 2-5 R.

**4.** The compound of any of claims 1-3, or a pharmaceutically acceptable salt thereof, wherein X is a bond and $R^1$ is a $C_6$-$C_{12}$ aryl further substituted with 2-5 R.

**5.** The compound of claim 4, or a pharmaceutically acceptable salt thereof, wherein $R^1$ is phenyl further substituted with 2-5 R and at least one of the R is not a halogen.

**6.** The compound of any of claims 1-5, or a pharmaceutically acceptable salt thereof, wherein R is selected from the group consisting of F, Cl, Br, -OH, -CN, $C_1$-$C_3$ alkyl, $C_1$-$C_3$ perfluoroalkyl, -($C_1$-$C_6$ alkylene)-OH, -O-($C_1$-$C_6$ alkyl), -($C_1$-$C_6$ alkylene)-O-($C_1$-$C_6$ alkyl), -($C_1$-$C_6$ alkylene)$_p$-O-($C_1$-$C_6$ alkylene)$_p$-($C_6$-$C_{10}$ aryl), -($C_1$-$C_6$ alkylene)$_p$-O-($C_1$-$C_6$ alkylene)$_p$-($C_3$-$C_{10}$ cycloalkyl), -($C_1$-$C_6$ alkylene)$_p$-O-($C_1$-$C_6$ alkylene)$_p$-(5-10 member heteroaryl), -($C_1$-$C_6$ alkylene)$_p$-O-($C_1$-$C_6$ alkylene)p-(3-10 member heterocyclyl), -($C_1$-$C_6$ alkylene)$_p$-O-($C_2$-$C_6$ alkenyl), -($C_1$-$C_6$ alkylene)$_p$-O-($C_2$-$C_6$ alkenylene)$_p$-($C_6$-$C_{10}$ aryl), ($C_1$-$C_6$ alkylene)$_p$-O-($C_2$-$C_6$ alkenylene)$_p$-($C_3$-$C_{10}$ cycloalkyl), -($C_1$-$C_6$ alkylene)$_p$-O-($C_2$-$C_6$ alkenylene)$_p$-(5-10 member heteroaryl), -($C_1$-$C_6$ alkylene)$_p$-O-($C_2$-$C_6$ alkenylene)$_p$-(3-10 member heterocyclyl), -($C_1$-$C_6$ alkylene)$_p$-O-($C_2$-$C_6$ alkynyl), -($C_1$-$C_6$ alkylene)$_p$-O-($C_2$-$C_6$ alkynylene)$_p$-($C_6$-$C_{10}$ aryl), -($C_1$-$C_6$ alkylene)$_p$-O-($C_2$-$C_6$ alkynylene)$_p$-($C_3$-$C_{10}$ cycloalkyl), -($C_1$-$C_6$ alkylene)$_p$-O-($C_2$-$C_6$ alkynylene)$_p$-(5-10 member heteroaryl) and -($C_1$-$C_6$ alkylene)p-O-($C_2$-$C_6$ alkynylene)$_p$-(3-10 member heterocyclyl); wherein each R is optionally further substituted by 1-5 $R^x$.

**7.** The compound of claim 2, of formula V,

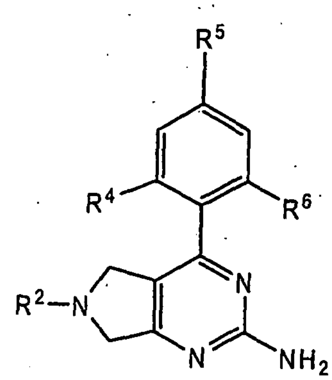

V

wherein

$R^4$ and $R^5$ are independently F, Cl, Br, -OH, -CN, unsubstituted $C_1$-$C_3$ alkyl, $C_1$-$C_3$ perfluoroalkyl, unsubstituted -($C_1$-$C_6$ alkylene)-OH or unsubstituted -O-($C_1$-$C_6$ alkyl);

$R^6$ is selected from the group consisting of -($C_1$-$C_6$ alkylene)-OH, -O-($C_1$-$C_6$ alkyl), -($C_1$-$C_6$ alkylene)-O-($C_1$-$C_6$ alkyl), -($C_1$-$C_6$ alkylene)$_p$-O-$C_1$-$C_6$ alkylene)$_p$-($C_6$-$C_{10}$ aryl), -($C_1$-$C_6$ alkylene)$_p$-O-($C_1$-$C_6$ alkylene)$_p$-($C_3$-$C_{10}$ cycloalkyl), -($C_1$-$C_6$ alkylene)$_p$-O-($C_1$-$C_6$ alkylene)$_p$-(5-10 member heteroaryl), -($C_1$-$C_6$ alkylene)$_p$-O-($C_1$-$C_6$ alkylene)$_p$-(3-10 member heterocyclyl), -($C_1$-$C_6$ alkylene)$_p$-O-($C_2$-$C_6$ alkenyl), -($C_1$-$C_6$ alkylene)$_p$-O-($C_2$-$C_6$ alkenytene)$_p$-($C_6$-$C_{10}$ aryl), -($C_1$-$C_6$ alkylene)$_p$-O-($C_2$-$C_6$ alkenylene)$_p$-($C_3$-$C_{10}$ cycloalkyl), -($C_1$-$C_6$ alkylene)$_p$-O-($C_2$-$C_6$ alkenylene)$_p$-(5-10 member heteroaryl), -($C_1$-$C_6$ alkylene)$_p$-O-($C_2$-$C_6$ alkenylene)$_p$-(3-10 member heterocyclyl), -($C_1$-$C_6$ alkylene)$_p$-O-($C_2$-$C_6$ alkynyl), -($C_1$-$C_6$ alkylene)$_p$-O-($C_2$-$C_6$ alkyhylene)$_p$-($C_6$-$C_{10}$ aryl), -($C_1$-$C_6$ alkylene)$_p$-O-($C_2$-$C_6$ alkynylene)$_p$-($C_3$-$C_{10}$ cycloalkyl), -($C_1$-$C_8$ alkylene)$_p$-O-($C_2$-$C_6$ alkynylene)$_p$-(5-10 member heteroaryl) and -($C_1$-$C_6$ alkylene)$_p$-O-($C_2$-$C_6$ alkynylene)$_p$-(3-10 member heterocyclyl); and $R^6$ is optionally further substituted with 1-5 $R^x$;

or a pharmaceutically acceptable salt thereof.

**8.** The compound of claim 2, of formula VI

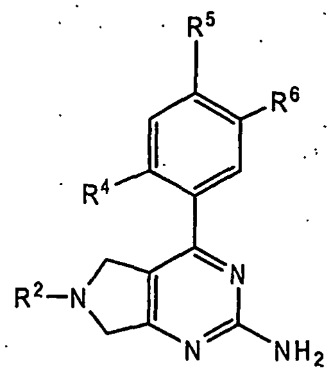

VI

wherein

$R^4$ and $R^5$ are independently F, Cl, Br, -OH, -CN, unsubstituted $C_1$-$C_3$ alkyl, $C_1$-$C_3$ perfluoroalkyl, unsubstituted -($C_1$-$C_6$ alkylene)-OH or unsubstituted -O-($C_1$-$C_6$ alkyl);

$R^6$ is selected from the group consisting of -($C_1$-$C_6$ alkylene)-OH, -O-($C_1$-$C_6$ alkyl), -($C_1$-$C_6$ alkylene)-O-($C_1$-$C_6$ alkyl), -($C_1$-$C_6$ alkylene)$_p$-O-($C_1$-$C_6$ alkylene)p-($C_6$-$C_{10}$ aryl), -($C_1$-$C_6$ alkylene)$_p$-O-($C_1$-$C_6$ alkylene)$_p$-($C_3$-$C_{10}$ cycloalkyl), -($C_1$-$C_6$ alkylene)$_p$-O-($C_1$-$C_6$ alkylene)$_p$-(5-10 member heteroaryl), -($C_1$-$C_6$ alkylene)$_p$-O-($C_1$-$C_6$ alkylene)$_p$-(3-10 member heterocyclyl), -($C_1$-$C_6$ alkylene)$_p$-O-($C_2$-$C_6$ alkenyl), -($C_1$-$C_6$ alkylene)$_p$-O-($C_2$-$C_6$ alkenylene)$_p$-($C_6$-$C_{10}$ aryl), -($C_1$-$C_6$ alkylene)$_p$-O-($C_2$-$C_6$, alkenylene)$_p$-($C_3$-$C_{10}$ cycloalkyl), -($C_1$-$C_6$alkylene)$_p$-O-($C_2$-$C_6$ alkenylene)$_p$-(5-10 member heteroaryl), -($C_1$-$C_6$ alkylene)$_p$-O-($C_2$-$C_6$ alkenylene)$_p$-(3-10 member heterocyclyl),

$-(C_1-C_6\ alkylene)_p-O-(C_2-C_6\ alkynyl)$, $-(C_1-C_6\ alkylene)_p-O-(C_2-C_6\ alkynylene)_p-(C_6-C_{10}\ aryl)$; $-(C_1-C_6\ alkylene)_p-O-(C_2-C_6\ alkynylene)_p-(C_3-C_{10}\ cycloalkyl)$, $-(C_1-C_6\ alkylene)_p-O-(C_2-C_6\ alkynylene)_p$-(5-10 member heteroaryl) and $-(C_1-C_6\ alkylene)_p-O-(C_2-C_6\ alkynylene)_p$-(3-10 member heterocyclyl); and $R^6$ is optionally further substituted with 1-5 $R^x$;
or a pharmaceutically acceptable salt thereof.

**9.** The compound of claim 7 or claim 8, or a pharmaceutically acceptable salt thereof, wherein $R^6$ is $-O-(C_1-C_6$ alkylene)-(5-10 member heteroaryl), and $R^6$ is optionally further substituted with 1-5 $R^x$.

**10.** The compound of claim 7 or claim 8, or a pharmaceutically acceptable salt thereof, wherein $R^6$ is $-O-(C_1-C_6$ alkylene)-(5 member heteroaryl), and $R^6$ is optionally further substituted with 1-5 $R^x$.

**11.** The compound of claim 7 or claim 8, or a pharmaceutically acceptable salt thereof, wherein $R^6$ is $-O-(C_1-C_6$ alkylene)-(3-10 member heterocyclyl), and $R^6$ is optionally further substituted with 1-5 $R^X$.

**12.** The compound of claim 7 or claim 8, or a pharmaceutically acceptable salt thereof, wherein $R^6$ is $-O-(C_1-C_6$ alkyl) or $-O-(C_2-C_6$ alkenyl), and $R^6$ is optionally further substututited with 1-5 $R^x$.

**13.** The compound of any of claims 1-12, or a pharmaceutically acceptable salt thereof, wherein $R^2$ is $-C(O)-N(R)_2$.

**14.** The compound of any of claim 9-12, or a pharmaceutically acceptable salt thereof, wherein $R^2$ is $-C(O)-OR^a$.

**15.** A pharmaceutical composition comprising a compound as claimed in any one of claims 1 to 14, or a pharmaceutically acceptable salt thereof, and a pharmaceutically acceptable carrier or excipient.

**16.** A compound as claimed in any one of claims 1 to 14, or a pharmaceutically acceptable salt thereof, or a composition as claimed in claim 15, for use in the treatment of cancer.

**17.** A compound as claimed in any one of claims 1 to 14, or a pharmaceutically acceptable salt thereof, or a composition as claimed in claim 15, for use as a modulator or inhibitor of the activity of HSP-90.

**18.** A compound as claimed in any one of claims 1 to 14, or a pharmaceutically acceptable salt thereof, or a composition as claimed in claim 15, for use in the treatment of a disease associated with abnormal cell growth.

**19.** The use of a compound as claimed in any one of claims 1 to 14, or a pharmaceutically acceptable salt thereof, or a composition as claimed in claim 15, for the manufacture of a medicament for the treatment of cancer.

**20.** The use of a compound as claimed in any one of claims 1 to 14, or a pharmaceutically acceptable salt thereof, or a composition as claimed in claim 15, for the manufacture of a medicament for use as a modulator or inhibitor of the activity of HSP-90.

**21.** The use of a compound as claimed in any one of claims 1 to 14, or a pharmaceutically acceptable salt thereof, or a composition as claimed in claim 15, for the manufacture of a medicament for the treatment of a disease associated with abnormal cell growth.

**22.** A combination of a compound as claimed in any one of claims 1 to 14, or a pharmaceutically acceptable salt thereof, or a composition as claimed in claim 15, and another drug.

**23.** A combination of a compound as claimed in any one of claims 1 to 14, or a pharmaceutically acceptable salt thereof, or a composition as claimed in claim 15, and an anti-tumour agent selected from the group consisting of mitotic inhibitors, alkylating agents, anti-metabolites, intercalating antibiotics, growth factor inhibitors, cell cycle inhibitors, enzymes, topoisomerase inhibitors, biological response modifiers, antibodies, cytotoxics, anti-hormones, anti-androgens, anti-angiogenesis agents, kinase inhibitors, pan kinase inhibitors or growth factor inhibitors, for use in the treatment of a disease associated with abnormal cell growth.

## Patentansprüche

1. Verbindung der Formel (I)

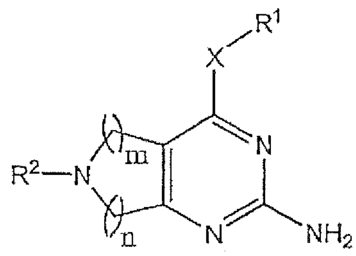

(I)

worin:

m 1 oder 2 ist, n 1 oder 2 ist, wenn m 2 ist, n 1 ist;
X eine Bindung oder ein zweiwertiger Rest ist, ausgewählt aus der Gruppe, bestehend aus -O-, -S-, -($C_1$-$C_3$-Alkylen)-, -O-($C_1$-$C_3$-Alkylen)-, -NH-($C_1$-$C_3$-Alkylen), -S-($C_1$-$C_3$-Alkylen)-, -C(O)-, -C(O)-O-, -C(O)-NH-, -OC(O)-NH-, -NH-C(O)-NH-, -S(O)-, -$S(O)_2$-, -$S(O)_2$-O- und $S(O)_2$-NH, wobei jedes Ende des zweiwertigen Rests an $R^1$ oder an den Aminopyrimidinring der Formel I gebunden sein kann;
wo zulässig, jedes Stickstoff oder Kohlenstoffatom von X außerdem gegebenenfalls mit einer Gruppe substituiert ist, die ausgewählt ist aus -($C_1$-$C_3$-Alkylen)$_t$-CN, -($C_1$-$C_3$-Alkylen)$_t$-F, -($C_1$-$C_3$-Alkylen)$_t$-($C_1$-$C_3$-perfluoralkyl), -($C_1$-$C_3$-Alkylen)$_t$-O-($C_1$-$C_6$-alkyl),-($C_1$-$C_3$-Alkylen)$_1$-OH,-($C_1$-$C_3$-Alkylen)$_t$-$NH_2$,-($C_1$-$C_3$-Alkylen)$_t$-NH($C_1$-$C_3$-alkyl) und -($C_1$-$C_3$-Alkylen)$_t$N($C_1$-$C_3$-alkyl)($C_1$-$C_3$-alkyl) und t 0 oder 1 ist;
$R^1$ ausgewählt ist aus der Gruppe, bestehend aus $C_6$-$C_{12}$-Aryl, 5- bis 12-gliedrigem Heteroaryl, $C_3$-$C_{12}$-Cycloalkyl, 3-12-gliedrigem Heterocyclyl und ungesättigtem nichtaromatischem $C_5$-$C_{12}$-Carbocyclyl, und jedes $R^1$ gegebenenfalls außerdem mit 1 bis 5 R weiter substituiert ist, mit der Maßgabe, dass, wenn $R^1$ Phenyl ist, dann $R^1$ mit wenigstens zwei R weiter substituiert ist und wenigstens eines der R nicht Halogen ist;
R ausgewählt ist aus der Gruppe, bestehend aus $R^x$ -($C_1$-$C_6$-Alkylen)$_p$-O-($C_1$-$C_6$-alkylen)$_p$-($C_6$-$C_{10}$-aryl), -($C_1$-$C_6$-Alkylen)$_p$-O-($C_1$-$C_6$-alkylen)$_p$-($C_7$-$C_{10}$-cycloalkyl) -($C_1$-$C_6$-Alkylen)$_p$-O-($C_1$-$C_6$-alkylen)$_p$-(7-10-gliedriges Heteroaryl),-($C_1$-$C_6$Alkylen)$_p$-O-($C_1$-$C_6$-alkylen)$_p$-(7-10-gliedriges Heterocyclyl),-($C_1$-$C_6$-Alkylen)$_p$-O-($C_2$-$C_6$-alkenyl), -($C_1$-$C_6$-Alkylen)$_p$-O-($C_2$-$C_6$-alkenylen)$_p$-(C6-$C_{10}$-aryl), -($C_1$-$C_6$-Alkylen)$_p$-O-($C_2$-$C_6$-alkenylen)$_p$-(C3-$C_{10}$-cycloalkyl), -($C_1$-$C_6$-Alkenylen)$_p$-O-($C_2$-$C_6$-alkenylen)$_p$-(5-10-gliedriges Heteroaryl), -($C_1$-$C_6$-Alkylen)$_p$-O-($C_2$-$C_6$-alkenylen)$_p$-(3-10-gliedriges Heterocyclyl), -($C_1$-$C_6$-Alkylen)$_p$-O-($C_2$-$C_6$-alkinyl), -($C_1$-$C_6$-Alkylen)$_p$-O-($C_2$-$C_6$-alkinylen)$_p$-($C_6$-$C_{10}$-aryl), -($C_1$-$C_6$-Alkylen)$_p$-O-($C_2$-$C_6$-alkinylen)$_p$-($C_3$-$C_{10}$-cycloalkyl),-($C_1$-$C_6$-Alkylen)$_p$-O-($C_2$-$C_6$-alkinylen)$_p$-(5-10-gliedriges Heteroaryl) und -($C_1$-$C_6$-Alkylen)$_p$-O-($C_2$-$C_6$-alkinylen)$_p$-(3-10-gliedriges Heterocyclyl);
$R^2$ ausgewählt ist aus der Gruppe, bestehend aus -($C_1$-$C_6$-Allcylen)$_p$-C(O)-$R^b$, -($C_1$-$C_6$-Alkylen)$_p$-C(O)-O-$R^a$, -($C_1$-$C_6$-Alkylen)$_p$-C(O)-N($R^a$)2, -($C_1$-$C_6$-Alkylen)$_p$-S(O)-$R^a$, -($C_1$-$C_6$-Alkylen)$_p$-$S(O)_2$-$R^a$, -($C_1$-$C_6$-Alkylen)$_p$-$S(O)_2$-N($R^a$)$_2$,-($C_1C_6$-Alkylen)$_p$-$S(O)_2$-O-$R^a$undR$^3$,wobeiR$^3$ausgewähltistaus-($C_1$-$C_6$-Alkylen)-($C_1$-$C_3$-perfluoralkyl), $C_2$-$C_8$-Alkenyl, $C_2$-$C_8$-Alkinyl, -($C_1$-$C_6$-Alkylen)$_p$-($C_3$-$C_{12}$-cycloalkyl), -($C_1$-$C_6$-Alkylen)$_p$-(3-12-gliedriges Heterocyclyl), -($C_1$-$C_6$-Alkylen)$_p$-(5-12-gliedriges Heteroaryl) und -($C_1$-$C_6$-Alkylen)$_p$-(ungesättigtes nichtaromatisches $C_5$-$C_{12}$-Carbocyclyl);
jedes $R^a$ unabhängig ausgewählt ist aus der Gruppe, bestehend aus H, $C_1$-$C_8$-Alkyl, $C_2$-$C_8$-Alkenyl, $C_2$-$C_8$-Alkinyl, $C_1$-$C_8$-Perfluoralkyl, -($C_1$-$C_6$-Alkylen)$_p$-($C_6$-$C_2$-Aryl), -($C_1C_6$-Alkylen)$_p$-(5-12-gliedriges Heteroaryl), -($C_1$-$C_6$-Alkylen)$_p$-($C_3$-$C_{12}$-cycloalkyl),-($C_1$-$C_6$-Alkylen)$_p$-(3-12-gliedriges Heterocyclyl),-($C_1$-$C_6$-Alkylen)$_p$-(ungesättigtes nichtaromatisches $C_5$-$C_{12}$-Carbocyclyl);
zwei $R^a$, die an das selbe Stickstoffatom gebunden sind, zusammen mit dem Stickstoffatom gegebenenfalls ein 3-12-gliedriges Heterocyclyl oder ein 5-12-gliedriges Heteroaryl bilden können; das 3-12-gliedrige Heterocyclyl und das 5-12-gliedrige Heteroaryl gegebenenfalls mit 1-5 $R^x$ weiter substituiert sind;
$R^b$ ausgewählt ist aus der Gruppe, bestehend aus -N$R^a$N($R^a$)$_2$, -N$R^a$O$R^a$, $C_2$-$C_8$-Alkenyl, $C_2$-$C_8$-Alkinyl, $C_1$-$C_8$-Perfluoralyl, ($C_3$-$C_6$-Alkylen)-($C_1$-$C_3$-perfluoralkyl), -($C_1$-$C_6$-Alkylen)$_p$-($C_6$-$C_{12}$-aryl), -($C_1$-$C_6$-Alkylen)$_p$-($C_3$-$C_{12}$-cycloalkyl), -($C_1$-$C_6$-Alkylen)$_p$-(3-12-gliedriges Heterocyclyl), -($C_1$-$C_6$-Alkylen)$_p$-(5-12-gliedriges Heteroaryl), -($C_1$-$C_6$-Alkylen)$_p$-(ungesättigtes nichtaromatisches $C_5$-$C_{12}$-Carbocyclyl);

p 0 oder 1 ist;
jedes R, $R^a$, $R^b$ und $R^3$ gegebenenfalls außerdem mit 1-5 $R^x$ substituiert ist;
jedes $R^x$ unabhängig ausgewählt ist aus der Gruppe, bestehend aus -Oxo-, -($C_1$-$C_4$-Alkylen)-, Halogen, -CN, -OH, $C_1$-$C_6$-Alkyl, $C_2$-$C_6$-Alkenyl, $C_2$-$C_6$-Alkinyl, $C_1$-$C_6$-Perfluoralkyl, -($C_1$-$C_6$-Alkylen)-halogen, -($C_1$-$C_6$-Alkylen)-OH, -($C_1$-$C_6$-Alkylen)-CN, -($C_1$-$C_6$-Alkylen)$_q$-($C_3$-$C_6$-cycloalkyl), -($C_1$-$C_6$-Alkylen)$_q$-(3-6-gliedriges Heterocyclyl), -($C_1$-C6-Allcylen)$_q$-(5-6-gliedriges Heteroaryl), -($C_1$-$C_6$-Alkylen)$_q$-C(O)-($C_1$-$C_6$-alkyl), -($C_1$-$C_6$-Alkylen)$_q$-C(O)-($C_3$-$C_6$-cycloalkyl), -($C_1$-$C_6$-Alkylen)$_q$-C(O)-($C_1$-$C_6$-alkylen)-($C_3$-$C_6$-cycloalkyl), -($C_1$-$C_6$-Alkylen)$_q$-C(O)-O-($C_1$-$C_6$-alkyl), -($C_1$-$C_6$-Alkylen)$_q$-C(O)-NH-($C_1$-$C_6$-alkyl), -($C_1$-$C_6$-Alkylen)$_q$-C(O)-N($C_1$-$C_6$-alkyl)($C_1$-$C_6$-alkyl)-($C_1$-$C_6$-Alkylen)$_q$-O-($C_1$-$C_6$-alkyl), -($C_1$-$C_6$-Alkylen)$_q$-O-($C_1$-$C_6$-alkylen)-halogen, -($C_1$-$C_6$-Alkylen)$_q$-O-($C_1$-$C_6$-alkylen)-($C_1$-$C_3$-perfluoralkyl), -($C_1$-$C_6$-Alkylen)$_q$-O-($C_1$-$C_6$-alkylen)$_q$-($C_3$-$C_6$-cycloalkyl), -($C_1$-$C_6$-Alkylen)$_q$-O-($C_1$-$C_6$-alkylen)$_q$-(3-6-gliedriges Heterocyclyl), -($C_1$-$C_6$-Alkylen)$_q$-O-($C_1$-$C_6$-alkylen)$_q$-(5-6-gliedriges Heteroaryl), -($C_1$-$C_6$-Alkylen)$_q$-O-($C_1$-$C_6$-alkylen)-$NH_2$, -($C_1$-$C_6$-Alkylen)$_q$-O-($C_1$-$C_6$-alkylen)-NH-($C_1$-$C_6$-alkyl), - ($C_1$-$C_6$-Alkylen)$_q$-O-($C_1$-$C_6$-alkylen)-NH-($C_3$-$C_6$-cycloalkyl), -($C_1$-$C_6$-Alkylen)$_q$-O-($C_1$-$C_6$-alkylen)-N($C_1$-$C_6$-alkyl)$_2$, -($C_1$-$C_6$-Alkylen)$_q$-$NH_2$, -($C_1$-$C_6$-Alkylen)$_q$-NH-($C_1$-$C_6$-alkyl), -($C_1$-$C_6$-Alkylen)$_q$-NH-($C_3$-$C_6$-cycloalkyl), -($C_1$-$C_6$-Alkylen)$_q$-N($C_1$-$C_6$-alkyl)($C_1$-$C_6$-alkyl), -($C_1$-$C_6$-Alkylen)$_q$-NHC(O)-($C_1$-$C_6$-alkyl), -($C_1$-$C_6$-Alkylen)$_q$-NH-$SO_2$-($C_1$-$C_6$-alkyl), -($C_1$-$C_6$-Alkylen)$_q$-$SO_2$-($C_1$-$C_6$-alkyl), -($C_1$-$C_6$-Alkylen)$_q$-$SO_2$-($C_1$-$C_3$alkylen)$_q$-($C_3$-$C_6$-cycloalkyl), -($C_1$$C_6$-Alkylen)$_q$-$SO_2$-$NH_2$, -($C_1$-$C_6$-Alkylen)$_q$-$SO_2$-NH($C_1$-$C_3$-alkyl), -($C_1$-$C_6$-Alkylen)$_q$-$SO_2$-NH-($C_1$-$C_3$-alkylen)$_q$-($C_3$-$C_6$-cycloalkyl) und -($C_1$-$C_6$-Alkylen)$_q$-$SO_2$N($C_1$-$C_3$-alkyl)$_2$; jedes q unabhängig 0 oder 1; wo zulässig, jedes Kohlenstoffatom von $R^x$ gegebenenfalls außerdem mit 1-3 Fluor substituiert ist;

oder ein pharmazeutisch verträgliches Salz davon.

2. Verbindung nach Anspruch 1 oder ein pharmazeutisch verträgliches Salz davon, wobei m 1 ist und n 1 ist, und die Verbindung der Formel II

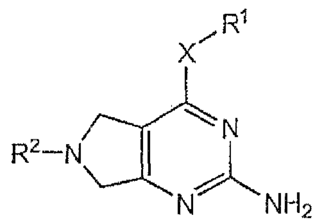

II

oder ein pharmazeutisch verträgliches Salz davon.

3. Verbindung nach einem der Ansprüche 1-2 oder ein pharmazeutisch verträgliches Salz davon, wobei X eine Bindung oder -O- ist, $R^1$ $C_6$-$C_{12}$-Aryl, 5- bis 12-gliedriges Heteroaryl oder 3-12-gliedriges Heterocyclyl ist und $R^1$ außerdem mit 2-5 R substituiert ist.

4. Verbindung nach einem der Ansprüche 1-3 oder ein pharmazeutisch verträgliches Salz davon, wobei X eine Bindung ist und $R^1$ ein $C_6$-$C_{12}$-Aryl ist, das außerdem mit 2-5 R substituiert ist.

5. Verbindung nach Anspruch 4 oder ein pharmazeutisch verträgliches Salz davon, wobei $R^1$ Phenyl ist, das außerdem mit 2-5 R substituiert ist, und wenigstens eines der R nicht Halogen ist.

6. Verbindung nach einem der Ansprüche 1-5 oder ein pharmazeutisch verträgliches Salz davon, wobei R ausgewählt ist aus der Gruppe, bestehend aus F, Cl, Br, -OH, -CN, $C_1$-$C_3$-Alkyl, $C_1$-$C_3$-Perfluoralkyl, -($C_1$-$C_6$-Alkylen)-OH, -O-($C_1$-$C_6$-Alkyl), -($C_1$-$C_6$-Alkylen)-O-($C_1$-$C_6$-alkyl), -($C_1$-$C_6$-Alkylen)$_p$-O-($C_1$-$C_6$-alkylen)$_p$-($C_6$-$C_{10}$-aryl), -($C_1$-$C_6$-Alkylen)$_p$-O-($C_1$-$C_6$-alkylen)$_p$-($C_3$-$C_{10}$-cycloalkyl), -($C_1$-$C_6$-Alkylen)$_p$-O-($C_1$-$C_6$-alkylen)$_p$-(5-10-gliedriges Heteroaryl), -($C_1$-$C_6$-Alkylen)$_p$-O-($C_1$-$C_6$-alkylen)$_p$-(3-10-gliedriges Heterocyclyl), -($C_1$-$C_6$-Alkylen)$_p$-O-($C_2$-$C_6$-alkenyl), -($C_1$-$C_6$-Alkylen)$_p$-O-($C_2$-$C_6$-alkylen)$_p$-($C_6$-$C_6$-aryl), -($C_1$-$C_6$-Alkylen)$_p$-O-($C_2$-$C_6$-alkenylen)$_p$-($C_3$-$C_1$-cycloalkyl), -($C_1$-$C_6$-Alkylen)$_p$-O-($C_2$-$C_6$-alkenylen)$_p$-(5-10-gliedriges Heteroaryl), -($C_1$-$C_6$-Alkylen)$_p$-O-($C_2$-$C_6$-alkenylen)$_p$-(3-10-gliedriges Heterocyclyl), -($C_1$-$C_6$-Alkylen)$_p$-O-($C_2$-$C_6$-alkinyl), -($C_1$-$C_6$-Alkylen)$_p$-O-($C_2$-$C_6$-alkinylen)$_p$-($C_6$-$C_{10}$-aryl), -($C_1$-$C_6$-Alkylen)$_p$-O-($C_2$-$C_6$-alkinylen)$_p$-($C_3$-$C_{10}$-cycloalkyl), -($C_1$-$C_6$-Alkylen)$_p$-O-($C_2$-$C_6$-alkinylen)$_p$-(5-10-gliedriges Heteroaryl) und -($C_1$-$C_6$-Alkylen)$_p$-O-($C_9$-$C_6$-alkinylen)$_p$-(3-10-gliedriges Heterocyclyl); wobei

jedes R gegebenenfalls außerdem mit 1-5 $R^x$ substituiert ist.

**7.** Verbindung nach Anspruch 2 der Formel V

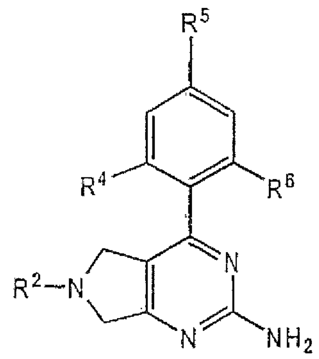

V

worin
$R^4$ und $R^5$ unabhängig F, Cl, Br, -OH, -CN, unsubstituiertes $C_1$-$C_3$-Alkyl, $C_1$-$C_3$-Perfluoralkyl, unsubstituiertes -($C_1$-$C_6$-Alkylen)-OH oder unsubstituiertes -O-($C_1$-$C_6$-Alkyl) sind;
$R^6$ausgewähltistausderGruppe,bestehendaus-($C_1$-$C_6$-Alkylen)-OH,-O-($C_1$-$C_6$-Alkyl),-($C_1$-$C_6$-Alkylen)-O-($C_1$-$C_6$-alkyl), -($C_1$-$C_6$-Alkylen)$_p$-O-($C_1$-$C_6$-alkylen)$_p$-($C_6$-$C_{10}$-aryl), -($C_1$-$C_6$-Alkylen)$_p$-O-($C_1$-$C_6$-alkylen)$_p$-($C_3$-$C_{10}$-cycloalkyl), -($C_1$-$C_6$-Alkylen)$_p$-O-($C_1$-$C_6$-alkylen)p-(5-10-gliedriges Heteroaryl), -($C_1$-$C_6$-Alkylen)$_p$-O-($C_1$-$C_6$-alkylen)$_p$-(3-10-gliedriges Heterocyclyl), -($C_1C_6$-Alkylen)$_p$-O-($C_2$-$C_6$-alkenyl), -($C_1$-$C_6$-Alkylen)$_p$-O-($C_2$-$C_6$-alkenylen)$_p$-($C_6$-$C_{10}$-aryl),-($C_1$-$C_6$-Alkylen)$_p$-O-($C_2$-$C_6$-alkenylen)$_p$-($C_3$-$C_{10}$-cycloalkyl),-($C_1$-$C_6$-Alkylen)$_p$-O-($C_2$-$C_6$-alkenylen)$_p$-(5-10-gliedrigesHeteroaryl),-($C_1$-$C_6$-Alkylen)$_p$-O-($C_2$-$C_6$-alkenylen)$_p$-(3-10-gliedrigesHeterocyclyl),-($C_1$-$C_6$-Alkylen)$_p$-O-($C_2$-$C_6$-alkinyl),-($C_1$-$C_6$-Alkylen)$_p$-O-($C_2$-$C_6$-alkinylen)$_p$-($C_6$-$C_{10}$-aryl),-($C_1$-$C_6$-Alkylen)$_p$-O-($C_2$-$C_6$-alkinylen)$_p$-($C_3$-$C_{10}$-cycloalkyl),- ($C_1$-$C_6$-Alkylen)$_p$-O-($C_2$-$C_6$alkinylen)$_p$-(5-10-gliedriges Heteroaryl) und -($C_1$-$C_6$-Alkylen)$_p$-O-($C_2$-$C_6$-alkinylen)$_p$-(3-10-gliedriges Heterocyclyl), und $R^6$ gegebenenfalls außerdem mit 1-5 $R^x$ substituiert ist,
oder ein pharmazeutisch verträgliches Salz davon.

**8.** Verbindung nach Anspruch 2 der Formel VI

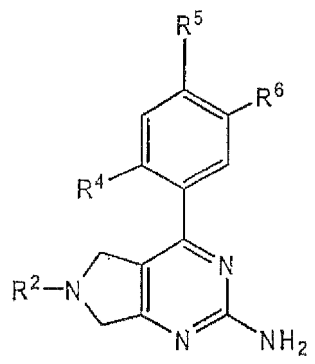

VI

worin
$R^4$ und $R^5$ unabhängig F, Cl, Br, -OH, -CN, unsubstituiertes $C_1$-$C_3$-Alkyl, $C_1$-$C_3$-Perfluoralkyl, unsubstituiertes -($C_1$-$C_6$-Alkylen)-OH oder unsubstituiertes -O-($C_1$-$C_6$-Alkyl) sind;
$R^6$ausgewähltistausderGruppe,bestehendaus-($C_1$-$C_6$-Alkylen)-OH,-O-($C_1$-$C_6$-Alkyl),-($C_1$-$C_6$-Alkylen)-O-($C_1$-$C_6$-alkyl), -($C_1$-$C_6$-Alkylen)$_p$-O-($C_1$-$C_6$-alkylen)$_p$-($C_6$-$C_{10}$-aryl), -($C_1$-$C_6$-Alkylen)$_p$-O-($C_1$-$C_6$-alkylen)$_p$-($C_3$-$C_{10}$-cycloalkyl),

-($C_1$-$C_6$-Alkylen)$_p$-O-($C_1$-$C_6$-alkylen)$_p$-(5-10-gliedriges Heteroaryl), -($C_1$-$C_6$-Alkylen)$_p$-O-($C_1$-$C_6$-alkylen)$_p$-(3-10-gliedriges Heterocyclyl), -($C_1$-$C_6$-Alkylen)$_p$-O-($C_1$-$C_6$-alkenyl), -($C_1$-$C_6$-Alkylen)$_p$-O-($C_2$-$C_6$-alkenylen)$_p$-($C_6$-$C_{10}$-aryl),-($C_1$-$C_6$-Alkylen)$_p$-O-($C_2$-$C_6$-alkenylen)$_p$-($C_2$-$C_{10}$-cycloalkyl),-($C_1$-$C_6$-Alkylen)$_p$-O-($C_2$-$C_6$-alkenylen)$_p$-(5-10-gliedrigesHeteroaryl),-($C_1$-$C_6$-Alkylen)$_p$-O-($C_2$-$C_6$-alkenylen)p-(3-10-gliedrigesHeterocyclyl),-($C_1$-$C_6$-Alkylen)$_p$-O-($C_2$-$C_6$-alkinyl),-($C_1$-$C_6$-Alkylen)$_p$-O-($C_2$-$C_2$-alkinylen)$_p$-($C_6$-$C_{10}$-aryl),-($C_1$-$C_6$-Alkylen)$_p$-O-($C_2$-$C_6$-alkinylen)$_p$-($C_3$-$C_{10}$-cycloalkyl), -($C_1$-$C_6$-Alkylen)$_p$-O-($C_2$-$C_6$-alkinylen)$_p$-(5-10-gliedriges Heteroaryl) und -($C_1$-$C_6$-Alkylen)$_p$-O-($C_2$-$C_6$-alkinylen)$_p$-(3-10-gliedriges Heterocyclyl), und $R^6$ gegebenenfalls außerdem mit 1-5 $R^x$ substituiert ist,

oder ein pharmazeutisch verträgliches Salz davon.

**9.** Verbindung nach Anspruch 7 oder Anspruch 8 oder ein pharmazeutisch verträgliches Salz davon, wobei $R^6$ -O-($C_1$-$C_6$-Alkylen)-(5-10-gliedriges Heteroaryl) ist und $R^6$ gegebenenfalls außerdem mit 1-5 $R^x$ substituiert ist.

**10.** Verbindung nach Anspruch 7 oder Anspruch 8 oder ein pharmazeutisch verträgliches Salz davon, wobei $R^6$ -O-($C_1$-$C_6$-Alkylen)-(5-gliedriges Heteroaryl) ist und $R^6$ gegebenenfalls außerdem mit 1-5 $R^x$ substituiert ist.

**11.** Verbindung nach Anspruch 7 oder Anspruch 8 oder ein pharmazeutisch verträgliches Salz davon, wobei $R^6$ -O-($C_1$-$C_6$-Alkylen)-(3-10-gliedriges Heterocyclyl) ist und $R^6$ gegebenenfalls außerdem mit 1-5 $R^x$ substituiert ist.

**12.** Verbindung nach Anspruch 7 oder Anspruch 8 oder ein pharmazeutisch verträgliches Salz davon, wobei $R^6$ -O-($C_1$-$C_6$-Alkyl) oder -O-($C_2$-$C_6$-Alkenyl) ist und $R^6$ gegebenenfalls außerdem mit 1-5 $R^x$ substituiert ist.

**13.** Verbindung nach einem der Ansprüche 1-12 oder ein pharmazeutisch verträgliches Salz davon, wobei $R^2$ -C(O)-N($R^a$)$_2$ ist.

**14.** Verbindung nach einem der Ansprüche 1-12 oder ein pharmazeutisch verträgliches Salz davon, wobei $R^2$ -C(O)-OR$^a$ ist.

**15.** Pharmazeutische Zusammensetzung, die eine Verbindung, wie sie in einem der Ansprüche 1-14 beansprucht ist, oder ein pharmazeutisch verträgliches Salz davon und einen pharmazeutisch verträglichen Träger oder ein pharmazeutisch verträgliches Exzipiens uinfasst.

**16.** Verbindung, wie sie in einem der Ansprüche 1-14 beansprucht ist, oder ein pharmazeutisch verträgliches Salz davon oder eine Zusammensetzung, wie sie in Anspruch 15 beansprucht ist, zur Verwendung bei der Behandlung von Krebs.

**17.** Verbindung, wie sie in einem der Ansprüche 1-14 beansprucht ist, oder ein pharmazeutisch verträgliches Salz davon oder eine Zusammensetzung, wie sie in Anspruch 15 beansprucht ist, zur Verwendung als Modulator oder Inhibitor der Aktivität von HSP-90.

**18.** Verbindung, wie sie in einem der Ansprüche 1-14 beansprucht ist, oder ein pharmazeutisch verträgliches Salz davon oder eine Zusammensetzung, wie sie in Anspruch 15 beansprucht ist, zur Verwendung bei der Behandlung einer Erkrankung, die mit abnormalem Zellwachstum assoziiert ist.

**19.** Verwendung einer Verbindung, wie sie in einem der Ansprüche 1-14 beansprucht ist, oder eines pharmazeutisch verträglichen Salzes davon oder einer Zusammensetzung, wie sie in Anspruch 15 beansprucht ist, für die Herstellung eines Medikaments für die Behandlung von Krebs.

**20.** Verwendung einer Verbindung, wie sie in einem der Ansprüche 1-14 beansprucht ist, oder eines pharmazeutisch verträglichen Salzes davon oder einer Zusammensetzung, wie sie in Anspruch 15 beansprucht ist, für die Herstellung eines Medikaments zur Verwendung als Modulator oder Inhibitor der Aktivität von HSP-90.

**21.** Verwendung einer Verbindung, wie sie in einem der Ansprüche 1 bis 14 beansprucht ist, oder eines pharmazeutisch verträglichen Salzes davon oder einer Zusammensetzung, wie sie in Anspruch 15 beansprucht ist, für die Herstellung eines Medikaments für die Behandlung einer Erkrankung, die mit abnormalem Zellwachstum assoziiert ist.

**22.** Kombination einer Verbindung, wie sie in einem der Ansprüche 1-14 beansprucht ist, oder eines pharmazeutisch verträglichen Salzes davon oder einer Zusammensetzung, wie sie in Anspruch 15 beansprucht ist, und eines anderen

Wirkstoffs.

**23.** Kombination einer Verbindung, wie sie in einem der Ansprüche 1-14 beansprucht ist, oder eines pharmazeutisch verträglichen Salzes davon oder einer Zusammensetzung, wie sie in Anspruch 15 beansprucht ist, und eines Antitumormittels, ausgewählt aus der Gruppe, bestehend aus Mitoseinhibitoren, Alkylierungsmitteln, Antimetaboliten, interkalierenden Antibiotika, Wachstumsfaktor-Inhibitoren, Zellzyklusinhibitoren, Enzymen, Topoisomerasc-Inhibitoren, Modifizierungsmitteln der biologischen Antwort, Antikörpern, zytotoxischen Mitteln, Antihormonen, Antiandrogenen, Antiangiogenesemitteln, Kinase-Inhibitoren, pan-Kinase-Inhibitoren oder Wachstumsfaktor-Inhibitoren, zur Verwendung bei der Behandlung einer Erkrankung, die mit abnormalen Zellwachstum assoziiert ist.

## Revendications

**1.** Composé de formule (I)

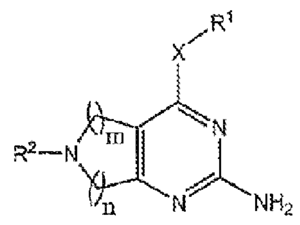

(I)

dans lequel :

m représente 1 ou 2, n représente 1 ou 2, lorsque m représente 2, n représente 1 ;
X représente une liaison ou un diradical sélectionné dans le groupe consistant en -O-, -S-, -(alkylène en $C_1$-$C_3$)-, -O-(alkylène en $C_1$-$C_3$)-, -NH-(alkylène en $C_1$-$C_3$)-, -S-(alkylène en $C_1$-$C_3$)-, -C(O)-, -C(O)-O-, -C(O)-NH-, -OC (O)-NH-, -NH-C(O)-NH-, -S(O)-, -$S(O)_2$-, -S $(O)_2$-O- et -$S(O)_2$-NH-, chaque extrémité du diradical pouvant être connectée à $R^1$ ou au noyau aminopyrimidine de formule I ;
lorsque cela est possible, chaque atome d'azote ou de carbone de X est, en outre, facultativement substitué par un groupe sélectionné parmi -(alkylène en $C_1$-$C_3$)$_t$-CN, -(alkylène en $C_1$-C3)$_t$-F, -(alkylène e n $C_1$-C3)$_t$-(perfluoroalkyle en $C_1$-$C_3$), -(alkylène en $C_1$-$C_3$)-O-(alkyle en $C_1$-$C_6$),-(alkylène en $C_1$-$C_3$)$_t$-OH, -(alkylène en $C_1$-$C_3$)$_t$-$NH_2$, -(alkylène en $C_1$-$C_3$)$_t$-NH(alkyle en $C_1$-$C_3$) et - (alkylène en $C_1$-$C_3$)$_t$-N(alkyle en $C_1$-$C_3$) (alkyle en $C_1$-$C_3$), et t représente 0 ou 1 ;
$R^1$ est sélectionné dans le groupe consistant en aryle en $C_6$-$C_{12}$, hétéroaryle ayant de 5 à 12 éléments, cycloalkyle en $C_3$-$C_{12}$, hétérocyclyle ayant de 3 à 12 éléments et carbocyclyle non-aromatique insaturé en $C_5$-$C_{12}$, et chaque $R^1$ est, de plus, facultativement substitué par 1-5 R, pour autant que, lorsque $R^1$ représente phényle, $R^1$ est, en outre, substitué par au moins deux R et un R au moins ne représente pas un halogène ;
R est sélectionné dans le groupe consistant en $R^X$, - (alkylène en $C_1$-$C_6$)$_p$-O- (alkylène en $C_1$-$C_6$)$_p$- (aryle en $C_6$-$C_{10}$), - (alkylène en $C_1$-$C_6$)$_p$-O- (alkylène en $C_1$-$C_6$)$_p$- (cycloalkyle en $C_7$-$C_{10}$), -(alkylène en $C_1$-$C_6$)$_p$-O-(alkylène en $C_1$-$C_6$)$_p$-(hétéroaryle ayant de 7 à 10 éléments), -(alkylène en $C_1$-$C_6$)$_p$-O-(alkylène en $C_1$-$C_6$)$_p$-(hétérocyclyle ayant de 7 à 10 éléments), - (alkylène en $C_1$-$C_6$)$_p$-O-(alkényle en $C_2$-$C_6$), -(alkylène en $C_1$-$C_6$)p-O-(alkénylène en $C_2$-$C_6$)$_p$-(aryle en $C_6$-$C_{10}$), -(alkylène en $C_1$-$C_6$)$_p$-O-(alkénylène en $C_2$-$C_6$)$_p$-(cycloalkyle en $C_3$-$C_{10}$), -(alkylène en $C_1$-$C_6$)$_p$-O-(alkénylène e n $C_2$-$C_6$)$_p$-(hétéroaryle ayant de 5 à 10 éléments), - (alkylène en $C_1$-$C_6$)$_p$-O-(alkénylène en $C_2$-$C_6$)$_p$-(hétérocyclyle ayant de 3 à 10 éléments), -(alkylène en $C_1$-$C_6$)$_p$-O-(alkynyle en $C_2$-$C_6$), -(alkylène en $C_1$-$C_6$)$_p$-O-(alkynylène en $C_2$-$C_6$)$_p$-(aryle en $C_6$-$C_{10}$), - (alkylène en $C_1$-$C_6$)p-O-(alkynylène en $C_2$-$C_6$)$_p$-(cycloalkyle en $C_3$-$C_{10}$), -(alkylène en $C_1$-$C_6$)$_p$-O-(alkynylène en $C_2$-$C_6$)$_p$-(hétéroaryle ayant de 5 à 10 éléments) et -(alkylène en $C_1$-$C_6$)$_p$-O-(alkynylène en $C_2$-$C_6$)$_p$-(hétérocyclyle ayant de 3 à 10 éléments) ;
$R^2$ est sélectionné dans le groupe consistant en - (alkylène en $C_1$-$C_6$)$_p$-C(O)-$R^b$, -(alkylène en $C_1$-$C_6$)$_p$-C (O)-$OR^a$, - (alkylène en $C_1$-$C_6$)$_p$-C(O)-N($R^a$)$_2$, - (alkylène en $C_1$-$C_6$)$_p$-S(O)-$R^a$, - (alkylène en $C_1$-$C_6$)$_p$-$S(O)_2$-$R^a$, -(alkylène en $C_1$-$C_6$)$_p$-$S(O)_2$-N($R^a$)$_2$, -(alkylène en $C_1$-$C_6$)$_p$-$S(O)_2$-O-$R^a$, et $R^3$, dans lequel $R^3$ est sélectionné parmi -(alkylène en $C_1$-$C_6$)-(perfluoroalkyle en $C_1$-$C_3$) , alkényle en $C_2$-$C_8$, alkynyle en $C_2$-$C_8$, -(alkylène en $C_1$-$C_6$)$_p$-(cycloalkyle en $C_3$-$C_{12}$), -(alkylène en $C_1$-$C_6$)$_p$-(hétérocyclyle ayant de 3 à 12 éléments), -(alkylène en $C_1$-$C_6$)$_p$-(hétéroaryle ayant de 5 à 12 éléments) et -(alkylène en $C_1$-$C_6$)$_p$-(carbocyclyle non-aromatique insaturé

en $C_5$-$C_{12}$) ;
chaque $R^a$ est indépendamment sélectionné dans le groupe consistant en H, alkyle en $C_1$-$C_8$, alkényle en $C_2$-$C_8$, alkynyle en $C_2$-$C_8$, perfluoroalkyle en $C_1$-$C_8$, -(alkylène en $C_1$-$C_6$)$_p$-(aryle en $C_6$-$C_{12}$), -(alkylène en $C_1$-$C_6$)$_p$-(hétéroaryle ayant de 5 à 12 éléments), -(alkylène en $C_1$-$C_6$)$_p$-(cycloalkyle en $C_3$-$C_{12}$), -(alkylène en $C_1$-$C_6$)$_p$-(hétérocyclyle ayant de 3 à 12 éléments), -(alkylène en $C_1$-$C_6$)$_p$-(carbocyclyle non-aromatique insaturé en $C_5$-$C_{12}$) ;
deux $R^a$ fixés au même atome d'azote peuvent facultativement former, avec ledit atome d'azote, un hétérocyclyle ayant de 3 à 12 éléments ou un hétéroaryle ayant de 5 à 12 éléments ; ledit hétérocyclyle ayant de 3 à 12 éléments et ledit hétéroaryle ayant de 5 à 12 éléments étant facultativement substitués, en outre, par 1-5 $R^x$ ;
$R^b$ est sélectionné dans le groupe consistant en -$NR^aN(R^a)_2$, -$NR^aOR^a$, alkényle en $C_2$-$C_8$, alkynyle en $C_2$-$C_8$, perfluoroalkyle en $C_1$-$C_8$, -(alkylène en $C_3$-$C_6$)-(perfluoroalkyle en $C_1$-$C_3$), -(alkylène en $C_1$-$C_6$)$_p$-(aryle en $C_6$-$C_{12}$), -(alkylène en $C_1$-$C_6$)$_p$-(cycloalkyle en $C_3$-$C_{12}$), -(alkylène en $C_1$-$C_6$)$_p$-(hétérocyclyle ayant de 3 à 12 éléments), -(alkylène en $C_1$-$C_6$)$_p$-(hétéroaryle ayant de 5 à 1 2 éléments), - (alkylène e n $C_1$-$C_6$)$_p$- (carbocyclyle non-aromatique insaturé en $C_5$-$C_{12}$) ;
p représente 0 ou 1 ;
chaque R, $R^a$, $R^b$ et $R^3$ est, de plus, facultativement substitué par 1-5 $R^x$ ;
chaque $R^x$ est indépendamment sélectionné dans le groupe consistant en -oxo-, -(alkylène en $C_1$-$C_4$)-, halogéno, -CN, -OH, alkyle en $C_1$-$C_6$, alkényle en $C_2$-$C_6$, alkynyle en $C_2$-$C_6$, perfluoroalkyle en $C_1$-$C_6$, -(alkylène en $C_1$-$C_6$)-halogéno, - (alkylène en $C_1$-$C_6$)-OH, -(alkylène en $C_1$-$C_6$)-CN, -(alkylène en $C_1$-$C_6$)$_q$-(cycloalkyle en $C_3$-$C_6$), -(alkylène en $C_1$-$C_6$)$_q$-(hétérocyclyle ayant de 3 à 6 éléments), -(alkylène en $C_1$-$C_6$)$_q$-(hétéroaryle ayant de 5 à 6 éléments), -(alkylène en $C_1$-$C_6$)$_q$-C(O)-(alkyle en $C_1$-$C_6$), -(alkylène en $C_1$-$C_6$)$_q$-C(O)-(cycloalkyle en $C_3$-$C_6$), -(alkylène en $C_1$-$C_6$)$_q$-C(O)-(alkylène en $C_1$-$C_6$)-(cycloalkyle en $C_3$-$C_6$), -(alkylène en $C_1$-$C_6$)$_q$-C (O)-O-(alkyle en $C_1$-$C_6$), -(alkylène en $C_1$-$C_6$)$_q$-C(O)-NH-(alkyle en $C_1$-$C_6$), - (alkylène en $C_1$-$C_6$)$_q$-C(O)-N(alkyle en $C_1$-$C_6$) (alkyle en $C_1$-$C_6$), -(alkylène en $C_1$-$C_6$)$_q$-O-(alkyle en $C_1$-$C_6$), -(alkylène en $C_1$-$C_6$)$_q$-O-(alkylène en $C_1$-$C_6$)-halogéno, - (alkylène en $C_1$-$C_6$)$_q$-O-(alkylène en $C_1$-$C_6$)-(perfluoroalkyle en $C_1$-$C_3$), -(alkylène en $C_1$-$C_6$)$_q$-O-(alkylène en $C_1$-$C_6$)$_q$-(cycloalkyle en $C_3$-$C_6$), -(alkylène en $C_1$-$C_6$)$_q$-O-(alkylène en $C_1$-$C_6$)$_q$-(hétérocyclyle ayant de 3 à 6 éléments), -(alkylène en $C_1$-$C_6$)$_q$-O-(alkylène en $C_1$-$C_6$)$_q$-(hétéroaryle ayant de 5 à 6 éléments), -(alkylène en $C_{1-}C_6$)$_q$-O-(alkylène en $C_1$-$C_6$)-$NH_2$, -(alkylène en $C_1$-$C_6$)$_q$-O-(alkylène en $C_1$-$C_6$)-NH-(alkyle en $C_1$-$C_6$), -(alkylène en $C_1$-$C_6$)$_q$-O-(alkylène en $C_1$-$C_6$)-NH-(cycloalkyl en $C_3$-$C_6$), -(alkylène en $C_1$-$C_6$)$_q$-O-(alkylène en $C_1$-$C_6$)-N(alkyle en $C_1$-$C_6$)$_2$, -(alkylène en $C_1$-$C_6$)$_q$-$NH_2$, -(alkylène en $C_1$-$C_6$)$_q$-NH-(alkyle en $C_1$-$C_6$), -(alkylène en $C_1$-$C_6$)$_q$-NH-(cycloalkyle en $C_3$-$C_6$), -(alkylène en $C_1$-$C_6$)$_q$-N(alkyle en $C_1$-$C_6$) (alkyle en $C_1$-$C_6$), -(alkylène en $C_1$-$C_6$)$_q$-NHC(O)-(alkyle en $C_1$-$C_6$), -(alkylène en $C_1$-$C_6$)$_q$-NH-$SO_2$-(alkyle en $C_1$-$C_6$), -(alkylène en $C_1$-$C_6$)$_q$-$SO_2$-(alkyle en $C_1$-$C_6$), - (alkylène en $C_1$-$C_6$)q-$SO_2$-(alkylène en $C_1$-$C_3$)$_q$-(cycloalkyle en $C_3$-$C_6$), -(alkylène en $C_1$-$C_6$)$_q$-$SO_2$-$NH_2$, (alkylène en $C_1$-$C_6$)$_q$-$SO_2$-NH(alkyle en $C_1$-$C_3$), -(alkylène en $C_1$-$C_6$)$_q$-$SO_2$-NH-(alkylène en $C_1$-$C_3$)$_q$-(cycloalkyle en $C_3$-$C_6$) et - (alkylène en $C_1$-$C_6$)$_q$-$SO_2$-N(alkyle en $C_1$-$C_3$)$_2$ ; chaque q représente indépendamment 0 ou 1 ; lorsque cela est possible, chaque atome de carbone de $R^x$ est facultativement substitué, en outre, par 1-3 atomes de fluor ; ou sel pharmaceutiquement acceptable dudit composé.

2. Composé selon la revendication 1, ou sel pharmaceutiquement acceptable dudit composé, dans lequel m représente 1 et n représente 1, et le composé est de formule II,

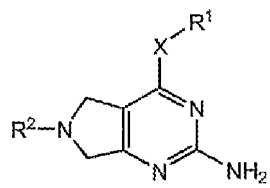

II

ou sel pharmaceutiquement acceptable dudit composé.

3. Composé selon l'une quelconque des revendications 1-2, ou sel pharmaceutiquement acceptable dudit composé, dans lequel X représente une liaison ou -O-, $R^1$ représente aryle en $C_6$-$C_{12}$, hétéroaryle ayant de 5 à 12 éléments ou hétérocyclyle ayant de 3 à 12 éléments, et $R^1$ est, en outre, substitué par 2-5 R.

**4.** Composé selon l'une quelconque des revendications 1-3, ou sel pharmaceutiquement acceptable dudit composé, dans lequel X représente une liaison et $R^1$ représente un groupe aryle en $C_6$-$C_{12}$, en outre, substitué par 2-5 R.

**5.** Composé selon la revendication 4, ou sel pharmaceutiquement acceptable dudit composé, dans lequel $R^1$ représente phényle, en outre, substitué par 2-5 R et un R au moins ne représente pas un halogène.

**6.** Composé selon l'une quelconque des revendications 1-5, ou sel pharmaceutiquement acceptable dudit composé, dans lequel R est sélectionné dans le groupe consistant en F, Cl, Br, -OH, -CN, alkyle en $C_1$-$C_3$, perfluoroalkyle en $C_1$-$C_3$, -(alkylène en $C_1$-$C_6$)-OH, -O-(alkyle en $C_1$-$C_6$), - (alkylène en $C_1$-$C_6$)-O-(alkyle en $C_1$-$C_6$), -(alkylène en $C_1$-$C_6$)$_p$-O-(alkylène en $C_1$-$C_6$)$_p$-(aryle en $C_6$-$C_{10}$), -(alkylène en $C_1$-$C_6$)$_p$-O-(alkylène en $C_1$-$C_6$)$_p$-(cycloalkyle en $C_3$-$C_{10}$), -(alkylène en $C_1$-$C_6$)$_p$-O-(alkylène en $C_1$-$C_6$)$_p$-(hétéroaryle ayant de 5 à 10 éléments), -(alkylène en $C_1$-$C_6$)$_p$-O-(alkylène en $C_1$-$C_6$)$_p$-(hétérocyclyle ayant de 3 à 10 éléments), -(alkylène en $C_1$-$C_6$)$_p$-O-(alkényle en $C_2$-$C_6$), -(alkylène en $C_1$-$C_6$)$_p$-O-(alkénylène en $C_2$-$C_6$)$_p$-(aryle en $C_6$-$C_{10}$), -(alkylène en $C_1$-$C_6$)$_p$-O-(alkénylène en $C_2$-$C_6$)$_p$-(cycloalkyle en $C_3$-$C_{10}$), -(alkylène en $C_1$-$C_6$)$_p$-O-(alkénylène en $C_2$-$C_6$)$_p$-(hétéroaryle ayant de 5 à 10 éléments), -(alkylène en $C_1$-$C_6$)$_p$-O-(alkénylène en $C_2$-$C_6$)$_p$-(hétérocyclyle ayant de 3 à 10 éléments), -(alkylène en $C_1$-$C_6$)$_p$-O-(alkynyle en $C_2$-$C_6$), -(alkylène en $C_1$-$C_6$)$_p$-O-(alkynylène en $C_2$-$C_6$)$_p$-(aryle en $C_6$-$C_{10}$), -(alkylène en $C_1$-$C_6$)$_p$-O-(alkynylène en $C_2$-$C_6$)$_p$-(cycloalkyl en $C_3$-$C_{10}$), -(alkylène en $C_1$-$C_6$)$_p$-O-(alkynylène en $C_2$-$C_6$)$_p$-(hétéroaryle ayant de 5 à 10 éléments) et -(alkylène en $C_1$-$C_6$)$_p$-O-(alkynylène en $C_2$-$C_6$)$_p$-(hétérocyclyle ayant de 3 à 10 éléments); chaque R étant, en outre, facultativement substitué par 1-5 $R^x$.

**7.** Composé selon la revendication 2, de formule V,

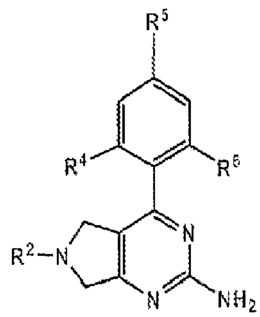

V

dans lequel $R^4$ et $R^5$ représentent indépendamment F, Cl, Br, -OH, -CN, alkyle en $C_1$-$C_3$ non substitué, perfluoroalkyle en $C_1$-$C_3$, (alkylène en $C_1$-$C_6$ non substitué)-OH ou -0- (alkyle en $C_1$-$C_6$ non substitué) ; $R^6$ est sélectionné dans le groupe consistant en - (alkylène en $C_1$-$C_6$)-OH, -O-(alkyle en $C_1$-$C_6$), -(alkylène en $C_1$-$C_6$)-O-(alkyle en $C_1$-$C_6$), - (alkylène en $C_1$-$C_6$)$_p$-O-(alkylène en $C_1$-$C_6$)$_p$-(aryle en $C_6$-$C_{10}$), -(alkylène en $C_1$-$C_6$)$_p$-O-(alkylène en $C_1$-$C_6$)$_p$-(cycloalkyle en $C_3$-$C_{10}$), -(alkylène en $C_1$-$C_6$)$_p$-O-(alkylène en $C_1$-$C_6$)$_p$-(hétéroaryle ayant de 5 à 10 éléments), -(alkylène en $C_1$-$C_6$)$_p$-O-(alkylène en $C_1$-$C_6$)$_p$-(hétérocyclyle ayant de 3 à 10 éléments), - (alkylène en $C_1$-$C_6$)$_p$-O-(alkényle en $C_2$-$C_6$), -(alkylène en $C_1$-$C_6$)$_p$-O-(alkénylène en $C_2$-$C_6$)$_p$-(aryle en $C_6$-$C_{10}$), -(alkylène en $C_1$-$C_6$)$_p$-O-(alkénylène en $C_2$-$C_6$)$_p$-(cycloalkyle en $C_3$-$C_{10}$), -(alkylène en $C_1$-$C_6$)$_p$-O-(alkénylène en $C_2$-$C_6$)$_p$-(hétéroaryle ayant de 5 à 10 éléments), -(alkylène en $C_1$-$C_6$)$_p$-O-(alkénylène en $C_2$-$C_6$)$_p$-(hétérocyclyle ayant de 3 à 10 éléments), -(alkylène en $C_1$-$C_6$)$_p$-O-(alkynyle en $C_2$-$C_6$), -(alkylène en $C_1$-$C_6$)$_p$-O-(alkynylène en $C_2$-$C_6$)$_p$-(aryle en $C_6$-$C_{10}$), -(alkylène en $C_1$-$C_6$)$_p$-O-(alkynylène en $C_2$-$C_6$)$_p$-(cycloalkyle en $C_3$-$C_{10}$), -(alkylène en $C_1$-$C_6$)$_p$-O-(alkynylène en $C_2$-$C_6$)$_p$-(hétéroaryle ayant de 5 à 10 éléments) et - (alkylène en $C_1$-$C_6$)$_p$-O-(alkynylène en $C_2$-$C_6$)$_p$-(hétérocyclyle ayant de 3 à 1 0 éléments) ; $R^6$ étant, en outre, facultativement substitué par 1-5 $R^x$; ou sel pharmaceutiquement acceptable dudit composé.

**8.** Composé selon la revendication 2, de formule VI

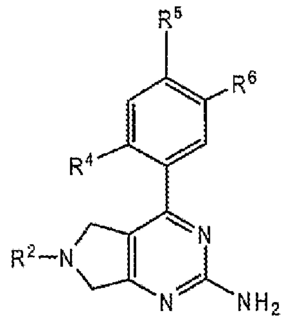

VI

dans lequel
$R^4$ et $R^5$ représentent indépendamment F, Cl, Br, -OH, -CN, alkyle en $C_1$-$C_3$ non substitué, perfluoroalkyle en $C_1$-$C_3$, (alkylène en $C_1$-$C_6$ non substitué)-OH ou -0-(alkyle en $C_1$-$C_6$ non substitué) ;
$R^6$ est sélectionné dans le groupe consistant en - (alkylène en $C_1$-$C_6$)-OH, -O-(alkyle en $C_1$-$C_6$), -(alkylène en $C_1$-$C_6$)-O-(alkyle en $C_1$-$C_6$), -(alkylène en $C_1$-$C_6$)$_p$-(alkylène en $C_1$-$C_6$)$_p$-(aryle en $C_6$-$C_{10}$), -(alkylène en $C_1$-$C_6$)$_p$-(alkylène en $C_1$-$C_6$)$_p$-(cycloalkyle en $C_3$-$C_{10}$), -(alkylène en $C_1$-$C_6$)$_p$-O-(alkylène en $C_1$-$C_6$)$_p$-(alkylène en $C_1$-$C_6$)$_p$-(hétéroaryle ayant de 5 à 10 éléments), -(alkylène en $C_1$-$C_6$)$_p$-(hétérocyclyle ayant de 3 à 10 éléments), -(alkylène en $C_1$-$C_6$)$_p$-O-(alkényle en $C_2$-$C_6$), -(alkylène en $C_1$-$C_6$)$_p$-O-(alkénylène en $C_2$-$C_6$)$_p$-(aryle en $C_6$-$C_{10}$), - (alkylène en $C_1$-$C_6$)$_p$-O-(alkénylène en $C_2$-$C_6$)$_p$-(cycloalkyle en $C_3$-$C_{10}$), -(alkylène en $C_1$-$C_6$)$_p$-O-(alkénylène en $C_2$-$C_6$)$_p$-(hétéroaryle ayant de 5 à 10 éléments), -(alkylène en $C_1$-$C_6$)$_p$-O-(alkénylène en $C_2$-$C_6$)$_p$-(hétérocyclyle ayant de 3 à 10 éléments), -(alkylène en $C_1$-$C_6$)$_p$-O-(alkynyle en $C_2$-$C_6$), -(alkylène en $C_1$-$C_6$)$_p$-O-(alkynylène en $C_2$-$C_6$)$_p$-(aryle en $C_6$-$C_{10}$), -(alkylène en $C_1$-$C_6$)$_p$-O-(alkynylène en $C_2$-$C_6$)$_p$-(cycloalkyle en $C_3$-$C_{10}$), -(alkylène en $C_1$-$C_6$)$_p$-O-(alkynylène en $C_2$-$C_6$)$_p$-(hétéroaryle ayant de 5 à 10 éléments) et -(alkylène en $C_1$-$C_6$)$_p$-O-(alkynylène en $C_2$-$C_6$)$_p$-(hétérocyclyle ayant de 3 à 10 éléments); $R^6$ étant, de plus, facultativement substitué par 1-5 $R^x$ ; ou sel pharmaceutiquement acceptable dudit composé.

**9.** Composé selon la revendication 7 ou la revendication 8, ou sel pharmaceutiquement acceptable dudit composé, dans lequel $R^6$ représente -O-(alkylène en $C_1$-$C_6$)-(hétéroaryle ayant de 5 à 10 éléments), et $R^6$ est, de plus, facultativement substitué par 1-5 $R^x$.

**10.** Composé selon la revendication 7 ou la revendication 8, ou sel pharmaceutiquement acceptable dudit composé, dans lequel $R^6$ représente -O-(alkylène en $C_1$-$C_6$)-(hétéroaryle ayant 5 éléments), et $R^6$ est, de plus, facultativement substitué par 1-5 $R^x$.

**11.** Composé selon la revendication 7 ou la revendication 8, ou sel pharmaceutiquement acceptable dudit composé, dans lequel $R^6$ représente -O-(alkylène en $C_1$-$C_6$)-(hétérocyclyle ayant de 3 à 10 éléments), et $R^6$ est, de plus, facultativement substitué par 1-5 $R^x$.

**12.** Composé selon la revendication 7 ou la revendication 8, ou sel pharmaceutiquement acceptable dudit composé, dans lequel $R^6$ représente -O-(alkyle en $C_1$-$C_6$) ou -0-(alkényle en $C_2$-$C_6$), et $R^6$ est, de plus, facultativement substitué par 1-5 $R^x$.

**13.** Composé selon l'une quelconque des revendications 1-12, ou sel pharmaceutiquement acceptable dudit composé, dans lequel $R^2$ représente -C(O)-N($R^a$)$_2$.

**14.** Composé selon l'une quelconque des revendications 1-12, ou sel pharmaceutiquement acceptable dudit composé, dans lequel $R^2$ représente -C(O)-O$R^a$.

**15.** Composition pharmaceutique comprenant un composé selon l'une quelconque des revendications 1 à 14, ou un sel pharmaceutiquement acceptable dudit composé, et un support ou excipient pharmaceutiquement acceptable.

**16.** Composé selon l'une quelconque des revendications 1 à 14, ou sel pharmaceutiquement acceptable dudit composé, ou composition telle que revendiquée dans la revendication 15, pour une utilisation dans le traitement du cancer.

**17.** Composé selon l'une quelconque des revendications 1 à 14, ou sel pharmaceutiquement acceptable dudit composé, ou composition telle que revendiquée dans la revendication 15, pour une utilisation comme modulateur ou inhibiteur de l'activité de HSP-90.

**18.** Composé selon l'une quelconque des revendications 1 à 14, ou sel pharmaceutiquement acceptable dudit composé, ou composition telle que revendiquée dans la revendication 15, pour une utilisation dans le traitement d'une maladie associée à une croissance cellulaire anormale.

**19.** Utilisation d'un composé selon l'une quelconque des revendications 1 à 14, ou d'un sel pharmaceutiquement acceptable dudit composé, ou d'une composition telle que revendiquée dans la revendication 15, pour la fabrication d'un médicament pour le traitement du cancer.

**20.** Utilisation d'un composé selon l'une quelconque des revendications 1 à 14, ou d'un sel pharmaceutiquement acceptable dudit composé, ou d'une composition telle que revendiquée dans la revendication 15, pour la fabrication d'un médicament en vue d'une utilisation comme modulateur ou inhibiteur de l'activité de HSP-90.

**21.** Utilisation d'un composé selon l'une quelconque des revendications 1 à 14, ou d'un sel pharmaceutiquement acceptable dudit composé, ou d'une composition telle que revendiquée dans la revendication 15, pour la fabrication d'un médicament pour le traitement d'une maladie associée à une croissance cellulaire anormale.

**22.** Combinaison d'un composé selon l'une quelconque des revendications 1 à 14, ou d'un sel pharmaceutiquement acceptable dudit composé, ou d'une composition telle que revendiquée dans la revendication 15, et d'un autre médicament.

**23.** Combinaison d'un composé selon l'une quelconque des revendications 1 à 14, ou d'un sel pharmaceutiquement acceptable dudit composé, ou d'une composition telle que revendiquée dans la revendication 15, et d'un agent antitumoral sélectionné dans le groupe consistant en les inhibiteurs mitotiques, les agents alkylants, les anti-métabolites, les antibiotiques intercalants, les inhibiteurs de facteurs de croissance, les inhibiteurs de cycles cellulaires, les enzymes, les inhibiteurs de topoisomérases, les modificateurs de réponses biologiques, les anticorps, les agents cytotoxiques, les anti-hormones, les anti-androgènes, les agents anti-angiogénèse, les inhibiteurs de kinases, les inhibiteurs de pan-kinases ou les inhibiteurs de facteurs de croissance, pour une utilisation dans le traitement d'une maladie associée à une croissance cellulaire anormale.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6106864 A
- WO 0035298 A
- WO 9111172 A
- WO 9402518 A
- WO 9855148 A
- US 6573293 B
- US 6534524 B
- US 6235764 B
- WO 9924440 A
- WO 9521613 A
- WO 9961422 A
- US 5834504 A
- WO 9850356 A
- US 5883113 A
- US 5886020 A
- US 5792783 A
- US 6653308 B
- WO 9910349 A
- WO 9732856 A
- WO 9722596 A
- WO 9854093 A
- WO 9802438 A
- WO 9916755 A
- WO 9802437 A
- US 6080769 A
- US 6194438 B
- US 6258824 B
- US 6586447 B
- US 6071935 A
- US 6495564 B
- US 6150377 A
- US 6596735 B
- US 6479513 B
- WO 0140217 A
- US 20030166675 A
- WO 2004020431 A
- US 20050148627 A
- US 20050148777 A
- US 5466823 A
- US 5633272 A
- US 5932598 A
- US 5521207 A
- US 6034256 A
- WO 200224719 A
- WO 199803484 A
- WO 199911605 A
- US 6180651 B
- WO 9802434 A
- WO 9935146 A
- WO 9935132 A
- WO 9713760 A
- WO 9519970 A
- US 5587458 A
- US 5877305 A
- US 6465449 B
- US 6284764 B
- WO 200198277 A
- EP 239362 A
- US 6682736 B
- WO 2002053596 A
- WO 2003040170 A
- US 4261989 A
- WO 2006117669 A


### Non-patent literature cited in the description


- **Jolly et al.** *J. Natl. Cancer Inst.,* 2000, vol. 92, 1564-1572
- **Martin et al.** *Cancer Res.,* 2000, vol. 60, 2232-2238
- **Young et al.** *J. Cell Biol.,* 2001, vol. 154, 267-273
- **Xu et al.** *Proc. Natl. Acad. Sci,* 1993, vol. 90, 7074-7078
- **Holstein et al.** *Cancer Res.,* 2001, vol. 61, 4003-4009
- **Viscontini ; Buhler.** *Helvetica Chimica Acta,* 1967, vol. 50 (5), 1289-93
- **Rosowsky.** *J. Heterocyclic Chem.,* 1989, vol. 26, 509-16
- **T. Greene ; P. Wuts.** Protective Groups in Organic Synthesis. John Wiley & Sons, 1999
- Stereochemistry of Organic Compounds. Wiley, 1994
- **T: Higuchi ; W. Stella.** Pro-drugs as Novel Delivery Systems. ACS Symposium Series, vol. 14
- Bioreversible Carriers in Drug Design. American Pharmaceutical Association, 1987
- **H. Bundgaard.** Design of Prodrugs. Elsevier, 1985

- Remington's Pharmaceutical Sciences'. Mack Publishing Company, 1995
- **Liang ; Chen.** *Expert Opinion in Therapeutic Patents,* 2001, vol. 11 (6), 981-986
- **H. Lieberman ; L. Lachman.** Pharmaceutical Dosage Forms: Tablets. Marcel Dekker, 1980, vol. 1
- **Verma et al.** *Pharmaceutical Technology On-line,* 2001, vol. 25 (2), 1-14
- **Finnin ; Morgan.** *J Pharm Sci,* October 1999, vol. 88 (10), 955-958
- **Buhler.** *Helvetica Chimica Acta,* 1967, vol. 50 (5), 1289-93
- **Young.** *Eur. J. Med. Chem.,* 1993, vol. 28, 201-211